# EUROPEAN PATENT APPLICATION

(11) **EP 3 795 160 A1**
(43) Date of publication of application: **24.03.2021**
(21) Application number: 19198308.9
(22) Date of filing: 19.09.2019
(51) Int. Cl.: A61K 35/768, A61K 39/395, C07K 14/125, C07K 16/22, C07K 16/28, C12N 7/00, C12N 7/02, C12N 15/86

(54) **RECOMBINANT ONCOLYTIC NEWCASTLE DISEASE VIRUSES WITH INCREASED ACTIVITY**

(71) Applicant: Thaller, Arno, 91801 Markt Berolzheim (DE)
(72) Inventor: Thaller, Arno, 91801 Markt Berolzheim (DE)
(74) Representative: Becker & Kurig Partnerschaft Patentanwälte PartmbB

(57) **Abstract**

The invention relates to transgene expressing Newcastle Disease Viruses (NDV), which have been demonstrated to possess significant oncolytic activity against mammalian cancers and/or an improved safety profile. The invention provides novel oncolytic viruses through the use of genetic engineering, including the transfer of foreign genes or parts thereof, such as genes encoding Atezolizumab or Bevacizumab. The present invention also provides nucleic acids encoding a reverse genetically engineered (rg-)NDV comprising one or more of these foreign genes and having a mutation in the HN gene, said mutation allowing replication of said rgNDV in a cancer cell to a higher level than replication of an otherwise identical rgNDV not having said mutation in the HN gene.

SEQUENCE LISTING

<110> Dr. Arno Thaller
<120> Recombinant oncolytic Newcastle Disease Viruses with increased activity

<160> 45

<210> 1
<211> 15186
<212> DNA
<213> Newcastle disease virus

<220>
<223> STRAIN MTH-68/H

<400> 1
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgagccctc ctctataaac ccaagattca aggctccaag cgataacctt 3180

ctcccgcctc ttccatccca ctgaatgatc gcgcagctgc aattaatcta gcaacattaa 3240

agattaagaa aaaatacggg tagaatcgga gtgcaccgat tgtgccaaga tggactcatc 3300

taggacaatt gggttatact ttgattctgc ccttccttct agcaacctgt tagcattccc 3360

gattgtctta cgagatgtag gagatggaaa gaagcaaatt actccgcaat ataggatccg 3420

gcgtcttgac tcgtggacag acagtaaaga agactcggta ttcatcacta cctacggatt 3480

catcttccag gttgggaatg aagaagtcac tgtcggcatg atcaatgata atcccaagcg 3540

agagttactc tccgctgcga tgctctgcct aggaagcgtc ccgaatgttg gagatcctgt 3600

tgagctggca agggcctgcc tcactatggt ggtaacatgc aagaagagtg caactaatac 3660

tgagagaatg gtcttctcag tagtgcaggc accccgggtg ctgcaaagct gtagggtcgt 3720

ggcagacaaa tactcgtcag tgaatgcagt taagcacgtg aaagcaccag agaagatccc 3780

tgggagtgaa accctagagt acaaggtgaa ttttgtctct ttgactgtgg tgccgaagaa 3840

ggatgtctac aagatcccaa ccgcagtatt gaaggtatcc ggctcgagcc tgtataatct 3900

tgcgctcaat gtcactattg atgtggaggt agacccaaag agcccgttag tcaagtccct 3960

ttctaggtcc gacagtggat actatgctaa tctcttctta catatcggac ttatgtccac 4020

tgtagataag aggggaaaga aagtgacatt tgaccagctg gagaggaaga taaggagact 4080

tgatctatct gtcgggctca gtgatgtgct cggaccttct gtgcttgtga aggcgagagg 4140

tgcacggact aggctgctgg cacctttctt ctctaacagc gggacagcct gctaccctat 4200

agcaaatgcc tctcctcagg tagctaagat actctggagt cagaccgcgt gcctgcggag 4260

tgtaaaaatc attattcaag cgggcaccca acgcgctgtc gcagtgactg ctgaccatga 4320

ggttacctct actaagatag agaagaggca taccatcgct aaatacaatc ccttcaagaa 4380

ataggccgta tccctgagac tgcaattcac ccgccttccc aaaacaccat gacaccagat 4440

aatgatctgt cttgattact tacagttagt ttccctgtct atcaaattag aaaaaacacg 4500

ggtagaagag ttcggatccc ggccggcgca ccaaaagcgc aagatgggcc ccagatcttc 4560

taccaggatc ccagtacctc taatgctgac catacggatc acgctggcac tgagttatgt 4620

ccgtctgaca agttctcttg atggcaggcc tcttgcagcc gcagggatcg tggtaacagg 4680

ggataaagca gttaacatat acacctcatc ccagacaggg tcaatcatag tcaagttact 4740

cccaaatatg cccaaggaca aagaggcatg tgcaaaagcc ccattggagg cttacaacag 4800

gacactgact actttgctta ccccccttgg tgattctatc cgcaggatac aagagtctgt 4860

gactacatcc ggaggaagga gacagagacg ctttataggt gccattattg gcagtgtagc 4920

tctaggggtt gcaacagctg cacagataac ggcagcctcg gctctgatac aagccaacca 4980

gaatgctgct aacatcctcc ggcttaagga gagcattgct gcaaccaatg aagctgtaca 5040

cgaggtcact ggcggattgt cacagttagc agtggcagtt ggaaagatgc agcaatttgt 5100

taatgaccag tttaataaca cagctcaaga gttggactgt ataaaaatta cacagcaggt 5160

tggtgtagaa ctcaacttat acttaactga attgactaca gtgttcgggc cacaaatcac 5220

ttcccctgcc ttaactcagt tgactgttca ggctctttac aatctggctg gtggtaatgt 5280

agattacttg ttgactaagt taggtgtagg gaacaaccag ctcagctcat tgattggtag 5340

cggcttgatc accggtaacc ctatttttta cgactcacag actcaactct tgggcataca 5400

ggtgacttta ccctcagtcg ggaacctaaa taatatgcgt gccacctact tggagacctt 5460

gtctgtaagc acaacaaagg gatttgcctc agcacttgtc ccaaaagtag tgacacaggt 5520

cggttctgtg atagaagagc ttgacacctc atactgtata gaagctgatt tggatttata 5580

ttgtacaaga atagtgacat tccctatgtc ccctggtatt tattcctgtt tgagcggcaa 5640

tacatcggct tgcatgtatt caaggactga aggcgcactt actacaccat acatgactct 5700

caaaggctca gttgttgcca attgccagat gacaacatgt agatgtgcag acccccgggg 5760

tatcatatca caaaattatg gagaggctgt gtctctaata gataagcact catgcaatgt 5820

cgtatcctta gacgggataa ctttgaggct cagtggggaa tttgatgcaa cttatcaaaa 5880

gaatatctca atattagatt ctcaagtact agtgacaggc aatctcgata tctcaactga 5940

gcttgggaat gtcaaccact caataagtaa tgctttggat aagttagagg aaagcaacag 6000

caaactagac aaagtcaatg tcaaactgac cagcacatct gctctcatta cctatattgt 6060

tttaactgtc atatctcttg ttcttggtat gcttagcctg gttctagcat gctatctgat 6120

gtacaagcaa aaagcgcaac gaaagacctt gttgtggctt ggaaataata ccctagatca 6180

gatgagagct actacaaaaa tgtgaatgca gatgagaggc agaggtatcc ccaatagcaa 6240

tctgtgtgtc aattctggca gcctgttaat cagaagaatt aagaaaaaac taccggatgt 6300

aggtgaacaa aagggaatat acgggtagaa cggcctgaga ggccacccct caatcgggag 6360

ccaggcccca ctacgtccgc tctaccgcat caccaacagc agtcttcagt catggacagc 6420

gcagttagcc aagttgcgct agagaatgat agaagagaag cgaataatac atggcgcttg 6480

gttttccgga tcgcagcctt acttttactg gtaataacct tagccgtctc tgcagtcgcc 6540

ctggcatata gtatggaggc tagcacacct ggcgaccttg taagcatacc gactgcgatc 6600

tctagggcag aggaaaggat tacatctgca ctcggttcca atcaagatgt agtagatagg 6660

atatacaagc aagtggccct tgagtctcca ttggcactgc taaataccga atctataatt 6720

atgaatgcaa taacgtctct ctcttatcaa atcaatggag ccacaaataa tagcgggtgt 6780

ggggcacctg ttcatgaccc agattacatc ggggggatag gtcaagaact tattgtagat 6840

gatacgagtg acgtcacatc attttatccc tctgcattcc aagaacacct gaattttatc 6900

ccggcgccca ctacaggatc aggctgcact cggataccct cattcgacat gagtgctacc 6960

cattactgtt acactcacaa tgtgatattg tctggctgca gagatcactc acactcacat 7020
cagtatttag cacttggtgt gcttcggaca tctgcaacag ggagggtatt cttttctact 7080
ctgcgttcca tcaacttgga tgacgcccaa aatcggaagt cttgcagtgt gagtgcaact 7140
cctttaggtt gtgatatgct gtgctctaaa atcacagaga ctgaggaaga ggattataaa 7200
tcagttatcc ccacatcgat ggtacatgga aggttagggt ttgacggcca ataccatgag 7260
aaggacctag acgtcacaac actatttagg gactgggtgg caaattaccc aggagtagga 7320
ggtgggtctt ttattaacaa tcgcgtatgg ttcccggtct acggagggct aaaacccagc 7380
tcgcctagtg acactgcaca agaagggaga tacgtaatat acaagcgata caatgacaca 7440
tgcccggatg agcaagatta ccagattcgg atggctaagt catcgtataa gcctaggcgg 7500
tttggtggaa aacgcgtaca gcaggccatc ctatccatca aggtgtcaac atccttgggt 7560
gaggacccgg tgttgactgt accgcctaat acggtcgcac tcatgggggc cgaaggcaga 7620
gttctcacag tggggacatc tcatttctta tatcagcgag ggtcatcata cttctctccc 7680
gctttgttat accctatgac agtcaacaac aaaacagcca ctcttcataa tccttataca 7740
ttcaatgctt tcactcggcc aggtagtgtc ccttgccagg cttcagcaag atgccccaac 7800
tcatgtgtta ccggagtcta tactgatcca tatcccttag tcttccatag gaaccacacc 7860
ttgcgagggg tattcggaac aatgcttgat gacgaacaag caagactcaa ccctgtatct 7920
gcagtatttg ataacatatc ccgcagtcgc ataactcggg taagttcaag cagtaccagg 7980
gcagcataca cgacatcaac gtgttttaaa gttgtcaaga ccaataaaac ctattgcctc 8040
agcattgcag aaatatccaa taccctcttc ggggaattca gaattgtccc cttactagtt 8100
gagattctca aggatggtgg ggtttaagaa gctaggaatg gtcggttgag tcaaccgtga 8160
gaaggcgggg aagatgatat tgcatcatct atcttctgta acaccaagaa tcaaatcgaa 8220
taccagtgcg agctcgaatc ctacgctgcc agtcagccat aatcggctgg tgctaatgtg 8280
gttagcctga atcttgtcga tagtcacttg attaagaaaa aatgtggacg gtagtgaaat 8340
ataaggcaaa gcagctcaca gaggacagca cgggtaggac atggcgggct ccggacccga 8400
aagggcagag catcagatta tcctaccaga gtcacatctg tcctcaccat tggtcaagca 8460
taaattgctt tattactgga aattaactgg gctaccgctt cctgacgaat gtgacttcga 8520
ccatcttatt atcagccgac aatggaagaa ggtacttgaa tcggccaccc ctgacattga 8580
gagaatgata aaactagggc gggcagtaca ccagactctc aaccacaatt ccaggataac 8640
cggagtactc catccccggt gtttagagga actggctagt attgaggttc ctgattcaac 8700
caacaaattt cggaagatcg aaaaaaagat ccagattcac aacacaaggt atggagaact 8760
gttcacaaga ttatgcacgc atgtagaaaa gaaactattg gggtcatctt ggtctaacaa 8820
tgtcccacgg tcagaggaat tcaacagcat ccgtacagat ccggcatttt ggtttcactc 8880

aaaatggtcc acagccaaat ttgcatggct ccatataaaa caggtccaga ggcatctgat 8940

tgtagcagca agaacaaggt ccgcagtcaa caaattagtg acgctgaccc ataaggtagg 9000

ccaaatcttt gttactcctg agcttgtcat tgtgacacat acagatgaga acaagttcac 9060

gtgtcttacc caggaacttg tgttgatgta tgcagatatg atggagggca gagatatggt 9120

cagcataata tcatccacgg cggcacatct taggagctta tcagagaaaa ttgatgatat 9180

tctgcggtta gtagatgctc tggcaagaga tttgggcaat caagtctacg atgttgtagc 9240

actaatggag ggattcgcat acggcgctgt tcagctgctt gaaccgtcag gtacatttgc 9300

gggggatttc ttcgcattca acctgcagga gctcaaagat actctaaccg gactcctccc 9360

caaggatatc gcagaatctg tgactcacgc aatcgcaacc atattctctg gcttagaaca 9420

aaatcaagca gctgagatgt tgtgcctgtt gcgtctgtgg ggtcacccac tactcgagtc 9480

ccgtattgca gcaaaagcag ttaggagtca aatgtgcgca ccaaaaatgg tagactttga 9540

tatgatcctc caggtattat ctttctttaa aggaacaatc atcaatggat atagaaagaa 9600

gaatgcaggc gtgtggccac gtgtcaagat agatacgata tacggaaggg tcatagggca 9660

gctacacgca gattctgcag agatttcaca tgatgtcatg ttgagggaat acaagagttt 9720

atctgcactt gaattcgagc catgtataga gtatgaccct gtcaccaatc tgagcatgtt 9780

tttaaaagac aaggcaatcg cacacccgaa agacaactgg ctcgcttcgt ttaggcgaaa 9840

ccttctctct gaggaccaga agaaacatgt aagagaggca acctcaacta accgcctctt 9900

gatagagttc ttagagtcaa atgattttga tccatataaa gagatggaat atctgacgac 9960

ccttgagtac ctaagagatg ataatgtggc agtatcatac tcactcaaag aaaaagaggt 10020

gaaagttaat gggcggattt tcgcaaagct aacaaataga ttaaggaatt gtcaggtaat 10080

ggcagaaggg atcctagctg accagattgc acctttcttc cagggaaatg gggtcattca 10140

ggatagcata tctttgacta aaagtatgct agcgatgagt caactgtctt tcaacagcaa 10200

caagaaacgt attactgact gcaaagaaag agtatcctca aaccgcaatc atgatccgaa 10260

gagcaagaat cgtcggagag ttgccacttt tgtaactacc gacctgcaaa agtattgtct 10320

caattggaga tatcagacag tcaagctgtt cgctcatgcc atcaatcagc tgatgggctt 10380

acctcacttc ttcgagtgga ttcatcttag actaatggat actacgatgt ttgtagggga 10440

ccctttcaat cctccaagtg accctacgga ctgtgatcta tcaagagtcc caaatgatga 10500

catatatatt gtcagtgcta ggggaggcat tgagggatta tgccagaagc tatggacaat 10560

gatctcaatt gctgcaatcc agcttgctgc agcaagatca cattgtcgcg ttgcctgtat 10620

ggtacaaggc gacaatcaag taatagctgt aacgagagag gtaagatcag atgactcccc 10680

agagatggtg ttaacacaat tgcatcaagc cagtgataat ttcttcaagg aattgattca 10740

tgtcaatcat ttgatcggcc ataatttgaa ggatcgtgaa accatcaggt cagacacgtt 10800
cttcatatac agcaaacgaa tattcaaaga tggagcaata ctcagtcagg tcctcaaaaa 10860
ctcatctaaa ttagtgctaa tatcaggcga ccttagtgaa aacactgtaa tgtcttgtgc 10920
caacattgca tctactgtag cacggttatg cgagaacggg cttcctaagg atttctgtta 10980
ttacttaaac tacttaatga gttgcgtgca gacatacttc gattccgaat tttccatcac 11040
caacaactcg caactcgatt ctaaccagtc gtggatagag gacatttctt ttgtgcactc 11100
atatgtcctg acccctgctc aactgggggg actgagtaac cttcaatact caaggctcta 11160
cacaaggaac atcggcgacc cgggaaccac tgctttcgca gagatcaaga gattagaggc 11220
agtggggtta ctgagtccta gcattatgac taacatctta actaggccgc ctggaaatgg 11280
agattgggcc agtctgtgca acgatccata ctcctttaat tttgagactg tcgcaagccc 11340
aaatattgtc cttaagaaac atacacaaag agtcctattt gaaacttgct cgaatccctt 11400
attatctgga gtgcacacag aggataatga ggcagaagag aaggcattgg ctgaattctt 11460
gctcaatcaa gaaatgattc atccacgtgt cgcgcatgct atcatggaag caagctctgt 11520
aggtaggaga aagcaaattc aggggcttgt tgacacaaca aacaccgtga ttaagattgc 11580
actgactagg aggccactcg gcatcaagag gctgatgcgg atagtcaatt actcgagcat 11640
gcatgcaatg ctatttagag atgatgtttt ctcgtctaat agatccaacc atcccttagt 11700
ctcttctgat atgtgttctc tgacactggc agactatgca cggaacagaa gctggtcacc 11760
tttgacaggg ggtagaaaaa tactgggtgt atctaatcct gataccatag aacttgtaga 11820
gggtgagatc cttagtgtca gtggagggtg cactagatgt gacagtgggg atgaacagtt 11880
tacttggttc catcttccaa gcaatataga gctaaacgat gacaccagca agaatcctcc 11940
aatgagagtg ccatatctcg ggtcaaagac tcaagagagg agagccgcct cgcttgcgaa 12000
aatagctcat atgtcaccac acgtgaaggc ggctctaagg gcgtcatctg tgttaatctg 12060
ggcttatggg gacaacgaaa taaactggac tgctgccctt aagattgcaa ggtctcggtg 12120
caacataagc tcagagtatc ttcgactatt gtcacccttg cctacagctg ggaatctcca 12180
acatagattg gacgacggca taactcagat gacattcacc cctgcatctc tatacagggt 12240
gtcaccttac attcatatat ctaatgattc tcaaaggcta tttactgaag aaggagtcaa 12300
agaggggaat gtggtttatc agcaaatcat gctcttgggt ttgtccttaa ttgagtcact 12360
cttcccaatg acaacaacca agacatatga tgaaatcaca ttgcacctcc acagtaaatt 12420
tagctgctgt atcagggaag cacctgttgc agttcctttc gagctactcg ggtggcaccc 12480
ggaactaagg gcagtaacct caaataagtt tatgtatgat cctagccctg tatcagaggg 12540
agactttgcg agacttgact tagctatctt taagagttat gaacttaatt tagagtcata 12600
ttccacaata gagctaatga acgttctttc aatatctagt gggaagttga ttggccagtc 12660

tgtggtttct tatgatgaag atacctccat aaagaatgac gctataatag tgtatgacaa 12720

cacacggaat tggatcagcg aagctcagaa ttcagatgtg gtccgcctat tcgagtatgc 12780

ggcactcgaa gtgctcctcg actgttctta tcaactctac tatctgagag taagaggcct 12840

aaacaatatc gtcctgtaca tgagtgattt atacaagaat atgccaggaa ttctactctc 12900

taatattgca gccacaatat ctcaccctgt catccattca aggttgaatg cagtaggtct 12960

ggtcaaccat gacgggtcac accaacttgc agacacagat ttcattgaaa tgtctgcaaa 13020

gctgctagtc tcttgcactc gacgcgtggt ctcaggttta catgcaggga ataagtatga 13080

cctgctgttt ccatctgtct tggatgataa cctaagtgag aagatgcttc agttgatatc 13140

ccaattatgc tgtctgtata cggtgctctt tgctacaaca agagaaatcc cgaaaataag 13200

aggcttatct gcagaagaga aatgttcagt acttactgag tacctactgt cagatgctgt 13260

gaaaccgtta cttgggtccg agcaagtgag ctctatcatg tctcccaaca tagttacgtt 13320

cccagccaat ctgtattaca tgtctaggaa gagccttaat ttgatcaggg agagagagga 13380

cagggatact atcttggcat tgttgttccc tcaagaaccg ctgctcgagt ttcctctggt 13440

acgagatatt ggtgctcgtg taaaagatcc atttacccga caacctgcgg cgtttttaca 13500

agagttagat ttgagtgctc cggcaaggta tgacgcattc acaatcagtc aggcgcattc 13560

tgaacacata ttgccaaacc cagaggaaga tcacttagta cgatacttgt tcagaggaat 13620

agggactgcg tcctcctctt ggtataaggc atcccatctt ctttctgtac ccgaggtcag 13680

atgtgcaagg catgggaact ccttatattt agcagaagga agtggagcca tcatgagtct 13740

tctcgaattg catataccac acgaaactat ctattacaat acacttttct cgaacgagat 13800

gaacccccca cagcgacatt tcggaccaac tccaacacag tttctgaatt cggttgtttt 13860

taggaattta caggcggaag taccatgcaa ggatggattt gtccaggagt tccgtccgtt 13920

atggagagag aatacagaag aaagcgatct gacctcagat aaagcagtgg gatatatcac 13980

atctgtagtg ccgtacaggt ctgtatcatt gctgcattgt gacattgaaa tccctccagg 14040

atctaatcaa agcttactag atcagctggc taccaatctg tctctgattg ccatgcattc 14100

tgtgaaggag ggcggggtcg tgattatcaa agtactgtat gcaatgggat attacttcca 14160

tctactcatg aacttgttta ctccatgttc cacgaaagga tatattctct ctaatggcta 14220

tgcctgtaga ggggatatgg agtgttacct gatatttgtc atgggctacc taggcgggcc 14280

tacatttgta catgaggtgg tgaggatggc aaaaacgcta gtacagcggc atggcacact 14340

tttgtctaag tcagatgaga ttacactgac taggttattt acttcacagc aacagcgtgt 14400

aacagacatc ctatccagcc ctttaccgag actaatgaag tacttgagag agaatattga 14460

tactgcactg attgaagccg ggggacagcc tgtccgtcca ttctgtgcag agagtttagt 14520

gagcacacta acagacatga ctcagacaac ccagatcatc gccagccaca ttgatacagt 14580

cattcgatct gtgatctata tggaagcaga gggtgatctt gctgacacag tattcttatt 14640

taccccttac aacctctcta tggacgggaa aaagagaaca tcacttaaac agtgcacaag 14700

acagatctta gaggtcacaa tactgggtct cagagtcaaa aatctcaata aagtaggtga 14760

tgtaatcagc ctagtactca gaggtatgat ttctctggag gaccttctcc cactgagaac 14820

ctacttgaag tgtagtacct gccctaagta tttgaaggct gtcctaggta ttaccaaact 14880

caaagaaatg ttcacagacg cctctttatt atacttgact cgtgctcaac aaaaattcta 14940

catgaaaact ataggcaatg cagtcaaggg atactatagt aactgtgact cttaaaggca 15000

accacatatc aataggccct ctttctagcc gatcgtattc ttgttgactt cattatacca 15060

tattagaaaa aaattgaatt ccgacccttt aagactcgta ttcggattca aataattatc 15120

tcagaaaaaa agtgcacgta gttgttcttg attatagtcc cgtcattcac caagtctttg 15180

tttggt 15186

<210> 2
<211> 15186
<212> DNA
<213> Newcastle disease virus

<220>
<223> Mut HN (F277L)/M(G165W)

<400> 2
   accaaacaga gaatccgtga gctacgataa aaggcgaagg agcaatcgaa gtcgtacggg 60
   tagaaggtgt gaatctcgag tgcgagaccg aagcttaaac tcgagagagc cttctgccaa 120
   catgtcttcc gtatttgacg agtatgaaca gctcctcgcg gctcagactc gccctaatgg 180
   agctcacgga ggaggagaga aggggagcac cttaaaagtt gaagtcccgg tattcactct 240
   taacagtgac gacccagaag atagatggaa ctttgcagta ttctgtctta ggattgctgt 300
   tagcgaagat gccaacaaac cactcagaca aggtgctctc atatctctct tatgctccca 360
   ttctcaagtg atgaggaacc atgtcgccct tgcagggaaa cagaatgagg ccacactggc 420
   tgttcttgag atcgatggtt tcaccaacag cgtgcctcaa tttaacaaca ggagtggagt 480
   gtctgaggag agagcacaga gattcttgat gatagcaggg tccctccctc gggcatgcag 540
   caacggtacc ccgttcatca cagctggggt tgaagatgat gcaccagaag acatcactga 600
   tactctggaa agaatcatct ctatccaggc tcaagtatgg gtcacggtag caaaggccat 660
   gactgcatat gagacagcgg atgagtcgga aacaagaaga atcaataagt atatgcagca 720
   aggcagagtc caaaagaagt acatcctcca ccccgtatgt aggagcgcaa ttcaactcac 780
   gattagacaa tctctggcag tccgcatttt cttggttagc gagcttaaga gaggccgcaa 840
   cacggcaggt ggaagttcca cttattatag cttggtaggg gacatagact catacatcag 900
   gaacaccggg cttactgcat tcttcctgac actcaggtat ggaattaaca ccaagacatc 960
   agcccttgca ctcagcagcc tcgcaggcga tatccaaaaa atgaagcagc tcatgcgtct 1020
   atatcggatg aaaggagata atgcgccgta catgacactg ctcggtgaca gtgaccagat 1080
   gagctttgca ccggctgagt atgcacaact ctactctttt gcaatgggta tggcatccgt 1140
   cctagacaaa ggaactggca agtaccagtt cgccagagac tttatgagca catcattctg 1200
   gagacttgga gtagagtatg ctcgggctca gggaagcagc atcaatgagg atatggctgc 1260
   cgagctaaag ctgaccccag cggcaaggag gggcctggca gctgctgccc aacgagcatc 1320
   cgaggagacc ggcagcatgg acattcccac tcaacaagct ggggtcctca cagggctcag 1380
   cgacggaggc cctcaagccc cacaaggtgg actgaacagg tcgcaagggc aaccggacgc 1440
   cggagatggg gagacccaat ttttagattt gatgagagca gtggcaaata gtatgagaga 1500
   agcgccaaac tctgtgcaga acaccactca gcaggagcct ccatctactc ctgggccatc 1560
   tcaagacaac gacaccgact ggggatactg accgacaaca ctcagcctgc ctccatcgaa 1620
   ttatctcgat tcttctgcct gtaacctaac ccctggtcca cagtctcaca cggccaaacc 1680
   cgcaaacgag cccccccctc tctccccccc cctccagcca cacggctcca accgcccaaa 1740
   acaacacggg cacaactcga ctcactcata atctacacag agccgaagat cttagaaaaa 1800
   aatacgggta gaagagagac attcagagat caggacaaat catcagggtc tccgctctcc 1860
   cctctaccca gcagaccaga gtgaagatgg ccaccttcac ggatgcagag atcgacgatc 1920
   tatttgaaac cagtggaact gtcattgaca gcataattac ggcccaaggc aacccagtag 1980
   agactgttgg aaggagcgca atcccacaag gcaaaactaa agcattgagt gcagcatggg 2040
   agaaacatgg aagcacccaa ccgccggccg gccaagacac ccctgatcga atggacagat 2100
   cagacaaaca accatcccca cccgaacagg caaccacaca caacagcccg ccagccacat 2160
   ccaccgacca gccccccacc caggccgcag gcgaagccgg tgacacacag ctcaagactg 2220
   gagcaagcaa ctccctcctg tcgatgctcg acaagcttag caataaatcg tccaatgcta 2280
   aaaagggccc acggtcgagt ccccaggaag ggcaccacca acctccgacc cagcagcagg 2340
   ggagtcaaca gagccgcgga aacagtcagg agagatcgca aaaccaggtc aaggccgcct 2400
   ctggagacca gggcacagac gcgaacacag catatcatgg acaatggaag gagtcacaac 2460
   catcagctgg tgcaatccct catgctctcc ggtcagggca gagccaagcc aatactcctg 2520
   catctgtgga tcatgtccag ctacctgtcg actttgtgca ggcgatgatg tctatgatgg 2580
   aggcgatatc acagaaggta aataaagtcg actatcagct agaccttgtc tcaaagcaga 2640
   catcctccat ccccatgatg cgatctgaaa tccaacagct gaaaacatcc gttgcggtca 2700
   tggaagccaa cctgggcatg atgaagattc tggaccctgg ttgtgctaac gtttcatctt 2760
   taagtgacct acgggcggtc gcccgctctc acccagtttt agtttcaggc cccggagacc 2820
   catctcctta tgtgacacaa gggggtgaat tgatactcaa taaactctcg caaccagtgc 2880
   aacatccttc tgagttgatt aaacctgcca cgataagcgg gcctgatata ggagtggaga 2940
   aggacactgt ccgtgcattg atcacctcac gcccaatgca tccgagctct tcagctaagc 3000
   tcctgagtaa gctggatgca gccgggtcga tagaagaaat taggaaaatc aaacgccttg 3060
   cgctgaatgg ctgatcacta ctacaacccg caacgggttc ccgtttatct attgtcacaa 3120
   ggaacccgcc ccgagccctc ctctataaac ccaagattca aggctccaag cgataacctt 3180
   ctcccgcctc ttccatccca ctgaatgatc gcgcagctgc aattaatcta gcaacattaa 3240
   agattaagaa aaaatacggg tagaatcgga gtgcaccgat tgtgccaaga tggactcatc 3300
   taggacaatt gggttatact ttgattctgc ccttccttct agcaacctgt tagcattccc 3360
   gattgtctta cgagatgtag gagatggaaa gaagcaaatt actccgcaat ataggatccg 3420
   gcgtcttgac tcgtggacag acagtaaaga agactcggta ttcatcacta cctacggatt 3480
   catcttccag gttgggaatg aagaagtcac tgtcggcatg atcaatgata atcccaagcg 3540
   agagttactc tccgctgcga tgctctgcct aggaagcgtc ccgaatgttg gagatcctgt 3600
   tgagctggca agggcctgcc tcactatggt ggtaacatgc aagaagagtg caactaatac 3660
   tgagagaatg gtcttctcag tagtgcaggc accccgggtg ctgcaaagct gtagggtcgt 3720
   ggcagacaaa tactcgtcag tgaatgcagt taagcacgtg aaagcaccag agaagatccc 3780
   ttggagtgaa accctagagt acaaggtgaa ttttgtctct ttgactgtgg tgccgaagaa 3840
   ggatgtctac aagatcccaa ccgcagtatt gaaggtatcc ggctcgagcc tgtataatct 3900
   tgcgctcaat gtcactattg atgtggaggt agacccaaag agcccgttag tcaagtccct 3960
   ttctaggtcc gacagtggat actatgctaa tctcttctta catatcggac ttatgtccac 4020
   tgtagataag aggggaaaga aagtgacatt tgaccagctg gagaggaaga taaggagact 4080
   tgatctatct gtcgggctca gtgatgtgct cggaccttct gtgcttgtga aggcgagagg 4140
   tgcacggact aggctgctgg cacctttctt ctctaacagc gggacagcct gctaccctat 4200
   agcaaatgcc tctcctcagg tagctaagat actctggagt cagaccgcgt gcctgcggag 4260
   tgtaaaaatc attattcaag cgggcaccca acgcgctgtc gcagtgactg ctgaccatga 4320
   ggttacctct actaagatag agaagaggca taccatcgct aaatacaatc ccttcaagaa 4380
   ataggccgta tccctgagac tgcaattcac ccgccttccc aaaacaccat gacaccagat 4440
   aatgatctgt cttgattact tacagttagt ttccctgtct atcaaattag aaaaaacacg 4500
   ggtagaagag ttcggatccc ggccggcgca ccaaaagcgc aagatgggcc ccagatcttc 4560
   taccaggatc ccagtacctc taatgctgac catacggatc acgctggcac tgagttatgt 4620
   ccgtctgaca agttctcttg atggcaggcc tcttgcagcc gcagggatcg tggtaacagg 4680
   ggataaagca gttaacatat acacctcatc ccagacaggg tcaatcatag tcaagttact 4740
   cccaaatatg cccaaggaca aagaggcatg tgcaaaagcc ccattggagg cttacaacag 4800
   gacactgact actttgctta ccccccttgg tgattctatc cgcaggatac aagagtctgt 4860
   gactacatcc ggaggaagga gacagagacg ctttataggt gccattattg gcagtgtagc 4920
   tctaggggtt gcaacagctg cacagataac ggcagcctcg gctctgatac aagccaacca 4980
   gaatgctgct aacatcctcc ggcttaagga gagcattgct gcaaccaatg aagctgtaca 5040
   cgaggtcact ggcggattgt cacagttagc agtggcagtt ggaaagatgc agcaatttgt 5100
   taatgaccag tttaataaca cagctcaaga gttggactgt ataaaaatta cacagcaggt 5160
   tggtgtagaa ctcaacttat acttaactga attgactaca gtgttcggcc cacaaatcac 5220
   ttcccctgcc ttaactcagt tgactgttca ggctctttac aatctggctg gtggtaatgt 5280
   agattacttg ttgactaagt taggtgtagg gaacaaccag ctcagctcat tgattggtag 5340
   cggcttgatc accggtaacc ctatttttta cgactcacag actcaactct tgggcataca 5400
   ggtgacttta ccctcagtcg ggaacctaaa taatatgcgt gccacctact tggagacctt 5460
   gtctgtaagc acaacaaagg gatttgcctc agcacttgtc ccaaaagtag tgacacaggt 5520
   cggttctgtg atagaagagc ttgacacctc atactgtata gaagctgatt tggatttata 5580
   ttgtacaaga atagtgacat tccctatgtc ccctggtatt tattcctgtt tgagcggcaa 5640
   tacatcggct tgcatgtatt caaggactga aggcgcactt actacaccat acatgactct 5700
   caaaggctca gttgttgcca attgccagat gacaacatgt agatgtgcag acccccgggg 5760
   tatcatatca caaaattatg gagaggctgt gtctctaata gataagcact catgcaatgt 5820
   cgtatcctta gacgggataa ctttgaggct cagtggggaa tttgatgcaa cttatcaaaa 5880
   gaatatctca atattagatt ctcaagtact agtgacaggc aatctcgata tctcaactga 5940
   gcttgggaat gtcaaccact caataagtaa tgctttggat aagttagagg aaagcaacag 6000
   caaactagac aaagtcaatg tcaaactgac cagcacatct gctctcatta cctatattgt 6060
   tttaactgtc atatctcttg ttcttggtat gcttagcctg gttctagcat gctatctgat 6120
   gtacaagcaa aaagcgcaac gaaagacctt gttgtggctt ggaaataata ccctagatca 6180
   gatgagagct actacaaaaa tgtgaatgca gatgagaggc agaggtatcc ccaatagcaa 6240
   tctgtgtgtc aattctggca gcctgttaat cagaagaatt aagaaaaaac taccggatgt 6300
   aggtgaacaa aagggaatat acgggtagaa cggcctgaga ggccacccct caatcgggag 6360
   ccaggcccca ctacgtccgc tctaccgcat caccaacagc agtcttcagt catggacagc 6420
   gcagttagcc aagttgcgct agagaatgat agaagagaag cgaataatac atggcgcttg 6480
   gttttccgga tcgcagcctt acttttactg gtaataacct tagccgtctc tgcagtcgcc 6540
   ctggcatata gtatggaggc tagcacacct ggcgaccttg taagcatacc gactgcgatc 6600
   tctagggcag aggaaaggat tacatctgca ctcggttcca atcaagatgt agtagatagg 6660
   atatacaagc aagtggccct tgagtctcca ttggcactgc taaataccga atctataatt 6720
   atgaatgcaa taacgtctct ctcttatcaa atcaatggag ccacaaataa tagcgggtgt 6780
   ggggcacctg ttcatgaccc agattacatc ggggggatag gtcaagaact tattgtagat 6840
   gatacgagtg acgtcacatc attttatccc tctgcattcc aagaacacct gaattttatc 6900
   ccggcgccca ctacaggatc aggctgcact cggataccct cattcgacat gagtgctacc 6960
   cattactgtt acactcacaa tgtgatattg tctggctgca gagatcactc acactcacat 7020
   cagtatttag cacttggtgt gcttcggaca tctgcaacag ggagggtatt cttttctact 7080
   ctgcgttcca tcaacttgga tgacgcccaa aatcggaagt cttgcagtgt gagtgcaact 7140
   cctttaggtt gtgatatgct gtgctctaaa atcacagaga ctgaggaaga ggattataaa 7200
   tcagttatcc ccacatcgat ggtacatgga aggttagggc ttgacggcca ataccatgag 7260
   aaggacctag acgtcacaac actatttagg gactgggtgg caaattaccc aggagtagga 7320
   ggtgggtctt ttattaacaa tcgcgtatgg ttcccggtct acggagggct aaaacccagc 7380
   tcgcctagtg acactgcaca agaaggagaa tacgtaatat acaagcgata caatgacaca 7440
   tgcccggatg agcaagatta ccagattcgg atggctaagt catcgtataa gcctaggcgg 7500
   tttggtggaa aacgcgtaca gcaggccatc ctatccatca aggtgtcaac atccttgggt 7560
   gaggacccgg tgttgactgt accgcctaat acggtcgcac tcatgggggc cgaaggcaga 7620
   gttctcacag tggggacatc tcatttctta tatcagcgag ggtcatcata cttctctccc 7680
   gctttgttat accctatgac agtcaacaac aaaacagcca ctcttcataa tccttataca 7740
   ttcaatgctt tcactcggcc aggtagtgtc ccttgccagg cttcagcaag atgccccaac 7800
   tcatgtgtta ccggagtcta tactgatcca tatcccttag tcttccatag gaaccacacc 7860
   ttgcgagggg tattcggaac aatgcttgat gacgaacaag caagactcaa ccctgtatct 7920
   gcagtatttg ataacatatc ccgcagtcgc ataactcggg taagttcaag cagtaccagg 7980
   gcagcataca cgacatcaac gtgttttaaa gttgtcaaga ccaataaaac ctattgcctc 8040
   agcattgcag aaatatccaa taccctcttc ggggaattca gaattgtccc cttactagtt 8100
   gagattctca aggatggtgg ggtttaagaa gctaggaatg gtcggttgag tcaaccgtga 8160
   gaaggcgggg aagatgatat tgcatcatct atcttctgta acaccaagaa tcaaatcgaa 8220
   taccagtgcg agctcgaatc ctacgctgcc agtcagccat aatcggctgg tgctaatgtg 8280
   gttagcctga atcttgtcga tagtcacttg attaagaaaa aatgtggacg gtagtgaaat 8340
   ataaggcaaa gcagctcaca gaggacagca cgggtaggac atggcgggct ccggacccga 8400
   aagggcagag catcagatta tcctaccaga gtcacatctg tcctcaccat tggtcaagca 8460
   taaattgctt tattactgga aattaactgg gctaccgctt cctgacgaat gtgacttcga 8520
   ccatcttatt atcagccgac aatggaagaa ggtacttgaa tcggccaccc ctgacattga 8580
   gagaatgata aaactagggc gggcagtaca ccagactctc aaccacaatt ccaggataac 8640
   cggagtactc catccccggt gtttagagga actggctagt attgaggttc ctgattcaac 8700
   caacaaattt cggaagatcg aaaaaaagat ccagattcac aacacaaggt atggagaact 8760
   gttcacaaga ttatgcacgc atgtagaaaa gaaactattg gggtcatctt ggtctaacaa 8820
   tgtcccacgg tcagaggaat tcaacagcat ccgtacagat ccggcatttt ggtttcactc 8880
   aaaatggtcc acagccaaat ttgcatggct ccatataaaa caggtccaga ggcatctgat 8940
   tgtagcagca agaacaaggt ccgcagtcaa caaattagtg acgctgaccc ataaggtagg 9000
   ccaaatcttt gttactcctg agcttgtcat tgtgacacat acagatgaga acaagttcac 9060
   gtgtcttacc caggaacttg tgttgatgta tgcagatatg atggagggca gagatatggt 9120
   cagcataata tcatccacgg cggcacatct taggagctta tcagagaaaa ttgatgatat 9180
   tctgcggtta gtagatgctc tggcaagaga tttgggcaat caagtctacg atgttgtagc 9240
   actaatggag ggattcgcat acggcgctgt tcagctgctt gaaccgtcag gtacatttgc 9300
   gggggatttc ttcgcattca acctgcagga gctcaaagat actctaaccg gactcctccc 9360
   caaggatatc gcagaatctg tgactcacgc aatcgcaacc atattctctg gcttagaaca 9420
   aaatcaagca gctgagatgt tgtgcctgtt gcgtctgtgg ggtcacccac tactcgagtc 9480
   ccgtattgca gcaaaagcag ttaggagtca aatgtgcgca ccaaaaatgg tagactttga 9540
   tatgatcctc caggtattat ctttctttaa aggaacaatc atcaatggat atagaaagaa 9600
   gaatgcaggc gtgtggccac gtgtcaagat agatacgata tacggaaggg tcatagggca 9660
   gctacacgca gattctgcag agatttcaca tgatgtcatg ttgagggaat acaagagttt 9720
   atctgcactt gaattcgagc catgtataga gtatgaccct gtcaccaatc tgagcatgtt 9780
   tttaaaagac aaggcaatcg cacacccgaa agacaactgg ctcgcttcgt ttaggcgaaa 9840
   ccttctctct gaggaccaga agaaacatgt aagagaggca acctcaacta accgcctctt 9900
   gatagagttc ttagagtcaa atgattttga tccatataaa gagatggaat atctgacgac 9960
   ccttgagtac ctaagagatg ataatgtggc agtatcatac tcactcaaag aaaaagaggt 10020
   gaaagttaat gggcggattt tcgcaaagct aacaaataga ttaaggaatt gtcaggtaat 10080
   ggcagaaggg atcctagctg accagattgc acctttcttc cagggaaatg gggtcattca 10140
   ggatagcata tctttgacta aaagtatgct agcgatgagt caactgtctt ttcaacagca 10200
   caagaaacgt attactgact gcaaagaaag agtatcctca aaccgcaatc atgatccgaa 10260
   gagcaagaat cgtcggagag ttgccacttt tgtaactacc gacctgcaaa agtattgtct 10320
   caattggaga tatcagacag tcaagctgtt cgctcatgcc atcaatcagc tgatgggctt 10380
   acctcacttc ttcgagtgga ttcatcttag actaatggat actacgatgt ttgtagggga 10440
   ccctttcaat cctccaagtg accctacgga ctgtgatcta tcaagagtcc caaatgatga 10500
   catatatatt gtcagtgcta ggggaggcat tgagggatta tgccagaagc tatggacaat 10560
   gatctcaatt gctgcaatcc agcttgctgc agcaagatca cattgtcgcg ttgcctgtat 10620
   ggtacaaggc gacaatcaag taatagctgt aacgagagag gtaagatcag atgactcccc 10680
   agagatggtg ttaacacaat tgcatcaagc cagtgataat ttcttcaagg aattgattca 10740
   tgtcaatcat ttgatcggcc ataatttgaa ggatcgtgaa accatcaggt cagacacgtt 10800
   cttcatatac agcaaacgaa tattcaaaga tggagcaata ctcagtcagg tcctcaaaaa 10860
   ctcatctaaa ttagtgctaa tatcaggcga ccttagtgaa aacactgtaa tgtcttgtgc 10920
   caacattgca tctactgtag cacggttatg cgagaacggg cttcctaagg atttctgtta 10980
   ttacttaaac tacttaatga gttgcgtgca gacatacttc gattccgaat tttccatcac 11040
   caacaactcg caactcgatt ctaaccagtc gtggatagag gacatttctt ttgtgcactc 11100
   atatgtcctg acccctgctc aactgggggg actgagtaac cttcaatact caaggctcta 11160
   cacaaggaac atcggcgacc cgggaaccac tgctttcgca gagatcaaga gattagaggc 11220
   agtggggtta ctgagtccta gcattatgac taacatctta actaggccgc ctggaaatgg 11280
   agattgggcc agtctgtgca acgatccata ctcctttaat tttgagactg tcgcaagccc 11340
   aaatattgtc cttaagaaac atacacaaag agtcctattt gaaacttgct cgaatccctt 11400
   attatctgga gtgcacacag aggataatga ggcagaagag aaggcattgg ctgaattctt 11460
   gctcaatcaa gaaatgattc atccacgtgt cgcgcatgct atcatggaag caagctctgt 11520
   aggtaggaga aagcaaattc aggggcttgt tgacacaaca aacaccgtga ttaagattgc 11580
   actgactagg aggccactcg gcatcaagag gctgatgcgg atagtcaatt actcgagcat 11640
   gcatgcaatg ctatttagag atgatgtttt ctcgtctaat agatccaacc atcccttagt 11700
   ctcttctgat atgtgttctc tgacactggc agactatgca cggaacagaa gctggtcacc 11760
   tttgacaggg ggtagaaaaa tactgggtgt atctaatcct gataccatag aacttgtaga 11820
   gggtgagatc cttagtgtca gtggagggtg cactagatgt gacagtgggg atgaacagtt 11880
   tacttggttc catcttccaa gcaatataga gctaaacgat gacaccagca agaatcctcc 11940
   aatgagagtg ccatatctcg ggtcaaagac tcaagagagg agagccgcct cgcttgcgaa 12000
   aatagctcat atgtcaccac acgtgaaggc ggctctaagg gcgtcatctg tgttaatctg 12060
   ggcttatggg gacaacgaaa taaactggac tgctgccctt aagattgcaa ggtctcggtg 12120
   caacataagc tcagagtatc ttcgactatt gtcacccttg cctacagctg ggaatctcca 12180
   acatagattg gacgacggca taactcagat gacattcacc cctgcatctc tatacagggt 12240
   gtcaccttac attcatatat ctaatgattc tcaaaggcta tttactgaag aaggagtcaa 12300
   agaggggaat gtggtttatc agcaaatcat gctcttgggt ttgtccttaa ttgagtcact 12360
   cttcccaatg acaacaacca agacatatga tgaaatcaca ttgcacctcc acagtaaatt 12420
   tagctgctgt atcagggaag cacctgttgc agttcctttc gagctactcg gggtggcacc 12480
   ggaactaagg gcagtaacct caaataagtt tatgtatgat cctagccctg tatcagaggg 12540
   agactttgcg agacttgact tagctatctt taagagttat gaacttaatt tagagtcata 12600
   ttccacaata gagctaatga acgttctttc aatatctagt gggaagttga ttggccagtc 12660
   tgtggtttct tatgatgaag atacctccat aaagaatgac gctataatag tgtatgacaa 12720
   cacacggaat tggatcagcg aagctcagaa ttcagatgtg gtccgcctat tcgagtatgc 12780
   ggcactcgaa gtgctcctcg actgttctta tcaactctac tatctgagag taagaggcct 12840
   aaacaatatc gtcctgtaca tgagtgattt atacaagaat atgccaggaa ttctactctc 12900
   taatattgca gccacaatat ctcaccctgt catccattca aggttgaatg cagtaggtct 12960
   ggtcaaccat gacgggtcac accaacttgc agacacagat ttcattgaaa tgtctgcaaa 13020
   gctgctagtc ttttgcactc gacgcgtggt ctcaggttta catgcaggga ataagtatga 13080
   cctgctgttt ccatctgtct tggatgataa cctaagtgag aagatgcttc agttgatatc 13140
   ccaattatgc tgtctgtata cggtgctctt tgctacaaca agagaaatcc cgaaaataag 13200
   aggcttatct gcagaagaga aatgttcagt acttactgag tacctactgt cagatgctgt 13260
   gaaaccgtta cttgggtccg agcaagtgag ctctatcatg tctcccaaca tagttacgtt 13320
   cccagccaat ctgtattaca tgtctaggaa gagccttaat ttgatcaggg agagagagga 13380
   cagggatact atcttggcat tgttgttccc tcaagaaccg ctgctcgagt ttcctctggt 13440
   acgagatatt ggtgctcgtg taaaagatcc atttacccga caacctgcgg cgtttttaca 13500
   agagttagat ttgagtgctc cggcaaggca tgacgcattc acaatcagtc aggcgcattc 13560
   tgaacacata ttgccaaacc cagaggaaga tcacttagta cgatacttgt tcagaggaat 13620
   agggactgcg tcctcctctt ggtataaggc atcccatctt ctttctgtac ccgaggtcag 13680
   atgtgcaagg catgggaact ccttatattt agcagaagga agtggagcca tcatgagtct 13740
   tctcgaattg catataccac acgaaactat ctattacaat acacttttct cgaacgagat 13800
   gaacccccca cagcgacatt tcggaccaac tccaacacag tttctgaatt cggttgtttt 13860
   taggaattta caggcggaag taccatgcaa ggatggattt gtccaggagt tccgtccgtt 13920
   atggagagag aatacagaag aaagcgatct gacctcagat aaagcagtgg gatatatcac 13980
   atctgtagtg ccgtacaggt ctgtatcatt gctgcattgt gacattgaaa tccctccagg 14040
   atctaatcaa agcttactag atcagctggc taccaatctg tctctgattg ccatgcattc 14100
   tgtgaaggag ggcggggtcg tgattatcaa agtactgtat gcaatgggat attacttcca 14160
   tctactcatg aacttgttta ctccatgttc cacgaaagga tatattctct ctaatggcta 14220
   tgcctgtaga ggggatatgg agtgttacct gatatttgtc atgggctacc taggcgggcc 14280
   tacatttgta catgaggtgg tgaggatggc aaaaacgcta gtacagcggc atggcacact 14340
   tttgtctaag tcagatgaga ttacactgac taggttattt acttcacagc aacagcgtgt 14400
   aacagacatc ctatccagcc ctttaccgag actaatgaag tacttgagag agaatattga 14460
   tactgcactg attgaagccg ggggacagcc tgtccgtcca ttctgtgcag agagtttagt 14520
   gagcacacta acagacatga ctcagacaac ccagatcatc gccagccaca ttgatacagt 14580
   cattcgatct gtgatctata tggaagcaga gggtgatctt gctgacacag tattcttatt 14640
   taccccttac aacctctcta tggacgggaa aaagagaaca tcacttaaac agtgcacaag 14700
   acagatctta gaggtcacaa tactgggtct cagagtcaaa aatctcaata aagtaggtga 14760
   tgtaatcagc ctagtactca gaggtatgat ttctctggag gaccttctcc cactgagaac 14820
   ctacttgaag tgtagtacct gccctaagta tttgaaggct gtcctaggta ttaccaaact 14880
   caaagaaatg ttcacagacg cctctttatt atacttgact cgtgctcaac aaaaattcta 14940
   catgaaaact ataggcaatg cagtcaaggg atactatagt aactgtgact cttaaaggca 15000
   accacatatc aataggccct ctttctagcc gatcgtattc ttgttgactt cattatacca 15060
   tattagaaaa aaattgaatt ccgacccttt aagactcgta ttcggattca aataattatc 15120
   tcagaaaaaa agtgcacgta gttgttcttg attatagtcc cgtcattcac caagtctttg 15180
   tttggt 15186

<210> 3
<211> 15186
<212> DNA
<213> Newcastle disease virus

<220>
<223> Mut HN
(F277L)/M(G165W)/F(F117S)/F(F190L)/L(V757I)/L(F1551S)/L(R1700L)

<400> 3
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360
ttctcaagtg atgaggaacc atgtcgccct tgcagggaaa cagaatgagg ccacactggc 420
tgttcttgag atcgatggtt tcaccaacag cgtgcctcaa tttaacaaca ggagtggagt 480
gtctgaggag agagcacaga gattcttgat gatagcaggg tccctccctc gggcatgcag 540
caacggtacc ccgttcatca cagctggggt tgaagatgat gcaccagaag acatcactga 600
tactctggaa agaatcatct ctatccaggc tcaagtatgg gtcacggtag caaaggccat 660
gactgcatat gagacagcgg atgagtcgga aacaagaaga atcaataagt atatgcagca 720
aggcagagtc caaaagaagt acatcctcca ccccgtatgt aggagcgcaa ttcaactcac 780
gattagacaa tctctggcag tccgcatttt cttggttagc gagcttaaga gaggccgcaa 840
cacggcaggt ggaagttcca cttattatag cttggtaggg gacatagact catacatcag 900
gaacaccggg cttactgcat tcttcctgac actcaggtat ggaattaaca ccaagacatc 960
agcccttgca ctcagcagcc tcgcaggcga tatccaaaaa atgaagcagc tcatgcgtct 1020
atatcggatg aaaggagata atgcgccgta catgacactg ctcggtgaca gtgaccagat 1080
gagctttgca ccggctgagt atgcacaact ctactctttt gcaatgggta tggcatccgt 1140
cctagacaaa ggaactggca agtaccagtt cgccagagac tttatgagca catcattctg 1200
gagacttgga gtagagtatg ctcgggctca gggaagcagc atcaatgagg atatggctgc 1260
cgagctaaag ctgaccccag cggcaaggag gggcctggca gctgctgccc aacgagcgtc 1320
cgaggagacc ggcagcatgg acattcccac tcaacaagct ggggtcctca cagggctcag 1380
cgacggaggc cctcaagccc cacaaggtgg actgaacagg tcgcaagggc aaccggacgc 1440
cggagatggg gagacccaat ttttagattt gatgagagca gtggcaaata gtatgagaga 1500
agcgccaaac tctgtgcaga acaccactca gcaggagcct ccatctactc ctgggccatc 1560
tcaagacaac gacaccgact ggggatactg accgacaaca ctcagcctgc ctccatcgaa 1620
ttatctcgat tcttctgcct gtaacctaac ccctggtcca cagtctcaca cggccaaacc 1680
cgcaaacgag cccccccctc tctccccccc cctccagcca cacggctcca accgcccaaa 1740
acaacacggg cacaactcga ctcactcata atctacacag agccgaagat cttagaaaaa 1800
aatacgggta gaagagagac attcagagat caggacaaat catcagggtc tccgctctcc 1860
cctctaccca gcagaccaga gtgaagatgg ccaccttcac ggatgcagag atcgacgatc 1920
tatttgaaac cagtggaact gtcattgaca gcataattac ggcccaaggc aacccagtag 1980
agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgagccctc ctctataaac ccaagattca aggctccaag cgataacctt 3180

ctcccgcctc ttccatccca ctgaatgatc gcgcagctgc aattaatcta gcaacattaa 3240

agattaagaa aaaatacggg tagaatcgga gtgcaccgat tgtgccaaga tggactcatc 3300

taggacaatt gggttatact ttgattctgc ccttccttct agcaacctgt tagcattccc 3360

gattgtctta cgagatgtag gagatggaaa gaagcaaatt actccgcaat ataggatccg 3420

gcgtcttgac tcgtggacag acagtaaaga agactcggta ttcatcacta cctacggatt 3480

catcttccag gttgggaatg aagaagtcac tgtcggcatg atcaatgata atcccaagcg 3540

agagttactc tccgctgcga tgctctgcct aggaagcgtc ccgaatgttg gagatcctgt 3600

tgagctggca agggcctgcc tcactatggt ggtaacatgc aagaagagtg caactaatac 3660

tgagagaatg gtcttctcag tagtgcaggc accccgggtg ctgcaaagct gtagggtcgt 3720

ggcagacaaa tactcgtcag tgaatgcagt taagcacgtg aaagcaccag agaagatccc 3780

ttggagtgaa accctagagt acaaggtgaa ttttgtctct ttgactgtgg tgccgaagaa 3840

ggatgtctac aagatcccaa ccgcagtatt gaaggtatcc ggctcgagcc tgtataatct 3900

tgcgctcaat gtcactattg atgtggaggt agacccaaag agcccgttag tcaagtccct 3960

ttctaggtcc gacagtggat actatgctaa tctcttctta catatcggac ttatgtccac 4020

tgtagataag aggggaaaga aagtgacatt tgaccagctg gagaggaaga taaggagact 4080

tgatctatct gtcgggctca gtgatgtgct cggaccttct gtgcttgtga aggcgagagg 4140

tgcacggact aggctgctgg cacctttctt ctctaacagc gggacagcct gctaccctat 4200

agcaaatgcc tctcctcagg tagctaagat actctggagt cagaccgcgt gcctgcggag 4260

tgtaaaaatc attattcaag cgggcaccca acgcgctgtc gcagtgactg ctgaccatga 4320

ggttacctct actaagatag agaagaggca taccatcgct aaatacaatc ccttcaagaa 4380

ataggccgta tccctgagac tgcaattcac ccgccttccc aaaacaccat gacaccagat 4440

aatgatctgt cttgattact tacagttagt ttccctgtct atcaaattag aaaaaacacg 4500

ggtagaagag ttcggatccc ggccggcgca ccaaaagcgc aagatgggcc ccagatcttc 4560

taccaggatc ccagtacctc taatgctgac catacggatc acgctggcac tgagttatgt 4620

ccgtctgaca agttctcttg atggcaggcc tcttgcagcc gcagggatcg tggtaacagg 4680

ggataaagca gttaacatat acacctcatc ccagacaggg tcaatcatag tcaagttact 4740

cccaaatatg cccaaggaca aagaggcatg tgcaaaagcc ccattggagg cttacaacag 4800

gacactgact actttgctta ccccccttgg tgattctatc cgcaggatac aagagtctgt 4860

gactacatcc ggaggaagga gacagagacg ctctataggt gccattattg gcagtgtagc 4920

tctaggggtt gcaacagctg cacagataac ggcagcctcg gctctgatac aagccaacca 4980

gaatgctgct aacatcctcc ggcttaagga gagcattgct gcaaccaatg aagctgtaca 5040

cgaggtcact ggcggattgt cacagttagc agtggcagtt ggaaagatgc agcaatttgt 5100

taatgaccag cttaataaca cagctcaaga gttggactgt ataaaaatta cacagcaggt 5160

tggtgtagaa ctcaacttat acttaactga attgactaca gtgttcgggc cacaaatcac 5220

ttcccctgcc ttaactcagt tgactgttca ggctctttac aatctggctg gtggtaatgt 5280

agattacttg ttgactaagt taggtgtagg gaacaaccag ctcagctcat tgattggtag 5340

cggcttgatc accggtaacc ctatttttta cgactcacag actcaactct tgggcataca 5400

ggtgacttta ccctcagtcg ggaacctaaa taatatgcgt gccacctact tggagacctt 5460

gtctgtaagc acaacaaagg gatttgcctc agcacttgtc ccaaaagtag tgacacaggt 5520

cggttctgtg atagaagagc ttgacacctc atactgtata gaagctgatt tggatttata 5580

ttgtacaaga atagtgacat tccctatgtc ccctggtatt tattcctgtt tgagcggcaa 5640

tacatcggct tgcatgtatt caaggactga aggcgcactt actacaccat acatgactct 5700

caaaggctca gttgttgcca attgccagat gacaacatgt agatgtgcag acccccgggg 5760

tatcatatca caaaattatg gagaggctgt gtctctaata gataagcact catgcaatgt 5820

cgtatcctta gacgggataa ctttgaggct cagtggggaa tttgatgcaa cttatcaaaa 5880

gaatatctca atattagatt ctcaagtact agtgacaggc aatctcgata tctcaactga 5940

gcttgggaat gtcaaccact caataagtaa tgctttggat aagttagagg aaagcaacag 6000

caaactagac aaagtcaatg tcaaactgac cagcacatct gctctcatta cctatattgt 6060

tttaactgtc atatctcttg ttcttggtat gcttagcctg gttctagcat gctatctgat 6120

gtacaagcaa aaagcgcaac gaaagacctt gttgtggctt ggaaataata ccctagatca 6180

gatgagagct actacaaaaa tgtgaatgca gatgagaggc agaggtatcc ccaatagcaa 6240

tctgtgtgtc aattctggca gcctgttaat cagaagaatt aagaaaaaac taccggatgt 6300

aggtgaacaa aagggaatat acgggtagaa cggcctgaga ggccacccct caatcgggag 6360

ccaggcccca ctacgtccgc tctaccgcat caccaacagc agtcttcagt catggacagc 6420

gcagttagcc aagttgcgct agagaatgat agaagagaag cgaataatac atggcgcttg 6480

gttttccgga tcgcagcctt acttttactg gtaataacct tagccgtctc tgcagtcgcc 6540

ctggcatata gtatggaggc tagcacacct ggcgaccttg taagcatacc gactgcgatc 6600

tctagggcag aggaaaggat tacatctgca ctcggttcca atcaagatgt agtagatagg 6660

atatacaagc aagtggccct tgagtctcca ttggcactgc taaataccga atctataatt 6720

atgaatgcaa taacgtctct ctcttatcaa atcaatggag ccacaaataa tagcgggtgt 6780

ggggcacctg ttcatgaccc agattacatc ggggggatag gtcaagaact tattgtagat 6840

gatacgagtg acgtcacatc attttatccc tctgcattcc aagaacacct gaattttatc 6900

ccggcgccca ctacaggatc aggctgcact cggataccct cattcgacat gagtgctacc 6960

cattactgtt acactcacaa tgtgatattg tctggctgca gagatcactc acactcacat 7020

cagtatttag cacttggtgt gcttcggaca tctgcaacag ggagggtatt cttttctact 7080

ctgcgttcca tcaacttgga tgacgcccaa aatcggaagt cttgcagtgt gagtgcaact 7140

cctttaggtt gtgatatgct gtgctctaaa atcacagaga ctgaggaaga ggattataaa 7200

tcagttatcc ccacatcgat ggtacatgga aggttagggc ttgacggcca ataccatgag 7260

aaggacctag acgtcacaac actatttagg gactgggtgg caaattaccc aggagtagga 7320

ggtgggtctt ttattaacaa tcgcgtatgg ttcccggtct acggagggct aaaacccagc 7380

tcgcctagtg acactgcaca agaagggaga tacgtaatat acaagcgata caatgacaca 7440

tgcccggatg agcaagatta ccagattcgg atggctaagt catcgtataa gcctaggcgg 7500

tttggtggaa aacgcgtaca gcaggccatc ctatccatca aggtgtcaac atccttgggt 7560

gaggacccgg tgttgactgt accgcctaat acggtcgcac tcatgggggc cgaaggcaga 7620

gttctcacag tggggacatc tcatttctta tatcagcgag ggtcatcata cttctctccc 7680

gctttgttat accctatgac agtcaacaac aaaacagcca ctcttcataa tccttataca 7740

ttcaatgctt tcactcggcc aggtagtgtc ccttgccagg cttcagcaag atgccccaac 7800

tcatgtgtta ccggagtcta tactgatcca tatcccttag tcttccatag gaaccacacc 7860

ttgcgagggg tattcggaac aatgcttgat gacgaacaag caagactcaa ccctgtatct 7920

gcagtatttg ataacatatc ccgcagtcgc ataactcggg taagttcaag cagtaccagg 7980

gcagcataca cgacatcaac gtgttttaaa gttgtcaaga ccaataaaac ctattgcctc 8040

agcattgcag aaatatccaa taccctcttc ggggaattca gaattgtccc cttactagtt 8100

gagattctca aggatggtgg ggtttaagaa gctaggaatg gtcggttgag tcaaccgtga 8160

gaaggcgggg aagatgatat tgcatcatct atcttctgta acaccaagaa tcaaatcgaa 8220

taccagtgcg agctcgaatc ctacgctgcc agtcagccat aatcggctgg tgctaatgtg 8280

gttagcctga atcttgtcga tagtcacttg attaagaaaa aatgtggacg gtagtgaaat 8340

ataaggcaaa gcagctcaca gaggacagca cgggtaggac atggcgggct ccggacccga 8400

aagggcagag catcagatta tcctaccaga gtcacatctg tcctcaccat tggtcaagca 8460

taaattgctt tattactgga aattaactgg gctaccgctt cctgacgaat gtgacttcga 8520

ccatcttatt atcagccgac aatggaagaa ggtacttgaa tcggccaccc ctgacattga 8580

gagaatgata aaactagggc gggcagtaca ccagactctc aaccacaatt ccaggataac 8640

cggagtactc catccccggt gtttagagga actggctagt attgaggttc ctgattcaac 8700

caacaaattt cggaagatcg aaaaaaagat ccagattcac aacacaaggt atggagaact 8760

gttcacaaga ttatgcacgc atgtagaaaa gaaactattg gggtcatctt ggtctaacaa 8820

tgtcccacgg tcagaggaat tcaacagcat ccgtacagat ccggcatttt ggtttcactc 8880

aaaatggtcc acagccaaat ttgcatggct ccatataaaa caggtccaga ggcatctgat 8940

tgtagcagca agaacaaggt ccgcagtcaa caaattagtg acgctgaccc ataaggtagg 9000

ccaaatcttt gttactcctg agcttgtcat tgtgacacat acagatgaga acaagttcac 9060

gtgtcttacc caggaacttg tgttgatgta tgcagatatg atggagggca gagatatggt 9120

cagcataata tcatccacgg cggcacatct taggagctta tcagagaaaa ttgatgatat 9180

tctgcggtta gtagatgctc tggcaagaga tttgggcaat caagtctacg atgttgtagc 9240

actaatggag ggattcgcat acggcgctgt tcagctgctt gaaccgtcag gtacatttgc 9300

gggggatttc ttcgcattca acctgcagga gctcaaagat actctaaccg gactcctccc 9360

caaggatatc gcagaatctg tgactcacgc aatcgcaacc atattctctg gcttagaaca 9420

aaatcaagca gctgagatgt tgtgcctgtt gcgtctgtgg ggtcacccac tactcgagtc 9480

ccgtattgca gcaaaagcag ttaggagtca aatgtgcgca ccaaaaatgg tagactttga 9540

tatgatcctc caggtattat ctttctttaa aggaacaatc atcaatggat atagaaagaa 9600

gaatgcaggc gtgtggccac gtgtcaagat agatacgata tacgggaagg tcatagggca 9660

gctacacgca gattctgcag agatttcaca tgatgtcatg ttgagggaat acaagagttt 9720

atctgcactt gaattcgagc catgtataga gtatgaccct gtcaccaatc tgagcatgtt 9780

tttaaaagac aaggcaatcg cacacccgaa agacaactgg ctcgcttcgt ttaggcgaaa 9840

ccttctctct gaggaccaga agaaacatgt aagagaggca acctcaacta accgcctctt 9900

gatagagttc ttagagtcaa atgattttga tccatataaa gagatggaat atctgacgac 9960

ccttgagtac ctaagagatg ataatgtggc agtatcatac tcactcaaag aaaaagaggt 10020

gaaagttaat gggcggattt tcgcaaagct aacaaataga ttaaggaatt gtcaggtaat 10080

ggcagaaggg atcctagctg accagattgc acctttcttc cagggaaatg gggtcattca 10140

ggatagcata tctttgacta aaagtatgct agcgatgagt caactgtctt tcaacagcaa 10200

caagaaacgt attactgact gcaaagaaag agtatcctca aaccgcaatc atgatccgaa 10260

gagcaagaat cgtcggagag ttgccacttt tgtaactacc gacctgcaaa agtattgtct 10320

caattggaga tatcagacag tcaagctgtt cgctcatgcc atcaatcagc tgatgggctt 10380

acctcacttc ttcgagtgga ttcatcttag actaatggat actacgatgt ttgtagggga 10440

ccctttcaat cctccaagtg accctacgga ctgtgatcta tcaagagtcc caaatgatga 10500

catatatatt gtcagtgcta ggggaggcat tgagggatta tgccagaagc tatggacaat 10560

gatctcaatt gctgcaatcc agcttgctgc agcaagatca cattgtcgcg ttgcctgtat 10620

ggtacaaggc gacaatcaag taatagctat aacgagagag gtaagatcag atgactcccc 10680

agagatggtg ttaacacaat tgcatcaagc cagtgataat ttcttcaagg aattgattca 10740

tgtcaatcat ttgatcggcc ataatttgaa ggatcgtgaa accatcaggt cagacacgtt 10800

cttcatatac agcaaacgaa tattcaaaga tggagcaata ctcagtcagg tcctcaaaaa 10860

ctcatctaaa ttagtgctaa tatcaggcga ccttagtgaa aacactgtaa tgtcttgtgc 10920

caacattgca tctactgtag cacggttatg cgagaacggg cttcctaagg atttctgtta 10980

ttacttaaac tacttaatga gttgcgtgca gacatacttc gattccgaat tttccatcac 11040

caacaactcg caactcgatt ctaaccagtc gtggatagag gacatttctt ttgtgcactc 11100

atatgtcctg acccctgctc aactgggggg actgagtaac cttcaatact caaggctcta 11160

cacaaggaac atcggcgacc cgggaaccac tgctttcgca gagatcaaga gattagaggc 11220

agtggggtta ctgagtccta gcattatgac taacatctta actaggccgc ctggaaatgg 11280

agattgggcc agtctgtgca acgatccata ctcctttaat tttgagactg tcgcaagccc 11340

aaatattgtc cttaagaaac atacacaaag agtcctattt gaaacttgct cgaatccctt 11400

attatctgga gtgcacacag aggataatga ggcagaagag aaggcattgg ctgaattctt 11460

gctcaatcaa gaaatgattc atccacgtgt cgcgcatgct atcatggaag caagctctgt 11520

aggtaggaga aagcaaattc aggggcttgt tgacacaaca aacaccgtga ttaagattgc 11580

actgactagg aggccactcg gcatcaagag gctgatgcgg atagtcaatt actcgagcat 11640

gcatgcaatg ctatttagag atgatgtttt ctcgtctaat agatccaacc atcccttagt 11700

ctcttctgat atgtgttctc agacactggc agactatgca cggaacagaa gctggtcacc 11760

tttgacaggg ggtagaaaaa tactgggtgt atctaatcct gataccatag aacttgtaga 11820

gggtgagatc cttagtgtca gtggagggtg cactagatgt gacagtgggg atgaacagtt 11880

tacttggttc catcttccaa gcaatataga gctaaacgat gacaccagca agaatcctcc 11940

aatgagagtg ccatatctcg ggtcaaagac tcaagagagg agagccgcct cgcttgcgaa 12000

aatagctcat atgtcaccac acgtgaaggc ggctctaagg gcgtcatctg tgttaatctg 12060

ggcttatggg gacaacgaaa taaactggac tgctgccctt aagattgcaa ggtctcggtg 12120

caacataagc tcagagtatc ttcgactatt gtcacccttg cctacagctg ggaatctcca 12180

acatagattg gacgacggca taactcagat gacattcacc cctgcatctc tatacagggt 12240

gtcaccttac attcatatat ctaatgattc tcaaaggcta tttactgaag aaggagtcaa 12300

agaggggaat gtggtttatc agcaaatcat gctcttgggt ttgtccttaa ttgagtcact 12360

cttcccaatg acaacaacca agacatatga tgaaatcaca ttgcacctcc acagtaaatt 12420

tagctgctgt atcagggaag cacctgttgc agttcctttc gagctactcg gggtggcacc 12480

ggaactaagg gcagtaacct caaataagtt tatgtatgat cctagccctg tatcagaggg 12540

agactttgcg agacttgact tagctatctt taagagttat gaacttaatt tagagtcata 12600

ttccacaata gagctaatga acgttctttc aatatctagt gggaagttga ttggccagtc 12660

tgtggtttct tatgatgaag atacctccat aaagaatgac gctataatag tgtatgacaa 12720

cacacggaat tggatcagcg aagctcagaa ttcagatgtg gtccgcctat tcgagtatgc 12780

ggcactcgaa gtgctcctcg actgttctta tcaactctac tatctgagag taagaggcct 12840

aaacaatatc gtcctgtaca tgagtgattt atacaagaat atgccaggaa ttctactctc 12900

taatattgca gccacaatat ctcaccctgt catccattca aggttgaatg cagtaggtct 12960

ggtcaaccat gacgggtcac accaacttgc agacacagat ttcattgaaa tgtctgcaaa 13020

gctgctagtc tcttgcactc gacgcgtggt ctcaggttta catgcaggga ataagtatga 13080

cctgctgttt ccatctgtct tggatgataa cctaagtgag aagatgcttc agttgatatc 13140

ccaattatgc tgtctgtata cggtgctctt tgctacaaca agagaaatcc cgaaaataag 13200

aggcttatct gcagaagaga aatgttcagt acttactgag tacctactgt cagatgctgt 13260

gaaaccgtta cttgggtccg agcaagtgag ctctatcatg tctcccaaca tagttacgtt 13320

cccagccaat ctgtattaca tgtctaggaa gagccttaat ttgatcaggg agagagagga 13380

cagggatact atcttggcat tgttgttccc tcaagaaccg ctgctcgagt ttcctctggt 13440

acgagatatt ggtgctcgtg taaaagatcc atttacccta caacctgcgg cgtttttaca 13500

agagttagat ttgagtgctc cggcaaggta tgacgcattc acaatcagtc aggcgcattc 13560

tgaacacata ttgccaaacc cagaggaaga tcacttagta cgatacttgt tcagaggaat 13620

agggactgcg tcctcctctt ggtataaggc atcccatctt ctttctgtac ccgaggtcag 13680

atgtgcaagg catgggaact ccttatattt agcagaagga agtggagcca tcatgagtct 13740

tctcgaattg catataccac acgaaactat ctattacaat acacttttct cgaacgagat 13800

gaacccccca cagcgacatt tcggaccaac tccaacacag tttctgaatt cggttgtttt 13860

taggaattta caggcggaag taccatgcaa ggatggattt gtccaggagt tccgtccgtt 13920

atggagagag aatacagaag aaagcgatct gacctcagat aaagcagtgg gatatatcac 13980

atctgtagtg ccgtacaggt ctgtatcatt gctgcattgt gacattgaaa tccctccagg 14040

atctaatcaa agcttactag atcagctggc taccaatctg tctctgattg ccatgcattc 14100

tgtgaaggag ggcggggtcg tgattatcaa agtactgtat gcaatgggat attacttcca 14160

tctactcatg aacttgttta ctccatgttc cacgaaagga tatattctct ctaatggcta 14220

tgcctgtaga ggggatatgg agtgttacct gatatttgtc atgggctacc taggcgggcc 14280

tacatttgta catgaggtgg tgaggatggc aaaaacgcta gtacagcggc atggcacact 14340

tttgtctaag tcagatgaga ttacactgac taggttattt acttcacagc aacagcgtgt 14400

aacagacatc ctatccagcc ctttaccgag actaatgaag tacttgagag agaatattga 14460

tactgcactg attgaagccg ggggacagcc tgtccgtcca ttctgtgcag agagtttagt 14520

gagcacacta acagacatga ctcagacaac ccagatcatc gccagccaca ttgatacagt 14580

cattcgatct gtgatctata tggaagcaga gggtgatctt gctgacacag tattcttatt 14640

taccccttac aacctctcta tggacgggaa aaagagaaca tcacttaaac agtgcacaag 14700

acagatctta gaggtcacaa tactgggtct cagagtcaaa aatctcaata aagtaggtga 14760

tgtaatcagc ctagtactca gaggtatgat ttctctggag gaccttctcc cactgagaac 14820

ctacttgaag tgtagtacct gccctaagta tttgaaggct gtcctaggta ttaccaaact 14880

caaagaaatg ttcacagacg cctctttatt atacttgact cgtgctcaac aaaaattcta 14940

catgaaaact ataggcaatg cagtcaaggg atactatagt aactgtgact cttaaaggca 15000

accacatatc aataggccct ctttctagcc gatcgtattc ttgttgactt cattatacca 15060

tattagaaaa aaattgaatt ccgacccttt aagactcgta ttcggattca aataattatc 15120

tcagaaaaaa agtgcacgta gttgttcttg attatagtcc cgtcattcac caagtctttg 15180

tttggt 15186

<210> 4
<211> 17436
<212> DNA
<213> Newcastle disease virus

<220>
<223> Mut HN (F277L)/M(G165W)-Atezolizumab

<400> 4
   accaaacaga gaatccgtga gatacgataa aaggcgaagg agcaatcgaa gtcgtacggg 60
   tagaaggtgt gaatctcgag tgcgagaccg aagcttaaac tcgagagagc cttctgccaa 120
   catgtcttcc gtatttgacg agtatgaaca gctcctcgcg gctcagactc gccctaatgg 180
   agctcacgga ggaggagaga aggggagcac cttaaaagtt gaagtcccgg tattcactct 240
   taacagtgac gacccagaag atagatggaa ctttgcagta ttctgtctta ggattgctgt 300
   tagcgaagat gccaacaaac cactcagaca aggtgctctc atatctctct tatgctccca 360
   ttctcaagtg atgaggaacc atgtcgccct tgcagggaaa cagaatgagg ccacactggc 420
   tgttcttgag atcgatggtt tcaccaacag cgtgcctcaa tttaacaaca ggagtggagt 480
   gtctgaggag agagcacaga gattcttgat gatagcaggg tccctccctc gggcatgcag 540
   caacggtacc ccgttcatca cagctggggt tgaagatgat gcaccagaag acatcactga 600
   tactctggaa agaatcatct ctatccaggc tcaagtatgg gtcacggtag caaaggccat 660
   gactgcatat gagacagcgg atgagtcgga aacaagaaga atcaataagt atatgcagca 720
   aggcagagtc caaaagaagt acatcctcca ccccgtatgt aggagcgcaa ttcaactcac 780
   gattagacaa tctctggcag tccgcatttt cttggttagc gagcttaaga gaggccgcaa 840
   cacggcaggt ggaagttcca cttattatag cttggtaggg gacatagact catacatcag 900
   gaacaccggg cttactgcat tcttcctgac actcaggtat ggaattaaca ccaagacatc 960
   agcccttgca ctcagcagcc tcgcaggcga tatccaaaaa atgaagcagc tcatgcgtct 1020
   atatcggatg aaaggagata atgcgccgta catgacactg ctcggtgaca gtgaccagat 1080
   gagctttgca ccggctgagt atgcacaact ctactctttt gcaatgggta tggcatccgt 1140
   cctagacaaa ggaactggca agtaccagtt cgccagagac tttatgagca catcattctg 1200
   gagacttgga gtagagtatg ctcgggctca gggaagcagc atcaatgagg atatggctgc 1260
   cgagctaaag ctgaccccag cggcaaggag gggcctggca gctgctgccc aacgagcgtc 1320
   cgaggagacc ggcagcatgg acattcccac tcaacaagct ggggtcctca cagggctcag 1380
   cgacggaggc cctcaagccc cacaaggtgg actgaacagg tcgcaagggc aaccggacgc 1440
   cggagatggg gagacccaat ttttagattt gatgagagca gtggcaaata gtatgagaga 1500
   agcgccaaac tctgtgcaga acaccactca gcaggagcct ccatctactc ctgggccatc 1560
   tcaagacaac gacaccgact ggggatactg accgacaaca ctcagcctgc ctccatcgaa 1620
   ttatctcgat tcttctgcct gtaacctaac ccctggtcca cagtctcaca cggccaaacc 1680
   cgcaaacgag cccccccctc tctccccccc cctccagcca cacggctcca accgcccaaa 1740
   acaacacggg cacaactcga ctcactcata atctacacag agccgaagat cttagaaaaa 1800
   aatacgggta gaagagagac attcagagat caggacaaat catcagggtc tccgctctcc 1860
   cctctaccca gcagaccaga gtgaagatgg ccaccttcac ggatgcagag atcgacgatc 1920
   tatttgaaac cagtggaact gtcattgaca gcataattac ggcccaaggc aacccagtag 1980
   agactgttgg aaggagcgca atcccacaag gcaaaactaa agcattgagt gcagcatggg 2040
   agaaacatgg aagcacccaa ccgccggccg gccaagacac ccctgatcga atggacagat 2100
   cagacaaaca accatcccca cccgaacagg caaccacaca caacagcccg ccagccacat 2160
   ccaccgacca gccccccacc caggccgcag gcgaagccgg tgacacacag ctcaagactg 2220
   gagcaagcaa ctccctcctg tcgatgctcg acaagcttag caataaatcg tccaatgcta 2280
   aaaagggccc acggtcgagt ccccaggaag ggcaccacca acctccgacc cagcagcaag 2340
   ggagtcaaca gagccgcgga aacagtcagg agagatcgca aaaccaggtc aaggccgcct 2400
   ctggagacca gggcacagac gcgaacacag catatcatgg acaatggaag gagtcacaac 2460
   catcagctgg tgcaatccct catgctctcc ggtcagggca gagccaagcc aatactcctg 2520
   catctgtgga tcatgtccag ctacctgtcg actttgtgca ggcgatgatg tctatgatgg 2580
   aggcgatatc acagaaggta aataaagtcg actatcagct agaccttgtc tcaaagcaga 2640
   catcctccat ccccatgatg cgatctgaaa tccaacagct gaaaacatcc gttgcggtca 2700
   tggaagccaa cctgggcatg atgaagattc tggaccctgg ttgtgctaac gtttcatctt 2760
   taagtgacct acgggcggtc gcccgctctc acccagtttt agtttcaggc cccggagacc 2820
   catctcctta tgtgacacaa gggggtgaat tgatactcaa taaactctcg caaccagtgc 2880
   aacatccttc tgagttgatt aaacctgcca cgataagcgg gcctgatata ggagtggaga 2940
   aggacactgt ccgtgcattg atcacctcac gcccaatgca tccgagctct tcagctaagc 3000
   tcctgagtaa gctggatgca gccgggtcga tagaagaaat taggaaaatc aaacgccttg 3060
   cgctgaatgg ctgatcacta ctacaacccg caacgggttc ccgtttatct attgtcacaa 3120
   ggaacccgcc ccgaagctta agaaaaaata cgggtagaag cagtttgaga gctccgttcg 3180
   ccaccatgga gttcggcctg tcctgggtgt tcctggtggc cctgttccgc ggcgtgcagt 3240
   gcgaggtgca gctggtggag agcggcggcg gcctggtgca gcccggcggc agcctgaggc 3300
   tgagctgcgc cgccagcggc ttcaccttca gcgacagctg gatccactgg gtgaggcagg 3360
   cccccggcaa gggcctggag tgggtggcct ggatcagccc ctacggcggc agcacctact 3420
   acgccgacag cgtgaagggc aggttcacca tcagcgccga caccagcaag aacaccgcct 3480
   acctgcagat gaacagcctg agggccgagg acaccgccgt gtactactgc gccaggaggc 3540
   actggcccgg cggcttcgac tactggggcc agggcaccct ggtgaccgtg agcagcgcca 3600
   gcaccaaggg ccccagcgtg ttccccctgg cccccagcag caagagcacc agcggcggca 3660
   ccgccgccct gggctgcctg gtgaaggact acttccccga gcccgtgacc gtgagctgga 3720
   acagcggcgc cctgaccagc ggcgtgcaca ccttccccgc cgtgctgcag agcagcggcc 3780
   tgtacagcct gagcagcgtg gtgaccgtgc ccagcagcag cctgggcacc cagacctaca 3840
   tctgcaacgt gaaccacaag cccagcaaca ccaaggtgga caagaaggtg gagcccaaga 3900
   gctgcgacaa gacccacacc tgccccccct gccccgcccc cgagctgctg ggcggcccca 3960
   gcgtgttcct gttccccccc aagcccaagg acaccctgat gatcagcagg acccccgagg 4020
   tgacctgcgt ggtggtggac gtgagccacg aggaccccga ggtgaagttc aactggtacg 4080
   tggacggcgt ggaggtgcac aacgccaaga ccaagcccag ggaggagcag tacgccagca 4140
   cctacagggt ggtgagcgtg ctgaccgtgc tgcaccagga ctggctgaac ggcaaggagt 4200
   acaagtgcaa ggtgagcaac aaggccctgc ccgcccccat cgagaagacc atcagcaagg 4260
   ccaagggcca gcccagggag ccccaggtgt acaccctgcc ccccagcagg gaggagatga 4320
   ccaagaacca ggtgagcctg acctgcctgg tgaagggctt ctaccccagc gacatcgccg 4380
   tggagtggga gagcaacggc cagcccgaga acaactacaa gaccaccccc cccgtgctgg 4440
   acagcgacgg cagcttcttc ctgtacagca agctgaccgt ggacaagagc aggtggcagc 4500
   agggcaacgt gttcagctgc agcgtgatgc acgaggccct gcacaaccac tacacccaga 4560
   agagcctgag cctgagcccc ggcaagcgcc gcaagcgcgg ctccggcgag ggccgcggct 4620
   ccctgctgac ctgcggcgac gtggaggaga accccggccc catgaagtac ctgctgccca 4680
   ccgccgccgc cggcctgctg ctgctggccg cccagcccgc catggccgac atccagatga 4740
   cccagagccc cagcagcctg agcgccagcg tgggcgacag ggtgaccatc acctgcaggg 4800
   ccagccagga cgtgagcacc gccgtggcct ggtaccagca gaagcccggc aaggccccca 4860
   agctgctgat ctacagcgcc agcttcctgt acagcggcgt gcccagcagg ttcagcggca 4920
   gcggcagcgg caccgacttc accctgacca tcagcagcct gcagcccgag gacttcgcca 4980
   cctactactg ccagcagtac ctgtaccacc ccgccacctt cggccagggc accaaggtgg 5040
   agatcaagag gaccgtggcc gcccccagcg tgttcatctt cccccccagc gacgagcagc 5100
   tgaagagcgg caccgccagc gtggtgtgcc tgctgaacaa cttctacccc agggaggcca 5160
   aggtgcagtg gaaggtggac aacgccctgc agagcggcaa cagccaggag agcgtgaccg 5220
   agcaggacag caaggacagc acctacagcc tgagcagcac cctgaccctg agcaaggccg 5280
   actacgagaa gcacaaggtg tacgcctgcg aggtgaccca ccagggcctg agcagccccg 5340
   tgaccaagag cttcaacagg ggcgagtgct gataagctga ccgagccctc ctctataaac 5400
   ccaagattca aggctccaag cgataacctt ctcccgcctc ttccatccca ctgaatgatc 5460
   gcgcagctgc aattaatcta gcaacattaa agattaagaa aaatacgggt tagaatcgga 5520
   gtgcaccgat tgtgccaaga tggactcatc taggacaatt gggttatact ttgattctgc 5580
   ccttccttct agcaacctgt tagcattccc gattgtctta cgagatgtag gagatggaaa 5640
   gaagcaaatt actccgcaat ataggatccg gcgtcttgac tcgtggacag acagtaaaga 5700
   agactcggta ttcatcacta cctacggatt catcttccag gttgggaatg aagaagtcac 5760
   tgtcggcatg atcaatgata atcccaagcg agagttactc tccgctgcga tgctctgcct 5820
   aggaagcgtc ccgaatgttg gagatcctgt tgagctggca agggcctgcc tcactatggt 5880
   ggtaacatgc aagaagagtg caactaatac tgagagaatg gtcttctcag tagtgcaggc 5940
   accccgggtg ctgcaaagct gtagggtcgt ggcagacaaa tactcgtcag tgaatgcagt 6000
   taagcacgtg aaagcaccag agaagatccc ttggagtgaa accctagagt acaaggtgaa 6060
   ttttgtctct ttgactgtgg tgccgaagaa ggatgtctac aagatcccaa ccgcagtatt 6120
   gaaggtatcc ggctcgagcc tgtataatct tgcgctcaat gtcactattg atgtggaggt 6180
   agacccaaag agcccgttag tcaagtccct ttctaggtcc gacagtggat actatgctaa 6240
   tctcttctta catatcggac ttatgtccac tgtagataag aggggaaaga aagtgacatt 6300
   tgaccagctg gagaggaaga taaggagact tgatctatct gtcgggctca gtgatgtgct 6360
   cggaccttct gtgcttgtga aggcgagagg tgcacggact aggctgctgg cacctttctt 6420
   ctctaacagc gggacagcct gctaccctat agcaaatgcc tctcctcagg tagctaagat 6480
   actctggagt cagaccgcgt gcctgcggag tgtaaaaatc attattcaag cgggcaccca 6540
   acgcgctgtc gcagtgactg ctgaccatga ggttacctct actaagatag agaagaggca 6600
   taccatcgct aaatacaatc ccttcaagaa ataggccgta tccctgagac tgcaattcac 6660
   ccgccttccc aaaacaccat gacaccagat aatgatctgt cttgattact tacagttagt 6720
   ttccctgtct atcaaattag aaaaaacacg ggtagaagag ttcggatccc ggccggcgca 6780
   ccaaaagcgc aagatgggcc ccagatcttc taccaggatc ccagtacctc taatgctgac 6840
   catacggatc acgctggcac tgagttatgt ccgtctgaca agttctcttg atggcaggcc 6900
   tcttgcagcc gcagggatcg tggtaacagg ggataaagca gttaacatat acacctcatc 6960
   ccagacaggg tcaatcatag tcaagttact cccaaatatg cccaaggaca aagaggcatg 7020
   tgcaaaagcc ccattggagg cttacaacag gacactgact actttgctta ccccccttgg 7080
   tgattctatc cgcaggatac aagagtctgt gactacatcc ggaggaagga gacagagacg 7140
   ctttataggt gccattattg gcagtgtagc tctaggggtt gcaacagctg cacagataac 7200
   ggcagcctcg gctctgatac aagccaacca gaatgctgct aacatcctcc ggcttaagga 7260
   gagcattgct gcaaccaatg aagctgtaca cgaggtcact ggcggattgt cacagttagc 7320
   agtggcagtt ggaaagatgc agcaatttgt taatgaccag tttaataaca cagctcaaga 7380
   gttggactgt ataaaaatta cacagcaggt tggtgtagaa ctcaacttat acttaactga 7440
   attgactaca gtgttcgggc cacaaatcac ttcccctgcc ttaactcagt tgactgttca 7500
   ggctctttac aatctggctg gtggtaatgt agattacttg ttgactaagt taggtgtagg 7560
   gaacaaccag ctcagctcat tgattggtag cggcttgatc accggtaacc ctatttttta 7620
   cgactcacag actcaactct tgggcataca ggtgacttta ccctcagtcg ggaacctaaa 7680
   taatatgcgt gccacctact tggagacctt gtctgtaagc acaacaaagg gatttgcctc 7740
   agcacttgtc ccaaaagtag tgacacaggt cggttctgtg atagaagagc ttgacacctc 7800
   atactgtata gaagctgatt tggatttata ttgtacaaga atagtgacat tccctatgtc 7860
   ccctggtatt tattcctgtt tgagcggcaa tacatcggct tgcatgtatt caaggactga 7920
   aggcgcactt actacaccat acatgactct caaaggctca gttgttgcca attgccagat 7980
   gacaacatgt agatgtgcag acccccgggg tatcatatca caaaattatg gagaggctgt 8040
   gtctctaata gataagcact catgcaatgt cgtatcctta gacgggataa ctttgaggct 8100
   cagtggggaa tttgatgcaa cttatcaaaa gaatatctca atattagatt ctcaagtact 8160
   agtgacaggc aatctcgata tctcaactga gcttgggaat gtcaaccact caataagtaa 8220
   tgctttggat aagttagagg aaagcaacag caaactagac aaagtcaatg tcaaactgac 8280
   cagcacatct gctctcatta cctatattgt tttaactgtc atatctcttg ttcttggtat 8340
   gcttagcctg gttctagcat gctatctgat gtacaagcaa aaagcgcaac gaaagacctt 8400
   gttgtggctt ggaaataata ccctagatca gatgagagct actacaaaaa tgtgaatgca 8460
   gatgagaggc agaggtatcc ccaatagcaa tctgtgtgtc aattctggca gcctgttaat 8520
   cagaagaatt aagaaaaaac taccggatgt aggtgaacaa aagggaatat acgggtagaa 8580
   cggcctgaga ggccacccct caatcgggag ccaggcccca ctacgtccgc tctaccgcat 8640
   caccaacagc agtcttcagt catggacagc gcagttagcc aagttgcgct agagaatgat 8700
   agaagagaag cgaataatac atggcgcttg gttttccgga tcgcagcctt acttttactg 8760
   gtaataacct tagccgtctc tgcagtcgcc ctggcatata gtatggaggc tagcacacct 8820
   ggcgaccttg taagcatacc gactgcgatc tctagggcag aggaaaggat tacatctgca 8880
   ctcggttcca atcaagatgt agtagatagg atatacaagc aagtggccct tgagtctcca 8940
   ttggcactgc taaataccga atctataatt atgaatgcaa taacgtctct ctcttatcaa 9000
   atcaatggag ccacaaataa tagcgggtgt ggggcacctg ttcatgaccc agattacatc 9060
   ggggggatag gtcaagaact tattgtagat gatacgagtg acgtcacatc attttatccc 9120
   tctgcattcc aagaacacct gaattttatc ccggcgccca ctacaggatc aggctgcact 9180
   cggataccct cattcgacat gagtgctacc cattactgtt acactcacaa tgtgatattg 9240
   tctggctgca gagatcactc acactcacat cagtatttag cacttggtgt gcttcggaca 9300
   tctgcaacag ggagggtatt cttttctact ctgcgttcca tcaacttgga tgacgcccaa 9360
   aatcggaagt cttgcagtgt gagtgcaact cctttaggtt gtgatatgct gtgctctaaa 9420
   atcacagaga ctgaggaaga ggattataaa tcagttatcc ccacatcgat ggtacatgga 9480
   aggttagggc ttgacggcca ataccatgag aaggacctag acgtcacaac actatttagg 9540
   gactgggtgg caaattaccc aggagtagga ggtgggtctt ttattaacaa tcgcgtatgg 9600
   ttcccggtct acggagggct aaaacccagc tcgcctagtg acactgcaca agaaggagga 9660
   tacgtaatat acaagcgata caatgacaca tgcccggatg agcaagatta ccagattcgg 9720
   atggctaagt catcgtataa gcctaggcgg tttggtggaa aacgcgtaca gcaggccatc 9780
   ctatccatca aggtgtcaac atccttgggt gaggacccgg tgttgactgt accgcctaat 9840
   acggtcgcac tcatgggggc cgaaggcaga gttctcacag tggggacatc tcatttctta 9900
   tatcagcgag ggtcatcata cttctctccc gctttgttat accctatgac agtcaacaac 9960
   aaaacagcca ctcttcataa tccttataca ttcaatgctt tcactcggcc aggtagtgtc 10020
   ccttgccagg cttcagcaag atgccccaac tcatgtgtta ccggagtcta tactgatcca 10080
   tatcccttag tcttccatag gaaccacacc ttgcgagggg tattcggaac aatgcttgat 10140
   gacgaacaag caagactcaa ccctgtatct gcagtatttg ataacatatc ccgcagtcgc 10200
   ataactcggg taagttcaag cagtaccagg gcagcataca cgacatcaac gtgttttaaa 10260
   gttgtcaaga ccaataaaac ctattgcctc agcattgcag aaatatccaa taccctcttc 10320
   ggggaattca gaattgtccc cttactagtt gagattctca aggatggtgg ggtttaagaa 10380
   gctaggaatg gtcggttgag tcaaccgtga gaaggcgggg aagatgatat tgcatcatct 10440
   atcttctgta acaccaagaa tcaaatcgaa taccagtgcg agctcgaatc ctacgctgcc 10500
   agtcagccat aatcggctgg tgctaatgtg gttagcctga atcttgtcga tagtcacttg 10560
   attaagaaaa aatgtggacg gtagtgaaat ataaggcaaa gcagctcaca gaggacagca 10620
   cgggtaggac atggcgggct ccggacccga aagggcagag catcagatta tcctaccaga 10680
   gtcacatctg tcctcaccat tggtcaagca taaattgctt tattactgga aattaactgg 10740
   gctaccgctt cctgacgaat gtgacttcga ccatcttatt atcagccgac aatggaagaa 10800
   ggtacttgaa tcggccaccc ctgacattga gagaatgata aaactagggc gggcagtaca 10860
   ccagactctc aaccacaatt ccaggataac cggagtactc catccccggt gtttagagga 10920
   actggctagt attgaggttc ctgattcaac caacaaattt cggaagatcg aaaaaaagat 10980
   ccagattcac aacacaaggt atggagaact gttcacaaga ttatgcacgc atgtagaaaa 11040
   gaaactattg gggtcatctt ggtctaacaa tgtcccacgg tcagaggaat tcaacagcat 11100
   ccgtacagat ccggcatttt ggtttcactc aaaatggtcc acagccaaat ttgcatggct 11160
   ccatataaaa caggtccaga ggcatctgat tgtagcagca agaacaaggt ccgcagtcaa 11220
   caaattagtg acgctgaccc ataaggtagg ccaaatcttt gttactcctg agcttgtcat 11280
   tgtgacacat acagatgaga acaagttcac gtgtcttacc caggaacttg tgttgatgta 11340
   tgcagatatg atggagggca gagatatggt cagcataata tcatccacgg cggcacatct 11400
   taggagctta tcagagaaaa ttgatgatat tctgcggtta gtagatgctc tggcaagaga 11460
   tttgggcaat caagtctacg atgttgtagc actaatggag ggattcgcat acggcgctgt 11520
   tcagctgctt gaaccgtcag gtacatttgc gggggatttc ttcgcattca acctgcagga 11580
   gctcaaagat actctaaccg gactcctccc caaggatatc gcagaatctg tgactcacgc 11640
   aatcgcaacc atattctctg gcttagaaca aaatcaagca gctgagatgt tgtgcctgtt 11700
   gcgtctgtgg ggtcacccac tactcgagtc ccgtattgca gcaaaagcag ttaggagtca 11760
   aatgtgcgca ccaaaaatgg tagactttga tatgatcctc caggtattat ctttctttaa 11820
   aggaacaatc atcaatggat atagaaagaa gaatgcaggc gtgtggccac gtgtcaagat 11880
   agatacgata tacgggaagg tcatagggca gctacacgca gattctgcag agatttcaca 11940
   tgatgtcatg ttgagggaat acaagagttt atctgcactt gaattcgagc catgtataga 12000
   gtatgaccct gtcaccaatc tgagcatgtt tttaaaagac aaggcaatcg cacacccgaa 12060
   agacaactgg ctcgcttcgt ttaggcgaaa ccttctctct gaggaccaga agaaacatgt 12120
   aagagaggca acctcaacta accgcctctt gatagagttc ttagagtcaa atgattttga 12180
   tccatataaa gagatggaat atctgacgac ccttgagtac ctaagagatg ataatgtggc 12240
   agtatcatac tcactcaaag aaaaagaggt gaaagttaat gggcggattt tcgcaaagct 12300
   aacaaataga ttaaggaatt gtcaggtaat ggcagaaggg atcctagctg accagattgc 12360
   acctttcttc cagggaaatg gggtcattca ggatagcata tctttgacta aaagtatgct 12420
   agcgatgagt caactgtctt tcaacagcaa caagaaacgt attactgact gcaaagaaag 12480
   agtatcctca aaccgcaatc atgatccgaa gagcaagaat cgtcggagag ttgccacttt 12540
   tgtaactacc gacctgcaaa agtattgtct caattggaga tatcagacag tcaagctgtt 12600
   cgctcatgcc atcaatcagc tgatgggctt acctcacttc ttcgagtgga ttcatcttag 12660
   actaatggat actacgatgt ttgtagggga ccctttcaat cctccaagtg accctacgga 12720
   ctgtgatcta tcaagagtcc caaatgatga catatatatt gtcagtgcta ggggaggcat 12780
   tgagggatta tgccagaagc tatggacaat gatctcaatt gctgcaatcc agcttgctgc 12840
   agcaagatca cattgtcgcg ttgcctgtat ggtacaaggc gacaatcaag taatagctgt 12900
   aacgagagag gtaagatcag atgactcccc agagatggtg ttaacacaat tgcatcaagc 12960
   cagtgataat ttcttcaagg aattgattca tgtcaatcat ttgatcggcc ataatttgaa 13020
   ggatcgtgaa accatcaggt cagacacgtt cttcatatac agcaaacgaa tattcaaaga 13080
   tggagcaata ctcagtcagg tcctcaaaaa ctcatctaaa ttagtgctaa tatcaggcga 13140
   ccttagtgaa aacactgtaa tgtcttgtgc caacattgca tctactgtag cacggttatg 13200
   cgagaacggg cttcctaagg atttctgtta ttacttaaac tacttaatga gttgcgtgca 13260
   gacatacttc gattccgaat tttccatcac caacaactcg caactcgatt ctaaccagtc 13320
   gtggatagag gacatttctt ttgtgcactc atatgtcctg acccctgctc aactgggggg 13380
   actgagtaac cttcaatact caaggctcta cacaaggaac atcggcgacc cgggaaccac 13440
   tgctttcgca gagatcaaga gattagaggc agtggggtta ctgagtccta gcattatgac 13500
   taacatctta actaggccgc ctggaaatgg agattgggcc agtctgtgca acgatccata 13560
   ctcctttaat tttgagactg tcgcaagccc aaatattgtc cttaagaaac atacacaaag 13620
   agtcctattt gaaacttgct cgaatccctt attatctgga gtgcacacag aggataatga 13680
   ggcagaagag aaggcattgg ctgaattctt gctcaatcaa gaaatgattc atccacgtgt 13740
   cgcgcatgct atcatggaag caagctctgt aggtaggaga aagcaaattc aggggcttgt 13800
   tgacacaaca aacaccgtga ttaagattgc actgactagg aggccactcg gcatcaagag 13860
   gctgatgcgg atagtcaatt actcgagcat gcatgcaatg ctatttagag atgatgtttt 13920
   ctcgtctaat agatccaacc atcccttagt ctcttctgat atgtgttctc agacactggc 13980
   agactatgca cggaacagaa gctggtcacc tttgacaggg ggtagaaaaa tactgggtgt 14040
   atctaatcct gataccatag aacttgtaga gggtgagatc cttagtgtca gtggagggtg 14100
   cactagatgt gacagtgggg atgaacagtt tacttggttc catcttccaa gcaatataga 14160
   gctaaacgat gacaccagca agaatcctcc aatgagagtg ccatatctcg ggtcaaagac 14220
   tcaagagagg agagccgcct cgcttgcgaa aatagctcat atgtcaccac acgtgaaggc 14280
   ggctctaagg gcgtcatctg tgttaatctg ggcttatggg gacaacgaaa taaactggac 14340
   tgctgccctt aagattgcaa ggtctcggtg caacataagc tcagagtatc ttcgactatt 14400
   gtcacccttg cctacagctg ggaatctcca acatagattg gacgacggca taactcagat 14460
   gacattcacc cctgcatctc tatacagggt gtcaccttac attcatatat ctaatgattc 14520
   tcaaaggcta tttactgaag aaggagtcaa agaggggaat gtggtttatc agcaaatcat 14580
   gctcttgggt ttgtccttaa ttgagtcact cttcccaatg acaacaacca agacatatga 14640
   tgaaatcaca ttgcacctcc acagtaaatt tagctgctgt atcagggaag cacctgttgc 14700
   agttcctttc gagctactcg ggtggcaccc ggaactaagg gcagtaacct caaataagtt 14760
   tatgtatgat cctagccctg tatcagaggg agactttgcg agacttgact tagctatctt 14820
   taagagttat gaacttaatt tagagtcata ttccacaata gagctaatga acgttctttc 14880
   aatatctagt gggaagttga ttggccagtc tgtggtttct tatgatgaag atacctccat 14940
   aaagaatgac gctataatag tgtatgacaa cacacggaat tggatcagcg aagctcagaa 15000
   ttcagatgtg gtccgcctat tcgagtatgc ggcactcgaa gtgctcctcg actgttctta 15060
   tcaactctac tatctgagag taagaggcct aaacaatatc gtcctgtaca tgagtgattt 15120
   atacaagaat atgccaggaa ttctactctc taatattgca gccacaatat ctcaccctgt 15180
   catccattca aggttgaatg cagtaggtct ggtcaaccat gacgggtcac accaacttgc 15240
   agacacagat ttcattgaaa tgtctgcaaa gctgctagtc ttttgcactc gacgcgtggt 15300
   ctcaggttta catgcaggga ataagtatga cctgctgttt ccatctgtct tggatgataa 15360
   cctaagtgag aagatgcttc agttgatatc ccaattatgc tgtctgtata cggtgctctt 15420
   tgctacaaca agagaaatcc cgaaaataag aggcttatct gcagaagaga aatgttcagt 15480
   acttactgag tacctactgt cagatgctgt gaaaccgtta cttgggtccg agcaagtgag 15540
   ctctatcatg tctcccaaca tagttacgtt cccagccaat ctgtattaca tgtctaggaa 15600
   gagccttaat ttgatcaggg agagagagga cagggatact atcttggcat tgttgttccc 15660
   tcaagaaccg ctgctcgagt ttcctctggt acgagatatt ggtgctcgtg taaaagatcc 15720
   atttacccga caacctgcgg cgtttttaca agagttagat ttgagtgctc cggcaaggta 15780
   tgacgcattc acaatcagtc aggcgcattc tgaacacata ttgccaaacc cagaggaaga 15840
   tcacttagta cgatacttgt tcagaggaat agggactgcg tcctcctctt ggtataaggc 15900
   atcccatctt ctttctgtac ccgaggtcag atgtgcaagg catgggaact ccttatattt 15960
   agcagaagga agtggagcca tcatgagtct tctcgaattg catataccac acgaaactat 16020
   ctattacaat acacttttct cgaacgagat gaacccccca cagcgacatt tcggaccaac 16080
   tccaacacag tttctgaatt cggttgtttt taggaattta caggcggaag taccatgcaa 16140
   ggatggattt gtccaggagt tccgtccgtt atggagagag aatacagaag aaagcgatct 16200
   gacctcagat aaagcagtgg gatatatcac atctgtagtg ccgtacaggt ctgtatcatt 16260
   gctgcattgt gacattgaaa tccctccagg atctaatcaa agcttactag atcagctggc 16320
   taccaatctg tctctgattg ccatgcattc tgtgaaggag ggcggggtcg tgattatcaa 16380
   agtactgtat gcaatgggat attacttcca tctactcatg aacttgttta ctccatgttc 16440
   cacgaaagga tatattctct ctaatggcta tgcctgtaga ggggatatgg agtgttacct 16500
   gatatttgtc atgggctacc taggcgggcc tacatttgta catgaggtgg tgaggatggc 16560
   aaaaacgcta gtacagcggc atggcacact tttgtctaag tcagatgaga ttacactgac 16620
   taggttattt acttcacagc aacagcgtgt aacagacatc ctatccagcc ctttaccgag 16680
   actaatgaag tacttgagag agaatattga tactgcactg attgaagccg ggggacagcc 16740
   tgtccgtcca ttctgtgcag agagtttagt gagcacacta acagacatga ctcagacaac 16800
   ccagatcatc gccagccaca ttgatacagt cattcgatct gtgatctata tggaagcaga 16860
   gggtgatctt gctgacacag tattcttatt taccccttac aacctctcta tggacgggaa 16920
   aaagagaaca tcacttaaac agtgcacaag acagatctta gaggtcacaa tactgggtct 16980
   cagagtcaaa aatctcaata aagtaggtga tgtaatcagc ctagtactca gaggtatgat 17040
   ttctctggag gaccttctcc cactgagaac ctacttgaag tgtagtacct gccctaagta 17100
   tttgaaggct gtcctaggta ttaccaaact caaagaaatg ttcacagacg cctctttatt 17160
   atacttgact cgtgctcaac aaaaattcta catgaaaact ataggcaatg cagtcaaggg 17220
   atactatagt aactgtgact cttaaaggca accacatatc aataggccct ctttctagcc 17280
   gatcgtattc ttgttgactt cattatacca tattagaaaa aaattgaatt ccgacccttt 17340
   aagactcgta ttcggattca aataattatc tcagaaaaaa agtgcacgta gttgttcttg 17400
   attatagtcc cgtcattcac caagtctttg tttggt 17436

<210> 5
<211> 17448
<212> DNA
<213> Newcastle disease virus

<220>
<223> Mut HN (F277L)/M(G165W)-Bevacizumab

<400> 5
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960
agcccttgca ctcagcagcc tcgcaggcga tatccaaaaa atgaagcagc tcatgcgtct 1020
atatcggatg aaaggagata atgcgccgta catgacactg ctcggtgaca gtgaccagat 1080
gagctttgca ccggctgagt atgcacaact ctactctttt gcaatgggta tggcatccgt 1140
cctagacaaa ggaactggca agtaccagtt cgccagagac tttatgagca catcattctg 1200
gagacttgga gtagagtatg ctcgggctca gggaagcagc atcaatgagg atatggctgc 1260
cgagctaaag ctgaccccag cggcaaggag gggcctggca gctgctgccc aacgagcgtc 1320
cgaggagacc ggcagcatgg acattcccac tcaacaagct ggggtcctca cagggctcag 1380
cgacggaggc cctcaagccc cacaaggtgg actgaacagg tcgcaagggc aaccggacgc 1440
cggagatggg gagacccaat ttttagattt gatgagagca gtggcaaata gtatgagaga 1500
agcgccaaac tctgtgcaga acaccactca gcaggagcct ccatctactc ctgggccatc 1560
tcaagacaac gacaccgact ggggatactg accgacaaca ctcagcctgc ctccatcgaa 1620
ttatctcgat tcttctgcct gtaacctaac ccctggtcca cagtctcaca cggccaaacc 1680
cgcaaacgag cccccccctc tctccccccc cctccagcca cacggctcca accgcccaaa 1740
acaacacggg cacaactcga ctcactcata atctacacag agccgaagat cttagaaaaa 1800
aatacgggta gaagagagac attcagagat caggacaaat catcagggtc tccgctctcc 1860
cctctaccca gcagaccaga gtgaagatgg ccaccttcac ggatgcagag atcgacgatc 1920
tatttgaaac cagtggaact gtcattgaca gcataattac ggcccaaggc aacccagtag 1980
agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gccccccacc caggccgcag gcgaagccgg tgacacacag ctcaagactg 2220
gagcaagcaa ctccctcctg tcgatgctcg acaagcttag caataaatcg tccaatgcta 2280
aaaagggccc acggtcgagt ccccaggaag ggcaccacca acctccgacc cagcagcaag 2340
ggagtcaaca gagccgcgga aacagtcagg agagatcgca aaaccaggtc aaggccgcct 2400
ctggagacca gggcacagac gcgaacacag catatcatgg acaatggaag gagtcacaac 2460
catcagctgg tgcaatccct catgctctcc ggtcagggca gagccaagcc aatactcctg 2520
catctgtgga tcatgtccag ctacctgtcg actttgtgca ggcgatgatg tctatgatgg 2580
aggcgatatc acagaaggta aataaagtcg actatcagct agaccttgtc tcaaagcaga 2640
catcctccat ccccatgatg cgatctgaaa tccaacagct gaaaacatcc gttgcggtca 2700
tggaagccaa cctgggcatg atgaagattc tggaccctgg ttgtgctaac gtttcatctt 2760
taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgttccgc ggcgtgcagt 3240

gcgaggtgca gctggtggag agcggcggcg gcctggtgca gcccggcggc agcctgaggc 3300

tgagctgcgc cgccagcggc tacaccttca ccaactacgg catgaactgg gtgaggcagg 3360

cccccggcaa gggcctggag tgggtgggct ggatcaacac ctacaccggc gagcccacct 3420

acgccgccga cttcaagagg aggttcacct tcagcctgga caccagcaag agcaccgcct 3480

acctgcagat gaacagcctg agggccgagg acaccgccgt gtactactgc gccaagtacc 3540

cccactacta cggcagcagc cactggtact tcgacgtgtg gggccagggc accctggtga 3600

ccgtgagcag cgccagcacc aagggcccca gcgtgttccc cctggccccc agcagcaaga 3660

gcaccagcgg cggcaccgcc gccctgggct gcctggtgaa ggactacttc cccgagcccg 3720

tgaccgtgag ctggaacagc ggcgccctga ccagcggcgt gcacaccttc cccgccgtgc 3780

tgcagagcag cggcctgtac agcctgagca gcgtggtgac cgtgcccagc agcagcctgg 3840

gcacccagac ctacatctgc aacgtgaacc acaagcccag caacaccaag gtggacaaga 3900

aggtggagcc caagagctgc gacaagaccc acacctgccc cccctgcccc gcccccgagc 3960

tgctgggcgg ccccagcgtg ttcctgttcc cccccaagcc caaggacacc ctgatgatca 4020

gcaggacccc cgaggtgacc tgcgtggtgg tggacgtgag ccacgaggac cccgaggtga 4080

agttcaactg gtacgtggac ggcgtggagg tgcacaacgc caagaccaag cccagggagg 4140

agcagtacaa cagcacctac agggtggtga gcgtgctgac cgtgctgcac caggactggc 4200

tgaacggcaa ggagtacaag tgcaaggtga gcaacaaggc cctgcccgcc cccatcgaga 4260

agaccatcag caaggccaag ggccagccca gggagcccca ggtgtacacc ctgcccccca 4320

gcagggagga gatgaccaag aaccaggtga gcctgacctg cctggtgaag ggcttctacc 4380

ccagcgacat cgccgtggag tgggagagca acggccagcc cgagaacaac tacaagacca 4440

ccccccccgt gctggacagc gacggcagct tcttcctgta cagcaagctg accgtggaca 4500

agagcaggtg gcagcagggc aacgtgttca gctgcagcgt gatgcacgag gccctgcaca 4560

accactacac ccagaagagc ctgagcctga gccccggcaa gcgccgcaag cgcggctccg 4620

gcgagggccg cggctccctg ctgacctgcg gcgacgtgga ggagaacccc ggccccatga 4680

agtacctgct gcccaccgcc gccgccggcc tgctgctgct ggccgcccag cccgccatgg 4740

ccgacatcca gatgacccag agccccagca gcctgagcgc cagcgtgggc gacagggtga 4800

ccatcacctg cagcgccagc caggacatca gcaactacct gaactggtac cagcagaagc 4860

ccggcaaggc ccccaaggtg ctgatctact tcaccagcag cctgcacagc ggcgtgccca 4920

gcaggttcag cggcagcggc agcggcaccg acttcaccct gaccatcagc agcctgcagc 4980

ccgaggactt cgccacctac tactgccagc agtacagcac cgtgccctgg accttcggcc 5040

agggcaccaa ggtggagatc aagaggaccg tggccgcccc cagcgtgttc atcttccccc 5100

ccagcgacga gcagctgaag agcggcaccg ccagcgtggt gtgcctgctg aacaacttct 5160

accccaggga ggccaaggtg cagtggaagg tggacaacgc cctgcagagc ggcaacagcc 5220

aggagagcgt gaccgagcag gacagcaagg acagcaccta cagcctgagc agcaccctga 5280

ccctgagcaa ggccgactac gagaagcaca aggtgtacgc ctgcgaggtg acccaccagg 5340

gcctgagcag ccccgtgacc aagagcttca acaggggcga gtgctgagct gaccgagccc 5400

tcctctataa acccaagatt caaggctcca agcgataacc ttctcccgcc tcttccatcc 5460

cactgaatga tcgcgcagct gcaattaatc tagcaacatt aaagattaag aaaaaatacg 5520

ggtagaatcg gagtgcaccg attgtgccaa gatggactca tctaggacaa ttgggttata 5580

ctttgattct gcccttcctt ctagcaacct gttagcattc ccgattgtct tacgagatgt 5640

aggagatgga aagaagcaaa ttactccgca atataggatc cggcgtcttg actcgtggac 5700

agacagtaaa gaagactcgg tattcatcac tacctacgga ttcatcttcc aggttgggaa 5760

tgaagaagtc actgtcggca tgatcaatga taatcccaag cgagagttac tctccgctgc 5820

gatgctctgc ctaggaagcg tcccgaatgt tggagatcct gttgagctgg caagggcctg 5880

cctcactatg gtggtaacat gcaagaagag tgcaactaat actgagagaa tggtcttctc 5940

agtagtgcag gcaccccggg tgctgcaaag ctgtagggtc gtggcagaca aatactcgtc 6000

agtgaatgca gttaagcacg tgaaagcacc agagaagatc ccttggagtg aaaccctaga 6060

gtacaaggtg aattttgtct ctttgactgt ggtgccgaag aaggatgtct acaagatccc 6120

aaccgcagta ttgaaggtat ccggctcgag cctgtataat cttgcgctca atgtcactat 6180

tgatgtggag gtagacccaa agagcccgtt agtcaagtcc ctttctaggt ccgacagtgg 6240

atactatgct aatctcttct tacatatcgg acttatgtcc actgtagata agaggggaaa 6300

gaaagtgaca tttgaccagc tggagaggaa gataaggaga cttgatctat ctgtcgggct 6360

cagtgatgtg ctcggacctt ctgtgcttgt gaaggcgaga ggtgcacgga ctaggctgct 6420

ggcacctttc ttctctaaca gcgggacagc ctgctaccct atagcaaatg cctctcctca 6480

ggtagctaag atactctgga gtcagaccgc gtgcctgcgg agtgtaaaaa tcattattca 6540

agcgggcacc caacgcgctg tcgcagtgac tgctgaccat gaggttacct ctactaagat 6600

agagaagagg cataccatcg ctaaatacaa tcccttcaag aaataggccg tatccctgag 6660

actgcaattc acccgccttc ccaaaacacc atgacaccag ataatgatct gtcttgatta 6720

cttacagtta gtttccctgt ctatcaaatt agaaaaaaca cgggtagaag agttcggatc 6780

ccggccggcg caccaaaagc gcaagatggg ccccagatct tctaccagga tcccagtacc 6840

tctaatgctg accatacgga tcacgctggc actgagttat gtccgtctga caagttctct 6900

tgatggcagg cctcttgcag ccgcagggat cgtggtaaca ggggataaag cagttaacat 6960

atacacctca tcccagacag ggtcaatcat agtcaagtta ctcccaaata tgcccaagga 7020

caaagaggca tgtgcaaaag ccccattgga ggcttacaac aggacactga ctactttgct 7080

tacccccctt ggtgattcta tccgcaggat acaagagtct gtgactacat ccggaggaag 7140

gagacagaga cgctttatag gtgccattat tggcagtgta gctctagggg ttgcaacagc 7200

tgcacagata acggcagcct cggctctgat acaagccaac cagaatgctg ctaacatcct 7260

ccggcttaag gagagcattg ctgcaaccaa tgaagctgta cacgaggtca ctggcggatt 7320

gtcacagtta gcagtggcag ttggaaagat gcagcaattt gttaatgacc agtttaataa 7380

cacagctcaa gagttggact gtataaaaat tacacagcag gttggtgtag aactcaactt 7440

atacttaact gaattgacta cagtgttcgg gccacaaatc acttcccctg ccttaactca 7500

gttgactgtt caggctcttt acaatctggc tggtggtaat gtagattact tgttgactaa 7560

gttaggtgta gggaacaacc agctcagctc attgattggt agcggcttga tcaccggtaa 7620

ccctattttt tacgactcac agactcaact cttgggcata caggtgactt taccctcagt 7680

cgggaaccta aataatatgc gtgccaccta cttggagacc ttgtctgtaa gcacaacaaa 7740

gggatttgcc tcagcacttg tcccaaaagt agtgacacag gtcggttctg tgatagaaga 7800

gcttgacacc tcatactgta tagaagctga tttggattta tattgtacaa gaatagtgac 7860

attccctatg tcccctggta tttattcctg tttgagcggc aatacatcgg cttgcatgta 7920

ttcaaggact gaaggcgcac ttactacacc atacatgact ctcaaaggct cagttgttgc 7980

caattgccag atgacaacat gtagatgtgc agaccccccg ggtatcatat cacaaaatta 8040

tggagaggct gtgtctctaa tagataagca ctcatgcaat gtcgtatcct tagacgggat 8100

aactttgagg ctcagtgggg aatttgatgc aacttatcaa aagaatatct caatattaga 8160

ttctcaagta ctagtgacag gcaatctcga tatctcaact gagcttggga atgtcaacca 8220

ctcaataagt aatgctttgg ataagttaga ggaaagcaac agcaaactag acaaagtcaa 8280

tgtcaaactg accagcacat ctgctctcat tacctatatt gttttaactg tcatatctct 8340

tgttcttggt atgcttagcc tggttctagc atgctatctg atgtacaagc aaaaagcgca 8400

acgaaagacc ttgttgtggc ttggaaataa taccctagat cagatgagag ctactacaaa 8460

aatgtgaatg cagatgagag gcagaggtat ccccaatagc aatctgtgtg tcaattctgg 8520

cagcctgtta atcagaagaa ttaagaaaaa actaccggat gtaggtgaac aaaagggaat 8580

atacgggtag aacggcctga gaggccaccc ctcaatcggg agccaggccc cactacgtcc 8640

gctctaccgc atcaccaaca gcagtcttca gtcatggaca gcgcagttag ccaagttgcg 8700

ctagagaatg atagaagaga agcgaataat acatggcgct tggttttccg gatcgcagcc 8760

ttacttttac tggtaataac cttagccgtc tctgcagtcg ccctggcata tagtatggag 8820

gctagcacac ctggcgacct tgtaagcata ccgactgcga tctctagggc agaggaaagg 8880

attacatctg cactcggttc caatcaagat gtagtagata ggatatacaa gcaagtggcc 8940

cttgagtctc cattggcact gctaaatacc gaatctataa ttatgaatgc aataacgtct 9000

ctctcttatc aaatcaatgg agccacaaat aatagcgggt gtggggcacc tgttcatgac 9060

ccagattaca tcggggggat aggtcaagaa cttattgtag atgatacgag tgacgtcaca 9120

tcattttatc cctctgcatt ccaagaacac ctgaatttta tcccggcgcc cactacagga 9180

tcaggctgca ctcggatacc ctcattcgac atgagtgcta cccattactg ttacactcac 9240

aatgtgatat tgtctggctg cagagatcac tcacactcac atcagtattt agcacttggt 9300

gtgcttcgga catctgcaac agggagggta ttcttttcta ctctgcgttc catcaacttg 9360

gatgacgccc aaaatcggaa gtcttgcagt gtgagtgcaa ctcctttagg ttgtgatatg 9420

ctgtgctcta aaatcacaga gactgaggaa gaggattata aatcagttat ccccacatcg 9480

atggtacatg gaaggttagg gcttgacggc caataccatg agaaggacct agacgtcaca 9540

acactattta gggactgggt ggcaaattac ccaggagtag gaggtgggtc ttttattaac 9600

aatcgcgtat ggttcccggt ctacggaggg ctaaaaccca gctcgcctag tgacactgca 9660

caagaaggga gatacgtaat atacaagcga tacaatgaca catgcccgga tgagcaagat 9720

taccagattc ggatggctaa gtcatcgtat aagcctaggc ggtttggtgg aaaacgcgta 9780

cagcaggcca tcctatccat caaggtgtca acatccttgg gtgaggaccc ggtgttgact 9840

gtaccgccta atacggtcgc actcatgggg gccgaaggca gagttctcac agtggggaca 9900

tctcatttct tatatcagcg agggtcatca tacttctctc ccgctttgtt ataccctatg 9960

acagtcaaca acaaaacagc cactcttcat aatccttata cattcaatgc tttcactcgg 10020

ccaggtagtg tcccttgcca ggcttcagca agatgcccca actcatgtgt taccggagtc 10080

tatactgatc catatccctt agtcttccat aggaaccaca ccttgcgagg ggtattcgga 10140

acaatgcttg atgacgaaca agcaagactc aaccctgtat ctgcagtatt tgataacata 10200

tcccgcagtc gcataactcg ggtaagttca agcagtacca gggcagcata cacgacatca 10260

acgtgtttta aagttgtcaa gaccaataaa acctattgcc tcagcattgc agaaatatcc 10320

aataccctct tcggggaatt cagaattgtc cccttactag ttgagattct caaggatggt 10380

ggggtttaag aagctaggaa tggtcggttg agtcaaccgt gagaaggcgg ggaagatgat 10440

attgcatcat ctatcttctg taacaccaag aatcaaatcg aataccagtg cgagctcgaa 10500

tcctacgctg ccagtcagcc ataatcggct ggtgctaatg tggttagcct gaatcttgtc 10560

gatagtcact tgattaagaa aaaatgtgga cggtagtgaa atataaggca aagcagctca 10620

cagaggacag cacgggtagg acatggcggg ctccggaccc gaaagggcag agcatcagat 10680

tatcctacca gagtcacatc tgtcctcacc attggtcaag cataaattgc tttattactg 10740

gaaattaact gggctaccgc ttcctgacga atgtgacttc gaccatctta ttatcagccg 10800

acaatggaag aaggtacttg aatcggccac ccctgacatt gagagaatga taaaactagg 10860

gcgggcagta caccagactc tcaaccacaa ttccaggata accggagtac tccatccccg 10920

gtgtttagag gaactggcta gtattgaggt tcctgattca accaacaaat ttcggaagat 10980

cgaaaaaaag atccagattc acaacacaag gtatggagaa ctgttcacaa gattatgcac 11040

gcatgtagaa aagaaactat tggggtcatc ttggtctaac aatgtcccac ggtcagagga 11100

attcaacagc atccgtacag atccggcatt ttggtttcac tcaaaatggt ccacagccaa 11160

atttgcatgg ctccatataa aacaggtcca gaggcatctg attgtagcag caagaacaag 11220

gtccgcagtc aacaaattag tgacgctgac ccataaggta ggccaaatct ttgttactcc 11280

tgagcttgtc attgtgacac atacagatga gaacaagttc acgtgtctta cccaggaact 11340

tgtgttgatg tatgcagata tgatggaggg cagagatatg gtcagcataa tatcatccac 11400

ggcggcacat cttaggagct tatcagagaa aattgatgat attctgcggt tagtagatgc 11460

tctggcaaga gatttgggca atcaagtcta cgatgttgta gcactaatgg agggattcgc 11520

atacggcgct gttcagctgc ttgaaccgtc aggtacattt gcgggggatt tcttcgcatt 11580

caacctgcag gagctcaaag atactctaac cggactcctc cccaaggata tcgcagaatc 11640

tgtgactcac gcaatcgcaa ccatattctc tggcttagaa caaaatcaag cagctgagat 11700

gttgtgcctg ttgcgtctgt ggggtcaccc actactcgag tcccgtattg cagcaaaagc 11760

agttaggagt caaatgtgcg caccaaaaat ggtagacttt gatatgatcc tccaggtatt 11820

atctttcttt aaaggaacaa tcatcaatgg atatagaaag aagaatgcag gcgtgtggcc 11880

acgtgtcaag atagatacga tatacgggaa ggtcataggg cagctacacg cagattctgc 11940

agagatttca catgatgtca tgttgaggga atacaagagt ttatctgcac ttgaattcga 12000

gccatgtata gagtatgacc ctgtcaccaa tctgagcatg tttttaaaag acaaggcaat 12060

cgcacacccg aaagacaact ggctcgcttc gtttaggcga aaccttctct ctgaggacca 12120

gaagaaacat gtaagagagg caacctcaac taaccgcctc ttgatagagt tcttagagtc 12180

aaatgatttt gatccatata aagagatgga atatctgacg acccttgagt acctaagaga 12240

tgataatgtg gcagtatcat actcactcaa agaaaaagag gtgaaagtta atgggcggat 12300

tttcgcaaag ctaacaaata gattaaggaa ttgtcaggta atggcagaag ggatcctagc 12360

tgaccagatt gcacctttct tccagggaaa tggggtcatt caggatagca tatctttgac 12420

taaaagtatg ctagcgatga gtcaactgtc tttcaacagc aacaagaaac gtattactga 12480

ctgcaaagaa agagtatcct caaaccgcaa tcatgatccg aagagcaaga atcgtcggag 12540

agttgccact tttgtaacta ccgacctgca aaagtattgt ctcaattgga gatatcagac 12600

agtcaagctg ttcgctcatg ccatcaatca gctgatgggc ttacctcact tcttcgagtg 12660

gattcatctt agactaatgg atactacgat gtttgtaggg gaccctttca atcctccaag 12720

tgaccctacg gactgtgatc tatcaagagt cccaaatgat gacatatata ttgtcagtgc 12780

taggggaggc attgagggat tatgccagaa gctatggaca atgatctcaa ttgctgcaat 12840

ccagcttgct gcagcaagat cacattgtcg cgttgcctgt atggtacaag gcgacaatca 12900

agtaatagct gtaacgagag aggtaagatc agatgactcc ccagagatgg tgttaacaca 12960

attgcatcaa gccagtgata atttcttcaa ggaattgatt catgtcaatc atttgatcgg 13020

ccataatttg aaggatcgtg aaaccatcag gtcagacacg ttcttcatat acagcaaacg 13080

aatattcaaa gatggagcaa tactcagtca ggtcctcaaa aactcatcta aattagtgct 13140

aatatcaggc gaccttagtg aaaacactgt aatgtcttgt gccaacattg catctactgt 13200

agcacggtta tgcgagaacg ggcttcctaa ggatttctgt tattacttaa actacttaat 13260

gagttgcgtg cagacatact tcgattccga attttccatc accaacaact cgcaactcga 13320

ttctaaccag tcgtggatag aggacatttc ttttgtgcac tcatatgtcc tgacccctgc 13380

tcaactgggg ggactgagta accttcaata ctcaaggctc tacacaagga acatcggcga 13440

cccgggaacc actgctttcg cagagatcaa gagattagag gcagtggggt tactgagtcc 13500

tagcattatg actaacatct taactaggcc gcctggaaat ggagattggg ccagtctgtg 13560

caacgatcca tactccttta attttgagac tgtcgcaagc ccaaatattg tccttaagaa 13620

acatacacaa agagtcctat ttgaaacttg ctcgaatccc ttattatctg gagtgcacac 13680

agaggataat gaggcagaag agaaggcatt ggctgaattc ttgctcaatc aagaaatgat 13740

tcatccacgt gtcgcgcatg ctatcatgga agcaagctct gtaggtagga gaaagcaaat 13800

tcaggggctt gttgacacaa caaacaccgt gattaagatt gcactgacta ggaggccact 13860

cggcatcaag aggctgatgc ggatagtcaa ttactcgagc atgcatgcaa tgctatttag 13920

agatgatgtt ttctcgtcta atagatccaa ccatccctta gtctcttctg atatgtgttc 13980

tcagacactg gcagactatg cacggaacag aagctggtca cctttgacag ggggtagaaa 14040

aatactgggt gtatctaatc ctgataccat agaacttgta gagggtgaga tccttagtgt 14100

cagtggaggg tgcactagat gtgacagtgg ggatgaacag tttacttggt tccatcttcc 14160

aagcaatata gagctaaacg atgacaccag caagaatcct ccaatgagag tgccatatct 14220

cgggtcaaag actcaagaga ggagagccgc ctcgcttgcg aaaatagctc atatgtcacc 14280

acacgtgaag gcggctctaa gggcgtcatc tgtgttaatc tgggcttatg gggacaacga 14340

aataaactgg actgctgccc ttaagattgc aaggtctcgg tgcaacataa gctcagagta 14400

tcttcgacta ttgtcaccct tgcctacagc tgggaatctc caacatagat tggacgacgg 14460

cataactcag atgacattca cccctgcatc tctatacagg gtgtcacctt acattcatat 14520

atctaatgat tctcaaaggc tatttactga agaaggagtc aaagagggga atgtggttta 14580

tcagcaaatc atgctcttgg gtttgtcctt aattgagtca ctcttcccaa tgacaacaac 14640

caagacatat gatgaaatca cattgcacct ccacagtaaa tttagctgct gtatcaggga 14700

agcacctgtt gcagttcctt tcgagctact cggggtggca ccggaactaa gggcagtaac 14760

ctcaaataag tttatgtatg atcctagccc tgtatcagag ggagactttg cgagacttga 14820

cttagctatc tttaagagtt atgaacttaa tttagagtca tattccacaa tagagctaat 14880

gaacgttctt tcaatatcta gtgggaagtt gattggccag tctgtggttt cttatgatga 14940

agatacctcc ataaagaatg acgctataat agtgtatgac aacacacgga attggatcag 15000

cgaagctcag aattcagatg tggtccgcct attcgagtat gcggcactcg aagtgctcct 15060

cgactgttct tatcaactct actatctgag agtaagaggc ctaaacaata tcgtcctgta 15120

catgagtgat ttatacaaga atatgccagg aattctactc tctaatattg cagccacaat 15180

atctcaccct gtcatccatt caaggttgaa tgcagtaggt ctggtcaacc atgacgggtc 15240

acaccaactt gcagacacag atttcattga aatgtctgca aagctgctag tcttttgcac 15300

tcgacgcgtg gtctcaggtt tacatgcagg gaataagtat gacctgctgt ttccatctgt 15360

cttggatgat aacctaagtg agaagatgct tcagttgata tcccaattat gctgtctgta 15420

tacggtgctc tttgctacaa caagagaaat cccgaaaata agaggcttat ctgcagaaga 15480

gaaatgttca gtacttactg agtacctact gtcagatgct gtgaaaccgt tacttgggtc 15540

cgagcaagtg agctctatca tgtctcccaa catagttacg ttcccagcca atctgtatta 15600

catgtctagg aagagcctta atttgatcag ggagagagag gacagggata ctatcttggc 15660

attgttgttc cctcaagaac cgctgctcga gtttcctctg gtacgagata ttggtgctcg 15720

tgtaaaagat ccatttaccc gacaacctgc ggcgttttta caagagttag atttgagtgc 15780

tccggcaagg tatgacgcat tcacaatcag tcaggcgcat tctgaacaca tattgccaaa 15840

cccagaggaa gatcacttag tacgatactt gttcagagga atagggactg cgtcctcctc 15900

ttggtataag gcatcccatc ttctttctgt acccgaggtc agatgtgcaa ggcatgggaa 15960

ctccttatat ttagcagaag gaagtggagc catcatgagt cttctcgaat tgcatatacc 16020

acacgaaact atctattaca atacactttt ctcgaacgag atgaaccccc cacagcgaca 16080

tttcggacca actccaacac agtttctgaa ttcggttgtt tttaggaatt tacaggcgga 16140

agtaccatgc aaggatggat ttgtccagga gttccgtccg ttatggagag agaatacaga 16200

agaaagcgat ctgacctcag ataaagcagt gggatatatc acatctgtag tgccgtacag 16260

gtctgtatca ttgctgcatt gtgacattga aatccctcca ggatctaatc aaagcttact 16320

agatcagctg gctaccaatc tgtctctgat tgccatgcat tctgtgaagg agggcggggt 16380

cgtgattatc aaagtactgt atgcaatggg atattacttc catctactca tgaacttgtt 16440

tactccatgt tccacgaaag gatatattct ctctaatggc tatgcctgta gaggggatat 16500

ggagtgttac ctgatatttg tcatgggcta cctaggcggg cctacatttg tacatgaggt 16560

ggtgaggatg gcaaaaacgc tagtacagcg gcatggcaca cttttgtcta agtcagatga 16620

gattacactg actaggttat ttacttcaca gcaacagcgt gtaacagaca tcctatccag 16680

tcctttaccg agactaatga agtacttgag agagaatatt gatactgcac tgattgaagc 16740

cgggggacag cctgtccgtc cattctgtgc agagagttta gtgagcacac taacagacat 16800

gactcagaca acccagatca tcgccagcca cattgataca gtcattcgat ctgtgatcta 16860

tatggaagca gagggtgatc ttgctgacac agtattctta tttacccctt acaacctctc 16920

tatggacggg aaaaagagaa catcacttaa acagtgcaca agacagatct tagaggtcac 16980

aatactgggt ctcagagtca aaaatctcaa taaagtaggt gatgtaatca gcctagtact 17040

cagaggtatg atttctctgg aggaccttct cccactgaga acctacttga agtgtagtac 17100

ctgccctaag tatttgaagg ctgtcctagg tattaccaaa ctcaaagaaa tgttcacaga 17160

cgcctcttta ttatacttga ctcgtgctca acaaaaattc tacatgaaaa ctataggcaa 17220

tgcagtcaag ggatactata gtaactgtga ctcttaaagg caaccacata tcaataggcc 17280

ctctttctag ccgatcgtat tcttgttgac ttcattatac catattagaa aaaaattgaa 17340

ttccgaccct ttaagactcg tattcggatt caaataatta tctcagaaaa aaagtgcacg 17400

tagttgttct tgattatagt cccgtcattc accaagtctt tgtttggt 17448

<210> 6
<211> 17442
<212> DNA
<213> Newcastle disease virus

<220>
<223> Mut HN (F277L)/M(G165W)-Lirilumab

<400> 6
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gccccccacc caggccgcag gcgaagccgg tgacacacag ctcaagactg 2220
gagcaagcaa ctccctcctg tcgatgctcg acaagcttag caataaatcg tccaatgcta 2280
aaaagggccc acggtcgagt ccccaggaag ggcaccacca acctccgacc cagcagcaag 2340
ggagtcaaca gagccgcgga aacagtcagg agagatcgca aaaccaggtc aaggccgcct 2400
ctggagacca gggcacagac gcgaacacag catatcatgg acaatggaag gagtcacaac 2460
catcagctgg tgcaatccct catgctctcc ggtcagggca gagccaagcc aatactcctg 2520
catctgtgga tcatgtccag ctacctgtcg actttgtgca ggcgatgatg tctatgatgg 2580
aggcgatatc acagaaggta aataaagtcg actatcagct agaccttgtc tcaaagcaga 2640
catcctccat ccccatgatg cgatctgaaa tccaacagct gaaaacatcc gttgcggtca 2700
tggaagccaa cctgggcatg atgaagattc tggaccctgg ttgtgctaac gtttcatctt 2760
taagtgacct acgggcggtc gcccgctctc acccagtttt agtttcaggc cccggagacc 2820
catctcctta tgtgacacaa gggggtgaat tgatactcaa taaactctcg caaccagtgc 2880
aacatccttc tgagttgatt aaacctgcca cgataagcgg gcctgatata ggagtggaga 2940
aggacactgt ccgtgcattg atcacctcac gcccaatgca tccgagctct tcagctaagc 3000
tcctgagtaa gctggatgca gccgggtcga tagaagaaat taggaaaatc aaacgccttg 3060
cgctgaatgg ctgatcacta ctacaacccg caacgggttc ccgtttatct attgtcacaa 3120
ggaacccgcc ccgaagctta agaaaaaata cgggtagaag cagtttgaga gctccgttcg 3180
ccaccatgga gttcggcctg tcctgggtgt tcctggtggc cctgttccgc ggcgtgcagt 3240
gccaagtgca actggtgcag agcggtgcag aggtgaagaa gccaggatcc tctgtgaaag 3300
tctcatgcaa agccagtgga ggtacgttca gcttttacgc aatcagctgg gtgagacagg 3360
ctccgggcca aggtctggaa tggatgggcg gattcattcc aattttcggc gcagcaaact 3420
acgcacagaa gttccagggg cgggtcacga ttactgctga tgagtcgaca agcacagcat 3480
acatggagct cagttctttg agaagcgatg atacagcagt ttattactgt gctagaattc 3540
caagcggttc ttattactat gactacgata tggatgtctg gggacaggga acaacagtca 3600
ccgtatcttc cgcgagcacc aagggacctt ccgtgtttcc cctggcccct tgctccagat 3660
caacctcaga gtcgactgct gctctcggtt gcttggtgaa agattatttt ccagaacccg 3720
ttaccgtgtc ttggaactcc ggtgccctta cttcaggggt tcataccttc cccgcagtcc 3780
tacagtcatc tggtctgtat tctctcagct ccgtggtcac cgtcccaagc agctcactgg 3840
gcaccaagac ctatacctgc aacgtcgacc ataagcctag taatacaaag gtcgacaagc 3900

gagtcgaatc caaatacgga cctccttgtc caccttgccc cgctcctgaa ttcctgggtg 3960

gaccaagcgt ctttctgttt ccaccaaaac ctaaagacac acttatgatt tcccgcacac 4020

ccgaagtcac ctgcgtggtg gtggatgtca gccaggaaga ccctgaggtg caattcaact 4080

ggtacgttga tggcgtagaa gtccacaacg ccaagacaaa acccagagag gagcagttta 4140

atagtaccta tagggtggtg tccgtcctga ccgttctcca ccaggattgg ttgaatggca 4200

aggaatacaa gtgtaaagtg agcaacaaag gcctaccttc gagcatagaa aagactatct 4260

ccaaggcaaa gggacagcca agggagcccc aggtgtacac gctgccaccc tctcaggagg 4320

agatgactaa gaaccaggtg tccttgacct gcctcgtaaa ggggttttac ccctccgaca 4380

tagccgtcga atgggagagt aacggacaac ccgagaacaa ttataaaact acacctcccg 4440

tgctcgactc tgacggatcc ttcttcctgt actctaggct gacagtggat aaatcccgtt 4500

ggcaggaagg aaatgtattt tcctgtagtg tgatgcacga ggctctgcac aatcattata 4560

cacagaaatc actgtcctta agcctcggca aacgccgcaa gcgcggctcc ggcgagggcc 4620

gcggctccct gctgacctgc ggcgacgtgg aggagaaccc cggccccatg aagtacctgc 4680

tgcccaccgc cgccgccggc ctgctgctgc tggccgccca gcccgccatg gccgaaatcg 4740

tcctcacaca gagcccggtt acgctttcat tgtctccagg cgagcgcgct accctcagct 4800

gtagggcgtc tcagtccgtt tccagctatt tagcttggta ccagcaaaaa cctggccagg 4860

cccctagact gcttatttac gacgcctcta accgagcgac tggtataccc gcaaggttca 4920

gcggctcggg ctcaggcacc gactttactc tcaccatttc atcactggag cctgaggact 4980

tcgccgtgta ttactgtcag cagaggagta actggatgta caccttcggc caaggaccca 5040

aattggaaat caaaaggaca gtggccgcgc caagcgtgtt tatatttccc ccctccgacg 5100

aacagttaaa gagtggaacc gcatctgtgg tatgtctgct taacaatttt taccctcgcg 5160

aggcaaaggt tcaatggaaa gtcgacaacg ccttacagtc tggtaacagt caagagtctg 5220

ttacggagca agacagcaag gactccacct attcgctgtc ttcaacacta accctcagca 5280

aggctgatta cgagaagcac aaagtttacg cctgtgaggt cacccatcag ggcctttcta 5340

gccctgtcac aaagtcgttc aacaggggag agtgctaatg agctgaccga gccctcctct 5400

ataaacccaa gattcaaggc tccaagcgat aaccttctcc cgcctcttcc atcccactga 5460

atgatcgcgc agctgcaatt aatctagcaa cattaaagat taagaaaaaa tacgggtaga 5520

atcggagtgc accgattgtg ccaagatgga ctcatctagg acaattgggt tatactttga 5580

ttctgccctt ccttctagca acctgttagc attcccgatt gtcttacgag atgtaggaga 5640

tggaaagaag caaattactc cgcaatatag gatccggcgt cttgactcgt ggacagacag 5700

taaagaagac tcggtattca tcactaccta cggattcatc ttccaggttg ggaatgaaga 5760

agtcactgtc ggcatgatca atgataatcc caagcgagag ttactctccg ctgcgatgct 5820

ctgcctagga agcgtcccga atgttggaga tcctgttgag ctggcaaggg cctgcctcac 5880

tatggtggta acatgcaaga agagtgcaac taatactgag agaatggtct tctcagtagt 5940

gcaggcaccc cgggtgctgc aaagctgtag ggtcgtggca gacaaatact cgtcagtgaa 6000

tgcagttaag cacgtgaaag caccagagaa gatcccttgg agtgaaaccc tagagtacaa 6060

ggtgaatttt gtctctttga ctgtggtgcc gaagaaggat gtctacaaga tcccaaccgc 6120

agtattgaag gtatccggct cgagcctgta taatcttgcg ctcaatgtca ctattgatgt 6180

ggaggtagac ccaaagagcc cgttagtcaa gtccctttct aggtccgaca gtggatacta 6240

tgctaatctc ttcttacata tcggacttat gtccactgta gataagaggg gaaagaaagt 6300

gacatttgac cagctggaga ggaagataag gagacttgat ctatctgtcg ggctcagtga 6360

tgtgctcgga ccttctgtgc ttgtgaaggc gagaggtgca cggactaggc tgctggcacc 6420

tttcttctct aacagcggga cagcctgcta ccctatagca aatgcctctc ctcaggtagc 6480

taagatactc tggagtcaga ccgcgtgcct gcggagtgta aaaatcatta ttcaagcggg 6540

cacccaacgc gctgtcgcag tgactgctga ccatgaggtt acctctacta agatagagaa 6600

gaggcatacc atcgctaaat acaatccctt caagaaatag gccgtatccc tgagactgca 6660

attcacccgc cttcccaaaa caccatgaca ccagataatg atctgtcttg attacttaca 6720

gttagtttcc ctgtctatca aattagaaaa aacacgggta gaagagttcg gatcccggcc 6780

ggcgcaccaa aagcgcaaga tgggccccag atcttctacc aggatcccag tacctctaat 6840

gctgaccata cggatcacgc tggcactgag ttatgtccgt ctgacaagtt ctcttgatgg 6900

caggcctctt gcagccgcag ggatcgtggt aacaggggat aaagcagtta acatatacac 6960

ctcatcccag acagggtcaa tcatagtcaa gttactccca aatatgccca aggacaaaga 7020

ggcatgtgca aaagccccat tggaggctta caacaggaca ctgactactt tgcttacccc 7080

ccttggtgat tctatccgca ggatacaaga gtctgtgact acatccggag gaaggagaca 7140

gagacgcttt ataggtgcca ttattggcag tgtagctcta ggggttgcaa cagctgcaca 7200

gataacggca gcctcggctc tgatacaagc caaccagaat gctgctaaca tcctccggct 7260

taaggagagc attgctgcaa ccaatgaagc tgtacacgag gtcactggcg gattgtcaca 7320

gttagcagtg gcagttggaa agatgcagca atttgttaat gaccagttta ataacacagc 7380

tcaagagttg gactgtataa aaattacaca gcaggttggt gtagaactca acttatactt 7440

aactgaattg actacagtgt tcgggccaca aatcacttcc cctgccttaa ctcagttgac 7500

tgttcaggct ctttacaatc tggctggtgg taatgtagat tacttgttga ctaagttagg 7560

tgtagggaac aaccagctca gctcattgat tggtagcggc ttgatcaccg gtaaccctat 7620

tttttacgac tcacagactc aactcttggg catacaggtg actttaccct cagtcgggaa 7680

cctaaataat atgcgtgcca cctacttgga gaccttgtct gtaagcacaa caaagggatt 7740

tgcctcagca cttgtcccaa aagtagtgac acaggtcggt tctgtgatag aagagcttga 7800

cacctcatac tgtatagaag ctgatttgga tttatattgt acaagaatag tgacattccc 7860

tatgtcccct ggtatttatt cctgtttgag cggcaataca tcggcttgca tgtattcaag 7920

gactgaaggc gcacttacta caccatacat gactctcaaa ggctcagttg ttgccaattg 7980

ccagatgaca acatgtagat gtgcagaccc cccgggtatc atatcacaaa attatggaga 8040

ggctgtgtct ctaatagata agcactcatg caatgtcgta tccttagacg ggataacttt 8100

gaggctcagt ggggaatttg atgcaactta tcaaaagaat atctcaatat tagattctca 8160

agtactagtg acaggcaatc tcgatatctc aactgagctt gggaatgtca accactcaat 8220

aagtaatgct ttggataagt tagaggaaag caacagcaaa ctagacaaag tcaatgtcaa 8280

actgaccagc acatctgctc tcattaccta tattgtttta actgtcatat ctcttgttct 8340

tggtatgctt agcctggttc tagcatgcta tctgatgtac aagcaaaaag cgcaacgaaa 8400

gaccttgttg tggcttggaa ataataccct agatcagatg agagctacta caaaaatgtg 8460

aatgcagatg agaggcagag gtatccccaa tagcaatctg tgtgtcaatt ctggcagcct 8520

gttaatcaga agaattaaga aaaaactacc ggatgtaggt gaacaaaagg gaatatacgg 8580

gtagaacggc ctgagaggcc acccctcaat cgggagccag gccccactac gtccgctcta 8640

ccgcatcacc aacagcagtc ttcagtcatg gacagcgcag ttagccaagt tgcgctagag 8700

aatgatagaa gagaagcgaa taatacatgg cgcttggttt tccggatcgc agccttactt 8760

ttactggtaa taaccttagc cgtctctgca gtcgccctgg catatagtat ggaggctagc 8820

acacctggcg accttgtaag cataccgact gcgatctcta gggcagagga aaggattaca 8880

tctgcactcg gttccaatca agatgtagta gataggatat acaagcaagt ggcccttgag 8940

tctccattgg cactgctaaa taccgaatct ataattatga atgcaataac gtctctctct 9000

tatcaaatca atggagccac aaataatagc gggtgtgggg cacctgttca tgacccagat 9060

tacatcgggg ggataggtca agaacttatt gtagatgata cgagtgacgt cacatcattt 9120

tatccctctg cattccaaga acacctgaat tttatcccgg cgcccactac aggatcaggc 9180

tgcactcgga taccctcatt cgacatgagt gctacccatt actgttacac tcacaatgtg 9240

atattgtctg gctgcagaga tcactcacac tcacatcagt atttagcact tggtgtgctt 9300

cggacatctg caacagggag ggtattcttt tctactctgc gttccatcaa cttggatgac 9360

gcccaaaatc ggaagtcttg cagtgtgagt gcaactcctt taggttgtga tatgctgtgc 9420

tctaaaatca cagagactga ggaagaggat tataaatcag ttatccccac atcgatggta 9480

catggaaggt tagggcttga cggccaatac catgagaagg acctagacgt cacaacacta 9540

tttagggact gggtggcaaa ttacccagga gtaggaggtg ggtcttttat taacaatcgc 9600
gtatggttcc cggtctacgg agggctaaaa cccagctcgc ctagtgacac tgcacaagaa 9660
gggagatacg taatatacaa gcgatacaat gacacatgcc cggatgagca agattaccag 9720
attcggatgg ctaagtcatc gtataagcct aggcggtttg gtggaaaacg cgtacagcag 9780
gccatcctat ccatcaaggt gtcaacatcc ttgggtgagg acccggtgtt gactgtaccg 9840
cctaatacgg tcgcactcat gggggccgaa ggcagagttc tcacagtggg gacatctcat 9900
ttcttatatc agcgagggtc atcatacttc tctcccgctt tgttataccc tatgacagtc 9960
aacaacaaaa cagccactct tcataatcct tatacattca atgctttcac tcggccaggt 10020
agtgtccctt gccaggcttc agcaagatgc cccaactcat gtgttaccgg agtctatact 10080
gatccatatc ccttagtctt ccataggaac cacaccttgc gaggggtatt cggaacaatg 10140
cttgatgacg aacaagcaag actcaaccct gtatctgcag tatttgataa catatcccgc 10200
agtcgcataa ctcgggtaag ttcaagcagt accagggcag catacacgac atcaacgtgt 10260
tttaaagttg tcaagaccaa taaaacctat tgcctcagca ttgcagaaat atccaatacc 10320
ctcttcgggg aattcagaat tgtcccctta ctagttgaga ttctcaagga tggtggggtt 10380
taagaagcta ggaatggtcg gttgagtcaa ccgtgagaag gcggggaaga tgatattgca 10440
tcatctatct tctgtaacac caagaatcaa atcgaatacc agtgcgagct cgaatcctac 10500
gctgccagtc agccataatc ggctggtgct aatgtggtta gcctgaatct tgtcgatagt 10560
cacttgatta agaaaaaatg tggacggtag tgaaatataa ggcaaagcag ctcacagagg 10620
acagcacggg taggacatgg cgggctccgg acccgaaagg gcagagcatc agattatcct 10680
accagagtca catctgtcct caccattggt caagcataaa ttgctttatt actggaaatt 10740
aactgggcta ccgcttcctg acgaatgtga cttcgaccat cttattatca gccgacaatg 10800
gaagaaggta cttgaatcgg ccacccctga cattgagaga atgataaaac tagggcgggc 10860
agtacaccag actctcaacc acaattccag gataaccgga gtactccatc cccggtgttt 10920
agaggaactg gctagtattg aggttcctga ttcaaccaac aaatttcgga agatcgaaaa 10980
aaagatccag attcacaaca caaggtatgg agaactgttc acaagattat gcacgcatgt 11040
agaaaagaaa ctattggggt catcttggtc taacaatgtc ccacggtcag aggaattcaa 11100
cagcatccgt acagatccgg cattttggtt tcactcaaaa tggtccacag ccaaatttgc 11160
atggctccat ataaaacagg tccagaggca tctgattgta gcagcaagaa caaggtccgc 11220
agtcaacaaa ttagtgacgc tgacccataa ggtaggccaa atctttgtta ctcctgagct 11280
tgtcattgtg acacatacag atgagaacaa gttcacgtgt cttacccagg aacttgtgtt 11340
gatgtatgca gatatgatgg agggcagaga tatggtcagc ataatatcat ccacggcggc 11400
acatcttagg agcttatcag agaaaattga tgatattctg cggttagtag atgctctggc 11460

aagagatttg ggcaatcaag tctacgatgt tgtagcacta atggagggat tcgcatacgg 11520

cgctgttcag ctgcttgaac cgtcaggtac atttgcgggg gatttcttcg cattcaacct 11580

gcaggagctc aaagatactc taaccggact cctccccaag gatatcgcag aatctgtgac 11640

tcacgcaatc gcaaccatat tctctggctt agaacaaaat caagcagctg agatgttgtg 11700

cctgttgcgt ctgtggggtc acccactact cgagtcccgt attgcagcaa aagcagttag 11760

gagtcaaatg tgcgcaccaa aaatggtaga ctttgatatg atcctccagg tattatcttt 11820

ctttaaagga acaatcatca atggatatag aaagaagaat gcaggcgtgt ggccacgtgt 11880

caagatagat acgatatacg ggaaggtcat agggcagcta cacgcagatt ctgcagagat 11940

ttcacatgat gtcatgttga gggaatacaa gagtttatct gcacttgaat tcgagccatg 12000

tatagagtat gaccctgtca ccaatctgag catgttttta aaagacaagg caatcgcaca 12060

cccgaaagac aactggctcg cttcgtttag gcgaaacctt ctctctgagg accagaagaa 12120

acatgtaaga gaggcaacct caactaaccg cctcttgata gagttcttag agtcaaatga 12180

ttttgatcca tataaagaga tggaatatct gacgaccctt gagtacctaa gagatgataa 12240

tgtggcagta tcatactcac tcaaagaaaa agaggtgaaa gttaatgggc ggattttcgc 12300

aaagctaaca aatagattaa ggaattgtca ggtaatggca gaagggatcc tagctgacca 12360

gattgcacct ttcttccagg gaaatggggt cattcaggat agcatatctt tgactaaaag 12420

tatgctagcg atgagtcaac tgtctttcaa cagcaacaag aaacgtatta ctgactgcaa 12480

agaaagagta tcctcaaacc gcaatcatga tccgaagagc aagaatcgtc ggagagttgc 12540

cacttttgta actaccgacc tgcaaaagta ttgtctcaat tggagatatc agacagtcaa 12600

gctgttcgct catgccatca atcagctgat gggcttacct cacttcttcg agtggattca 12660

tcttagacta atggatacta cgatgtttgt aggggaccct ttcaatcctc caagtgaccc 12720

tacggactgt gatctatcaa gagtcccaaa tgatgacata tatattgtca gtgctagggg 12780

aggcattgag ggattatgcc agaagctatg gacaatgatc tcaattgctg caatccagct 12840

tgctgcagca agatcacatt gtcgcgttgc ctgtatggta caaggcgaca atcaagtaat 12900

agctgtaacg agagaggtaa gatcagatga ctccccagag atggtgttaa cacaattgca 12960

tcaagccagt gataatttct tcaaggaatt gattcatgtc aatcatttga tcggccataa 13020

tttgaaggat cgtgaaacca tcaggtcaga cacgttcttc atatacagca aacgaatatt 13080

caaagatgga gcaatactca gtcaggtcct caaaaactca tctaaattag tgctaatatc 13140

aggcgacctt agtgaaaaca ctgtaatgtc ttgtgccaac attgcatcta ctgtagcacg 13200

gttatgcgag aacgggcttc ctaaggattt ctgttattac ttaaactact taatgagttg 13260

cgtgcagaca tacttcgatt ccgaattttc catcaccaac aactcgcaac tcgattctaa 13320

ccagtcgtgg atagaggaca tttcttttgt gcactcatat gtcctgaccc ctgctcaact 13380
ggggggactg agtaaccttc aatactcaag gctctacaca aggaacatcg gcgacccggg 13440
aaccactgct ttcgcagaga tcaagagatt agaggcagtg gggttactga gtcctagcat 13500
tatgactaac atcttaacta ggccgcctgg aaatggagat tgggccagtc tgtgcaacga 13560
tccatactcc tttaattttg agactgtcgc aagcccaaat attgtcctta agaaacatac 13620
acaaagagtc ctatttgaaa cttgctcgaa tcccttatta tctggagtgc acacagagga 13680
taatgaggca gaagagaagg cattggctga attcttgctc aatcaagaaa tgattcatcc 13740
acgtgtcgcg catgctatca tggaagcaag ctctgtaggt aggagaaagc aaattcaggg 13800
gcttgttgac acaacaaaca ccgtgattaa gattgcactg actaggaggc cactcggcat 13860
caagaggctg atgcggatag tcaattactc gagcatgcat gcaatgctat ttagagatga 13920
tgttttctcg tctaatagat ccaaccatcc cttagtctct tctgatatgt gttctcagac 13980
actggcagac tatgcacgga acagaagctg gtcacctttg acagggggta gaaaaatact 14040
gggtgtatct aatcctgata ccatagaact tgtagagggt gagatcctta gtgtcagtgg 14100
agggtgcact agatgtgaca gtggggatga acagtttact tggttccatc ttccaagcaa 14160
tatagagcta aacgatgaca ccagcaagaa tcctccaatg agagtgccat atctcgggtc 14220
aaagactcaa gagaggagag ccgcctcgct tgcgaaaata gctcatatgt caccacacgt 14280
gaaggcggct ctaagggcgt catctgtgtt aatctgggct tatggggaca acgaaataaa 14340
ctggactgct gcccttaaga ttgcaaggtc tcggtgcaac ataagctcag agtatcttcg 14400
actattgtca cccttgccta cagctgggaa tctccaacat agattggacg acggcataac 14460
tcagatgaca ttcacccctg catctctata cagggtgtca ccttacattc atatatctaa 14520
tgattctcaa aggctattta ctgaagaagg agtcaaagag gggaatgtgg tttatcagca 14580
aatcatgctc ttgggtttgt ccttaattga gtcactcttc ccaatgacaa caaccaagac 14640
atatgatgaa atcacattgc acctccacag taaatttagc tgctgtatca gggaagcacc 14700
tgttgcagtt cctttcgagc tactcggggt ggcaccggaa ctaagggcag taacctcaaa 14760
taagtttatg tatgatccta gccctgtatc agagggagac tttgcgagac ttgacttagc 14820
tatctttaag agttatgaac ttaatttaga gtcatattcc acaatagagc taatgaacgt 14880
tctttcaata tctagtggga agttgattgg ccagtctgtg gtttcttatg atgaagatac 14940
ctccataaag aatgacgcta taatagtgta tgacaacaca cggaattgga tcagcgaagc 15000
tcagaattca gatgtggtcc gcctattcga gtatgcggca ctcgaagtgc tcctcgactg 15060
ttcttatcaa ctctactatc tgagagtaag aggcctaaac aatatcgtcc tgtacatgag 15120
tgatttatac aagaatatgc caggaattct actctctaat attgcagcca caatatctca 15180
ccctgtcatc cattcaaggt tgaatgcagt aggtctggtc aaccatgacg ggtcacacca 15240

acttgcagac acagatttca ttgaaatgtc tgcaaagctg ctagtctttt gcactcgacg 15300

cgtggtctca ggtttacatg cagggaataa gtatgacctg ctgtttccat ctgtcttgga 15360

tgataaccta agtgagaaga tgcttcagtt gatatcccaa ttatgctgtc tgtatacggt 15420

gctctttgct acaacaagag aaatcccgaa aataagaggc ttatctgcag aagagaaatg 15480

ttcagtactt actgagtacc tactgtcaga tgctgtgaaa ccgttacttg ggtccgagca 15540

agtgagctct atcatgtctc ccaacatagt tacgttccca gccaatctgt attacatgtc 15600

taggaagagc cttaatttga tcagggagag agaggacagg gatactatct tggcattgtt 15660

gttccctcaa gaaccgctgc tcgagtttcc tctggtacga gatattggtg ctcgtgtaaa 15720

agatccattt acccgacaac ctgcggcgtt tttacaagag ttagatttga gtgctccggc 15780

aaggtatgac gcattcacaa tcagtcaggc gcattctgaa cacatattgc caaacccaga 15840

ggaagatcac ttagtacgat acttgttcag aggaataggg actgcgtcct cctcttggta 15900

taaggcatcc catcttcttt ctgtacccga ggtcagatgt gcaaggcatg ggaactcctt 15960

atatttagca gaaggaagtg gagccatcat gagtcttctc gaattgcata taccacacga 16020

aactatctat tacaatacac ttttctcgaa cgagatgaac cccccacagc gacatttcgg 16080

accaactcca acacagtttc tgaattcggt tgtttttagg aatttacagg cggaagtacc 16140

atgcaaggat ggatttgtcc aggagttccg tccgttatgg agagagaata cagaagaaag 16200

cgatctgacc tcagataaag cagtgggata tatcacatct gtagtgccgt acaggtctgt 16260

atcattgctg cattgtgaca ttgaaatccc tccaggatct aatcaaagct tactagatca 16320

gctggctacc aatctgtctc tgattgccat gcattctgtg aaggagggcg gggtcgtgat 16380

tatcaaagta ctgtatgcaa tgggatatta cttccatcta ctcatgaact tgtttactcc 16440

atgttccacg aaaggatata ttctctctaa tggctatgcc tgtagagggg atatggagtg 16500

ttacctgata tttgtcatgg gctacctagg cgggcctaca tttgtacatg aggtggtgag 16560

gatggcaaaa acgctagtac agcggcatgg cacacttttg tctaagtcag atgagattac 16620

actgactagg ttatttactt cacagcaaca gcgtgtaaca gacatcctat ccagcccttt 16680

accgagacta atgaagtact tgagagagaa tattgatact gcactgattg aagccggggg 16740

acagcctgtc cgtccattct gtgcagagag tttagtgagc acactaacag acatgactca 16800

gacaacccag atcatcgcca gccacattga tacagtcatt cgatctgtga tctatatgga 16860

agcagagggt gatcttgctg acacagtatt cttatttacc ccttacaacc tctctatgga 16920

cgggaaaaag agaacatcac ttaaacagtg cacaagacag atcttagagg tcacaatact 16980

gggtctcaga gtcaaaaatc tcaataaagt aggtgatgta atcagcctag tactcagagg 17040

tatgatttct ctggaggacc ttctcccact gagaacctac ttgaagtgta gtacctgccc 17100

taagtatttg aaggctgtcc taggtattac caaactcaaa gaaatgttca cagacgcctc 17160

tttattatac ttgactcgtg ctcaacaaaa attctacatg aaaactatag gcaatgcagt 17220

caagggatac tatagtaact gtgactctta aaggcaacca catatcaata ggccctcttt 17280

ctagccgatc gtattcttgt tgacttcatt ataccatatt agaaaaaaat tgaattccga 17340

ccctttaaga ctcgtattcg gattcaaata attatctcag aaaaaaagtg cacgtagttg 17400

ttcttgatta tagtcccgtc attcaccaag tctttgtttg gt 17442

<210> 7
<211> 17430
<212> DNA
<213> Newcastle disease virus

<220>
<223> Mut HN (F277L)/M(G165W)-Relatlimab

<400> 7
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gcccccaccc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgttccgc ggcgtgcagt 3240

gccaggtgca actgcagcag tggggggccg gactcctgaa accatccgaa actctctcat 3300

tgacttgcgc cgtatacggc gggtcatttt ccgattacta ctggaattgg atccgtcagc 3360

ctccaggtaa aggacttgaa tggataggtg aaataaatca tcgtggaagt actaacagca 3420

atcctagtct gaaatcaaga gtaacgctga gtttggacac atctaagaac cagttttccc 3480

ttaaacttag aagtgttacc gctgctgaca cagctgtata ctactgtgct ttcggttact 3540

ccgattatga gtacaattgg ttcgaccctt ggggacaggg cacactagtg acagtgtcca 3600

gtgcttcaac caagggccca agcgtgtttc ccttggcccc ctgttcacgc tccactagcg 3660

aaagcacagc cgcgttaggc tgtctggtga aagactattt tccagagcca gtcacggtgt 3720

cttggaactc gggtgccctg acatcaggtg ttcatacttt ccccgctgtc ctccagtcca 3780

gtgggctgta ctcattatct tcggtggtga ctgtcccctc ctcatcgctg gggacgaaga 3840

cctatacctg taacgtggac cacaaaccaa gcaacactaa ggtggacaag cgcgtggaat 3900

ccaaatacgg gcccccatgc ccaccgtgcc ccgcacctga gttcctgggc ggtcctagcg 3960

tctttttgtt cccaccgaaa ccaaaggata ccctcatgat ttccaggacg ccggaggtca 4020

cctgcgtggt cgtggacgtg agtcaggagg accccgaagt gcaatttaac tggtatgtgg 4080

acggtgtgga agtccataat gcaaagacca agcctagaga ggaacaattt aatagcacgt 4140

accgggtggt tagtgtcctt accgtcttgc accaggattg gctgaacggg aaggagtata 4200

agtgcaaggt gtcaaacaag ggactgccct catcaatcga gaagacaata agtaaggcga 4260

agggccagcc acgggagcct caggtctaca cattgccgcc aagtcaagag gagatgacca 4320

aaaaccaggt gtctttgact tgcctcgtga agggttttta cccaagtgac atcgccgtag 4380

agtgggagtc taacggccag ccagagaata actataaaac gacacctcca gtgcttgatt 4440

ctgatggatc ttttttcttg tactcaaggt tgaccgtgga taagagcagg tggcaagaag 4500

gcaacgtttt tagttgttcc gttatgcacg aggccttgca taatcactat actcagaagt 4560

ccctatccct gtctctcggg cgccgcaagc gcggctccgg cgagggccgc ggctccctgc 4620

tgacctgcgg cgacgtggag gagaaccccg gccccatgaa gtacctgctg cccaccgccg 4680

ccgccggcct gctgctgctg gccgcccagc ccgccatggc cgaaattgtg ctgacacaat 4740

cgccagccac tctgtctctg agccctggtg agcgggccac tctgtcctgc agggccagtc 4800

agtctatttc cagctacttg gcttggtatc agcaaaaacc cggccaagct cctagattgc 4860

ttatttacga cgcgagcaat agggccaccg gtattccagc tcgctttagc ggctccggaa 4920

gtggtactga tttcacgctg acaatttcaa gcttggaacc cgaggatttt gcagtgtatt 4980

actgccagca gagaagcaat tggccgctga ccttcggcca ggggactaat cttgaaataa 5040
aacgcacggt ggctgccccc tccgtcttca tctttccacc ttctgacgag caattgaaga 5100
gcggtaccgc tagcgtagtg tgtcttctga acaatttcta cccacgcgag gccaaagtgc 5160
aatggaaagt cgacaatgct ctgcagtcag gaaactccca ggagagcgtc acagagcaag 5220
attctaaaga ttccacttac agtctgtcat cgacattgac actgagcaag gccgattacg 5280
aaaagcataa agtgtacgct tgtgaggtca ctcaccaagg actgagctca ccagttacca 5340
aatcgtttaa tagaggtgag tgttaatgag ctgaccgagc cctcctctat aaacccaaga 5400
ttcaaggctc caagcgataa ccttctcccg cctcttccat cccactgaat gatcgcgcag 5460
ctgcaattaa tctagcaaca ttaaagatta agaaaaaata cgggtagaat cggagtgcac 5520
cgattgtgcc aagatggact catctaggac aattgggtta tactttgatt ctgcccttcc 5580
ttctagcaac ctgttagcat tcccgattgt cttacgagat gtaggagatg gaaagaagca 5640
aattactccg caatatagga tccggcgtct tgactcgtgg acagacagta aagaagactc 5700
ggtattcatc actacctacg gattcatctt ccaggttggg aatgaagaag tcactgtcgg 5760
catgatcaat gataatccca agcgagagtt actctccgct gcgatgctct gcctaggaag 5820
cgtcccgaat gttggagatc ctgttgagct ggcaagggcc tgcctcacta tggtggtaac 5880
atgcaagaag agtgcaacta atactgagag aatggtcttc tcagtagtgc aggcaccccg 5940
ggtgctgcaa agctgtaggg tcgtggcaga caaatactcg tcagtgaatg cagttaagca 6000
cgtgaaagca ccagagaaga tcccttggag tgaaacccta gagtacaagg tgaattttgt 6060
ctctttgact gtggtgccga agaaggatgt ctacaagatc ccaaccgcag tattgaaggt 6120
atccggctcg agcctgtata atcttgcgct caatgtcact attgatgtgg aggtagaccc 6180
aaagagcccg ttagtcaagt ccctttctag gtccgacagt ggatactatg ctaatctctt 6240
cttacatatc ggacttatgt ccactgtaga taagagggga aagaaagtga catttgacca 6300
gctggagagg aagataagga gacttgatct atctgtcggg ctcagtgatg tgctcggacc 6360
ttctgtgctt gtgaaggcga gaggtgcacg gactaggctg ctggcacctt tcttctctaa 6420
cagcgggaca gcctgctacc ctatagcaaa tgcctctcct caggtagcta agatactctg 6480
gagtcagacc gcgtgcctgc ggagtgtaaa aatcattatt caagcgggca cccaacgcgc 6540
tgtcgcagtg actgctgacc atgaggttac ctctactaag atagagaaga ggcataccat 6600
cgctaaatac aatcccttca agaaataggc cgtatccctg agactgcaat tcacccgcct 6660
tcccaaaaca ccatgacacc agataatgat ctgtcttgat tacttacagt tagtttccct 6720
gtctatcaaa ttagaaaaaa cacgggtaga agagttcgga tcccggccgg cgcaccaaaa 6780
gcgcaagatg ggccccagat cttctaccag gatcccagta cctctaatgc tgaccatacg 6840

gatcacgctg gcactgagtt atgtccgtct gacaagttct cttgatggca ggcctcttgc 6900

agccgcaggg atcgtggtaa caggggataa agcagttaac atatacacct catcccagac 6960

agggtcaatc atagtcaagt tactcccaaa tatgcccaag gacaaagagg catgtgcaaa 7020

agccccattg gaggcttaca acaggacact gactactttg cttacccccc ttggtgattc 7080

tatccgcagg atacaagagt ctgtgactac atccggagga aggagacaga gacgctttat 7140

aggtgccatt attggcagtg tagctctagg ggttgcaaca gctgcacaga taacggcagc 7200

ctcggctctg atacaagcca accagaatgc tgctaacatc ctccggctta aggagagcat 7260

tgctgcaacc aatgaagctg tacacgaggt cactggcgga ttgtcacagt tagcagtggc 7320

agttggaaag atgcagcaat ttgttaatga ccagtttaat aacacagctc aagagttgga 7380

ctgtataaaa attacacagc aggttggtgt agaactcaac ttatacttaa ctgaattgac 7440

tacagtgttc gggccacaaa tcacttcccc tgccttaact cagttgactg ttcaggctct 7500

ttacaatctg gctggtggta atgtagatta cttgttgact aagttaggtg tagggaacaa 7560

ccagctcagc tcattgattg gtagcggctt gatcaccggt aaccctattt tttacgactc 7620

acagactcaa ctcttgggca tacaggtgac tttaccctca gtcgggaacc taaataatat 7680

gcgtgccacc tacttggaga ccttgtctgt aagcacaaca aagggatttg cctcagcact 7740

tgtcccaaaa gtagtgacac aggtcggttc tgtgatagaa gagcttgaca cctcatactg 7800

tatagaagct gatttggatt tatattgtac aagaatagtg acattcccta tgtcccctgg 7860

tatttattcc tgtttgagcg gcaatacatc ggcttgcatg tattcaagga ctgaaggcgc 7920

acttactaca ccatacatga ctctcaaagg ctcagttgtt gccaattgcc agatgacaac 7980

atgtagatgt gcagaccccc cgggtatcat atcacaaaat tatggagagg ctgtgtctct 8040

aatagataag cactcatgca atgtcgtatc cttagacggg ataactttga ggctcagtgg 8100

ggaatttgat gcaacttatc aaaagaatat ctcaatatta gattctcaag tactagtgac 8160

aggcaatctc gatatctcaa ctgagcttgg gaatgtcaac cactcaataa gtaatgcttt 8220

ggataagtta gaggaaagca acagcaaact agacaaagtc aatgtcaaac tgaccagcac 8280

atctgctctc attacctata ttgttttaac tgtcatatct cttgttcttg gtatgcttag 8340

cctggttcta gcatgctatc tgatgtacaa gcaaaaagcg caacgaaaga ccttgttgtg 8400

gcttggaaat aataccctag atcagatgag agctactaca aaaatgtgaa tgcagatgag 8460

aggcagaggt atccccaata gcaatctgtg tgtcaattct ggcagcctgt taatcagaag 8520

aattaagaaa aaactaccgg atgtaggtga acaaaaggga atatacgggt agaacggcct 8580

gagaggccac ccctcaatcg ggagccaggc cccactacgt ccgctctacc gcatcaccaa 8640

cagcagtctt cagtcatgga cagcgcagtt agccaagttg cgctagagaa tgatagaaga 8700

gaagcgaata atacatggcg cttggttttc cggatcgcag ccttactttt actggtaata 8760

accttagccg tctctgcagt cgccctggca tatagtatgg aggctagcac acctggcgac 8820
cttgtaagca taccgactgc gatctctagg gcagaggaaa ggattacatc tgcactcggt 8880
tccaatcaag atgtagtaga taggatatac aagcaagtgg cccttgagtc tccattggca 8940
ctgctaaata ccgaatctat aattatgaat gcaataacgt ctctctctta tcaaatcaat 9000
ggagccacaa ataatagcgg gtgtggggca cctgttcatg acccagatta catcgggggg 9060
ataggtcaag aacttattgt agatgatacg agtgacgtca catcatttta tccctctgca 9120
ttccaagaac acctgaattt tatcccggcg cccactacag gatcaggctg cactcggata 9180
ccctcattcg acatgagtgc tacccattac tgttacactc acaatgtgat attgtctggc 9240
tgcagagatc actcacactc acatcagtat ttagcacttg gtgtgcttcg gacatctgca 9300
acagggaggg tattcttttc tactctgcgt tccatcaact tggatgacgc ccaaaatcgg 9360
aagtcttgca gtgtgagtgc aactccttta ggttgtgata tgctgtgctc taaaatcaca 9420
gagactgagg aagaggatta taaatcagtt atccccacat cgatggtaca tggaaggtta 9480
gggcttgacg gccaatacca tgagaaggac ctagacgtca caacactatt tagggactgg 9540
gtggcaaatt acccaggagt aggaggtggg tcttttatta acaatcgcgt atggttcccg 9600
gtctacggag ggctaaaacc cagctcgcct agtgacactg cacaagaagg gagatacgta 9660
atatacaagc gatacaatga cacatgcccg gatgagcaag attaccagat tcggatggct 9720
aagtcatcgt ataagcctag gcggtttggt ggaaaacgcg tacagcaggc catcctatcc 9780
atcaaggtgt caacatcctt gggtgaggac ccggtgttga ctgtaccgcc taatacggtc 9840
gcactcatgg gggccgaagg cagagttctc acagtgggga catctcattt cttatatcag 9900
cgagggtcat catacttctc tcccgctttg ttatacccta tgacagtcaa caacaaaaca 9960
gccactcttc ataatcctta tacattcaat gctttcactc ggccaggtag tgtcccttgc 10020
caggcttcag caagatgccc caactcatgt gttaccggag tctatactga tccatatccc 10080
ttagtcttcc ataggaacca caccttgcga ggggtattcg gaacaatgct tgatgacgaa 10140
caagcaagac tcaaccctgt atctgcagta tttgataaca tatcccgcag tcgcataact 10200
cgggtaagtt caagcagtac cagggcagca tacacgacat caacgtgttt taaagttgtc 10260
aagaccaata aaacctattg cctcagcatt gcagaaatat ccaataccct cttcggggaa 10320
ttcagaattg tccccttact agttgagatt ctcaaggatg gtggggttta agaagctagg 10380
aatggtcggt tgagtcaacc gtgagaaggc ggggaagatg atattgcatc atctatcttc 10440
tgtaacacca agaatcaaat cgaataccag tgcgagctcg aatcctacgc tgccagtcag 10500
ccataatcgg ctggtgctaa tgtggttagc ctgaatcttg tcgatagtca cttgattaag 10560
aaaaaatgtg gacggtagtg aaatataagg caaagcagct cacagaggac agcacgggta 10620

ggacatggcg ggctccggac ccgaaagggc agagcatcag attatcctac cagagtcaca 10680

tctgtcctca ccattggtca agcataaatt gctttattac tggaaattaa ctgggctacc 10740

gcttcctgac gaatgtgact tcgaccatct tattatcagc cgacaatgga agaaggtact 10800

tgaatcggcc acccctgaca ttgagagaat gataaaacta gggcgggcag tacaccagac 10860

tctcaaccac aattccagga taaccggagt actccatccc cggtgtttag aggaactggc 10920

tagtattgag gttcctgatt caaccaacaa atttcggaag atcgaaaaaa agatccagat 10980

tcacaacaca aggtatggag aactgttcac aagattatgc acgcatgtag aaaagaaact 11040

attggggtca tcttggtcta acaatgtccc acggtcagag gaattcaaca gcatccgtac 11100

agatccggca ttttggtttc actcaaaatg gtccacagcc aaatttgcat ggctccatat 11160

aaaacaggtc cagaggcatc tgattgtagc agcaagaaca aggtccgcag tcaacaaatt 11220

agtgacgctg acccataagg taggccaaat ctttgttact cctgagcttg tcattgtgac 11280

acatacagat gagaacaagt tcacgtgtct tacccaggaa cttgtgttga tgtatgcaga 11340

tatgatggag ggcagagata tggtcagcat aatatcatcc acggcggcac atcttaggag 11400

cttatcagag aaaattgatg atattctgcg gttagtagat gctctggcaa gagatttggg 11460

caatcaagtc tacgatgttg tagcactaat ggagggattc gcatacggcg ctgttcagct 11520

gcttgaaccg tcaggtacat ttgcggggga tttcttcgca ttcaacctgc aggagctcaa 11580

agatactcta accggactcc tccccaagga tatcgcagaa tctgtgactc acgcaatcgc 11640

aaccatattc tctggcttag aacaaaatca agcagctgag atgttgtgcc tgttgcgtct 11700

gtggggtcac ccactactcg agtcccgtat tgcagcaaaa gcagttagga gtcaaatgtg 11760

cgcaccaaaa atggtagact ttgatatgat cctccaggta ttatctttct ttaaaggaac 11820

aatcatcaat ggatatagaa agaagaatgc aggcgtgtgg ccacgtgtca agatagatac 11880

gatatacggg aaggtcatag ggcagctaca cgcagattct gcagagattt cacatgatgt 11940

catgttgagg gaatacaaga gtttatctgc acttgaattc gagccatgta tagagtatga 12000

ccctgtcacc aatctgagca tgtttttaaa agacaaggca atcgcacacc cgaaagacaa 12060

ctggctcgct tcgtttaggc gaaaccttct ctctgaggac cagaagaaac atgtaagaga 12120

ggcaacctca actaaccgcc tcttgataga gttcttagag tcaaatgatt ttgatccata 12180

taaagagatg gaatatctga cgacccttga gtacctaaga gatgataatg tggcagtatc 12240

atactcactc aaagaaaaag aggtgaaagt taatgggcgg attttcgcaa agctaacaaa 12300

tagattaagg aattgtcagg taatggcaga agggatccta gctgaccaga ttgcaccttt 12360

cttccaggga aatggggtca ttcaggatag catatctttg actaaaagta tgctagcgat 12420

gagtcaactg tctttcaaca gcaacaagaa acgtattact gactgcaaag aaagagtatc 12480

ctcaaaccgc aatcatgatc cgaagagcaa gaatcgtcgg agagttgcca cttttgtaac 12540

taccgacctg caaaagtatt gtctcaattg gagatatcag acagtcaagc tgttcgctca 12600
tgccatcaat cagctgatgg gcttacctca cttcttcgag tggattcatc ttagactaat 12660
ggatactacg atgtttgtag ggaccctttt caatcctcca agtgacccta cggactgtga 12720
tctatcaaga gtcccaaatg atgacatata tattgtcagt gctaggggag gcattgaggg 12780
attatgccag aagctatgga caatgatctc aattgctgca atccagcttg ctgcagcaag 12840
atcacattgt cgcgttgcct gtatggtaca aggcgacaat caagtaatag ctgtaacgag 12900
agaggtaaga tcagatgact ccccagagat ggtgttaaca caattgcatc aagccagtga 12960
taatttcttc aaggaattga ttcatgtcaa tcatttgatc ggccataatt tgaaggatcg 13020
tgaaaccatc aggtcagaca cgttcttcat atacagcaaa cgaatattca aagatggagc 13080
aatactcagt caggtcctca aaaactcatc taaattagtg ctaatatcag gcgaccttag 13140
tgaaaacact gtaatgtctt gtgccaacat tgcatctact gtagcacggt tatgcgagaa 13200
cgggcttcct aaggatttct gttattactt aaactactta atgagttgcg tgcagacata 13260
cttcgattcc gaattttcca tcaccaacaa ctcgcaactc gattctaacc agtcgtggat 13320
agaggacatt tcttttgtgc actcatatgt cctgacccct gctcaactgg ggggactgag 13380
taaccttcaa tactcaaggc tctacacaag gaacatcggc gacccgggaa ccactgcttt 13440
cgcagagatc aagagattag aggcagtggg gttactgagt cctagcatta tgactaacat 13500
cttaactagg ccgcctggaa atggagattg ggccagtctg tgcaacgatc catactcctt 13560
taattttgag actgtcgcaa gcccaaatat tgtccttaag aaacatacac aaagagtcct 13620
atttgaaact tgctcgaatc ccttattatc tggagtgcac acagaggata atgaggcaga 13680
agagaaggca ttggctgaat tcttgctcaa tcaagaaatg attcatccac gtgtcgcgca 13740
tgctatcatg gaagcaagct ctgtaggtag gagaaagcaa attcaggggc ttgttgacac 13800
aacaaacacc gtgattaaga ttgcactgac taggaggcca ctcggcatca agaggctgat 13860
gcggatagtc aattactcga gcatgcatgc aatgctattt agagatgatg ttttctcgtc 13920
taatagatcc aaccatccct tagtctcttc tgatatgtgt tctcagacac tggcagacta 13980
tgcacggaac agaagctggt cacctttgac agggggtaga aaaatactgg gtgtatctaa 14040
tcctgatacc atagaacttg tagagggtga gatccttagt gtcagtggag ggtgcactag 14100
atgtgacagt ggggatgaac agtttacttg gttccatctt ccaagcaata tagagctaaa 14160
cgatgacacc agcaagaatc ctccaatgag agtgccatat ctcgggtcaa agactcaaga 14220
gaggagagcc gcctcgcttg cgaaaatagc tcatatgtca ccacacgtga aggcggctct 14280
aagggcgtca tctgtgttaa tctgggctta tggggacaac gaaataaact ggactgctgc 14340
ccttaagatt gcaaggtctc ggtgcaacat aagctcagag tatcttcgac tattgtcacc 14400

cttgcctaca gctgggaatc tccaacatag attggacgac ggcataactc agatgacatt 14460
cacccctgca tctctataca gggtgtcacc ttacattcat atatctaatg attctcaaag 14520
gctatttact gaagaaggag tcaaagaggg gaatgtggtt tatcagcaaa tcatgctctt 14580
gggtttgtcc ttaattgagt cactcttccc aatgacaaca accaagacat atgatgaaat 14640
cacattgcac ctccacagta aatttagctg ctgtatcagg gaagcacctg ttgcagttcc 14700
tttcgagcta ctcggggtgg caccggaact aagggcagta acctcaaata agtttatgta 14760
tgatcctagc cctgtatcag agggagactt tgcgagactt gacttagcta tctttaagag 14820
ttatgaactt aatttagagt catattccac aatagagcta atgaacgttc tttcaatatc 14880
tagtgggaag ttgattggcc agtctgtggt ttcttatgat gaagatacct ccataaagaa 14940
tgacgctata atagtgtatg acaacacacg gaattggatc agcgaagctc agaattcaga 15000
tgtggtccgc ctattcgagt atgcggcact cgaagtgctc ctcgactgtt cttatcaact 15060
ctactatctg agagtaagag gcctaaacaa tatcgtcctg tacatgagtg atttatacaa 15120
gaatatgcca ggaattctac tctctaatat tgcagccaca atatctcacc ctgtcatcca 15180
ttcaaggttg aatgcagtag gtctggtcaa ccatgacggg tcacaccaac ttgcagacac 15240
agatttcatt gaaatgtctg caaagctgct agtcttttgc actcgacgcg tggtctcagg 15300
tttacatgca gggaataagt atgacctgct gtttccatct gtcttggatg ataacctaag 15360
tgagaagatg cttcagttga tatcccaatt atgctgtctg tatacggtgc tctttgctac 15420
aacaagagaa atcccgaaaa taagaggctt atctgcagaa gagaaatgtt cagtacttac 15480
tgagtaccta ctgtcagatg ctgtgaaacc gttacttggg tccgagcaag tgagctctat 15540
catgtctccc aacatagtta cgttcccagc caatctgtat tacatgtcta ggaagagcct 15600
taatttgatc agggagagag aggacaggga tactatcttg gcattgttgt tccctcaaga 15660
accgctgctc gagtttcctc tggtacgaga tattggtgct cgtgtaaaag atccatttac 15720
ccgacaacct gcggcgtttt tacaagagtt agatttgagt gctccggcaa ggtatgacgc 15780
attcacaatc agtcaggcgc attctgaaca catattgcca aacccagagg aagatcactt 15840
agtacgatac ttgttcagag gaatagggac tgcgtcctcc tcttggtata aggcatccca 15900
tcttctttct gtacccgagg tcagatgtgc aaggcatggg aactccttat atttagcaga 15960
aggaagtgga gccatcatga gtcttctcga attgcatata ccacacgaaa ctatctatta 16020
caatacactt ttctcgaacg agatgaaccc cccacagcga catttcggac caactccaac 16080
acagtttctg aattcggttg tttttaggaa tttacaggcg gaagtaccat gcaaggatgg 16140
atttgtccag gagttccgtc cgttatggag agagaataca gaagaaagcg atctgacctc 16200
agataaagca gtgggatata tcacatctgt agtgccgtac aggtctgtat cattgctgca 16260
ttgtgacatt gaaatccctc caggatctaa tcaaagctta ctagatcagc tggctaccaa 16320

tctgtctctg attgccatgc attctgtgaa ggagggcggg gtcgtgatta tcaaagtact 16380

gtatgcaatg ggatattact tccatctact catgaacttg tttactccat gttccacgaa 16440

aggatatatt ctctctaatg gctatgcctg tagaggggat atggagtgtt acctgatatt 16500

tgtcatgggc tacctaggcg ggcctacatt tgtacatgag gtggtgagga tggcaaaaac 16560

gctagtacag cggcatggca cacttttgtc taagtcagat gagattacac tgactaggtt 16620

atttacttca cagcaacagc gtgtaacaga catcctatcc agccctttac cgagactaat 16680

gaagtacttg agagagaata ttgatactgc actgattgaa gccgggggac agcctgtccg 16740

tccattctgt gcagagagtt tagtgagcac actaacagac atgactcaga caacccagat 16800

catcgccagc cacattgata cagtcattcg atctgtgatc tatatggaag cagagggtga 16860

tcttgctgac acagtattct tatttacccc ttacaacctc tctatggacg ggaaaaagag 16920

aacatcactt aaacagtgca caagacagat cttagaggtc acaatactgg gtctcagagt 16980

caaaaatctc aataaagtag gtgatgtaat cagcctagta ctcagaggta tgatttctct 17040

ggaggacctt ctcccactga gaacctactt gaagtgtagt acctgcccta agtatttgaa 17100

ggctgtccta ggtattacca aactcaaaga aatgttcaca gacgcctctt tattatactt 17160

gactcgtgct caacaaaaat tctacatgaa aactataggc aatgcagtca agggatacta 17220

tagtaactgt gactcttaaa ggcaaccaca tatcaatagg ccctctttct agccgatcgt 17280

attcttgttg acttcattat accatattag aaaaaaattg aattccgacc ctttaagact 17340

cgtattcgga ttcaaataat tatctcagaa aaaaagtgca cgtagttgtt cttgattata 17400

gtcccgtcat tcaccaagtc tttgtttggt 17430

<210> 8
<211> 17448
<212> DNA
<213> Newcastle disease virus

<220>
<223> Mut HN (F277L)/M(G165W)-Monalizumab

<400> 8
   accaaacaga gaatccgtga gatacgataa aaggcgaagg agcaatcgaa gtcgtacggg 60
   tagaaggtgt gaatctcgag tgcgagaccg aagcttaaac tcgagagagc cttctgccaa 120
   catgtcttcc gtatttgacg agtatgaaca gctcctcgcg gctcagactc gccctaatgg 180
   agctcacgga ggaggagaga aggggagcac cttaaaagtt gaagtcccgg tattcactct 240
   taacagtgac gacccagaag atagatggaa ctttgcagta ttctgtctta ggattgctgt 300
   tagcgaagat gccaacaaac cactcagaca aggtgctctc atatctctct tatgctccca 360
   ttctcaagtg atgaggaacc atgtcgccct tgcagggaaa cagaatgagg ccacactggc 420
   tgttcttgag atcgatggtt tcaccaacag cgtgcctcaa tttaacaaca ggagtggagt 480
   gtctgaggag agagcacaga gattcttgat gatagcaggg tccctccctc gggcatgcag 540
   caacggtacc ccgttcatca cagctggggt tgaagatgat gcaccagaag acatcactga 600
   tactctggaa agaatcatct ctatccaggc tcaagtatgg gtcacggtag caaaggccat 660
   gactgcatat gagacagcgg atgagtcgga aacaagaaga atcaataagt atatgcagca 720
   aggcagagtc caaaagaagt acatcctcca ccccgtatgt aggagcgcaa ttcaactcac 780
   gattagacaa tctctggcag tccgcatttt cttggttagc gagcttaaga gaggccgcaa 840
   cacggcaggt ggaagttcca cttattatag cttggtaggg gacatagact catacatcag 900
   gaacaccggg cttactgcat tcttcctgac actcaggtat ggaattaaca ccaagacatc 960
   agcccttgca ctcagcagcc tcgcaggcga tatccaaaaa atgaagcagc tcatgcgtct 1020
   atatcggatg aaaggagata atgcgccgta catgacactg ctcggtgaca gtgaccagat 1080
   gagctttgca ccggctgagt atgcacaact ctactctttt gcaatgggta tggcatccgt 1140
   cctagacaaa ggaactggca agtaccagtt cgccagagac tttatgagca catcattctg 1200
   gagacttgga gtagagtatg ctcgggctca gggaagcagc atcaatgagg atatggctgc 1260
   cgagctaaag ctgaccccag cggcaaggag gggcctggca gctgctgccc aacgagcgtc 1320
   cgaggagacc ggcagcatgg acattcccac tcaacaagct ggggtcctca cagggctcag 1380
   cgacggaggc cctcaagccc cacaaggtgg actgaacagg tcgcaagggc aaccggacgc 1440
   cggagatggg gagacccaat ttttagattt gatgagagca gtggcaaata gtatgagaga 1500
   agcgccaaac tctgtgcaga acaccactca gcaggagcct ccatctactc ctgggccatc 1560
   tcaagacaac gacaccgact ggggatactg accgacaaca ctcagcctgc ctccatcgaa 1620
   ttatctcgat tcttctgcct gtaacctaac ccctggtcca cagtctcaca cggccaaacc 1680
   cgcaaacgag cccccccctc tctccccccc cctccagcca cacggctcca accgcccaaa 1740
   acaacacggg cacaactcga ctcactcata atctacacag agccgaagat cttagaaaaa 1800
   aatacgggta gaagagagac attcagagat caggacaaat catcagggtc tccgctctcc 1860
   cctctaccca gcagaccaga gtgaagatgg ccaccttcac ggatgcagag atcgacgatc 1920
   tatttgaaac cagtggaact gtcattgaca gcataattac ggcccaaggc aacccagtag 1980
   agactgttgg aaggagcgca atcccacaag gcaaaactaa agcattgagt gcagcatggg 2040
   agaaacatgg aagcacccaa ccgccggccg gccaagacac ccctgatcga atggacagat 2100
   cagacaaaca accatcccca cccgaacagg caaccacaca caacagcccg ccagccacat 2160
   ccaccgacca gcccccaccc caggccgcag gcgaagccgg tgacacacag ctcaagactg 2220
   gagcaagcaa ctccctcctg tcgatgctcg acaagcttag caataaatcg tccaatgcta 2280
   aaaagggccc acggtcgagt ccccaggaag ggcaccacca acctccgacc cagcagcaag 2340
   ggagtcaaca gagccgcgga aacagtcagg agagatcgca aaaccaggtc aaggccgcct 2400
   ctggagacca gggcacagac gcgaacacag catatcatgg acaatggaag gagtcacaac 2460
   catcagctgg tgcaatccct catgctctcc ggtcagggca gagccaagcc aatactcctg 2520
   catctgtgga tcatgtccag ctacctgtcg actttgtgca ggcgatgatg tctatgatgg 2580
   aggcgatatc acagaaggta aataaagtcg actatcagct agaccttgtc tcaaagcaga 2640
   catcctccat ccccatgatg cgatctgaaa tccaacagct gaaaacatcc gttgcggtca 2700
   tggaagccaa cctgggcatg atgaagattc tggaccctgg ttgtgctaac gtttcatctt 2760
   taagtgacct acgggcggtc gcccgctctc acccagtttt agtttcaggc cccggagacc 2820
   catctcctta tgtgacacaa gggggtgaat tgatactcaa taaactctcg caaccagtgc 2880
   aacatccttc tgagttgatt aaacctgcca cgataagcgg gcctgatata ggagtggaga 2940
   aggacactgt ccgtgcattg atcacctcac gcccaatgca tccgagctct tcagctaagc 3000
   tcctgagtaa gctggatgca gccgggtcga tagaagaaat taggaaaatc aaacgccttg 3060
   cgctgaatgg ctgatcacta ctacaacccg caacgggttc ccgtttatct attgtcacaa 3120
   ggaacccgcc ccgaagctta agaaaaaata cgggtagaag cagtttgaga gctccgttcg 3180
   ccaccatgga gttcggcctg tcctgggtgt tcctggtggc cctgttccgc ggcgtgcagt 3240
   gccaggttca gttggtacag tctggcgcgg aggtgaaaaa acctggagca tctgtcaagg 3300
   taagttgtaa agcttcgggc tataccttta cctcatattg gatgaactgg gtgcggcagg 3360
   cccccggtca agggcttgaa tggatgggca gaattgatcc ttatgattcc gaaacacatt 3420
   acgctcagaa gctgcagggc agggttacca tgacgacaga caccagcact tccactgctt 3480
   acatggagct gcgatctctg cggtcagatg acacggctgt gtactattgc gccagaggcg 3540
   gatacgattt tgacgtgggg actctgtatt ggttctttga cgtctggggg cagggaacca 3600
   ctgtgacagt ctcatcggcg tcaactaaag ggccaagcgt gttccccttg gcaccatgta 3660
   gtcggtccac cagtgaaagt accgctgcac tcggttgtct ggtaaaagat tattttccag 3720
   agcccgttac tgtaagctgg aacagtgggg ccttgacgtc tggagtccat acctttccgg 3780
   cggtccttca gtcatctggg ctgtacagcc tttccagcgt tgtcactgtt ccaagctcct 3840
   ccctgggcac gaagacctat acatgtaatg tcgaccataa gccttcaaat acaaaagtag 3900
   acaaacgtgt ggaatcaaaa tacggacccc catgcccgcc ctgtccagcg cctgaattct 3960
   tgggaggccc gtctgtcttt ttattccccc cgaaaccgaa ggacaccctc atgatctcaa 4020
   ggacaccaga ggtgacctgc gtggtggtgg atgttagtca agaggatcca gaggtgcagt 4080
   ttaactggta tgtcgatggc gtcgaagtcc acaacgctaa aaccaagcca cgggaggagc 4140
   agtttaactc tacctatcga gtggtgtccg tactgactgt gttgcaccag gactggctta 4200
   atgggaaaga atacaagtgt aaagtttcaa acaaaggcct gccttcaagt atcgaaaaga 4260
   ctattagcaa agctaaaggg cagccaagag aaccacaggt gtacactttg cccccttccc 4320
   aagaggagat gactaagaat caagtttctc tgacatgtct ggtaaaaggc ttctaccctt 4380
   ctgatatagc agtggagtgg gagtctaacg gacagcccga aaacaattac aagacgacac 4440
   cccctgtgct agattcagac ggctcttttt ttctttacag tcggctcaca gtggataaat 4500
   cacgctggca ggaaggaaat gtctttagct gcagcgtcat gcatgaggct ttgcacaacc 4560
   attacactca gaaatctctg agcctctcac ttggtaaacg ccgcaagcgc ggctccggcg 4620
   agggccgcgg ctccctgctg acctgcggcg acgtggagga gaaccccggc cccatgaagt 4680
   acctgctgcc caccgccgcc gccggcctgc tgctgctggc cgcccagccc gccatggccg 4740
   atatccagat gacccagagc ccctccagcc ttagcgcttc agtcggggat agagtgacaa 4800
   ttacctgcag agcgtctgaa aatatctaca gctacttggc ctggtaccaa cagaaaccag 4860
   gcaaagctcc taagctcctg atttataacg caaaaacctt ggctgagggg gtgcccagtc 4920
   gcttctcggg ctctggctca ggaaccgact ttacactcac catttcttca ctgcagcctg 4980
   aggacttcgc tacgtactac tgccagcatc actatggtac cccacgcact tttggagggg 5040
   ggactaaggt ggaaatcaag aggaccgtgg ccgccccatc cgtatttatc ttccccccat 5100
   ccgatgagca gttaaaatca gggaccgcct cggttgtgtg tctgcttaac aactttaccc 5160
   cccgcgaagc taaggtgcag tggaaggttg ataacgcctt gcagtctgga aacagccagg 5220
   agagcgtcac agaacaagat tcaaaagaca gtacttattc cctgagtagt acgctgaccc 5280
   tgtccaaagc cgattacgaa aagcacaaag tatacgcgtg tgaggtcact caccagggac 5340
   tatcaagccc ggtcactaaa tcattcaaca ggggggagtg ctaatgagct gaccgagccc 5400
   tcctctataa acccaagatt caaggctcca agcgataacc ttctcccgcc tcttccatcc 5460
   cactgaatga tcgcgcagct gcaattaatc tagcaacatt aaagattaag aaaaaatacg 5520
   ggtagaatcg gagtgcaccg attgtgccaa gatggactca tctaggacaa ttgggttata 5580
   ctttgattct gcccttcctt ctagcaacct gttagcattc ccgattgtct tacgagatgt 5640
   aggagatgga aagaagcaaa ttactccgca atataggatc cggcgtcttg actcgtggac 5700
   agacagtaaa gaagactcgg tattcatcac tacctacgga ttcatcttcc aggttgggaa 5760
   tgaagaagtc actgtcggca tgatcaatga taatcccaag cgagagttac tctccgctgc 5820
   gatgctctgc ctaggaagcg tcccgaatgt tggagatcct gttgagctgg caagggcctg 5880
   cctcactatg gtggtaacat gcaagaagag tgcaactaat actgagagaa tggtcttctc 5940
   agtagtgcag gcaccccggg tgctgcaaag ctgtagggtc gtggcagaca aatactcgtc 6000
   agtgaatgca gttaagcacg tgaaagcacc agagaagatc ccttggagtg aaaccctaga 6060
   gtacaaggtg aattttgtct ctttgactgt ggtgccgaag aaggatgtct acaagatccc 6120
   aaccgcagta ttgaaggtat ccggctcgag cctgtataat cttgcgctca atgtcactat 6180
   tgatgtggag gtagacccaa agagcccgtt agtcaagtcc ctttctaggt ccgacagtgg 6240
   atactatgct aatctcttct tacatatcgg acttatgtcc actgtagata agaggggaaa 6300
   gaaagtgaca tttgaccagc tggagaggaa gataaggaga cttgatctat ctgtcgggct 6360
   cagtgatgtg ctcggacctt ctgtgcttgt gaaggcgaga ggtgcacgga ctaggctgct 6420
   ggcacctttc ttctctaaca gcgggacagc ctgctaccct atagcaaatg cctctcctca 6480
   ggtagctaag atactctgga gtcagaccgc gtgcctgcgg agtgtaaaaa tcattattca 6540
   agcgggcacc caacgcgctg tcgcagtgac tgctgaccat gaggttacct ctactaagat 6600
   agagaagagg cataccatcg ctaaatacaa tcccttcaag aaataggccg tatccctgag 6660
   actgcaattc acccgccttc ccaaaacacc atgacaccag ataatgatct gtcttgatta 6720
   cttacagtta gtttccctgt ctatcaaatt agaaaaaaca cgggtagaag agttcggatc 6780
   ccggccggcg caccaaaagc gcaagatggg ccccagatct tctaccagga tcccagtacc 6840
   tctaatgctg accatacgga tcacgctggc actgagttat gtccgtctga caagttctct 6900
   tgatggcagg cctcttgcag ccgcagggat cgtggtaaca ggggataaag cagttaacat 6960
   atacacctca tcccagacag ggtcaatcat agtcaagtta ctcccaaata tgcccaagga 7020
   caaagaggca tgtgcaaaag ccccattgga ggcttacaac aggacactga ctactttgct 7080
   tacccccctt ggtgattcta tccgcaggat acaagagtct gtgactacat ccggaggaag 7140
   gagacagaga cgctttatag gtgccattat tggcagtgta gctctagggg ttgcaacagc 7200
   tgcacagata acggcagcct cggctctgat acaagccaac cagaatgctg ctaacatcct 7260
   ccggcttaag gagagcattg ctgcaaccaa tgaagctgta cacgaggtca ctggcggatt 7320
   gtcacagtta gcagtggcag ttggaaagat gcagcaattt gttaatgacc agtttaataa 7380
   cacagctcaa gagttggact gtataaaaat tacacagcag gttggtgtag aactcaactt 7440
   atacttaact gaattgacta cagtgttcgg gccacaaatc acttcccctg ccttaactca 7500
   gttgactgtt caggctcttt acaatctggc tggtggtaat gtagattact tgttgactaa 7560
   gttaggtgta gggaacaacc agctcagctc attgattggt agcggcttga tcaccggtaa 7620
   ccctattttt tacgactcac agactcaact cttgggcata caggtgactt taccctcagt 7680
   cgggaaccta aataatatgc gtgccaccta cttggagacc ttgtctgtaa gcacaacaaa 7740
   gggatttgcc tcagcacttg tcccaaaagt agtgacacag gtcggttctg tgatagaaga 7800
   gcttgacacc tcatactgta tagaagctga tttggattta tattgtacaa gaatagtgac 7860
   attccctatg tcccctggta tttattcctg tttgagcggc aatacatcgg cttgcatgta 7920
   ttcaaggact gaaggcgcac ttactacacc atacatgact ctcaaaggct cagttgttgc 7980
   caattgccag atgacaacat gtagatgtgc agaccccccg ggtatcatat cacaaaatta 8040
   tggagaggct gtgtctctaa tagataagca ctcatgcaat gtcgtatcct tagacgggat 8100
   aactttgagg ctcagtgggg aatttgatgc aacttatcaa aagaatatct caatattaga 8160
   ttctcaagta ctagtgacag gcaatctcga tatctcaact gagcttggga atgtcaacca 8220
   ctcaataagt aatgctttgg ataagttaga ggaaagcaac agcaaactag acaaagtcaa 8280
   tgtcaaactg accagcacat ctgctctcat tacctatatt gttttaactg tcatatctct 8340
   tgttcttggt atgcttagcc tggttctagc atgctatctg atgtacaagc aaaaagcgca 8400
   acgaaagacc ttgttgtggc ttggaaataa taccctagat cagatgagag ctactacaaa 8460
   aatgtgaatg cagatgagag gcagaggtat ccccaatagc aatctgtgtg tcaattctgg 8520
   cagcctgtta atcagaagaa ttaagaaaaa actaccggat gtaggtgaac aaaagggaat 8580
   atacgggtag aacggcctga gaggccaccc ctcaatcggg agccaggccc cactacgtcc 8640
   gctctaccgc atcaccaaca gcagtcttca gtcatggaca gcgcagttag ccaagttgcg 8700
   ctagagaatg atagaagaga agcgaataat acatggcgct tggttttccg gatcgcagcc 8760
   ttacttttac tggtaataac cttagccgtc tctgcagtcg ccctggcata tagtatggag 8820
   gctagcacac ctggcgacct tgtaagcata ccgactgcga tctctagggc agaggaaagg 8880
   attacatctg cactcggttc caatcaagat gtagtagata ggatatacaa gcaagtggcc 8940
   cttgagtctc cattggcact gctaaatacc gaatctataa ttatgaatgc aataacgtct 9000
   ctctcttatc aaatcaatgg agccacaaat aatagcgggt gtggggcacc tgttcatgac 9060
   ccagattaca tcggggggat aggtcaagaa cttattgtag atgatacgag tgacgtcaca 9120
   tcattttatc cctctgcatt ccaagaacac ctgaatttta tcccggcgcc cactacagga 9180
   tcaggctgca ctcggatacc ctcattcgac atgagtgcta cccattactg ttacactcac 9240
   aatgtgatat tgtctggctg cagagatcac tcacactcac atcagtattt agcacttggt 9300
   gtgcttcgga catctgcaac agggagggta ttcttttcta ctctgcgttc catcaacttg 9360
   gatgacgccc aaaatcggaa gtcttgcagt gtgagtgcaa ctcctttagg ttgtgatatg 9420
   ctgtgctcta aaatcacaga gactgaggaa gaggattata aatcagttat ccccacatcg 9480
   atggtacatg gaaggttagg gcttgacggc caataccatg agaaggacct agacgtcaca 9540
   acactattta gggactgggt ggcaaattac ccaggagtag gaggtgggtc ttttattaac 9600
   aatcgcgtat ggttcccggt ctacggaggg ctaaaaccca gctcgcctag tgacactgca 9660
   caagaaggga gatacgtaat atacaagcga tacaatgaca catgcccgga tgagcaagat 9720
   taccagattc ggatggctaa gtcatcgtat aagcctaggc ggtttggtgg aaaacgcgta 9780
   cagcaggcca tcctatccat caaggtgtca acatccttgg gtgaggaccc ggtgttgact 9840
   gtaccgccta atacggtcgc actcatgggg gccgaaggca gagttctcac agtggggaca 9900
   tctcatttct tatatcagcg agggtcatca tacttctctc ccgctttgtt ataccctatg 9960
   acagtcaaca acaaaacagc cactcttcat aatccttata cattcaatgc tttcactcgg 10020
   ccaggtagtg tcccttgcca ggcttcagca agatgcccca actcatgtgt taccggagtc 10080
   tatactgatc catatccctt agtcttccat aggaaccaca ccttgcgagg ggtattcgga 10140
   acaatgcttg atgacgaaca agcaagactc aaccctgtat ctgcagtatt tgataacata 10200
   tcccgcagtc gcataactcg ggtaagttca agcagtacca gggcagcata cacgacatca 10260
   acgtgtttta aagttgtcaa gaccaataaa acctattgcc tcagcattgc agaaatatcc 10320
   aataccctct tcggggaatt cagaattgtc cccttactag ttgagattct caaggatggt 10380
   ggggtttaag aagctaggaa tggtcggttg agtcaaccgt gagaaggcgg ggaagatgat 10440
   attgcatcat ctatcttctg taacaccaag aatcaaatcg aataccagtg cgagctcgaa 10500
   tcctacgctg ccagtcagcc ataatcggct ggtgctaatg tggttagcct gaatcttgtc 10560
   gatagtcact tgattaagaa aaatgtggga cggtagtgaa atataaggca aagcagctca 10620
   cagaggacag cacgggtagg acatggcggg ctccggaccc gaaagggcag agcatcagat 10680
   tatcctacca gagtcacatc tgtcctcacc attggtcaag cataaattgc tttattactg 10740
   gaaattaact gggctaccgc ttcctgacga atgtgacttc gaccatctta ttatcagccg 10800
   acaatggaag aaggtacttg aatcggccac ccctgacatt gagagaatga taaaactagg 10860
   gcgggcagta caccagactc tcaaccacaa ttccaggata accggagtac tccatccccg 10920
   gtgtttagag gaactggcta gtattgaggt tcctgattca accaacaaat ttcggaagat 10980
   cgaaaaaaag atccagattc acaacacaag gtatggagaa ctgttcacaa gattatgcac 11040
   gcatgtagaa aagaaactat tggggtcatc ttggtctaac aatgtcccac ggtcagagga 11100
   attcaacagc atccgtacag atccggcatt ttggtttcac tcaaaatggt ccacagccaa 11160
   atttgcatgg ctccatataa aacaggtcca gaggcatctg attgtagcag caagaacaag 11220
   gtccgcagtc aacaaattag tgacgctgac ccataaggta ggccaaatct ttgttactcc 11280
   tgagcttgtc attgtgacac atacagatga gaacaagttc acgtgtctta cccaggaact 11340
   tgtgttgatg tatgcagata tgatggaggg cagagatatg gtcagcataa tatcatccac 11400
   ggcggcacat cttaggagct tatcagagaa aattgatgat attctgcggt tagtagatgc 11460
   tctggcaaga gatttgggca atcaagtcta cgatgttgta gcactaatgg agggattcgc 11520
   atacggcgct gttcagctgc ttgaaccgtc aggtacattt gcgggggatt tcttcgcatt 11580
   caacctgcag gagctcaaag atactctaac cggactcctc cccaaggata tcgcagaatc 11640
   tgtgactcac gcaatcgcaa ccatattctc tggcttagaa caaaatcaag cagctgagat 11700
   gttgtgcctg ttgcgtctgt ggggtcaccc actactcgag tcccgtattg cagcaaaagc 11760
   agttaggagt caaatgtgcg caccaaaaat ggtagacttt gatatgatcc tccaggtatt 11820
   atctttcttt aaaggaacaa tcatcaatgg atatagaaag aagaatgcag gcgtgtggcc 11880
   acgtgtcaag atagatacga tatacgggaa ggtcataggg cagctacacg cagattctgc 11940
   agagatttca catgatgtca tgttgaggga atacaagagt ttatctgcac ttgaattcga 12000
   gccatgtata gagtatgacc ctgtcaccaa tctgagcatg tttttaaaag acaaggcaat 12060
   cgcacacccg aaagacaact ggctcgcttc gtttaggcga aaccttctct ctgaggacca 12120
   gaagaaacat gtaagagagg caacctcaac taaccgcctc ttgatagagt tcttagagtc 12180
   aaatgatttt gatccatata aagagatgga atatctgacg acccttgagt acctaagaga 12240
   tgataatgtg gcagtatcat actcactcaa agaaaaagag gtgaaagtta atgggcggat 12300
   tttcgcaaag ctaacaaata gattaaggaa ttgtcaggta atggcagaag ggatcctagc 12360
   tgaccagatt gcacctttct tccagggaaa tggggtcatt caggatagca tatctttgac 12420
   taaaagtatg ctagcgatga gtcaactgtc tttcaacagc aacaagaaac gtattactga 12480
   ctgcaaagaa agagtatcct caaaccgcaa tcatgatccg aagagcaaga atcgtcggag 12540
   agttgccact tttgtaacta ccgacctgca aaagtattgt ctcaattgga gatatcagac 12600
   agtcaagctg ttcgctcatg ccatcaatca gctgatgggc ttacctcact tcttcgagtg 12660
   gattcatctt agactaatgg atactacgat gtttgtaggg gaccctttca atcctccaag 12720
   tgaccctacg gactgtgatc tatcaagagt cccaaatgat gacatatata ttgtcagtgc 12780
   taggggaggc attgagggat tatgccagaa gctatggaca atgatctcaa ttgctgcaat 12840
   ccagcttgct gcagcaagat cacattgtcg cgttgcctgt atggtacaag gcgacaatca 12900
   agtaatagct gtaacgagag aggtaagatc agatgactcc ccagagatgg tgttaacaca 12960
   attgcatcaa gccagtgata atttcttcaa ggaattgatt catgtcaatc atttgatcgg 13020
   ccataatttg aaggatcgtg aaaccatcag gtcagacacg ttcttcatat acagcaaacg 13080
   aatattcaaa gatggagcaa tactcagtca ggtcctcaaa aactcatcta aattagtgct 13140
   aatatcaggc gaccttagtg aaaacactgt aatgtcttgt gccaacattg catctactgt 13200
   agcacggtta tgcgagaacg ggcttcctaa ggatttctgt tattacttaa actacttaat 13260
   gagttgcgtg cagacatact tcgattccga attttccatc accaacaact cgcaactcga 13320
   ttctaaccag tcgtggatag aggacatttc ttttgtgcac tcatatgtcc tgacccctgc 13380
   tcaactgggg ggactgagta accttcaata ctcaaggctc tacacaagga acatcggcga 13440
   cccgggaacc actgctttcg cagagatcaa gagattagag gcagtggggt tactgagtcc 13500
   tagcattatg actaacatct taactaggcc gcctggaaat ggagattggg ccagtctgtg 13560
   caacgatcca tactccttta attttgagac tgtcgcaagc ccaaatattg tccttaagaa 13620
   acatacacaa agagtcctat ttgaaacttg ctcgaatccc ttattatctg gagtgcacac 13680
   agaggataat gaggcagaag agaaggcatt ggctgaattc ttgctcaatc aagaaatgat 13740
   tcatccacgt gtcgcgcatg ctatcatgga agcaagctct gtaggtagga gaaagcaaat 13800
   tcaggggctt gttgacacaa caaacaccgt gattaagatt gcactgacta ggaggccact 13860
   cggcatcaag aggctgatgc ggatagtcaa ttactcgagc atgcatgcaa tgctatttag 13920
   agatgatgtt ttctcgtcta atagatccaa ccatccctta gtctcttctg atatgtgttc 13980
   tcagacactg gcagactatg cacggaacag aagctggtca cctttgacag ggggtagaaa 14040
   aatactgggt gtatctaatc ctgataccat agaacttgta gagggtgaga tccttagtgt 14100
   cagtggaggg tgcactagat gtgacagtgg ggatgaacag tttacttggt tccatcttcc 14160
   aagcaatata gagctaaacg atgacaccag caagaatcct ccaatgagag tgccatatct 14220
   cgggtcaaag actcaagaga ggagagccgc ctcgcttgcg aaaatagctc atatgtcacc 14280
   acacgtgaag gcggctctaa gggcgtcatc tgtgttaatc tgggcttatg gggacaacga 14340
   aataaactgg actgctgccc ttaagattgc aaggtctcgg tgcaacataa gctcagagta 14400
   tcttcgacta ttgtcaccct tgcctacagc tgggaatctc caacatagat tggacgacgg 14460
   cataactcag atgacattca cccctgcatc tctatacagg gtgtcacctt acattcatat 14520
   atctaatgat tctcaaaggc tatttactga agaaggagtc aaagagggga atgtggttta 14580
   tcagcaaatc atgctcttgg gtttgtcctt aattgagtca ctcttcccaa tgacaacaac 14640
   caagacatat gatgaaatca cattgcacct ccacagtaaa tttagctgct gtatcaggga 14700
   agcacctgtt gcagttcctt tcgagctact cggggtggca ccggaactaa gggcagtaac 14760
   ctcaaataag tttatgtatg atcctagccc tgtatcagag ggagactttg cgagacttga 14820
   cttagctatc tttaagagtt atgaacttaa tttagagtca tattccacaa tagagctaat 14880
   gaacgttctt tcaatatcta gtgggaagtt gattggccag tctgtggttt cttatgatga 14940
   agatacctcc ataaagaatg acgctataat agtgtatgac aacacacgga attggatcag 15000
   cgaagctcag aattcagatg tggtccgcct attcgagtat gcggcactcg aagtgctcct 15060
   cgactgttct tatcaactct actatctgag agtaagaggc ctaaacaata tcgtcctgta 15120
   catgagtgat ttatacaaga atatgccagg aattctactc tctaatattg cagccacaat 15180
   atctcaccct gtcatccatt caaggttgaa tgcagtaggt ctggtcaacc atgacgggtc 15240
   acaccaactt gcagacacag atttcattga aatgtctgca aagctgctag tcttttgcac 15300
   tcgacgcgtg gtctcaggtt tacatgcagg gaataagtat gacctgctgt ttccatctgt 15360
   cttggatgat aacctaagtg agaagatgct tcagttgata tcccaattat gctgtctgta 15420
   tacggtgctc tttgctacaa caagagaaat cccgaaaata agaggcttat ctgcagaaga 15480
   gaaatgttca gtacttactg agtacctact gtcagatgct gtgaaaccgt tacttgggtc 15540
   cgagcaagtg agctctatca tgtctcccaa catagttacg ttcccagcca atctgtatta 15600
   catgtctagg aagagcctta atttgatcag ggagagagag gacagggata ctatcttggc 15660
   attgttgttc cctcaagaac cgctgctcga gtttcctctg gtacgagata ttggtgctcg 15720
   tgtaaaagat ccatttaccc gacaacctgc ggcgttttta caagagttag atttgagtgc 15780
   tccggcaagg tatgacgcat tcacaatcag tcaggcgcat tctgaacaca tattgccaaa 15840
   cccagaggaa gatcacttag tacgatactt gttcagagga atagggactg cgtcctcctc 15900
   ttggtataag gcatcccatc ttctttctgt acccgaggtc agatgtgcaa ggcatgggaa 15960
   ctccttatat ttagcagaag gaagtggagc catcatgagt cttctcgaat tgcatatacc 16020
   acacgaaact atctattaca atacactttt ctcgaacgag atgaaccccc cacagcgaca 16080
   tttcggacca actccaacac agtttctgaa ttcggttgtt tttaggaatt tacaggcgga 16140
   agtaccatgc aaggatggat ttgtccagga gttccgtccg ttatggagag agaatacaga 16200
   agaaagcgat ctgacctcag ataaagcagt gggatatatc acatctgtag tgccgtacag 16260
   gtctgtatca ttgctgcatt gtgacattga aatccctcca ggatctaatc aaagcttact 16320
   agatcagctg gctaccaatc tgtctctgat tgccatgcat tctgtgaagg agggcggggt 16380
   cgtgattatc aaagtactgt atgcaatggg atattacttc catctactca tgaacttgtt 16440
   tactccatgt tccacgaaag gatatattct ctctaatggc tatgcctgta gaggggatat 16500
   ggagtgttac ctgatatttg tcatgggcta cctaggcggg cctacatttg tacatgaggt 16560
   ggtgaggatg gcaaaaacgc tagtacagcg gcatggcaca cttttgtcta agtcagatga 16620
   gattacactg actaggttat ttacttcaca gcaacagcgt gtaacagaca tcctatccag 16680
   ccctttaccg agactaatga agtacttgag agagaatatt gatactgcac tgattgaagc 16740
   cgggggacag cctgtccgtc cattctgtgc agagagttta gtgagcacac taacagacat 16800
   gactcagaca acccagatca tcgccagcca cattgataca gtcattcgat ctgtgatcta 16860
   tatggaagca gagggtgatc ttgctgacac agtattctta tttacccctt acaacctctc 16920
   tatggacggg aaaaagagaa catcacttaa acagtgcaca agacagatct tagaggtcac 16980
   aatactgggt ctcagagtca aaaatctcaa taaagtaggt gatgtaatca gcctagtact 17040
   cagaggtatg atttctctgg aggaccttct cccactgaga acctacttga agtgtagtac 17100
   ctgccctaag tatttgaagg ctgtcctagg tattaccaaa ctcaaagaaa tgttcacaga 17160
   cgcctcttta ttatacttga ctcgtgctca acaaaaattc tacatgaaaa ctataggcaa 17220
   tgcagtcaag ggatactata gtaactgtga ctcttaaagg caaccacata tcaataggcc 17280
   ctctttctag ccgatcgtat tcttgttgac ttcattatac catattagaa aaaaattgaa 17340
   ttccgaccct ttaagactcg tattcggatt caaataatta tctcagaaaa aaagtgcacg 17400
   tagttgttct tgattatagt cccgtcattc accaagtctt tgtttggt 17448

<210> 9
<211> 17436
<212> DNA
<213> Newcastle disease virus

<220>
<223> Mut HN (F277L)/M(G165W)-TRX518

<400> 9
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500
agcgccaaac tctgtgcaga acaccactca gcaggagcct ccatctactc ctgggccatc 1560
tcaagacaac gacaccgact ggggatactg accgacaaca ctcagcctgc ctccatcgaa 1620
ttatctcgat tcttctgcct gtaacctaac ccctggtcca cagtctcaca cggccaaacc 1680
cgcaaacgag cccccccctc tctccccccc cctccagcca cacggctcca accgcccaaa 1740
acaacacggg cacaactcga ctcactcata atctacacag agccgaagat cttagaaaaa 1800
aatacgggta gaagagagac attcagagat caggacaaat catcagggtc tccgctctcc 1860
cctctaccca gcagaccaga gtgaagatgg ccaccttcac ggatgcagag atcgacgatc 1920
tatttgaaac cagtggaact gtcattgaca gcataattac ggcccaaggc aacccagtag 1980
agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gccccccacc caggccgcag gcgaagccgg tgacacacag ctcaagactg 2220
gagcaagcaa ctccctcctg tcgatgctcg acaagcttag caataaatcg tccaatgcta 2280
aaaagggccc acggtcgagt ccccaggaag ggcaccacca acctccgacc cagcagcaag 2340
ggagtcaaca gagccgcgga aacagtcagg agagatcgca aaaccaggtc aaggccgcct 2400
ctggagacca gggcacagac gcgaacacag catatcatgg acaatggaag gagtcacaac 2460
catcagctgg tgcaatccct catgctctcc ggtcagggca gagccaagcc aatactcctg 2520
catctgtgga tcatgtccag ctacctgtcg actttgtgca ggcgatgatg tctatgatgg 2580
aggcgatatc acagaaggta aataaagtcg actatcagct agaccttgtc tcaaagcaga 2640
catcctccat ccccatgatg cgatctgaaa tccaacagct gaaaacatcc gttgcggtca 2700
tggaagccaa cctgggcatg atgaagattc tggaccctgg ttgtgctaac gtttcatctt 2760
taagtgacct acgggcggtc gcccgctctc acccagtttt agtttcaggc cccggagacc 2820
catctcctta tgtgacacaa gggggtgaat tgatactcaa taaactctcg caaccagtgc 2880
aacatccttc tgagttgatt aaacctgcca cgataagcgg gcctgatata ggagtggaga 2940
aggacactgt ccgtgcattg atcacctcac gcccaatgca tccgagctct tcagctaagc 3000
tcctgagtaa gctggatgca gccgggtcga tagaagaaat taggaaaatc aaacgccttg 3060
cgctgaatgg ctgatcacta ctacaacccg caacgggttc ccgtttatct attgtcacaa 3120
ggaacccgcc ccgaagctta agaaaaaata cgggtagaag cagtttgaga gctccgttcg 3180
ccaccatgga gttcggcctg tcctgggtgt tcctggtggc cctgttccgc ggcgtgcagt 3240
gccaagtgac cttacgggaa tcaggtccag cgctggtaaa gcccacccaa actctaacat 3300
tgacctgtac tttcagcggc ttttcccttt cgacaagtgg catgggggtg ggatggatcc 3360

ggcaacctcc tgggaaggcc ctcgaatggc tggcccacat ttggtgggac gatgacaagt 3420

actacaaccc ctcactgaag tcacggctga cgatttcaaa ggacaccagc aaaaatcaag 3480

tggtactgac gatgaccaat atggatcccg tcgacactgc tacatactac tgcgctcgca 3540

caaggcgata ctttcccttc gcctattggg gacagggaac tctggttacc gtgagttccg 3600

catccactaa gggcccaagc gtcttcccgc tggccccttc cagtaagagc acctccggag 3660

ggaccgctgc acttggatgc ctggttaaag actatttccc tgaacctgtt acagtcagtt 3720

ggaactcggg ggcactaacc tctggagtgc ataccttccc agctgtactc caatcatctg 3780

gactatactc actttcgtct gtcgtgactg tgccctcttc ttcacttggg acccagacat 3840

atatttgcaa cgtcaatcat aagccatcga acaccaaggt ggataagaag gtggagccga 3900

aatcttgtga taagacccac acatgtcctc catgcccagc ccctgagctg ttgggaggtc 3960

cctctgtgtt cctgttcccc ccaaaaccaa aagacaccct catgatctcc agaacgcctg 4020

aggtcacctg tgtcgttgtg gacgtttctc acgaagaccc agaggtcaaa ttcaattggt 4080

atgtcgacgg ggtagaggtc cacaatgcta agaccaagcc tcgagaagag caatatgctt 4140

ccacttacag agtggtgtct gttctgaccg tcctgcacca agactggcta aatggaaaag 4200

agtataaatg caaggtcagc aacaaagctc tgcccgcccc aattgaaaag actatcagca 4260

aagcgaaagg ccagccacgg gaacctcagg tctatacgct gcccccaagc cgtgatgagc 4320

tcacaaagaa ccaggtgagt ctgacttgtc tagtgaaagg cttttacccc agtgacattg 4380

cagtggagtg ggagtccaac ggacaacccg aaaacaacta taaaactacc ccccccgttc 4440

tcgattctga tggcagcttc ttcttataca gcaaacttac ggtggataaa tcgcggtggc 4500

agcagggaaa tgtgttttcc tgctctgtca tgcatgaggc cctacataat cactatacac 4560

aaaagtctct ctctttgtcc cccggaaaac gccgcaagcg cggctccggc gagggccgcg 4620

gctccctgct gacctgcggc gacgtggagg agaaccccgg ccccatgaag tacctgctgc 4680

ccaccgccgc cgccggcctg ctgctgctgg ccgcccagcc cgccatggcc gagatagtga 4740

tgactcagtc ccccgccact ctctcagtaa gcccaggaga gagagctacg ctgagctgca 4800

aggcctccca gaacgtgggg accaacgtag cctggtacca acaaaagccc ggtcaggccc 4860

cccggctgct catttattcc gcaagttata gatattcagg cattccggcc agatttagtg 4920

gatccggctc aggcacggag tttaccttga ccattagctc tctccaatct gaggatttcg 4980

cggtgtatta ttgtcagcag tataacacag accccttgac cttcggcggt ggcacaaaag 5040

tcgaaattaa gaggacagtc gccgcccctt ccgtgttcat tttccccccc tcagacgaac 5100

agctgaaatc tggcacagct tcgtggtgtt tactgaacaa cttctatccc agagaagcaa 5160

aggtccagtg gaaggtcgat aatgccctgc agtctggtaa tagtcaggag tccgtaactg 5220

agcaggacag caaggactcc acctacagcc tgtcctctac cctgacactg agcaaagcag 5280

actacgaaaa acacaaagtg tacgcctgtg aggtcactca ccagggcctg agctctccag 5340

tgactaagtc ttttaatcgc ggcgagtgct aatgagctga ccgagccctc ctctataaac 5400

ccaagattca aggctccaag cgataacctt ctcccgcctc ttccatccca ctgaatgatc 5460

gcgcagctgc aattaatcta gcaacattaa agattaagaa aaaatacggg tagaatcgga 5520

gtgcaccgat tgtgccaaga tggactcatc taggacaatt gggttatact ttgattctgc 5580

ccttccttct agcaacctgt tagcattccc gattgtctta cgagatgtag gagatggaaa 5640

gaagcaaatt actccgcaat ataggatccg gcgtcttgac tcgtggacag acagtaaaga 5700

agactcggta ttcatcacta cctacggatt catcttccag gttgggaatg aagaagtcac 5760

tgtcggcatg atcaatgata atcccaagcg agagttactc tccgctgcga tgctctgcct 5820

aggaagcgtc ccgaatgttg gagatcctgt tgagctggca agggcctgcc tcactatggt 5880

ggtaacatgc aagaagagtg caactaatac tgagagaatg gtcttctcag tagtgcaggc 5940

accccgggtg ctgcaaagct gtagggtcgt ggcagacaaa tactcgtcag tgaatgcagt 6000

taagcacgtg aaagcaccag agaagatccc ttggagtgaa accctagagt acaaggtgaa 6060

ttttgtctct ttgactgtgg tgccgaagaa ggatgtctac aagatcccaa ccgcagtatt 6120

gaaggtatcc ggctcgagcc tgtataatct tgcgctcaat gtcactattg atgtggaggt 6180

agacccaaag agcccgttag tcaagtccct ttctaggtcc gacagtggat actatgctaa 6240

tctcttctta catatcggac ttatgtccac tgtagataag aggggaaaga aagtgacatt 6300

tgaccagctg gagaggaaga taaggagact tgatctatct gtcgggctca gtgatgtgct 6360

cggaccttct gtgcttgtga aggcgagagg tgcacggact aggctgctgg cacctttctt 6420

ctctaacagc gggacagcct gctaccctat agcaaatgcc tctcctcagg tagctaagat 6480

actctggagt cagaccgcgt gcctgcggag tgtaaaaatc attattcaag cgggcaccca 6540

acgcgctgtc gcagtgactg ctgaccatga ggttacctct actaagatag agaagaggca 6600

taccatcgct aaatacaatc ccttcaagaa ataggccgta tccctgagac tgcaattcac 6660

ccgccttccc aaaacaccat gacaccagat aatgatctgt cttgattact tacagttagt 6720

ttccctgtct atcaaattag aaaaaacacg ggtagaagag ttcggatccc ggccggcgca 6780

ccaaaagcgc aagatgggcc ccagatcttc taccaggatc ccagtacctc taatgctgac 6840

catacggatc acgctggcac tgagttatgt ccgtctgaca agttctcttg atggcaggcc 6900

tcttgcagcc gcagggatcg tggtaacagg ggataaagca gttaacatat acacctcatc 6960

ccagacaggg tcaatcatag tcaagttact cccaaatatg cccaaggaca aagaggcatg 7020

tgcaaaagcc ccattggagg cttacaacag gacactgact actttgctta cccccttggg 7080

tgattctatc cgcaggatac aagagtctgt gactacatcc ggaggaagga gacagagacg 7140

ctttataggt gccattattg gcagtgtagc tctaggggtt gcaacagctg cacagataac 7200

ggcagcctcg gctctgatac aagccaacca gaatgctgct aacatcctcc ggcttaagga 7260

gagcattgct gcaaccaatg aagctgtaca cgaggtcact ggcggattgt cacagttagc 7320

agtggcagtt ggaaagatgc agcaatttgt taatgaccag tttaataaca cagctcaaga 7380

gttggactgt ataaaaatta cacagcaggt tggtgtagaa ctcaacttat acttaactga 7440

attgactaca gtgttcgggc cacaaatcac ttcccctgcc ttaactcagt tgactgttca 7500

ggctctttac aatctggctg gtggtaatgt agattacttg ttgactaagt taggtgtagg 7560

gaacaaccag ctcagctcat tgattggtag cggcttgatc accggtaacc ctatttttta 7620

cgactcacag actcaactct tgggcataca ggtgacttta ccctcagtcg ggaacctaaa 7680

taatatgcgt gccacctact tggagacctt gtctgtaagc acaacaaagg gatttgcctc 7740

agcacttgtc ccaaaagtag tgacacaggt cggttctgtg atagaagagc ttgacacctc 7800

atactgtata gaagctgatt tggatttata ttgtacaaga atagtgacat tccctatgtc 7860

ccctggtatt tattcctgtt tgagcggcaa tacatcggct tgcatgtatt caaggactga 7920

aggcgcactt actacaccat acatgactct caaaggctca gttgttgcca attgccagat 7980

gacaacatgt agatgtgcag acccccgggg tatcatatca caaaattatg gagaggctgt 8040

gtctctaata gataagcact catgcaatgt cgtatcctta gacgggataa ctttgaggct 8100

cagtggggaa tttgatgcaa cttatcaaaa gaatatctca atattagatt ctcaagtact 8160

agtgacaggc aatctcgata tctcaactga gcttgggaat gtcaaccact caataagtaa 8220

tgctttggat aagttagagg aaagcaacag caaactagac aaagtcaatg tcaaactgac 8280

cagcacatct gctctcatta cctatattgt tttaactgtc atatctcttg ttcttggtat 8340

gcttagcctg gttctagcat gctatctgat gtacaagcaa aaagcgcaac gaaagacctt 8400

gttgtggctt ggaaataata ccctagatca gatgagagct actacaaaaa tgtgaatgca 8460

gatgagaggc agaggtatcc ccaatagcaa tctgtgtgtc aattctggca gcctgttaat 8520

cagaagaatt aagaaaaaac taccggatgt aggtgaacaa aagggaatat acgggtagaa 8580

cggcctgaga ggccacccct caatcgggag ccaggcccca ctacgtccgc tctaccgcat 8640

caccaacagc agtcttcagt catggacagc gcagttagcc aagttgcgct agagaatgat 8700

agaagagaag cgaataatac atggcgcttg gttttccgga tcgcagcctt acttttactg 8760

gtaataacct tagccgtctc tgcagtcgcc ctggcatata gtatggaggc tagcacacct 8820

ggcgaccttg taagcatacc gactgcgatc tctagggcag aggaaaggat tacatctgca 8880

ctcggttcca atcaagatgt agtagatagg atatacaagc aagtggccct tgagtctcca 8940

ttggcactgc taaataccga atctataatt atgaatgcaa taacgtctct ctcttatcaa 9000

atcaatggag ccacaaataa tagcgggtgt ggggcacctg ttcatgaccc agattacatc 9060

ggggggatag gtcaagaact tattgtagat gatacgagtg acgtcacatc attttatccc 9120

tctgcattcc aagaacacct gaattttatc ccggcgccca ctacaggatc aggctgcact 9180

cggataccct cattcgacat gagtgctacc cattactgtt acactcacaa tgtgatattg 9240

tctggctgca gagatcactc acactcacat cagtatttag cacttggtgt gcttcggaca 9300

tctgcaacag ggagggtatt cttttctact ctgcgttcca tcaacttgga tgacgcccaa 9360

aatcggaagt cttgcagtgt gagtgcaact cctttaggtt gtgatatgct gtgctctaaa 9420

atcacagaga ctgaggaaga ggattataaa tcagttatcc ccacatcgat ggtacatgga 9480

aggttagggc ttgacggcca ataccatgag aaggacctag acgtcacaac actatttagg 9540

gactgggtgg caaattaccc aggagtagga ggtgggtctt ttattaacaa tcgcgtatgg 9600

ttcccggtct acggagggct aaaacccagc tcgcctagtg acactgcaca agaaggagaa 9660

tacgtaatat acaagcgata caatgacaca tgcccggatg agcaagatta ccagattcgg 9720

atggctaagt catcgtataa gcctaggcgg tttggtggaa aacgcgtaca gcaggccatc 9780

ctatccatca aggtgtcaac atccttgggt gaggacccgg tgttgactgt accgcctaat 9840

acggtcgcac tcatgggggc cgaaggcaga gttctcacag tggggacatc tcatttctta 9900

tatcagcgag ggtcatcata cttctctccc gctttgttat accctatgac agtcaacaac 9960

aaaacagcca ctcttcataa tccttataca ttcaatgctt tcactcggcc aggtagtgtc 10020

ccttgccagg cttcagcaag atgccccaac tcatgtgtta ccggagtcta tactgatcca 10080

tatcccttag tcttccatag gaaccacacc ttgcgagggg tattcggaac aatgcttgat 10140

gacgaacaag caagactcaa ccctgtatct gcagtatttg ataacatatc ccgcagtcgc 10200

ataactcggg taagttcaag cagtaccagg gcagcataca cgacatcaac gtgttttaaa 10260

gttgtcaaga ccaataaaac ctattgcctc agcattgcag aaatatccaa taccctcttc 10320

ggggaattca gaattgtccc cttactagtt gagattctca aggatggtgg ggtttaagaa 10380

gctaggaatg gtcggttgag tcaaccgtga gaaggcgggg aagatgatat tgcatcatct 10440

atcttctgta acaccaagaa tcaaatcgaa taccagtgcg agctcgaatc ctacgctgcc 10500

agtcagccat aatcggctgg tgctaatgtg gttagcctga atcttgtcga tagtcacttg 10560

attaagaaaa aatgtggacg gtagtgaaat ataaggcaaa gcagctcaca gaggacagca 10620

cgggtaggac atggcgggct ccggacccga aagggcagag catcagatta tcctaccaga 10680

gtcacatctg tcctcaccat tggtcaagca taaattgctt tattactgga aattaactgg 10740

gctaccgctt cctgacgaat gtgacttcga ccatcttatt atcagccgac aatggaagaa 10800

ggtacttgaa tcggccaccc ctgacattga gagaatgata aaactagggc gggcagtaca 10860

ccagactctc aaccacaatt ccaggataac cggagtactc catccccggt gtttagagga 10920

actggctagt attgaggttc ctgattcaac caacaaattt cggaagatcg aaaaaaagat 10980

ccagattcac aacacaaggt atggagaact gttcacaaga ttatgcacgc atgtagaaaa 11040

gaaactattg gggtcatctt ggtctaacaa tgtcccacgg tcagaggaat tcaacagcat 11100

ccgtacagat ccggcatttt ggtttcactc aaaatggtcc acagccaaat ttgcatggct 11160

ccatataaaa caggtccaga ggcatctgat tgtagcagca agaacaaggt ccgcagtcaa 11220

caaattagtg acgctgaccc ataaggtagg ccaaatcttt gttactcctg agcttgtcat 11280

tgtgacacat acagatgaga acaagttcac gtgtcttacc caggaacttg tgttgatgta 11340

tgcagatatg atggagggca gagatatggt cagcataata tcatccacgg cggcacatct 11400

taggagctta tcagagaaaa ttgatgatat tctgcggtta gtagatgctc tggcaagaga 11460

tttgggcaat caagtctacg atgttgtagc actaatggag ggattcgcat acggcgctgt 11520

tcagctgctt gaaccgtcag gtacatttgc gggggatttc ttcgcattca acctgcagga 11580

gctcaaagat actctaaccg gactcctccc caaggatatc gcagaatctg tgactcacgc 11640

aatcgcaacc atattctctg gcttagaaca aaatcaagca gctgagatgt tgtgcctgtt 11700

gcgtctgtgg ggtcacccac tactcgagtc ccgtattgca gcaaaagcag ttaggagtca 11760

aatgtgcgca ccaaaaatgg tagactttga tatgatcctc caggtattat ctttctttaa 11820

aggaacaatc atcaatggat atagaaagaa gaatgcaggc gtgtggccac gtgtcaagat 11880

agatacgata tacgggaagg tcatagggca gctacacgca gattctgcag agatttcaca 11940

tgatgtcatg ttgagggaat acaagagttt atctgcactt gaattcgagc catgtataga 12000

gtatgaccct gtcaccaatc tgagcatgtt tttaaaagac aaggcaatcg cacacccgaa 12060

agacaactgg ctcgcttcgt ttaggcgaaa ccttctctct gaggaccaga agaaacatgt 12120

aagagaggca acctcaacta accgcctctt gatagagttc ttagagtcaa atgattttga 12180

tccatataaa gagatggaat atctgacgac ccttgagtac ctaagagatg ataatgtggc 12240

agtatcatac tcactcaaag aaaaagaggt gaaagttaat gggcggattt tcgcaaagct 12300

aacaaataga ttaaggaatt gtcaggtaat ggcagaaggg atcctagctg accagattgc 12360

acctttcttc cagggaaatg gggtcattca ggatagcata tctttgacta aaagtatgct 12420

agcgatgagt caactgtctt ttcaacagca caagaaacgt attactgact gcaaagaaag 12480

agtatcctca aaccgcaatc atgatccgaa gagcaagaat cgtcggagag ttgccacttt 12540

tgtaactacc gacctgcaaa agtattgtct caattggaga tatcagacag tcaagctgtt 12600

cgctcatgcc atcaatcagc tgatgggctt acctcacttc ttcgagtgga ttcatcttag 12660

actaatggat actacgatgt ttgtagggga ccctttcaat cctccaagtg accctacgga 12720

ctgtgatcta tcaagagtcc caaatgatga catatatatt gtcagtgcta ggggaggcat 12780

tgagggatta tgccagaagc tatggacaat gatctcaatt gctgcaatcc agcttgctgc 12840

agcaagatca cattgtcgcg ttgcctgtat ggtacaaggc gacaatcaag taatagctgt 12900

aacgagagag gtaagatcag atgactcccc agagatggtg ttaacacaat tgcatcaagc 12960

cagtgataat ttcttcaagg aattgattca tgtcaatcat ttgatcggcc ataatttgaa 13020

ggatcgtgaa accatcaggt cagacacgtt cttcatatac agcaaacgaa tattcaaaga 13080

tggagcaata ctcagtcagg tcctcaaaaa ctcatctaaa ttagtgctaa tatcaggcga 13140

ccttagtgaa aacactgtaa tgtcttgtgc caacattgca tctactgtag cacggttatg 13200

cgagaacggg cttcctaagg atttctgtta ttacttaaac tacttaatga gttgcgtgca 13260

gacatacttc gattccgaat tttccatcac caacaactcg caactcgatt ctaaccagtc 13320

gtggatagag gacatttctt ttgtgcactc atatgtcctg acccctgctc aactgggggg 13380

actgagtaac cttcaatact caaggctcta cacaaggaac atcggcgacc cgggaaccac 13440

tgctttcgca gagatcaaga gattagaggc agtggggtta ctgagtccta gcattatgac 13500

taacatctta actaggccgc ctggaaatgg agattgggcc agtctgtgca acgatccata 13560

ctcctttaat tttgagactg tcgcaagccc aaatattgtc cttaagaaac atacacaaag 13620

agtcctattt gaaacttgct cgaatccctt attatctgga gtgcacacag aggataatga 13680

ggcagaagag aaggcattgg ctgaattctt gctcaatcaa gaaatgattc atccacgtgt 13740

cgcgcatgct atcatggaag caagctctgt aggtaggaga aagcaaattc aggggcttgt 13800

tgacacaaca aacaccgtga ttaagattgc actgactagg aggccactcg gcatcaagag 13860

gctgatgcgg atagtcaatt actcgagcat gcatgcaatg ctatttagag atgatgtttt 13920

ctcgtctaat agatccaacc atcccttagt ctcttctgat atgtgttctc agacactggc 13980

agactatgca cggaacagaa gctggtcacc tttgacaggg ggtagaaaaa tactgggtgt 14040

atctaatcct gataccatag aacttgtaga gggtgagatc cttagtgtca gtggagggtg 14100

cactagatgt gacagtgggg atgaacagtt tacttggttc catcttccaa gcaatataga 14160

gctaaacgat gacaccagca agaatcctcc aatgagagtg ccatatctcg ggtcaaagac 14220

tcaagagagg agagccgcct cgcttgcgaa aatagctcat atgtcaccac acgtgaaggc 14280

ggctctaagg gcgtcatctg tgttaatctg ggcttatggg gacaacgaaa taaactggac 14340

tgctgccctt aagattgcaa ggtctcggtg caacataagc tcagagtatc ttcgactatt 14400

gtcacccttg cctacagctg ggaatctcca acatagattg gacgacggca taactcagat 14460

gacattcacc cctgcatctc tatacagggt gtcaccttac attcatatat ctaatgattc 14520

tcaaaggcta tttactgaag aaggagtcaa agaggggaat gtggtttatc agcaaatcat 14580

gctcttgggt ttgtccttaa ttgagtcact cttcccaatg acaacaacca agacatatga 14640

tgaaatcaca ttgcacctcc acagtaaatt tagctgctgt atcagggaag cacctgttgc 14700

agttcctttc gagctactcg gggtggcacc ggaactaagg gcagtaacct caaataagtt 14760

tatgtatgat cctagccctg tatcagaggg agactttgcg agacttgact tagctatctt 14820

taagagttat gaacttaatt tagagtcata ttccacaata gagctaatga acgttctttc 14880

aatatctagt gggaagttga ttggccagtc tgtggtttct tatgatgaag atacctccat 14940

aaagaatgac gctataatag tgtatgacaa cacacggaat tggatcagcg aagctcagaa 15000

ttcagatgtg gtccgcctat tcgagtatgc ggcactcgaa gtgctcctcg actgttctta 15060

tcaactctac tatctgagag taagaggcct aaacaatatc gtcctgtaca tgagtgattt 15120

atacaagaat atgccaggaa ttctactctc taatattgca gccacaatat ctcaccctgt 15180

catccattca aggttgaatg cagtaggtct ggtcaaccat gacgggtcac accaacttgc 15240

agacacagat ttcattgaaa tgtctgcaaa gctgctagtc ttttgcactc gacgcgtggt 15300

ctcaggttta catgcaggga ataagtatga cctgctgttt ccatctgtct tggatgataa 15360

cctaagtgag aagatgcttc agttgatatc ccaattatgc tgtctgtata cggtgctctt 15420

tgctacaaca agagaaatcc cgaaaataag aggcttatct gcagaagaga aatgttcagt 15480

acttactgag tacctactgt cagatgctgt gaaaccgtta cttgggtccg agcaagtgag 15540

ctctatcatg tctcccaaca tagttacgtt cccagccaat ctgtattaca tgtctaggaa 15600

gagccttaat ttgatcaggg agagagagga cagggatact atcttggcat tgttgttccc 15660

tcaagaaccg ctgctcgagt ttcctctggt acgagatatt ggtgctcgtg taaaagatcc 15720

atttacccga caacctgcgg cgtttttaca agagttagat ttgagtgctc cggcaaggta 15780

tgacgcattc acaatcagtc aggcgcattc tgaacacata ttgccaaacc cagaggaaga 15840

tcacttagta cgatacttgt tcagaggaat agggactgcg tcctcctctt ggtataaggc 15900

atcccatctt ctttctgtac ccgaggtcag atgtgcaagg catgggaact ccttatattt 15960

agcagaagga agtggagcca tcatgagtct tctcgaattg catataccac acgaaactat 16020

ctattacaat acacttttct cgaacgagat gaacccccca cagcgacatt tcggaccaac 16080

tccaacacag tttctgaatt cggttgtttt taggaattta caggcggaag taccatgcaa 16140

ggatggattt gtccaggagt tccgtccgtt atggagagag aatacagaag aaagcgatct 16200

gacctcagat aaagcagtgg gatatatcac atctgtagtg ccgtacaggt ctgtatcatt 16260

gctgcattgt gacattgaaa tccctccagg atctaatcaa agcttactag atcagctggc 16320

taccaatctg tctctgattg ccatgcattc tgtgaaggag ggcggggtcg tgattatcaa 16380

agtactgtat gcaatgggat attacttcca tctactcatg aacttgttta ctccatgttc 16440

cacgaaagga tatattctct ctaatggcta tgcctgtaga ggggatatgg agtgttacct 16500

gatatttgtc atgggctacc taggcgggcc tacatttgta catgaggtgg tgaggatggc 16560

aaaaacgcta gtacagcggc atggcacact tttgtctaag tcagatgaga ttacactgac 16620

taggttattt acttcacagc aacagcgtgt aacagacatc ctatccagcc ctttaccgag 16680

actaatgaag tacttgagag agaatattga tactgcactg attgaagccg ggggacagcc 16740

tgtccgtcca ttctgtgcag agagtttagt gagcacacta acagacatga ctcagacaac 16800

ccagatcatc gccagccaca ttgatacagt cattcgatct gtgatctata tggaagcaga 16860

gggtgatctt gctgacacag tattcttatt taccccttac aacctctcta tggacgggaa 16920

aaagagaaca tcacttaaac agtgcacaag acagatctta gaggtcacaa tactgggtct 16980

cagagtcaaa aatctcaata aagtaggtga tgtaatcagc ctagtactca gaggtatgat 17040

ttctctggag gaccttctcc cactgagaac ctacttgaag tgtagtacct gccctaagta 17100

tttgaaggct gtcctaggta ttaccaaact caaagaaatg ttcacagacg cctctttatt 17160

atacttgact cgtgctcaac aaaaattcta catgaaaact ataggcaatg cagtcaaggg 17220

atactatagt aactgtgact cttaaaggca accacatatc aataggccct ctttctagcc 17280

gatcgtattc ttgttgactt cattatacca tattagaaaa aaattgaatt ccgacccttt 17340

aagactcgta ttcggattca aataattatc tcagaaaaaa agtgcacgta gttgttcttg 17400

attatagtcc cgtcattcac caagtctttg tttggt 17436

<210> 10
<211> 17430
<212> DNA
<213> Newcastle disease virus

<220>
<223> Mut HN (F277L)/M(G165W)-BMS 986178

<400> 10
   accaaacaga gaatccgtga gatacgataa aaggcgaagg agcaatcgaa gtcgtacggg 60
   tagaaggtgt gaatctcgag tgcgagaccg aagcttaaac tcgagagagc cttctgccaa 120
   catgtcttcc gtatttgacg agtatgaaca gctcctcgcg gctcagactc gccctaatgg 180
   agctcacgga ggaggagaga aggggagcac cttaaaagtt gaagtcccgg tattcactct 240
   taacagtgac gacccagaag atagatggaa ctttgcagta ttctgtctta ggattgctgt 300
   tagcgaagat gccaacaaac cactcagaca aggtgctctc atatctctct tatgctccca 360
   ttctcaagtg atgaggaacc atgtcgccct tgcagggaaa cagaatgagg ccacactggc 420
   tgttcttgag atcgatggtt tcaccaacag cgtgcctcaa tttaacaaca ggagtggagt 480
   gtctgaggag agagcacaga gattcttgat gatagcaggg tccctccctc gggcatgcag 540
   caacggtacc ccgttcatca cagctggggt tgaagatgat gcaccagaag acatcactga 600
   tactctggaa agaatcatct ctatccaggc tcaagtatgg gtcacggtag caaaggccat 660
   gactgcatat gagacagcgg atgagtcgga aacaagaaga atcaataagt atatgcagca 720
   aggcagagtc caaaagaagt acatcctcca ccccgtatgt aggagcgcaa ttcaactcac 780
   gattagacaa tctctggcag tccgcatttt cttggttagc gagcttaaga gaggccgcaa 840
   cacggcaggt ggaagttcca cttattatag cttggtaggg gacatagact catacatcag 900
   gaacaccggg cttactgcat tcttcctgac actcaggtat ggaattaaca ccaagacatc 960
   agcccttgca ctcagcagcc tcgcaggcga tatccaaaaa atgaagcagc tcatgcgtct 1020
   atatcggatg aaaggagata atgcgccgta catgacactg ctcggtgaca gtgaccagat 1080
   gagctttgca ccggctgagt atgcacaact ctactctttt gcaatgggta tggcatccgt 1140
   cctagacaaa ggaactggca agtaccagtt cgccagagac tttatgagca catcattctg 1200
   gagacttgga gtagagtatg ctcgggctca gggaagcagc atcaatgagg atatggctgc 1260
   cgagctaaag ctgaccccag cggcaaggag gggcctggca gctgctgccc aacgagcgtc 1320
   cgaggagacc ggcagcatgg acattcccac tcaacaagct ggggtcctca cagggctcag 1380
   cgacggaggc cctcaagccc cacaaggtgg actgaacagg tcgcaagggc aaccggacgc 1440
   cggagatggg gagacccaat ttttagattt gatgagagca gtggcaaata gtatgagaga 1500
   agcgccaaac tctgtgcaga acaccactca gcaggagcct ccatctactc ctgggccatc 1560
   tcaagacaac gacaccgact ggggatactg accgacaaca ctcagcctgc ctccatcgaa 1620
   ttatctcgat tcttctgcct gtaacctaac ccctggtcca cagtctcaca cggccaaacc 1680
   cgcaaacgag cccccccctc tctccccccc cctccagcca cacggctcca accgcccaaa 1740
   acaacacggg cacaactcga ctcactcata atctacacag agccgaagat cttagaaaaa 1800
   aatacgggta gaagagagac attcagagat caggacaaat catcagggtc tccgctctcc 1860
   cctctaccca gcagaccaga gtgaagatgg ccaccttcac ggatgcagag atcgacgatc 1920
   tatttgaaac cagtggaact gtcattgaca gcataattac ggcccaaggc aacccagtag 1980
   agactgttgg aaggagcgca atcccacaag gcaaaactaa agcattgagt gcagcatggg 2040
   agaaacatgg aagcacccaa ccgccggccg gccaagacac ccctgatcga atggacagat 2100
   cagacaaaca accatcccca cccgaacagg caaccacaca caacagcccg ccagccacat 2160
   ccaccgacca gccccccacc caggccgcag gcgaagccgg tgacacacag ctcaagactg 2220
   gagcaagcaa ctccctcctg tcgatgctcg acaagcttag caataaatcg tccaatgcta 2280
   aaaagggccc acggtcgagt ccccaggaag ggcaccacca acctccgacc cagcagcaag 2340
   ggagtcaaca gagccgcgga aacagtcagg agagatcgca aaaccaggtc aaggccgcct 2400
   ctggagacca gggcacagac gcgaacacag catatcatgg acaatggaag gagtcacaac 2460
   catcagctgg tgcaatccct catgctctcc ggtcagggca gagccaagcc aatactcctg 2520
   catctgtgga tcatgtccag ctacctgtcg actttgtgca ggcgatgatg tctatgatgg 2580
   aggcgatatc acagaaggta aataaagtcg actatcagct agaccttgtc tcaaagcaga 2640
   catcctccat ccccatgatg cgatctgaaa tccaacagct gaaaacatcc gttgcggtca 2700
   tggaagccaa cctgggcatg atgaagattc tggaccctgg ttgtgctaac gtttcatctt 2760
   taagtgacct acgggcggtc gcccgctctc acccagtttt agtttcaggc cccggagacc 2820
   catctcctta tgtgacacaa gggggtgaat tgatactcaa taaactctcg caaccagtgc 2880
   aacatccttc tgagttgatt aaacctgcca cgataagcgg gcctgatata ggagtggaga 2940
   aggacactgt ccgtgcattg atcacctcac gcccaatgca tccgagctct tcagctaagc 3000
   tcctgagtaa gctggatgca gccgggtcga tagaagaaat taggaaaatc aaacgccttg 3060
   cgctgaatgg ctgatcacta ctacaacccg caacgggttc ccgtttatct attgtcacaa 3120
   ggaacccgcc ccgaagctta agaaaaaata cgggtagaag cagtttgaga gctccgttcg 3180
   ccaccatgga gttcggcctg tcctgggtgt tcctggtggc cctgttccgc ggcgtgcagt 3240
   gcgaagtgca gctagtgcag agtgggggcg ggctagtgca gccagggggg tctcttcgct 3300
   tgtcttgcgc cggtagcggt tttacctttt catcgtatgc aatgtactgg gtgaggcaag 3360
   cccctggcaa gggattggag tgggtgagcg cgatagacac tgacgccggt accttctatg 3420
   cggatagtgt aagggggcgc ttcaccatta gtcgcgacaa cgccaagaac tctctttatc 3480
   tccagatgaa ttcactccga gctgaggaca ccgcagtcta cttttgtgct aggctggggg 3540
   aaggctactt ctttgattac tggggacagg gaaccttggt gactgtgagc tctgcctcca 3600
   cgaaaggtcc ttctgttttc cccttgcccc cctcctcaaa atctacctct ggaggtaccg 3660
   ccgctctagg ctgcctagta aaggattact tccctgaacc cgtgacggtg tcttggaaca 3720
   gtggtgctct tacaagtggc gtgcacacct ttcctgccgt gctccaaagt tctgggcttt 3780
   attctctgtc ctccgtggtg accgtgccca gcagctctct tggtacccag acttacatct 3840
   gtaatgttaa ccacaaaccc tcaaatacaa aggtggacaa gagagtggag ccaaagagct 3900
   gcgataaaac ccacacctgt cccccctgtc ccgcacctga gcttcttggg ggccctagtg 3960
   tgtttctgtt cccacctaag cccaaagaca ccctgatgat ctctaggacc ccggaggtta 4020
   cctgcgtagt tgtggacgtc tcccacgagg atcccgaggt gaaattcaat tggtacgtcg 4080
   acggcgttga agtccataac gccaagacca agcctcgcga agaacagtat aactctacct 4140
   atcgcgtggt gtcggtcctg actgtgcttc accaggactg gcttaacggg aaggaatata 4200
   aatgcaaggt cagcaataag gccctccccg ctccaattga gaagaccatt tccaaagcta 4260
   aaggccagcc acgggagccc caggtctaca ctctaccacc ctcccgcgag gagatgacca 4320
   aaaaccaggt gtccctgacc tgtttggtga agggatttta cccgtccgat atcgctgtgg 4380
   agtgggaaag taatggccaa cctgaaaaca actataagac aaccccgcca gtcctagata 4440
   gcgatggatc cttcttcctg tatagtaaac ttacagtgga taaaagtagg tggcagcagg 4500
   gtaacgtttt ttcgtgtagt gtgatgcatg aagcactcca caatcactat acccaaaaaa 4560
   gcctttcgct gtcccctggc cgccgcaagc gcggctccgg cgagggccgc ggctccctgc 4620
   tgacctgcgg cgacgtggag gagaaccccg gccccatgaa gtacctgctg cccaccgccg 4680
   ccgccggcct gctgctgctg gccgcccagc ccgccatggc cgagattgtg ctgacgcaga 4740
   gcccagcaac tttgagtctc tcgcccggcg agcgcgccac actttcctgt cgagcctccc 4800
   aatccgtgag cagttacctg gcatggtacc aacagaagcc tgggcaagcc cccagattgc 4860
   tgatttatga tgcctccaat cgagcaacag gaataccagc tcggttcagt gggagcggaa 4920
   gcggtacaga tttcactctg acaatcagct cgctggagcc agaggacttc gctgtttatt 4980
   actgccagca gcgttcgaac tggcccccaa cgtttggagg aggcaccaaa gtggaaataa 5040
   aacgaacagt agcagcccca tctgtcttca tcttcccccc cagcgatgaa cagctgaagt 5100
   caggcactgc ttccgtcgtc tgcctactga acaattttta tcctagagag gccaaggtcc 5160
   aatggaaggt agacaacgca ctccaatcag ggaactcaca agagtctgtc accgagcagg 5220
   actctaagga ctcgacatac tcattatcta gtaccctgac actgagcaag gccgactacg 5280
   aaaaacataa agtctacgcc tgtgaagtga cgcaccaggg actcagctct cccgtcacta 5340
   agagcttcaa tcggggggag tgttaatgag ctgaccgagc cctcctctat aaacccaaga 5400
   ttcaaggctc caagcgataa ccttctcccg cctcttccat cccactgaat gatcgcgcag 5460
   ctgcaattaa tctagcaaca ttaaagatta agaaaaaata cgggtagaat cggagtgcac 5520
   cgattgtgcc aagatggact catctaggac aattgggtta tactttgatt ctgcccttcc 5580
   ttctagcaac ctgttagcat tcccgattgt cttacgagat gtaggagatg gaaagaagca 5640
   aattactccg caatatagga tccggcgtct tgactcgtgg acagacagta aagaagactc 5700
   ggtattcatc actacctacg gattcatctt ccaggttggg aatgaagaag tcactgtcgg 5760
   catgatcaat gataatccca agcgagagtt actctccgct gcgatgctct gcctaggaag 5820
   cgtcccgaat gttggagatc ctgttgagct ggcaagggcc tgcctcacta tggtggtaac 5880
   atgcaagaag agtgcaacta atactgagag aatggtcttc tcagtagtgc aggcaccccg 5940
   ggtgctgcaa agctgtaggg tcgtggcaga caaatactcg tcagtgaatg cagttaagca 6000
   cgtgaaagca ccagagaaga tcccttggag tgaaacccta gagtacaagg tgaattttgt 6060
   ctctttgact gtggtgccga agaaggatgt ctacaagatc ccaaccgcag tattgaaggt 6120
   atccggctcg agcctgtata atcttgcgct caatgtcact attgatgtgg aggtagaccc 6180
   aaagagcccg ttagtcaagt ccctttctag gtccgacagt ggatactatg ctaatctctt 6240
   cttacatatc ggacttatgt ccactgtaga taagagggga aagaaagtga catttgacca 6300
   gctggagagg aagataagga gacttgatct atctgtcggg ctcagtgatg tgctcggacc 6360
   ttctgtgctt gtgaaggcga gaggtgcacg gactaggctg ctggcacctt tcttctctaa 6420
   cagcgggaca gcctgctacc ctatagcaaa tgcctctcct caggtagcta agatactctg 6480
   gagtcagacc gcgtgcctgc ggagtgtaaa aatcattatt caagcgggca cccaacgcgc 6540
   tgtcgcagtg actgctgacc atgaggttac ctctactaag atagagaaga ggcataccat 6600
   cgctaaatac aatcccttca agaaataggc cgtatccctg agactgcaat tcacccgcct 6660
   tcccaaaaca ccatgacacc agataatgat ctgtcttgat tacttacagt tagtttccct 6720
   gtctatcaaa ttagaaaaaa cacgggtaga agagttcgga tcccggccgg cgcaccaaaa 6780
   gcgcaagatg ggccccagat cttctaccag gatcccagta cctctaatgc tgaccatacg 6840
   gatcacgctg gcactgagtt atgtccgtct gacaagttct cttgatggca ggcctcttgc 6900
   agccgcaggg atcgtggtaa caggggataa agcagttaac atatacacct catcccagac 6960
   agggtcaatc atagtcaagt tactcccaaa tatgcccaag gacaaagagg catgtgcaaa 7020
   agccccattg gaggcttaca acaggacact gactactttg cttacccccc ttggtgattc 7080
   tatccgcagg atacaagagt ctgtgactac atccggagga aggagacaga gacgctttat 7140
   aggtgccatt attggcagtg tagctctagg ggttgcaaca gctgcacaga taacggcagc 7200
   ctcggctctg atacaagcca accagaatgc tgctaacatc ctccggctta aggagagcat 7260
   tgctgcaacc aatgaagctg tacacgaggt cactggcgga ttgtcacagt tagcagtggc 7320
   agttggaaag atgcagcaat ttgttaatga ccagtttaat aacacagctc aagagttgga 7380
   ctgtataaaa attacacagc aggttggtgt agaactcaac ttatacttaa ctgaattgac 7440
   tacagtgttc gggccacaaa tcacttcccc tgccttaact cagttgactg ttcaggctct 7500
   ttacaatctg gctggtggta atgtagatta cttgttgact aagttaggtg tagggaacaa 7560
   ccagctcagc tcattgattg gtagcggctt gatcaccggt aaccctattt tttacgactc 7620
   acagactcaa ctcttgggca tacaggtgac tttaccctca gtcgggaacc taaataatat 7680
   gcgtgccacc tacttggaga ccttgtctgt aagcacaaca aaggatttgg cctcagcact 7740
   tgtcccaaaa gtagtgacac aggtcggttc tgtgatagaa gagcttgaca cctcatactg 7800
   tatagaagct gatttggatt tatattgtac aagaatagtg acattcccta tgtcccctgg 7860
   tatttattcc tgtttgagcg gcaatacatc ggcttgcatg tattcaagga ctgaaggcgc 7920
   acttactaca ccatacatga ctctcaaagg ctcagttgtt gccaattgcc agatgacaac 7980
   atgtagatgt gcagaccccc cgggtatcat atcacaaaat tatggagagg ctgtgtctct 8040
   aatagataag cactcatgca atgtcgtatc cttagacggg ataactttga ggctcagtgg 8100
   ggaatttgat gcaacttatc aaaagaatat ctcaatatta gattctcaag tactagtgac 8160
   aggcaatctc gatatctcaa ctgagcttgg gaatgtcaac cactcaataa gtaatgcttt 8220
   ggataagtta gaggaaagca acagcaaact agacaaagtc aatgtcaaac tgaccagcac 8280
   atctgctctc attacctata ttgttttaac tgtcatatct cttgttcttg gtatgcttag 8340
   cctggttcta gcatgctatc tgatgtacaa gcaaaaagcg caacgaaaga ccttgttgtg 8400
   gcttggaaat aataccctag atcagatgag agctactaca aaaatgtgaa tgcagatgag 8460
   aggcagaggt atccccaata gcaatctgtg tgtcaattct ggcagcctgt taatcagaag 8520
   aattaagaaa aaactaccgg atgtaggtga acaaaaggga atatacgggt agaacggcct 8580
   gagaggccac ccctcaatcg ggagccaggc cccactacgt ccgctctacc gcatcaccaa 8640
   cagcagtctt cagtcatgga cagcgcagtt agccaagttg cgctagagaa tgatagaaga 8700
   gaagcgaata atacatggcg cttggttttc cggatcgcag ccttactttt actggtaata 8760
   accttagccg tctctgcagt cgccctggca tatagtatgg aggctagcac acctggcgac 8820
   cttgtaagca taccgactgc gatctctagg gcagaggaaa ggattacatc tgcactcggt 8880
   tccaatcaag atgtagtaga taggatatac aagcaagtgg cccttgagtc tccattggca 8940
   ctgctaaata ccgaatctat aattatgaat gcaataacgt ctctctctta tcaaatcaat 9000
   ggagccacaa ataatagcgg gtgtggggca cctgttcatg acccagatta catcgggggg 9060
   ataggtcaag aacttattgt agatgatacg agtgacgtca catcatttta tccctctgca 9120
   ttccaagaac acctgaattt tatcccggcg cccactacag gatcaggctg cactcggata 9180
   ccctcattcg acatgagtgc tacccattac tgttacactc acaatgtgat attgtctggc 9240
   tgcagagatc actcacactc acatcagtat ttagcacttg gtgtgcttcg gacatctgca 9300
   acagggaggg tattcttttc tactctgcgt tccatcaact tggatgacgc ccaaaatcgg 9360
   aagtcttgca gtgtgagtgc aactccttta ggttgtgata tgctgtgctc taaaatcaca 9420
   gagactgagg aagaggatta taaatcagtt atccccacat cgatggtaca tggaaggtta 9480
   gggcttgacg gccaatacca tgagaaggac ctagacgtca caacactatt tagggactgg 9540
   gtggcaaatt acccaggagt aggaggtggg tcttttatta acaatcgcgt atggttcccg 9600
   gtctacggag ggctaaaacc cagctcgcct agtgacactg cacaagaagg gagatacgta 9660
   atatacaagc gatacaatga cacatgcccg gatgagcaag attaccagat tcggatggct 9720
   aagtcatcgt ataagcctag gcggtttggt ggaaaacgcg tacagcaggc catcctatcc 9780
   atcaaggtgt caacatcctt gggtgaggac ccggtgttga ctgtaccgcc taatacggtc 9840
   gcactcatgg gggccgaagg cagagttctc acagtgggga catctcattt cttatatcag 9900
   cgagggtcat catacttctc tcccgctttg ttatacccta tgacagtcaa caacaaaaca 9960
   gccactcttc ataatcctta tacattcaat gctttcactc ggccaggtag tgtcccttgc 10020
   caggcttcag caagatgccc caactcatgt gttaccggag tctatactga tccatatccc 10080
   ttagtcttcc ataggaacca caccttgcga ggggtattcg gaacaatgct tgatgacgaa 10140
   caagcaagac tcaaccctgt atctgcagta tttgataaca tatcccgcag tcgcataact 10200
   cgggtaagtt caagcagtac cagggcagca tacacgacat caacgtgttt taaagttgtc 10260
   aagaccaata aaacctattg cctcagcatt gcagaaatat ccaataccct cttcggggaa 10320
   ttcagaattg tccccttact agttgagatt ctcaaggatg gtggggttta agaagctagg 10380
   aatggtcggt tgagtcaacc gtgagaaggc ggggaagatg atattgcatc atctatcttc 10440
   tgtaacacca agaatcaaat cgaataccag tgcgagctcg aatcctacgc tgccagtcag 10500
   ccataatcgg ctggtgctaa tgtggttagc ctgaatcttg tcgatagtca cttgattaag 10560
   aaaaaatgtg gacggtagtg aaatataagg caaagcagct cacagaggac agcacgggta 10620
   ggacatggcg ggctccggac ccgaaagggc agagcatcag attatcctac cagagtcaca 10680
   tctgtcctca ccattggtca agcataaatt gctttattac tggaaattaa ctgggctacc 10740
   gcttcctgac gaatgtgact tcgaccatct tattatcagc cgacaatgga agaaggtact 10800
   tgaatcggcc acccctgaca ttgagagaat gataaaacta gggcgggcag tacaccagac 10860
   tctcaaccac aattccagga taaccggagt actccatccc cggtgtttag aggaactggc 10920
   tagtattgag gttcctgatt caaccaacaa atttcggaag atcgaaaaaa agatccagat 10980
   tcacaacaca aggtatggag aactgttcac aagattatgc acgcatgtag aaaagaaact 11040
   attggggtca tcttggtcta acaatgtccc acggtcagag gaattcaaca gcatccgtac 11100
   agatccggca ttttggtttc actcaaaatg gtccacagcc aaatttgcat ggctccatat 11160
   aaaacaggtc cagaggcatc tgattgtagc agcaagaaca aggtccgcag tcaacaaatt 11220
   agtgacgctg acccataagg taggccaaat ctttgttact cctgagcttg tcattgtgac 11280
   acatacagat gagaacaagt tcacgtgtct tacccaggaa cttgtgttga tgtatgcaga 11340
   tatgatggag ggcagagata tggtcagcat aatatcatcc acggcggcac atcttaggag 11400
   cttatcagag aaaattgatg atattctgcg gttagtagat gctctggcaa gagatttggg 11460
   caatcaagtc tacgatgttg tagcactaat ggagggattc gcatacggcg ctgttcagct 11520
   gcttgaaccg tcaggtacat ttgcggggga tttcttcgca ttcaacctgc aggagctcaa 11580
   agatactcta accggactcc tccccaagga tatcgcagaa tctgtgactc acgcaatcgc 11640
   aaccatattc tctggcttag aacaaaatca agcagctgag atgttgtgcc tgttgcgtct 11700
   gtggggtcac ccactactcg agtcccgtat tgcagcaaaa gcagttagga gtcaaatgtg 11760
   cgcaccaaaa atggtagact ttgatatgat cctccaggta ttatctttct ttaaaggaac 11820
   aatcatcaat ggatatagaa agaagaatgc aggcgtgtgg ccacgtgtca agatagatac 11880
   gatatacggg aaggtcatag ggcagctaca cgcagattct gcagagattt cacatgatgt 11940
   catgttgagg gaatacaaga gtttatctgc acttgaattc gagccatgta tagagtatga 12000
   ccctgtcacc aatctgagca tgtttttaaa agacaaggca atcgcacacc cgaaagacaa 12060
   ctggctcgct tcgtttaggc gaaaccttct ctctgaggac cagaagaaac atgtaagaga 12120
   ggcaacctca actaaccgcc tcttgataga gttcttagag tcaaatgatt ttgatccata 12180
   taaagagatg gaatatctga cgacccttga gtacctaaga gatgataatg tggcagtatc 12240
   atactcactc aaagaaaaag aggtgaaagt taatgggcgg attttcgcaa agctaacaaa 12300
   tagattaagg aattgtcagg taatggcaga agggatccta gctgaccaga ttgcaccttt 12360
   cttccaggga aatggggtca ttcaggatag catatctttg actaaaagta tgctagcgat 12420
   gagtcaactg tctttcaaca gcaacaagaa acgtattact gactgcaaag aaagagtatc 12480
   ctcaaaccgc aatcatgatc cgaagagcaa gaatcgtcgg agagttgcca cttttgtaac 12540
   taccgacctg caaaagtatt gtctcaattg gagatatcag acagtcaagc tgttcgctca 12600
   tgccatcaat cagctgatgg gcttacctca cttcttcgag tggattcatc ttagactaat 12660
   ggatactacg atgtttgtag gggacccttt caatcctcca agtgacccta cggactgtga 12720
   tctatcaaga gtcccaaatg atgacatata tattgtcagt gctaggggag gcattgaggg 12780
   attatgccag aagctatgga caatgatctc aattgctgca atccagcttg ctgcagcaag 12840
   atcacattgt cgcgttgcct gtatggtaca aggcgacaat caagtaatag ctgtaacgag 12900
   agaggtaaga tcagatgact ccccagagat ggtgttaaca caattgcatc aagccagtga 12960
   taatttcttc aaggaattga ttcatgtcaa tcatttgatc ggccataatt tgaaggatcg 13020
   tgaaaccatc aggtcagaca cgttcttcat atacagcaaa cgaatattca aagatggagc 13080
   aatactcagt caggtcctca aaaactcatc taaattagtg ctaatatcag gcgaccttag 13140
   tgaaaacact gtaatgtctt gtgccaacat tgcatctact gtagcacggt tatgcgagaa 13200
   cgggcttcct aaggatttct gttattactt aaactactta atgagttgcg tgcagacata 13260
   cttcgattcc gaattttcca tcaccaacaa ctcgcaactc gattctaacc agtcgtggat 13320
   agaggacatt tcttttgtgc actcatatgt cctgacccct gctcaactgg ggggactgag 13380
   taaccttcaa tactcaaggc tctacacaag gaacatcggc gacccgggaa ccactgcttt 13440
   cgcagagatc aagagattag aggcagtggg gttactgagt cctagcatta tgactaacat 13500
   cttaactagg ccgcctggaa atggagattg ggccagtctg tgcaacgatc catactcctt 13560
   taattttgag actgtcgcaa gcccaaatat tgtccttaag aaacatacac aaagagtcct 13620
   atttgaaact tgctcgaatc ccttattatc tggagtgcac acagaggata atgaggcaga 13680
   agagaaggca ttggctgaat tcttgctcaa tcaagaaatg attcatccac gtgtcgcgca 13740
   tgctatcatg gaagcaagct ctgtaggtag gagaaagcaa attcaggggc ttgttgacac 13800
   aacaaacacc gtgattaaga ttgcactgac taggaggcca ctcggcatca agaggctgat 13860
   gcggatagtc aattactcga gcatgcatgc aatgctattt agagatgatg ttttctcgtc 13920
   taatagatcc aaccatccct tagtctcttc tgatatgtgt tctcagacac tggcagacta 13980
   tgcacggaac agaagctggt cacctttgac aggggtagaa aaaatactgg gtgtatctaa 14040
   tcctgatacc atagaacttg tagagggtga gatccttagt gtcagtggag ggtgcactag 14100
   atgtgacagt ggggatgaac agtttacttg gttccatctt ccaagcaata tagagctaaa 14160
   cgatgacacc agcaagaatc ctccaatgag agtgccatat ctcgggtcaa agactcaaga 14220
   gaggagagcc gcctcgcttg cgaaaatagc tcatatgtca ccacacgtga aggcggctct 14280
   aagggcgtca tctgtgttaa tctgggctta tggggacaac gaaataaact ggactgctgc 14340
   ccttaagatt gcaaggtctc ggtgcaacat aagctcagag tatcttcgac tattgtcacc 14400
   cttgcctaca gctgggaatc tccaacatag attggacgac ggcataactc agatgacatt 14460
   cacccctgca tctctataca gggtgtcacc ttacattcat atatctaatg attctcaaag 14520
   gctatttact gaagaaggag tcaaagaggg gaatgtggtt tatcagcaaa tcatgctctt 14580
   gggtttgtcc ttaattgagt cactcttccc aatgacaaca accaagacat atgatgaaat 14640
   cacattgcac ctccacagta aatttagctg ctgtatcagg gaagcacctg ttgcagttcc 14700
   tttcgagcta ctcggggtgg caccggaact aagggcagta acctcaaata agtttatgta 14760
   tgatcctagc cctgtatcag agggagactt tgcgagactt gacttagcta tctttaagag 14820
   ttatgaactt aatttagagt catattccac aatagagcta atgaacgttc tttcaatatc 14880
   tagtgggaag ttgattggcc agtctgtggt ttcttatgat gaagatacct ccataaagaa 14940
   tgacgctata atagtgtatg acaacacacg gaattggatc agcgaagctc agaattcaga 15000
   tgtggtccgc ctattcgagt atgcggcact cgaagtgctc ctcgactgtt cttatcaact 15060
   ctactatctg agagtaagag gcctaaacaa tatcgtcctg tacatgagtg atttatacaa 15120
   gaatatgcca ggaattctac tctctaatat tgcagccaca atatctcacc ctgtcatcca 15180
   ttcaaggttg aatgcagtag gtctggtcaa ccatgacggg tcacaccaac ttgcagacac 15240
   agatttcatt gaaatgtctg caaagctgct agtcttttgc actcgacgcg tggtctcagg 15300
   tttacatgca gggaataagt atgacctgct gtttccatct gtcttggatg ataacctaag 15360
   tgagaagatg cttcagttga tatcccaatt atgctgtctg tatacggtgc tctttgctac 15420
   aacaagagaa atcccgaaaa taagaggctt atctgcagaa gagaaatgtt cagtacttac 15480
   tgagtaccta ctgtcagatg ctgtgaaacc gttacttggg tccgagcaag tgagctctat 15540
   catgtctccc aacatagtta cgttcccagc caatctgtat tacatgtcta ggaagagcct 15600
   taatttgatc agggagagag aggacaggga tactatcttg gcattgttgt tccctcaaga 15660
   accgctgctc gagtttcctc tggtacgaga tattggtgct cgtgtaaaag atccatttac 15720
   ccgacaacct gcggcgtttt tacaagagtt agatttgagt gctccggcaa ggtatgacgc 15780
   attcacaatc agtcaggcgc attctgaaca catattgcca aacccagagg aagatcactt 15840
   agtacgatac ttgttcagag gaatagggac tgcgtcctcc tcttggtata aggcatccca 15900
   tcttctttct gtacccgagg tcagatgtgc aaggcatggg aactccttat atttagcaga 15960
   aggaagtgga gccatcatga gtcttctcga attgcatata ccacacgaaa ctatctatta 16020
   caatacactt ttctcgaacg agatgaaccc cccacagcga catttcggac caactccaac 16080
   acagtttctg aattcggttg tttttaggaa tttacaggcg gaagtaccat gcaaggatgg 16140
   atttgtccag gagttccgtc cgttatggag agagaataca gaagaaagcg atctgacctc 16200
   agataaagca gtgggatata tcacatctgt agtgccgtac aggtctgtat cattgctgca 16260
   ttgtgacatt gaaatccctc caggatctaa tcaaagctta ctagatcagc tggctaccaa 16320
   tctgtctctg attgccatgc attctgtgaa ggagggcggg gtcgtgatta tcaaagtact 16380
   gtatgcaatg ggatattact tccatctact catgaacttg tttactccat gttccacgaa 16440
   aggatatatt ctctctaatg gctatgcctg tagaggggat atggagtgtt acctgatatt 16500
   tgtcatgggc tacctaggcg ggcctacatt tgtacatgag gtggtgagga tggcaaaaac 16560
   gctagtacag cggcatggca cacttttgtc taagtcagat gagattacac tgactaggtt 16620
   atttacttca cagcaacagc gtgtaacaga catcctatcc agccctttac cgagactaat 16680
   gaagtacttg agagagaata ttgatactgc actgattgaa gccgggggac agcctgtccg 16740
   tccattctgt gcagagagtt tagtgagcac actaacagac atgactcaga caacccagat 16800
   catcgccagc cacattgata cagtcattcg atctgtgatc tatatggaag cagagggtga 16860
   tcttgctgac acagtattct tatttacccc ttacaacctc tctatggacg ggaaaaagag 16920
   aacatcactt aaacagtgca caagacagat cttagaggtc acaatactgg gtctcagagt 16980
   caaaaatctc aataaagtag gtgatgtaat cagcctagta ctcagaggta tgatttctct 17040
   ggaggacctt ctcccactga gaacctactt gaagtgtagt acctgcccta agtatttgaa 17100
   ggctgtccta ggtattacca aactcaaaga aatgttcaca gacgcctctt tattatactt 17160
   gactcgtgct caacaaaaat tctacatgaa aactataggc aatgcagtca agggatacta 17220
   tagtaactgt gactcttaaa ggcaaccaca tatcaatagg ccctctttct agccgatcgt 17280
   attcttgttg acttcattat accatattag aaaaaaattg aattccgacc ctttaagact 17340
   cgtattcgga ttcaaataat tatctcagaa aaaaagtgca cgtagttgtt cttgattata 17400
   gtcccgtcat tcaccaagtc tttgtttggt 17430

<210> 11
<211> 16122
<212> DNA
<213> Newcastle disease virus

<220>
<223> Mut HN (F277L)/M(G165W)-CD80

<400> 11
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag tggctttcac ttttgaccct 3180

aagcatctgc caccatgggc cacacacgga ggcagggaac atcaccatcc aagtgtccat 3240

acctcaattt ctttcagctc ttggtgctgg ctggtctttc tcacttctgt tcaggtgtta 3300

tccacgtgac caaggaagtg aaagaagtgg caacgctgtc ctgtggtcac aatgtttctg 3360

ttgaagagct ggcacaaact cgcatctact ggcaaaagga gaagaaaatg gtgctgacta 3420

tgatgtctgg ggacatgaat atatggcccg agtacaagaa ccggaccatc tttgatatca 3480

ctaataacct ctccattgtg atcctggctc tgcgcccatc tgacgagggc acatacgagt 3540

gtgttgttct gaagtatgaa aaagacgctt tcaagcggga acacctggct gaagtgacgt 3600

tatcagtcaa agctgacttc cctacaccta gtatatctga ctttgaaatt ccaacttcta 3660

atattagaag gataatttgc tcaacctctg gaggttttcc agagcctcac ctctcctggt 3720

tggaaaatgg agaagaatta aatgccatca acacaacagt ttcccaagat cctgaaactg 3780

agctctatgc tgttagcagc aaactggatt tcaatatgac aaccaaccac agcttcatgt 3840

gtctcatcaa gtatggacat ttaagagtga atcagacctt caactggaat acaaccaagc 3900

aagagcattt tcctgataac ctgctcccat cctgggccat taccttaatc tcagtaaatg 3960

gaatttttgt gatatgctgc ctgacctact gctttgcccc aagatgcaga gagagaagga 4020

ggaatgagag attgagaagg gaaagtgtac gccctgtata agctgaccga gccctcctct 4080

ataaacccaa gattcaaggc tccaagcgat aaccttctcc cgcctcttcc atcccactga 4140

atgatcgcgc agctgcaatt aatctagcaa cattaaagat taagaaaaaa tacgggtaga 4200

atcggagtgc accgattgtg ccaagatgga ctcatctagg acaattgggt tatactttga 4260

ttctgccctt ccttctagca acctgttagc attcccgatt gtcttacgag atgtaggaga 4320

tggaaagaag caaattactc cgcaatatag gatccggcgt cttgactcgt ggacagacag 4380

taaagaagac tcggtattca tcactaccta cggattcatc ttccaggttg ggaatgaaga 4440

agtcactgtc ggcatgatca atgataatcc caagcgagag ttactctccg ctgcgatgct 4500

ctgcctagga agcgtcccga atgttggaga tcctgttgag ctggcaaggg cctgcctcac 4560

tatggtggta acatgcaaga agagtgcaac taatactgag agaatggtct tctcagtagt 4620

gcaggcaccc cgggtgctgc aaagctgtag ggtcgtggca gacaaatact cgtcagtgaa 4680

tgcagttaag cacgtgaaag caccagagaa gatcccttgg agtgaaaccc tagagtacaa 4740

ggtgaatttt gtctctttga ctgtggtgcc gaagaaggat gtctacaaga tcccaaccgc 4800

agtattgaag gtatccggct cgagcctgta taatcttgcg ctcaatgtca ctattgatgt 4860

ggaggtagac ccaaagagcc cgttagtcaa gtccctttct aggtccgaca gtggatacta 4920

tgctaatctc ttcttacata tcggacttat gtccactgta gataagaggg gaaagaaagt 4980

gacatttgac cagctggaga ggaagataag gagacttgat ctatctgtcg ggctcagtga 5040

tgtgctcgga ccttctgtgc ttgtgaaggc gagaggtgca cggactaggc tgctggcacc 5100

tttcttctct aacagcggga cagcctgcta ccctatagca aatgcctctc ctcaggtagc 5160

taagatactc tggagtcaga ccgcgtgcct gcggagtgta aaaatcatta ttcaagcggg 5220

cacccaacgc gctgtcgcag tgactgctga ccatgaggtt acctctacta agatagagaa 5280

gaggcatacc atcgctaaat acaatccctt caagaaatag gccgtatccc tgagactgca 5340

attcacccgc cttcccaaaa caccatgaca ccagataatg atctgtcttg attacttaca 5400

gttagtttcc ctgtctatca aattagaaaa aacacgggta gaagagttcg gatcccggcc 5460

ggcgcaccaa aagcgcaaga tgggccccag atcttctacc aggatcccag tacctctaat 5520

gctgaccata cggatcacgc tggcactgag ttatgtccgt ctgacaagtt ctcttgatgg 5580

caggcctctt gcagccgcag ggatcgtggt aacaggggat aaagcagtta acatatacac 5640

ctcatcccag acagggtcaa tcatagtcaa gttactccca aatatgccca aggacaaaga 5700

ggcatgtgca aaagccccat tggaggctta caacaggaca ctgactactt tgcttacccc 5760

ccttggtgat tctatccgca ggatacaaga gtctgtgact acatccggag gaaggagaca 5820

gagacgcttt ataggtgcca ttattggcag tgtagctcta ggggttgcaa cagctgcaca 5880

gataacggca gcctcggctc tgatacaagc caaccagaat gctgctaaca tcctccggct 5940

taaggagagc attgctgcaa ccaatgaagc tgtacacgag gtcactggcg gattgtcaca 6000

gttagcagtg gcagttggaa agatgcagca atttgttaat gaccagttta ataacacagc 6060

tcaagagttg gactgtataa aaattacaca gcaggttggt gtagaactca acttatactt 6120

aactgaattg actacagtgt tcgggccaca aatcacttcc cctgccttaa ctcagttgac 6180

tgttcaggct ctttacaatc tggctggtgg taatgtagat tacttgttga ctaagttagg 6240

tgtagggaac aaccagctca gctcattgat tggtagcggc ttgatcaccg gtaaccctat 6300

tttttacgac tcacagactc aactcttggg catacaggtg actttaccct cagtcgggaa 6360

cctaaataat atgcgtgcca cctacttgga gaccttgtct gtaagcacaa caaagggatt 6420

tgcctcagca cttgtcccaa aagtagtgac acaggtcggt tctgtgatag aagagcttga 6480

cacctcatac tgtatagaag ctgatttgga tttatattgt acaagaatag tgacattccc 6540

tatgtcccct ggtatttatt cctgtttgag cggcaataca tcggcttgca tgtattcaag 6600

gactgaaggc gcacttacta caccatacat gactctcaaa ggctcagttg ttgccaattg 6660

ccagatgaca acatgtagat gtgcagaccc cccgggtatc atatcacaaa attatggaga 6720

ggctgtgtct ctaatagata agcactcatg caatgtcgta tccttagacg ggataacttt 6780

gaggctcagt ggggaatttg atgcaactta tcaaaagaat atctcaatat tagattctca 6840

agtactagtg acaggcaatc tcgatatctc aactgagctt gggaatgtca accactcaat 6900

aagtaatgct ttggataagt tagaggaaag caacagcaaa ctagacaaag tcaatgtcaa 6960

actgaccagc acatctgctc tcattaccta tattgtttta actgtcatat ctcttgttct 7020

tggtatgctt agcctggttc tagcatgcta tctgatgtac aagcaaaaag cgcaacgaaa 7080

gaccttgttg tggcttggaa ataataccct agatcagatg agagctacta caaaaatgtg 7140

aatgcagatg agaggcagag gtatccccaa tagcaatctg tgtgtcaatt ctggcagcct 7200

gttaatcaga agaattaaga aaaaactacc ggatgtaggt gaacaaaagg gaatatacgg 7260

gtagaacggc ctgagaggcc acccctcaat cgggagccag gccccactac gtccgctcta 7320

ccgcatcacc aacagcagtc ttcagtcatg gacagcgcag ttagccaagt tgcgctagag 7380

aatgatagaa gagaagcgaa taatacatgg cgcttggttt tccggatcgc agccttactt 7440
ttactggtaa taaccttagc cgtctctgca gtcgccctgg catatagtat ggaggctagc 7500
acacctggcg accttgtaag cataccgact gcgatctcta gggcagagga aaggattaca 7560
tctgcactcg gttccaatca agatgtagta gataggatat acaagcaagt ggcccttgag 7620
tctccattgg cactgctaaa taccgaatct ataattatga atgcaataac gtctctctct 7680
tatcaaatca atggagccac aaataatagc gggtgtgggg cacctgttca tgacccagat 7740
tacatcgggg ggataggtca agaacttatt gtagatgata cgagtgacgt cacatcattt 7800
tatccctctg cattccaaga acacctgaat tttatcccgg cgcccactac aggatcaggc 7860
tgcactcgga taccctcatt cgacatgagt gctacccatt actgttacac tcacaatgtg 7920
atattgtctg gctgcagaga tcactcacac tcacatcagt atttagcact tggtgtgctt 7980
cggacatctg caacagggag ggtattcttt tctactctgc gttccatcaa cttggatgac 8040
gcccaaaatc ggaagtcttg cagtgtgagt gcaactcctt taggttgtga tatgctgtgc 8100
tctaaaatca cagagactga ggaagaggat tataaatcag ttatccccac atcgatggta 8160
catggaaggt tagggcttga cggccaatac catgagaagg acctagacgt cacaacacta 8220
tttagggact gggtggcaaa ttacccagga gtaggaggtg ggtcttttat taacaatcgc 8280
gtatggttcc cggtctacgg agggctaaaa cccagctcgc ctagtgacac tgcacaagaa 8340
gggagatacg taatatacaa gcgatacaat gacacatgcc cggatgagca agattaccag 8400
attcggatgg ctaagtcatc gtataagcct aggcggtttg gtggaaaacg cgtacagcag 8460
gccatcctat ccatcaaggt gtcaacatcc ttgggtgagg acccggtgtt gactgtaccg 8520
cctaatacgg tcgcactcat gggggccgaa ggcagagttc tcacagtggg gacatctcat 8580
ttcttatatc agcgagggtc atcatacttc tctcccgctt tgttataccc tatgacagtc 8640
aacaacaaaa cagccactct tcataatcct tatacattca atgctttcac tcggccaggt 8700
agtgtccctt gccaggcttc agcaagatgc cccaactcat gtgttaccgg agtctatact 8760
gatccatatc ccttagtctt ccataggaac cacaccttgc gaggggtatt cggaacaatg 8820
cttgatgacg aacaagcaag actcaaccct gtatctgcag tatttgataa catatcccgc 8880
agtcgcataa ctcgggtaag ttcaagcagt accagggcag catacacgac atcaacgtgt 8940
tttaaagttg tcaagaccaa taaaacctat tgcctcagca ttgcagaaat atccaatacc 9000
ctcttcgggg aattcagaat tgtcccctta ctagttgaga ttctcaagga tggtggggtt 9060
taagaagcta ggaatggtcg gttgagtcaa ccgtgagaag gcggggaaga tgatattgca 9120
tcatctatct tctgtaacac caagaatcaa atcgaatacc agtgcgagct cgaatcctac 9180
gctgccagtc agccataatc ggctggtgct aatgtggtta gcctgaatct tgtcgatagt 9240
cacttgatta agaaaaaatg tggacggtag tgaaatataa ggcaaagcag ctcacagagg 9300

acagcacggg taggacatgg cgggctccgg acccgaaagg gcagagcatc agattatcct 9360

accagagtca catctgtcct caccattggt caagcataaa ttgctttatt actggaaatt 9420

aactgggcta ccgcttcctg acgaatgtga cttcgaccat cttattatca gccgacaatg 9480

gaagaaggta cttgaatcgg ccacccctga cattgagaga atgataaaac tagggcgggc 9540

agtacaccag actctcaacc acaattccag gataaccgga gtactccatc cccggtgttt 9600

agaggaactg gctagtattg aggttcctga ttcaaccaac aaatttcgga agatcgaaaa 9660

aaagatccag attcacaaca caaggtatgg agaactgttc acaagattat gcacgcatgt 9720

agaaaagaaa ctattggggt catcttggtc taacaatgtc ccacggtcag aggaattcaa 9780

cagcatccgt acagatccgg cattttggtt tcactcaaaa tggtccacag ccaaatttgc 9840

atggctccat ataaaacagg tccagaggca tctgattgta gcagcaagaa caaggtccgc 9900

agtcaacaaa ttagtgacgc tgacccataa ggtaggccaa atctttgtta ctcctgagct 9960

tgtcattgtg acacatacag atgagaacaa gttcacgtgt cttacccagg aacttgtgtt 10020

gatgtatgca gatatgatgg agggcagaga tatggtcagc ataatatcat ccacggcggc 10080

acatcttagg agcttatcag agaaaattga tgatattctg cggttagtag atgctctggc 10140

aagagatttg ggcaatcaag tctacgatgt tgtagcacta atggagggat tcgcatacgg 10200

cgctgttcag ctgcttgaac cgtcaggtac atttgcgggg gatttcttcg cattcaacct 10260

gcaggagctc aaagatactc taaccggact cctccccaag gatatcgcag aatctgtgac 10320

tcacgcaatc gcaaccatat tctctggctt agaacaaaat caagcagctg agatgttgtg 10380

cctgttgcgt ctgtggggtc acccactact cgagtcccgt attgcagcaa aagcagttag 10440

gagtcaaatg tgcgcaccaa aaatggtaga ctttgatatg atcctccagg tattatcttt 10500

ctttaaagga acaatcatca atggatatag aaagaagaat gcaggcgtgt ggccacgtgt 10560

caagatagat acgatatacg ggaaggtcat agggcagcta cacgcagatt ctgcagagat 10620

ttcacatgat gtcatgttga gggaatacaa gagtttatct gcacttgaat tcgagccatg 10680

tatagagtat gaccctgtca ccaatctgag catgttttta aaagacaagg caatcgcaca 10740

cccgaaagac aactggctcg cttcgtttag gcgaaacctt ctctctgagg accagaagaa 10800

acatgtaaga gaggcaacct caactaaccg cctcttgata gagttcttag agtcaaatga 10860

ttttgatcca tataaagaga tggaatatct gacgaccctt gagtacctaa gagatgataa 10920

tgtggcagta tcatactcac tcaaagaaaa agaggtgaaa gttaatgggc ggattttcgc 10980

aaagctaaca aatagattaa ggaattgtca ggtaatggca gaagggatcc tagctgacca 11040

gattgcacct ttcttccagg gaaatggggt cattcaggat agcatatctt tgactaaaag 11100

tatgctagcg atgagtcaac tgtctttcaa cagcaacaag aaacgtatta ctgactgcaa 11160

agaaagagta tcctcaaacc gcaatcatga tccgaagagc aagaatcgtc ggagagttgc 11220

cacttttgta actaccgacc tgcaaaagta ttgtctcaat tggagatatc agacagtcaa 11280

gctgttcgct catgccatca atcagctgat gggcttacct cacttcttcg agtggattca 11340

tcttagacta atggatacta cgatgtttgt aggggaccct ttcaatcctc caagtgaccc 11400

tacggactgt gatctatcaa gagtcccaaa tgatgacata tatattgtca gtgctagggg 11460

aggcattgag ggattatgcc agaagctatg gacaatgatc tcaattgctg caatccagct 11520

tgctgcagca agatcacatt gtcgcgttgc ctgtatggta caaggcgaca atcaagtaat 11580

agctgtaacg agagaggtaa gatcagatga ctccccagag atggtgttaa cacaattgca 11640

tcaagccagt gataatttct tcaaggaatt gattcatgtc aatcatttga tcggccataa 11700

tttgaaggat cgtgaaacca tcaggtcaga cacgttcttc atatacagca aacgaatatt 11760

caaagatgga gcaatactca gtcaggtcct caaaaactca tctaaattag tgctaatatc 11820

aggcgacctt agtgaaaaca ctgtaatgtc ttgtgccaac attgcatcta ctgtagcacg 11880

gttatgcgag aacgggcttc ctaaggattt ctgttattac ttaaactact taatgagttg 11940

cgtgcagaca tacttcgatt ccgaattttc catcaccaac aactcgcaac tcgattctaa 12000

ccagtcgtgg atagaggaca tttcttttgt gcactcatat gtcctgaccc ctgctcaact 12060

ggggggactg agtaaccttc aatactcaag gctctacaca aggaacatcg gcgacccggg 12120

aaccactgct ttcgcagaga tcaagagatt agaggcagtg gggttactga gtcctagcat 12180

tatgactaac atcttaacta ggccgcctgg aaatggagat tgggccagtc tgtgcaacga 12240

tccatactcc tttaattttg agactgtcgc aagcccaaat attgtcctta agaaacatac 12300

acaaagagtc ctatttgaaa cttgctcgaa tcccttatta tctggagtgc acacagagga 12360

taatgaggca gaagagaagg cattggctga attcttgctc aatcaagaaa tgattcatcc 12420

acgtgtcgcg catgctatca tggaagcaag ctctgtaggt aggagaaagc aaattcaggg 12480

gcttgttgac acaacaaaca ccgtgattaa gattgcactg actaggaggc cactcggcat 12540

caagaggctg atgcggatag tcaattactc gagcatgcat gcaatgctat ttagagatga 12600

tgttttctcg tctaatagat ccaaccatcc cttagtctct tctgatatgt gttctcagac 12660

actggcagac tatgcacgga acagaagctg gtcacctttg acagggggta gaaaaatact 12720

gggtgtatct aatcctgata ccatagaact tgtagagggt gagatcctta gtgtcagtgg 12780

agggtgcact agatgtgaca gtggggatga acagtttact tggttccatc ttccaagcaa 12840

tatagagcta aacgatgaca ccagcaagaa tcctccaatg agagtgccat atctcgggtc 12900

aaagactcaa gagaggagag ccgcctcgct tgcgaaaata gctcatatgt caccacacgt 12960

gaaggcggct ctaaggcgtc catctgtgtt aatctgggct tatggggaca acgaaataaa 13020

ctggactgct gcccttaaga ttgcaaggtc tcggtgcaac ataagctcag agtatcttcg 13080

actattgtca cccttgccta cagctgggaa tctccaacat agattggacg acggcataac 13140

tcagatgaca ttcacccctg catctctata cagggtgtca ccttacattc atatatctaa 13200

tgattctcaa aggctattta ctgaagaagg agtcaaagag gggaatgtgg tttatcagca 13260

aatcatgctc ttgggtttgt ccttaattga gtcactcttc ccaatgacaa caaccaagac 13320

atatgatgaa atcacattgc acctccacag taaatttagc tgctgtatca gggaagcacc 13380

tgttgcagtt cctttcgagc tactcggggt ggcaccggaa ctaagggcag taacctcaaa 13440

taagtttatg tatgatccta gccctgtatc agagggagac tttgcgagac ttgacttagc 13500

tatctttaag agttatgaac ttaatttaga gtcatattcc acaatagagc taatgaacgt 13560

tctttcaata tctagtggga agttgattgg ccagtctgtg gtttcttatg atgaagatac 13620

ctccataaag aatgacgcta taatagtgta tgacaacaca cggaattgga tcagcgaagc 13680

tcagaattca gatgtggtcc gcctattcga gtatgcggca ctcgaagtgc tcctcgactg 13740

ttcttatcaa ctctactatc tgagagtaag aggcctaaac aatatcgtcc tgtacatgag 13800

tgatttatac aagaatatgc caggaattct actctctaat attgcagcca caatatctca 13860

ccctgtcatc cattcaaggt tgaatgcagt aggtctggtc aaccatgacg ggtcacacca 13920

acttgcagac acagatttca ttgaaatgtc tgcaaagctg ctagtctttt gcactcgacg 13980

cgtggtctca ggtttacatg cagggaataa gtatgacctg ctgtttccat ctgtcttgga 14040

tgataaccta agtgagaaga tgcttcagtt gatatcccaa ttatgctgtc tgtatacggt 14100

gctctttgct acaacaagag aaatcccgaa aataagaggc ttatctgcag aagagaaatg 14160

ttcagtactt actgagtacc tactgtcaga tgctgtgaaa ccgttacttg ggtccgagca 14220

agtgagctct atcatgtctc ccaacatagt tacgttccca gccaatctgt attacatgtc 14280

taggaagagc cttaatttga tcagggagag agaggacagg gatactatct tggcattgtt 14340

gttccctcaa gaaccgctgc tcgagtttcc tctggtacga gatattggtg ctcgtgtaaa 14400

agatccattt acccgacaac ctgcggcgtt tttacaagag ttagatttga gtgctccggc 14460

aaggtatgac gcattcacaa tcagtcaggc gcattctgaa cacatattgc caaacccaga 14520

ggaagatcac ttagtacgat acttgttcag aggaataggg actgcgtcct cctcttggta 14580

taaggcatcc catcttcttt ctgtacccga ggtcagatgt gcaaggcatg ggaactcctt 14640

atatttagca gaaggaagtg gagccatcat gagtcttctc gaattgcata taccacacga 14700

aactatctat tacaatacac ttttctcgaa cgagatgaac cccccacagc gacatttcgg 14760

accaactcca acacagtttc tgaattcggt tgtttttagg aatttacagg cggaagtacc 14820

atgcaaggat ggatttgtcc aggagttccg tccgttatgg agagagaata cagaagaaag 14880

cgatctgacc tcagataaag cagtgggata tatcacatct gtagtgccgt acaggtctgt 14940

atcattgctg cattgtgaca ttgaaatccc tccaggatct aatcaaagct tactagatca 15000

gctggctacc aatctgtctc tgattgccat gcattctgtg aaggagggcg gggtcgtgat 15060

tatcaaagta ctgtatgcaa tgggatatta cttccatcta ctcatgaact tgtttactcc 15120

atgttccacg aaaggatata ttctctctaa tggctatgcc tgtagagggg atatggagtg 15180

ttacctgata tttgtcatgg gctacctagg cgggcctaca tttgtacatg aggtggtgag 15240

gatggcaaaa acgctagtac agcggcatgg cacacttttg tctaagtcag atgagattac 15300

actgactagg ttatttactt cacagcaaca gcgtgtaaca gacatcctat ccagcccttt 15360

accgagacta atgaagtact tgagagagaa tattgatact gcactgattg aagccggggg 15420

acagcctgtc cgtccattct gtgcagagag tttagtgagc acactaacag acatgactca 15480

gacaacccag atcatcgcca gccacattga tacagtcatt cgatctgtga tctatatgga 15540

agcagagggt gatcttgctg acacagtatt cttatttacc ccttacaacc tctctatgga 15600

cgggaaaaag agaacatcac ttaaacagtg cacaagacag atcttagagg tcacaatact 15660

gggtctcaga gtcaaaaatc tcaataaagt aggtgatgta atcagcctag tactcagagg 15720

tatgatttct ctggaggacc ttctcccact gagaacctac ttgaagtgta gtacctgccc 15780

taagtatttg aaggctgtcc taggtattac caaactcaaa gaaatgttca cagacgcctc 15840

tttattatac ttgactcgtg ctcaacaaaa attctacatg aaaactatag gcaatgcagt 15900

caagggatac tatagtaact gtgactctta aaggcaacca catatcaata ggccctcttt 15960

ctagccgatc gtattcttgt tgacttcatt ataccatatt agaaaaaaat tgaattccga 16020

ccctttaaga ctcgtattcg gattcaaata attatctcag aaaaaaagtg cacgtagttg 16080

ttcttgatta tagtcccgtc attcaccaag tctttgtttg gt 16122

<210> 12
<211> 16824
<212> DNA
<213> Newcastle disease virus

<220>
<223> Mut HN (F277L)/M(G165W)-(ns)hIL-12

<400> 12
   accaaacaga gaatccgtga gatacgataa aaggcgaagg agcaatcgaa gtcgtacggg 60
   tagaaggtgt gaatctcgag tgcgagaccg aagcttaaac tcgagagagc cttctgccaa 120
   catgtcttcc gtatttgacg agtatgaaca gctcctcgcg gctcagactc gccctaatgg 180
   agctcacgga ggaggagaga aggggagcac cttaaaagtt gaagtcccgg tattcactct 240
   taacagtgac gacccagaag atagatggaa ctttgcagta ttctgtctta ggattgctgt 300
   tagcgaagat gccaacaaac cactcagaca aggtgctctc atatctctct tatgctccca 360
   ttctcaagtg atgaggaacc atgtcgccct tgcagggaaa cagaatgagg ccacactggc 420
   tgttcttgag atcgatggtt tcaccaacag cgtgcctcaa tttaacaaca ggagtggagt 480
   gtctgaggag agagcacaga gattcttgat gatagcaggg tccctccctc gggcatgcag 540
   caacggtacc ccgttcatca cagctggggt tgaagatgat gcaccagaag acatcactga 600
   tactctggaa agaatcatct ctatccaggc tcaagtatgg gtcacggtag caaaggccat 660
   gactgcatat gagacagcgg atgagtcgga aacaagaaga atcaataagt atatgcagca 720
   aggcagagtc caaaagaagt acatcctcca ccccgtatgt aggagcgcaa ttcaactcac 780
   gattagacaa tctctggcag tccgcatttt cttggttagc gagcttaaga gaggccgcaa 840
   cacggcaggt ggaagttcca cttattatag cttggtaggg gacatagact catacatcag 900
   gaacaccggg cttactgcat tcttcctgac actcaggtat ggaattaaca ccaagacatc 960
   agcccttgca ctcagcagcc tcgcaggcga tatccaaaaa atgaagcagc tcatgcgtct 1020
   atatcggatg aaaggagata atgcgccgta catgacactg ctcggtgaca gtgaccagat 1080
   gagctttgca ccggctgagt atgcacaact ctactctttt gcaatgggta tggcatccgt 1140
   cctagacaaa ggaactggca agtaccagtt cgccagagac tttatgagca catcattctg 1200
   gagacttgga gtagagtatg ctcggctcag gggaagcagc atcaatgagg atatggctgc 1260
   cgagctaaag ctgaccccag cggcaaggag gggcctggca gctgctgccc aacgagcgtc 1320
   cgaggagacc ggcagcatgg acattcccac tcaacaagct ggggtcctca cagggctcag 1380
   cgacggaggc cctcaagccc cacaaggtgg actgaacagg tcgcaagggc aaccggacgc 1440
   cggagatggg gagacccaat ttttagattt gatgagagca gtggcaaata gtatgagaga 1500
   agcgccaaac tctgtgcaga acaccactca gcaggagcct ccatctactc ctgggccatc 1560
   tcaagacaac gacaccgact ggggatactg accgacaaca ctcagcctgc ctccatcgaa 1620
   ttatctcgat tcttctgcct gtaacctaac ccctggtcca cagtctcaca cggccaaacc 1680
   cgcaaacgag cccccccctc tctccccccc cctccagcca cacggctcca accgcccaaa 1740
   acaacacggg cacaactcga ctcactcata atctacacag agccgaagat cttagaaaaa 1800
   aatacgggta gaagagagac attcagagat caggacaaat catcagggtc tccgctctcc 1860
   cctctaccca gcagaccaga gtgaagatgg ccaccttcac ggatgcagag atcgacgatc 1920
   tatttgaaac cagtggaact gtcattgaca gcataattac ggcccaaggc aacccagtag 1980
   agactgttgg aaggagcgca atcccacaag gcaaaactaa agcattgagt gcagcatggg 2040
   agaaacatgg aagcacccaa ccgccggccg gccaagacac ccctgatcga atggacagat 2100
   cagacaaaca accatcccca cccgaacagg caaccacaca caacagcccg ccagccacat 2160
   ccaccgacca gccccccacc caggccgcag gcgaagccgg tgacacacag ctcaagactg 2220
   gagcaagcaa ctccctcctg tcgatgctcg acaagcttag caataaatcg tccaatgcta 2280
   aaaagggccc acggtcgagt ccccaggaag ggcaccacca acctccgacc cagcagcaag 2340
   ggagtcaaca gagccgcgga aacagtcagg agagatcgca aaaccaggtc aaggccgcct 2400
   ctggagacca gggcacagac gcgaacacag catatcatgg acaatggaag gagtcacaac 2460
   catcagctgg tgcaatccct catgctctcc ggtcagggca gagccaagcc aatactcctg 2520
   catctgtgga tcatgtccag ctacctgtcg actttgtgca ggcgatgatg tctatgatgg 2580
   aggcgatatc acagaaggta aataaagtcg actatcagct agaccttgtc tcaaagcaga 2640
   catcctccat ccccatgatg cgatctgaaa tccaacagct gaaaacatcc gttgcggtca 2700
   tggaagccaa cctgggcatg atgaagattc tggaccctgg ttgtgctaac gtttcatctt 2760
   taagtgacct acgggcggtc gcccgctctc acccagtttt agtttcaggc cccggagacc 2820
   catctcctta tgtgacacaa gggggtgaat tgatactcaa taaactctcg caaccagtgc 2880
   aacatccttc tgagttgatt aaacctgcca cgataagcgg gcctgatata ggagtggaga 2940
   aggacactgt ccgtgcattg atcacctcac gcccaatgca tccgagctct tcagctaagc 3000
   tcctgagtaa gctggatgca gccgggtcga tagaagaaat taggaaaatc aaacgccttg 3060
   cgctgaatgg ctgatcacta ctacaacccg caacgggttc ccgtttatct attgtcacaa 3120
   ggaacccgcc ccgaagctta agaaaaaata cgggtagaag cagtttgaga gctccgttcg 3180
   ccaccatgag aaacctcccc gtggccactc cagacccagg aatgttccca tgccttcacc 3240
   actcccaaaa cctgctgagg gccgtcagca acatgctcca gaaggccaga caaactctag 3300
   aattttaccc ttgcacttct gaagagattg atcatgaaga tatcacaaaa gataaaacca 3360
   gcacagtgga ggcctgttta ccattggaat taaccaagaa tgagagttgc ctaaattcca 3420
   gagagacctc tttcataact aatgggagtt gcctggcctc cagaaagacc tcttttatga 3480
   tggccctgtg ccttagtagt atttatgaag acttgaagat gtaccaggtg gagttcaaga 3540
   ccatgaatgc aaagctgctg atggatccta agaggcagat ctttctagat caaaacatgc 3600
   tggcagttat tgatgagctg atgcaggccc tgaatttcaa cagtgagact gtgccacaaa 3660
   aatcctccct tgaagaaccg gatttttata aaactaaaat caagctctgc atacttcttc 3720
   atgctttcag aattcgggca gtgactattg atagagtgat gagctatctg aatgcttccg 3780
   gctccggcga gggccgcggc tccctgctga cctgcggcga cgtggaggag aaccccggcc 3840
   ccatatggga actgaagaaa gatgtttatg tcgtagaatt ggattggtat ccggatgccc 3900
   ctggagaaat ggtggtcctc acctgtgaca cccctgaaga agatggtatc acctggacct 3960
   tggaccagag cagtgaggtc ttaggctctg gcaaaaccct gaccatccaa gtcaaagagt 4020
   ttggagatgc tggccagtac acctgtcaca aaggaggcga ggttctaagc cattcgctcc 4080
   tgctgcttca caaaaaggaa gatggaattt ggtccactga tattttaaag gaccagaaag 4140
   aacccaaaaa taagaccttt ctaagatgcg aggccaagaa ttattctgga cgtttcacct 4200
   gctggtggct gacgacaatc agtactgatt tgacattcag tgtcaaaagc agcagaggct 4260
   cttctgaccc ccaaggggtg acgtgcggag ctgctacact ctctgcagag agagtcagag 4320
   gggacaacaa ggagtatgag tactcagtgg agtgccagga ggacagtgcc tgcccagctg 4380
   ctgaggagag tctgcccatt gaggtcatgg tggatgccgt tcacaagctc aagtatgaaa 4440
   actacaccag cagcttcttc atcagggaca tcatcaaacc tgacccaccc aagaacttgc 4500
   agctgaagcc attaaagaat tctcggcagg tggaggtcag ctgggagtac cctgacacct 4560
   ggagtactcc acattcctac ttctccctga cattctgcgt tcaggtccag ggcaagagca 4620
   agagagaaaa gaaagataga gtcttcacgg acaagacctc agccacggtc atctgccgca 4680
   aaaatgccag cattagcgtg cgggcccagg accgctacta tagctcatct tggagcgaat 4740
   gggcatctgt gccctgcagt taagctgacc gagccctcct ctataaaccc aagattcaag 4800
   gctccaagcg ataaccttct cccgcctctt ccatcccact gaatgatcgc gcagctgcaa 4860
   ttaatctagc aacattaaag attaagaaaa aatacgggta gaatcggagt gcaccgattg 4920
   tgccaagatg gactcatcta ggacaattgg gttatacttt gattctgccc ttccttctag 4980
   caacctgtta gcattcccga ttgtcttacg agatgtagga gatggaaaga agcaaattac 5040
   tccgcaatat aggatccggc gtcttgactc gtggacagac agtaaagaag actcggtatt 5100
   catcactacc tacggattca tcttccaggt tgggaatgaa gaagtcactg tcggcatgat 5160
   caatgataat cccaagcgag agttactctc cgctgcgatg ctctgcctag gaagcgtccc 5220
   gaatgttgga gatcctgttg agctggcaag ggcctgcctc actatggtgg taacatgcaa 5280
   gaagagtgca actaatactg agagaatggt cttctcagta gtgcaggcac cccgggtgct 5340
   gcaaagctgt agggtcgtgg cagacaaata ctcgtcagtg aatgcagtta agcacgtgaa 5400
   agcaccagag aagatccctt ggagtgaaac cctagagtac aaggtgaatt ttgtctcttt 5460
   gactgtggtg ccgaagaagg atgtctacaa gatcccaacc gcagtattga aggtatccgg 5520
   ctcgagcctg tataatcttg cgctcaatgt cactattgat gtggaggtag acccaaagag 5580
   cccgttagtc aagtcccttt ctaggtccga cagtggatac tatgctaatc tcttcttaca 5640
   tatcggactt atgtccactg tagataagag gggaaagaaa gtgacatttg accagctgga 5700
   gaggaagata aggagacttg atctatctgt cgggctcagt gatgtgctcg gaccttctgt 5760
   gcttgtgaag gcgagaggtg cacggactag gctgctggca cctttcttct ctaacagcgg 5820
   gacagcctgc taccctatag caaatgcctc tcctcaggta gctaagatac tctggagtca 5880
   gaccgcgtgc ctgcggagtg taaaaatcat tattcaagcg ggcacccaac gcgctgtcgc 5940
   agtgactgct gaccatgagg ttacctctac taagatagag aagaggcata ccatcgctaa 6000
   atacaatccc ttcaagaaat aggccgtatc cctgagactg caattcaccc gccttcccaa 6060
   aacaccatga caccagataa tgatctgtct tgattactta cagttagttt ccctgtctat 6120
   caaattagaa aaaacacggg tagaagagtt cggatcccgg ccggcgcacc aaaagcgcaa 6180
   gatgggcccc agatcttcta ccaggatccc agtacctcta atgctgacca tacggatcac 6240
   gctggcactg agttatgtcc gtctgacaag ttctcttgat ggcaggcctc ttgcagccgc 6300
   agggatcgtg gtaacagggg ataaagcagt taacatatac acctcatccc agacagggtc 6360
   aatcatagtc aagttactcc caaatatgcc caaggacaaa gaggcatgtg caaaagcccc 6420
   attggaggct tacaacagga cactgactac tttgcttacc ccccttggtg attctatccg 6480
   caggatacaa gagtctgtga ctacatccgg aggaaggaga cagagacgct ttataggtgc 6540
   cattattggc agtgtagctc taggggttgc aacagctgca cagataacgg cagcctcggc 6600
   tctgatacaa gccaaccaga atgctgctaa catcctccgg cttaaggaga gcattgctgc 6660
   aaccaatgaa gctgtacacg aggtcactgg cggattgtca cagttagcag tggcagttgg 6720
   aaagatgcag caatttgtta atgaccagtt taataacaca gctcaagagt tggactgtat 6780
   aaaaattaca cagcaggttg gtgtagaact caacttatac ttaactgaat tgactacagt 6840
   gttcgggcca caaatcactt cccctgcctt aactcagttg actgttcagg ctctttacaa 6900
   tctggctggt ggtaatgtag attacttgtt gactaagtta ggtgtaggga acaaccagct 6960
   cagctcattg attggtagcg gcttgatcac cggtaaccct attttttacg actcacagac 7020
   tcaactcttg ggcatacagg tgactttacc ctcagtcggg aacctaaata atatgcgtgc 7080
   cacctacttg gagaccttgt ctgtaagcac aacaaaggga tttgcctcag cacttgtccc 7140
   aaaagtagtg acacaggtcg gttctgtgat agaagagctt gacacctcat actgtataga 7200
   agctgatttg gatttatatt gtacaagaat agtgacattc cctatgtccc ctggtattta 7260
   ttcctgtttg agcggcaata catcggcttg catgtattca aggactgaag gcgcacttac 7320
   tacaccatac atgactctca aaggctcagt tgttgccaat tgccagatga caacatgtag 7380
   atgtgcagac cccccgggta tcatatcaca aaattatgga gaggctgtgt ctctaataga 7440
   taagcactca tgcaatgtcg tatccttaga cgggataact ttgaggctca gtggggaatt 7500
   tgatgcaact tatcaaaaga atatctcaat attagattct caagtactag tgacaggcaa 7560
   tctcgatatc tcaactgagc ttgggaatgt caaccactca ataagtaatg ctttggataa 7620
   gttagaggaa agcaacagca aactagacaa agtcaatgtc aaactgacca gcacatctgc 7680
   tctcattacc tatattgttt taactgtcat atctcttgtt cttggtatgc ttagcctggt 7740
   tctagcatgc tatctgatgt acaagcaaaa agcgcaacga aagaccttgt tgtggcttgg 7800
   aaataatacc ctagatcaga tgagagctac tacaaaaatg tgaatgcaga tgagaggcag 7860
   aggtatcccc aatagcaatc tgtgtgtcaa ttctggcagc ctgttaatca gaagaattaa 7920
   gaaaaaacta ccggatgtag gtgaacaaaa gggaatatac gggtagaacg gcctgagagg 7980
   ccacccctca atcgggagcc aggccccact acgtccgctc taccgcatca ccaacagcag 8040
   tcttcagtca tggacagcgc agttagccaa gttgcgctag agaatgatag aagagaagcg 8100
   aataatacat ggcgcttggt tttccggatc gcagccttac ttttactggt aataacctta 8160
   gccgtctctg cagtcgccct ggcatatagt atggaggcta gcacacctgg cgaccttgta 8220
   agcataccga ctgcgatctc tagggcagag gaaaggatta catctgcact cggttccaat 8280
   caagatgtag tagataggat atacaagcaa gtggcccttg agtctccatt ggcactgcta 8340
   aataccgaat ctataattat gaatgcaata acgtctctct cttatcaaat caatggagcc 8400
   acaaataata gcgggtgtgg ggcacctgtt catgacccag attacatcgg ggggataggt 8460
   caagaactta ttgtagatga tacgagtgac gtcacatcat tttatccctc tgcattccaa 8520
   gaacacctga attttatccc ggcgcccact acaggatcag gctgcactcg gataccctca 8580
   ttcgacatga gtgctaccca ttactgttac actcacaatg tgatattgtc tggctgcaga 8640
   gatcactcac actcacatca gtatttagca cttggtgtgc ttcggacatc tgcaacaggg 8700
   agggtattct tttctactct gcgttccatc aacttggatg acgcccaaaa tcggaagtct 8760
   tgcagtgtga gtgcaactcc tttaggttgt gatatgctgt gctctaaaat cacagagact 8820
   gaggaagagg attataaatc agttatcccc acatcgatgg tacatggaag gttagggctt 8880
   gacggccaat accatgagaa ggacctagac gtcacaacac tatttaggga ctgggtggca 8940
   aattacccag gagtaggagg tgggtctttt attaacaatc gcgtatggtt cccggtctac 9000
   ggagggctaa aacccagctc gcctagtgac actgcacaag aagggagata cgtaatatac 9060
   aagcgataca atgacacatg cccggatgag caagattacc agattcggat ggctaagtca 9120
   tcgtataagc ctaggcggtt tggtggaaaa cgcgtacagc aggccatcct atccatcaag 9180
   gtgtcaacat ccttgggtga ggacccggtg ttgactgtac cgcctaatac ggtcgcactc 9240
   atgggggccg aaggcagagt tctcacagtg gggacatctc atttcttata tcagcgaggg 9300
   tcatcatact tctctcccgc tttgttatac cctatgacag tcaacaacaa aacagccact 9360
   cttcataatc cttatacatt caatgctttc actcggccag gtagtgtccc ttgccaggct 9420
   tcagcaagat gccccaactc atgtgttacc ggagtctata ctgatccata tcccttagtc 9480
   ttccatagga accacacctt gcgaggggta ttcggaacaa tgcttgatga cgaacaagca 9540
   agactcaacc ctgtatctgc agtatttgat aacatatccc gcagtcgcat aactcgggta 9600
   agttcaagca gtaccagggc agcatacacg acatcaacgt gttttaaagt tgtcaagacc 9660
   aataaaacct attgcctcag cattgcagaa atatccaata ccctcttcgg ggaattcaga 9720
   attgtcccct tactagttga gattctcaag gatggtgggg tttaagaagc taggaatggt 9780
   cggttgagtc aaccgtgaga aggcggggaa gatgatattg catcatctat cttctgtaac 9840
   accaagaatc aaatcgaata ccagtgcgag ctcgaatcct acgctgccag tcagccataa 9900
   tcggctggtg ctaatgtggt tagcctgaat cttgtcgata gtcacttgat taagaaaaaa 9960
   tgtggacggt agtgaaatat aaggcaaagc agctcacaga ggacagcacg ggtaggacat 10020
   ggcgggctcc ggacccgaaa gggcagagca tcagattatc ctaccagagt cacatctgtc 10080
   ctcaccattg gtcaagcata aattgcttta ttactggaaa ttaactgggc taccgcttcc 10140
   tgacgaatgt gacttcgacc atcttattat cagccgacaa tggaagaagg tacttgaatc 10200
   ggccacccct gacattgaga gaatgataaa actagggcgg gcagtacacc agactctcaa 10260
   ccacaattcc aggataaccg gagtactcca tccccggtgt ttagaggaac tggctagtat 10320
   tgaggttcct gattcaacca acaaatttcg gaagatcgaa aaaaagatcc agattcacaa 10380
   cacaaggtat ggagaactgt tcacaagatt atgcacgcat gtagaaaaga aactattggg 10440
   gtcatcttgg tctaacaatg tcccacggtc agaggaattc aacagcatcc gtacagatcc 10500
   ggcattttgg tttcactcaa aatggtccac agccaaattt gcatggctcc atataaaaca 10560
   ggtccagagg catctgattg tagcagcaag aacaaggtcc gcagtcaaca aattagtgac 10620
   gctgacccat aaggtaggcc aaatctttgt tactcctgag cttgtcattg tgacacatac 10680
   agatgagaac aagttcacgt gtcttaccca ggaacttgtg ttgatgtatg cagatatgat 10740
   ggagggcaga gatatggtca gcataatatc atccacggcg gcacatctta ggagcttatc 10800
   agagaaaatt gatgatattc tgcggttagt agatgctctg gcaagagatt tgggcaatca 10860
   agtctacgat gttgtagcac taatggaggg attcgcatac ggcgctgttc agctgcttga 10920
   accgtcaggt acatttgcgg gggatttctt cgcattcaac ctgcaggagc tcaaagatac 10980
   tctaaccgga ctcctcccca aggatatcgc agaatctgtg actcacgcaa tcgcaaccat 11040
   attctctggc ttagaacaaa atcaagcagc tgagatgttg tgcctgttgc gtctgtgggg 11100
   tcacccacta ctcgagtccc gtattgcagc aaaagcagtt aggagtcaaa tgtgcgcacc 11160
   aaaaatggta gactttgata tgatcctcca ggtattatct ttctttaaag gaacaatcat 11220
   caatggatat agaaagaaga atgcaggcgt gtggccacgt gtcaagatag atacgatata 11280
   cgggaaggtc atagggcagc tacacgcaga ttctgcagag atttcacatg atgtcatgtt 11340
   gagggaatac aagagtttat ctgcacttga attcgagcca tgtatagagt atgaccctgt 11400
   caccaatctg agcatgtttt taaaagacaa ggcaatcgca cacccgaaag acaactggct 11460
   cgcttcgttt aggcgaaacc ttctctctga ggaccagaag aaacatgtaa gagaggcaac 11520
   ctcaactaac cgcctcttga tagagttctt agagtcaaat gattttgatc catataaaga 11580
   gatggaatat ctgacgaccc ttgagtacct aagagatgat aatgtggcag tatcatactc 11640
   actcaaagaa aaagaggtga aagttaatgg gcggattttc gcaaagctaa caaatagatt 11700
   aaggaattgt caggtaatgg cagaagggat cctagctgac cagattgcac ctttcttcca 11760
   gggaaatggg gtcattcagg atagcatatc tttgactaaa agtatgctag cgatgagtca 11820
   actgtctttc aacagcaaca agaaacgtat tactgactgc aaagaaagag tatcctcaaa 11880
   ccgcaatcat gatccgaaga gcaagaatcg tcggagagtt gccacttttg taactaccga 11940
   cctgcaaaag tattgtctca attggagata tcagacagtc aagctgttcg ctcatgccat 12000
   caatcagctg atgggcttac ctcacttctt cgagtggatt catcttagac taatggatac 12060
   tacgatgttt gtaggggacc ctttcaatcc tccaagtgac cctacggact gtgatctatc 12120
   aagagtccca aatgatgaca tatatattgt cagtgctagg ggaggcattg agggattatg 12180
   ccagaagcta tggacaatga tctcaattgc tgcaatccag cttgctgcag caagatcaca 12240
   ttgtcgcgtt gcctgtatgg tacaaggcga caatcaagta atagctgtaa cgagagaggt 12300
   aagatcagat gactccccag agatggtgtt aacacaattg catcaagcca gtgataattt 12360
   cttcaaggaa ttgattcatg tcaatcattt gatcggccat aatttgaagg atcgtgaaac 12420
   catcaggtca gacacgttct tcatatacag caaacgaata ttcaaagatg gagcaatact 12480
   cagtcaggtc ctcaaaaact catctaaatt agtgctaata tcaggcgacc ttagtgaaaa 12540
   cactgtaatg tcttgtgcca acattgcatc tactgtagca cggttatgcg agaacgggct 12600
   tcctaaggat ttctgttatt acttaaacta cttaatgagt tgcgtgcaga catacttcga 12660
   ttccgaattt tccatcacca acaactcgca actcgattct aaccagtcgt ggatagagga 12720
   catttctttt gtgcactcat atgtcctgac ccctgctcaa ctggggggac tgagtaacct 12780
   tcaatactca aggctctaca caaggaacat cggcgacccg ggaaccactg ctttcgcaga 12840
   gatcaagaga ttagaggcag tggggttact gagtcctagc attatgacta acatcttaac 12900
   taggccgcct ggaaatggag attgggccag tctgtgcaac gatccatact cctttaattt 12960
   tgagactgtc gcaagcccaa atattgtcct taagaaacat acacaaagag tcctatttga 13020
   aacttgctcg aatcccttat tatctggagt gcacacagag gataatgagg cagaagagaa 13080
   ggcattggct gaattcttgc tcaatcaaga aatgattcat ccacgtgtcg cgcatgctat 13140
   catggaagca agctctgtag gtaggagaaa gcaaattcag gggcttgttg acacaacaaa 13200
   caccgtgatt aagattgcac tgactaggag gccactcggc atcaagaggc tgatgcggat 13260
   agtcaattac tcgagcatgc atgcaatgct atttagagat gatgttttct cgtctaatag 13320
   atccaaccat cccttagtct cttctgatat gtgttctcag acactggcag actatgcacg 13380
   gaacagaagc tggtcacctt tgacaggggg tagaaaaata ctgggtgtat ctaatcctga 13440
   taccatagaa cttgtagagg gtgagatcct tagtgtcagt ggagggtgca ctagatgtga 13500
   cagtggggat gaacagttta cttggttcca tcttccaagc aatatagagc taaacgatga 13560
   caccagcaag aatcctccaa tgagagtgcc atatctcggg tcaaagactc aagagaggag 13620
   agccgcctcg cttgcgaaaa tagctcatat gtcaccacac gtgaaggcgg ctctaagggc 13680
   gtcatctgtg ttaatctggg cttatgggga caacgaaata aactggactg ctgcccttaa 13740
   gattgcaagg tctcggtgca acataagctc agagtatctt cgactattgt cacccttgcc 13800
   tacagctggg aatctccaac atagattgga cgacggcata actcagatga cattcacccc 13860
   tgcatctcta tacagggtgt caccttacat tcatatatct aatgattctc aaaggctatt 13920
   tactgaagaa ggagtcaaag aggggaatgt ggtttatcag caaatcatgc tcttgggttt 13980
   gtccttaatt gagtcactct tcccaatgac aacaaccaag acatatgatg aaatcacatt 14040
   gcacctccac agtaaattta gctgctgtat cagggaagca cctgttgcag ttcctttcga 14100
   gctactcggg gtggcaccgg aactaagggc agtaacctca aataagttta tgtatgatcc 14160
   tagccctgta tcagagggag actttgcgag acttgactta gctatcttta agagttatga 14220
   acttaattta gagtcatatt ccacaataga gctaatgaac gttctttcaa tatctagtgg 14280
   gaagttgatt ggccagtctg tggtttctta tgatgaagat acctccataa agaatgacgc 14340
   tataatagtg tatgacaaca cacggaattg gatcagcgaa gctcagaatt cagatgtggt 14400
   ccgcctattc gagtatgcgg cactcgaagt gctcctcgac tgttcttatc aactctacta 14460
   tctgagagta agaggcctaa acaatatcgt cctgtacatg agtgatttat acaagaatat 14520
   gccaggaatt ctactctcta atattgcagc cacaatatct caccctgtca tccattcaag 14580
   gttgaatgca gtaggtctgg tcaaccatga cgggtcacac caacttgcag acacagattt 14640
   cattgaaatg tctgcaaagc tgctagtctt ttgcactcga cgcgtggtct caggtttaca 14700
   tgcagggaat aagtatgacc tgctgtttcc atctgtcttg gatgataacc taagtgagaa 14760
   gatgcttcag ttgatatccc aattatgctg tctgtatacg gtgctctttg ctacaacaag 14820
   agaaatcccg aaaataagag gcttatctgc agaagagaaa tgttcagtac ttactgagta 14880
   cctactgtca gatgctgtga aaccgttact tgggtccgag caagtgagct ctatcatgtc 14940
   tcccaacata gttacgttcc cagccaatct gtattacatg tctaggaaga gccttaattt 15000
   gatcagggag agagaggaca gggatactat cttggcattg ttgttccctc aagaaccgct 15060
   gctcgagttt cctctggtac gagatattgg tgctcgtgta aaagatccat ttacccgaca 15120
   acctgcggcg tttttacaag agttagattt gagtgctccg gcaaggtatg acgcattcac 15180
   aatcagtcag gcgcattctg aacacatatt gccaaaccca gaggaagatc acttagtacg 15240
   atacttgttc agaggaatag ggactgcgtc ctcctcttgg tataaggcat cccatcttct 15300
   ttctgtaccc gaggtcagat gtgcaaggca tgggaactcc ttatatttag cagaaggaag 15360
   tggagccatc atgagtcttc tcgaattgca tataccacac gaaactatct attacaatac 15420
   acttttctcg aacgagatga accccccaca gcgacatttc ggaccaactc caacacagtt 15480
   tctgaattcg gttgttttta ggaatttaca ggcggaagta ccatgcaagg atggatttgt 15540
   ccaggagttc cgtccgttat ggagagagaa tacagaagaa agcgatctga cctcagataa 15600
   agcagtggga tatatcacat ctgtagtgcc gtacaggtct gtatcattgc tgcattgtga 15660
   cattgaaatc cctccaggat ctaatcaaag cttactagat cagctggcta ccaatctgtc 15720
   tctgattgcc atgcattctg tgaaggaggg cggggtcgtg attatcaaag tactgtatgc 15780
   aatgggatat tacttccatc tactcatgaa cttgtttact ccatgttcca cgaaaggata 15840
   tattctctct aatggctatg cctgtagagg ggatatggag tgttacctga tatttgtcat 15900
   gggctaccta ggcgggccta catttgtaca tgaggtggtg aggatggcaa aaacgctagt 15960
   acagcggcat ggcacacttt tgtctaagtc agatgagatt acactgacta ggttatttac 16020
   ttcacagcaa cagcgtgtaa cagacatcct atccagccct ttaccgagac taatgaagta 16080
   cttgagagag aatattgata ctgcactgat tgaagccggg ggacagcctg tccgtccatt 16140
   ctgtgcagag agtttagtga gcacactaac agacatgact cagacaaccc agatcatcgc 16200
   cagccacatt gatacagtca ttcgatctgt gatctatatg gaagcagagg gtgatcttgc 16260
   tgacacagta ttcttattta ccccttacaa cctctctatg gacgggaaaa agagaacatc 16320
   acttaaacag tgcacaagac agatcttaga ggtcacaata ctgggtctca gagtcaaaaa 16380
   tctcaataaa gtaggtgatg taatcagcct agtactcaga ggtatgattt ctctggagga 16440
   ccttctccca ctgagaacct acttgaagtg tagtacctgc cctaagtatt tgaaggctgt 16500
   cctaggtatt accaaactca aagaaatgtt cacagacgcc tctttattat acttgactcg 16560
   tgctcaacaa aaattctaca tgaaaactat aggcaatgca gtcaagggat actatagtaa 16620
   ctgtgactct taaaggcaac cacatatcaa taggccctct ttctagccga tcgtattctt 16680
   gttgacttca ttataccata ttagaaaaaa attgaattcc gaccctttaa gactcgtatt 16740
   cggattcaaa taattatctc agaaaaaaag tgcacgtagt tgttcttgat tatagtcccg 16800
   tcattcacca agtctttgtt tggt 16824

<210> 13
<211> 15966
<212> DNA
<213> Newcastle disease virus

<220>
<223> Mut HN (F277L)/M(G165W)-eGFP

<400> 13
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gcccccaccc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaat tggagcggcc gcattgtgcc 3180

aagatggtga gcaagggcga ggagctgttc accggggtgg tgcccatcct ggtcgagctg 3240

gacggcgacg taaacggcca caagttcagc gtgtccggcg agggcgaggg cgatgccacc 3300

tacggcaagc tgaccctgaa gttcatctgc accaccggca agctgcccgt gccctggccc 3360

accctcgtga ccaccctgac ctacggcgtg cagtgcttca gccgctaccc cgaccacatg 3420

aagcagcacg acttcttcaa gtccgccatg cccgaaggct acgtccagga gcgcaccatc 3480

ttcttcaagg acgacggcaa ctacaagacc cgcgccgagg tgaagttcga gggcgacacc 3540

ctggtgaacc gcatcgagct gaagggcatc gacttcaagg aggacggcaa catcctgggg 3600

cacaagctgg agtacaacta caacagccac aacgtctata tcatggccga caagcagaag 3660

aacggcatca aggtgaactt caagatccgc cacaacatcg aggacggcag cgtgcagctc 3720

gccgaccact accagcagaa cacccccatc ggcgacggcc ccgtgctgct gcccgacaac 3780

cactacctga gcacccagtc cgccctgagc aaagacccca acgagaagcg cgatcacatg 3840

gtcctgctgg agttcgtgac cgccgccggg atcactctcg gcatggacga gctgtacaag 3900

taagagctga ccgagccctc ctctataaac ccaagattca aggctccaag cgataacctt 3960

ctcccgcctc ttccatccca ctgaatgatc gcgcagctgc aattaatcta gcaacattaa 4020

agattaagaa aaaatacggg tagaatcgga gtgcaccgat tgtgccaaga tggactcatc 4080

taggacaatt gggttatact ttgattctgc ccttccttct agcaacctgt tagcattccc 4140

gattgtctta cgagatgtag gagatggaaa gaagcaaatt actccgcaat ataggatccg 4200

gcgtcttgac tcgtggacag acagtaaaga agactcggta ttcatcacta cctacggatt 4260

catcttccag gttgggaatg aagaagtcac tgtcggcatg atcaatgata atcccaagcg 4320

agagttactc tccgctgcga tgctctgcct aggaagcgtc ccgaatgttg gagatcctgt 4380

tgagctggca agggcctgcc tcactatggt ggtaacatgc aagaagagtg caactaatac 4440

tgagagaatg gtcttctcag tagtgcaggc accccgggtg ctgcaaagct gtagggtcgt 4500

ggcagacaaa tactcgtcag tgaatgcagt taagcacgtg aaagcaccag agaagatccc 4560

ttggagtgaa accctagagt acaaggtgaa ttttgtctct ttgactgtgg tgccgaagaa 4620

ggatgtctac aagatcccaa ccgcagtatt gaaggtatcc ggctcgagcc tgtataatct 4680

tgcgctcaat gtcactattg atgtggaggt agacccaaag agcccgttag tcaagtccct 4740

ttctaggtcc gacagtggat actatgctaa tctcttctta catatcggac ttatgtccac 4800

tgtagataag aggggaaaga aagtgacatt tgaccagctg gagaggaaga taaggagact 4860

tgatctatct gtcgggctca gtgatgtgct cggaccttct gtgcttgtga aggcgagagg 4920

tgcacggact aggctgctgg cacctttctt ctctaacagc gggacagcct gctaccctat 4980

agcaaatgcc tctcctcagg tagctaagat actctggagt cagaccgcgt gcctgcggag 5040

tgtaaaaatc attattcaag cgggcaccca acgcgctgtc gcagtgactg ctgaccatga 5100

ggttacctct actaagatag agaagaggca taccatcgct aaatacaatc ccttcaagaa 5160

ataggccgta tccctgagac tgcaattcac ccgccttccc aaaacaccat gacaccagat 5220

aatgatctgt cttgattact tacagttagt ttccctgtct atcaaattag aaaaaacacg 5280

ggtagaagag ttcggatccc ggccggcgca ccaaaagcgc aagatgggcc ccagatcttc 5340

taccaggatc ccagtacctc taatgctgac catacggatc acgctggcac tgagttatgt 5400

ccgtctgaca agttctcttg atggcaggcc tcttgcagcc gcagggatcg tggtaacagg 5460

ggataaagca gttaacatat acacctcatc ccagacaggg tcaatcatag tcaagttact 5520

cccaaatatg cccaaggaca aagaggcatg tgcaaaagcc ccattggagg cttacaacag 5580

gacactgact actttgctta ccccccttgg tgattctatc cgcaggatac aagagtctgt 5640

gactacatcc ggaggaagga gacagagacg ctttataggt gccattattg gcagtgtagc 5700

tctaggggtt gcaacagctg cacagataac ggcagcctcg gctctgatac aagccaacca 5760

gaatgctgct aacatcctcc ggcttaagga gagcattgct gcaaccaatg aagctgtaca 5820

cgaggtcact ggcggattgt cacagttagc agtggcagtt ggaaagatgc agcaatttgt 5880

taatgaccag tttaataaca cagctcaaga gttggactgt ataaaaatta cacagcaggt 5940

tggtgtagaa ctcaacttat acttaactga attgactaca gtgttcgggc cacaaatcac 6000

ttcccctgcc ttaactcagt tgactgttca ggctctttac aatctggctg gtggtaatgt 6060

agattacttg ttgactaagt taggtgtagg gaacaaccag ctcagctcat tgattggtag 6120

cggcttgatc accggtaacc ctatttttta cgactcacag actcaactct tgggcataca 6180

ggtgacttta ccctcagtcg ggaacctaaa taatatgcgt gccacctact tggagacctt 6240

gtctgtaagc acaacaaagg gatttgcctc agcacttgtc ccaaaagtag tgacacaggt 6300

cggttctgtg atagaagagc ttgacacctc atactgtata gaagctgatt tggatttata 6360

ttgtacaaga atagtgacat tccctatgtc ccctggtatt tattcctgtt tgagcggcaa 6420

tacatcggct tgcatgtatt caaggactga aggcgcactt actacaccat acatgactct 6480

caaaggctca gttgttgcca attgccagat gacaacatgt agatgtgcag acccccgggg 6540

tatcatatca caaaattatg gagaggctgt gtctctaata gataagcact catgcaatgt 6600

cgtatcctta gacgggataa ctttgaggct cagtggggaa tttgatgcaa cttatcaaaa 6660

gaatatctca atattagatt ctcaagtact agtgacaggc aatctcgata tctcaactga 6720

gcttgggaat gtcaaccact caataagtaa tgctttggat aagttagagg aaagcaacag 6780

caaactagac aaagtcaatg tcaaactgac cagcacatct gctctcatta cctatattgt 6840

tttaactgtc atatctcttg ttcttggtat gcttagcctg gttctagcat gctatctgat 6900

gtacaagcaa aaagcgcaac gaaagacctt gttgtggctt ggaaataata ccctagatca 6960

gatgagagct actacaaaaa tgtgaatgca gatgagaggc agaggtatcc ccaatagcaa 7020

tctgtgtgtc aattctggca gcctgttaat cagaagaatt aagaaaaaac taccggatgt 7080

aggtgaacaa aagggaatat acgggtagaa cggcctgaga ggccacccct caatcgggag 7140

ccaggcccca ctacgtccgc tctaccgcat caccaacagc agtcttcagt catggacagc 7200

gcagttagcc aagttgcgct agagaatgat agaagagaag cgaataatac atggcgcttg 7260

gttttccgga tcgcagcctt acttttactg gtaataacct tagccgtctc tgcagtcgcc 7320

ctggcatata gtatggaggc tagcacacct ggcgaccttg taagcatacc gactgcgatc 7380

tctagggcag aggaaaggat tacatctgca ctcggttcca atcaagatgt agtagatagg 7440

atatacaagc aagtggccct tgagtctcca ttggcactgc taaataccga atctataatt 7500

atgaatgcaa taacgtctct ctcttatcaa atcaatggag ccacaaataa tagcgggtgt 7560

ggggcacctg ttcatgaccc agattacatc ggggggatag gtcaagaact tattgtagat 7620

gatacgagtg acgtcacatc atttatcccc tctgcattcc aagaacacct gaattttatc 7680

ccggcgccca ctacaggatc aggctgcact cggataccct cattcgacat gagtgctacc 7740

cattactgtt acactcacaa tgtgatattg tctggctgca gagatcactc acactcacat 7800

cagtatttag cacttggtgt gcttcggaca tctgcaacag ggagggtatt cttttctact 7860

ctgcgttcca tcaacttgga tgacgcccaa aatcggaagt cttgcagtgt gagtgcaact 7920

cctttaggtt gtgatatgct gtgctctaaa atcacagaga ctgaggaaga ggattataaa 7980

tcagttatcc ccacatcgat ggtacatgga aggttagggc ttgacggcca ataccatgag 8040

aaggacctag acgtcacaac actatttagg gactgggtgg caaattaccc aggagtagga 8100

ggtgggtctt ttattaacaa tcgcgtatgg ttcccggtct acggagggct aaaacccagc 8160

tcgcctagtg acactgcaca agaaggagaa tacgtaatat acaagcgata caatgacaca 8220

tgcccggatg agcaagatta ccagattcgg atggctaagt catcgtataa gcctaggcgg 8280

tttggtggaa aacgcgtaca gcaggccatc ctatccatca aggtgtcaac atccttgggt 8340

gaggacccgg tgttgactgt accgcctaat acggtcgcac tcatgggggc cgaaggcaga 8400

gttctcacag tggggacatc tcatttctta tatcagcgag ggtcatcata cttctctccc 8460

gctttgttat accctatgac agtcaacaac aaaacagcca ctcttcataa tccttataca 8520

ttcaatgctt tcactcggcc aggtagtgtc ccttgccagg cttcagcaag atgccccaac 8580

tcatgtgtta ccggagtcta tactgatcca tatcccttag tcttccatag gaaccacacc 8640

ttgcgagggg tattcggaac aatgcttgat gacgaacaag caagactcaa ccctgtatct 8700

gcagtatttg ataacatatc ccgcagtcgc ataactcggg taagttcaag cagtaccagg 8760

gcagcataca cgacatcaac gtgttttaaa gttgtcaaga ccaataaaac ctattgcctc 8820

agcattgcag aaatatccaa taccctcttc ggggaattca gaattgtccc cttactagtt 8880

gagattctca aggatggtgg ggtttaagaa gctaggaatg gtcggttgag tcaaccgtga 8940

gaaggcgggg aagatgatat tgcatcatct atcttctgta acaccaagaa tcaaatcgaa 9000

taccagtgcg agctcgaatc ctacgctgcc agtcagccat aatcggctgg tgctaatgtg 9060

gttagcctga atcttgtcga tagtcacttg attaagaaaa aatgtggacg gtagtgaaat 9120

ataaggcaaa gcagctcaca gaggacagca cgggtaggac atggcgggct ccggacccga 9180

aagggcagag catcagatta tcctaccaga gtcacatctg tcctcaccat tggtcaagca 9240

taaattgctt tattactgga aattaactgg gctaccgctt cctgacgaat gtgacttcga 9300

ccatcttatt atcagccgac aatggaagaa ggtacttgaa tcggccaccc ctgacattga 9360

gagaatgata aaactagggc gggcagtaca ccagactctc aaccacaatt ccaggataac 9420

cggagtactc catccccggt gtttagagga actggctagt attgaggttc ctgattcaac 9480

caacaaattt cggaagatcg aaaaaaagat ccagattcac aacacaaggt atggagaact 9540

gttcacaaga ttatgcacgc atgtagaaaa gaaactattg gggtcatctt ggtctaacaa 9600

tgtcccacgg tcagaggaat tcaacagcat ccgtacagat ccggcatttt ggtttcactc 9660

aaaatggtcc acagccaaat ttgcatggct ccatataaaa caggtccaga ggcatctgat 9720

tgtagcagca agaacaaggt ccgcagtcaa caaattagtg acgctgaccc ataaggtagg 9780

ccaaatcttt gttactcctg agcttgtcat tgtgacacat acagatgaga acaagttcac 9840

gtgtcttacc caggaacttg tgttgatgta tgcagatatg atggagggca gagatatggt 9900

cagcataata tcatccacgg cggcacatct taggagctta tcagagaaaa ttgatgatat 9960

tctgcggtta gtagatgctc tggcaagaga tttgggcaat caagtctacg atgttgtagc 10020

actaatggag ggattcgcat acggcgctgt tcagctgctt gaaccgtcag gtacatttgc 10080

gggggatttc ttcgcattca acctgcagga gctcaaagat actctaaccg gactcctccc 10140

caaggatatc gcagaatctg tgactcacgc aatcgcaacc atattctctg gcttagaaca 10200

aaatcaagca gctgagatgt tgtgcctgtt gcgtctgtgg ggtcacccac tactcgagtc 10260

ccgtattgca gcaaaagcag ttaggagtca aatgtgcgca ccaaaaatgg tagactttga 10320

tatgatcctc caggtattat ctttctttaa aggaacaatc atcaatggat atagaaagaa 10380

gaatgcaggc gtgtggccac gtgtcaagat agatacgata tacgggaagg tcatagggca 10440

gctacacgca gattctgcag agatttcaca tgatgtcatg ttgagggaat acaagagttt 10500

atctgcactt gaattcgagc catgtataga gtatgaccct gtcaccaatc tgagcatgtt 10560

tttaaaagac aaggcaatcg cacacccgaa agacaactgg ctcgcttcgt ttaggcgaaa 10620

ccttctctct gaggaccaga agaaacatgt aagagaggca acctcaacta accgcctctt 10680

gatagagttc ttagagtcaa atgattttga tccatataaa gagatggaat atctgacgac 10740

ccttgagtac ctaagagatg ataatgtggc agtatcatac tcactcaaag aaaaagaggt 10800

gaaagttaat gggcggattt tcgcaaagct aacaaataga ttaaggaatt gtcaggtaat 10860

ggcagaaggg atcctagctg accagattgc acctttcttc cagggaaatg gggtcattca 10920

ggatagcata tctttgacta aaagtatgct agcgatgagt caactgtctt tcaacagcaa 10980

caagaaacgt attactgact gcaaagaaag agtatcctca aaccgcaatc atgatccgaa 11040

gagcaagaat cgtcggagag ttgccacttt tgtaactacc gacctgcaaa agtattgtct 11100

caattggaga tatcagacag tcaagctgtt cgctcatgcc atcaatcagc tgatgggctt 11160

acctcacttc ttcgagtgga ttcatcttag actaatggat actacgatgt ttgtagggga 11220

ccctttcaat cctccaagtg accctacgga ctgtgatcta tcaagagtcc caaatgatga 11280

catatatatt gtcagtgcta ggggaggcat tgagggatta tgccagaagc tatggacaat 11340

gatctcaatt gctgcaatcc agcttgctgc agcaagatca cattgtcgcg ttgcctgtat 11400

ggtacaaggc gacaatcaag taatagctgt aacgagagag gtaagatcag atgactcccc 11460

agagatggtg ttaacacaat tgcatcaagc cagtgataat ttcttcaagg aattgattca 11520
tgtcaatcat ttgatcggcc ataatttgaa ggatcgtgaa accatcaggt cagacacgtt 11580
cttcatatac agcaaacgaa tattcaaaga tggagcaata ctcagtcagg tcctcaaaaa 11640
ctcatctaaa ttagtgctaa tatcaggcga ccttagtgaa aacactgtaa tgtcttgtgc 11700
caacattgca tctactgtag cacggttatg cgagaacggg cttcctaagg atttctgtta 11760
ttacttaaac tacttaatga gttgcgtgca gacatacttc gattccgaat tttccatcac 11820
caacaactcg caactcgatt ctaaccagtc gtggatagag gacatttctt ttgtgcactc 11880
atatgtcctg acccctgctc aactgggggg actgagtaac cttcaatact caaggctcta 11940
cacaaggaac atcggcgacc cgggaaccac tgctttcgca gagatcaaga gattagaggc 12000
agtggggtta ctgagtccta gcattatgac taacatctta actaggccgc ctggaaatgg 12060
agattgggcc agtctgtgca acgatccata ctcctttaat tttgagactg tcgcaagccc 12120
aaatattgtc cttaagaaac atacacaaag agtcctattt gaaacttgct cgaatccctt 12180
attatctgga gtgcacacag aggataatga ggcagaagag aaggcattgg ctgaattctt 12240
gctcaatcaa gaaatgattc atccacgtgt cgcgcatgct atcatggaag caagctctgt 12300
aggtaggaga aagcaaattc aggggcttgt tgacacaaca aacaccgtga ttaagattgc 12360
actgactagg aggccactcg gcatcaagag gctgatgcgg atagtcaatt actcgagcat 12420
gcatgcaatg ctatttagag atgatgtttt ctcgtctaat agatccaacc atcccttagt 12480
ctcttctgat atgtgttctc agacactggc agactatgca cggaacagaa gctggtcacc 12540
tttgacaggg ggtagaaaaa tactgggtgt atctaatcct gataccatag aacttgtaga 12600
gggtgagatc cttagtgtca gtggagggtg cactagatgt gacagtgggg atgaacagtt 12660
tacttggttc catcttccaa gcaatataga gctaaacgat gacaccagca agaatcctcc 12720
aatgagagtg ccatatctcg ggtcaaagac tcaagagagg agagccgcct cgcttgcgaa 12780
aatagctcat atgtcaccac acgtgaaggc ggctctaagg gcgtcatctg tgttaatctg 12840
ggcttatggg gacaacgaaa taaactggac tgctgccctt aagattgcaa ggtctcggtg 12900
caacataagc tcagagtatc ttcgactatt gtcacccttg cctacagctg ggaatctcca 12960
acatagattg gacgacggca taactcagat gacattcacc cctgcatctc tatacagggt 13020
gtcaccttac attcatatat ctaatgattc tcaaaggcta tttactgaag aaggagtcaa 13080
agaggggaat gtggtttatc agcaaatcat gctcttgggt ttgtccttaa ttgagtcact 13140
cttcccaatg acaacaacca agacatatga tgaaatcaca ttgcacctcc acagtaaatt 13200
tagctgctgt atcagggaag cacctgttgc agttcctttc gagctactcg gggtggcacc 13260
ggaactaagg gcagtaacct caaataagtt tatgtatgat cctagccctg tatcagaggg 13320
agactttgcg agacttgact tagctatctt taagagttat gaacttaatt tagagtcata 13380

ttccacaata gagctaatga acgttctttc aatatctagt gggaagttga ttggccagtc 13440

tgtggtttct tatgatgaag atacctccat aaagaatgac gctataatag tgtatgacaa 13500

cacacggaat tggatcagcg aagctcagaa ttcagatgtg gtccgcctat tcgagtatgc 13560

ggcactcgaa gtgctcctcg actgttctta tcaactctac tatctgagag taagaggcct 13620

aaacaatatc gtcctgtaca tgagtgattt atacaagaat atgccaggaa ttctactctc 13680

taatattgca gccacaatat ctcaccctgt catccattca aggttgaatg cagtaggtct 13740

ggtcaaccat gacgggtcac accaacttgc agacacagat ttcattgaaa tgtctgcaaa 13800

gctgctagtc ttttgcactc gacgcgtggt ctcaggttta catgcaggga ataagtatga 13860

cctgctgttt ccatctgtct tggatgataa cctaagtgag aagatgcttc agttgatatc 13920

ccaattatgc tgtctgtata cggtgctctt tgctacaaca agagaaatcc cgaaaataag 13980

aggcttatct gcagaagaga aatgttcagt acttactgag tacctactgt cagatgctgt 14040

gaaaccgtta cttgggtccg agcaagtgag ctctatcatg tctcccaaca tagttacgtt 14100

cccagccaat ctgtattaca tgtctaggaa gagccttaat ttgatcaggg agagagagga 14160

cagggatact atcttggcat tgttgttccc tcaagaaccg ctgctcgagt ttcctctggt 14220

acgagatatt ggtgctcgtg taaaagatcc atttacccga caacctgcgg cgtttttaca 14280

agagttagat ttgagtgctc cggcaaggta tgacgcattc acaatcagtc aggcgcattc 14340

tgaacacata ttgccaaacc cagaggaaga tcacttagta cgatacttgt tcagaggaat 14400

agggactgcg tcctcctctt ggtataaggc atcccatctt ctttctgtac ccgaggtcag 14460

atgtgcaagg catgggaact ccttatattt agcagaagga agtggagcca tcatgagtct 14520

tctcgaattg catataccac acgaaactat ctattacaat acacttttct cgaacgagat 14580

gaacccccca cagcgacatt tcggaccaac tccaacacag tttctgaatt cggttgtttt 14640

taggaattta caggcggaag taccatgcaa ggatggattt gtccaggagt tccgtccgtt 14700

atggagagag aatacagaag aaagcgatct gacctcagat aaagcagtgg gatatatcac 14760

atctgtagtg ccgtacaggt ctgtatcatt gctgcattgt gacattgaaa tccctccagg 14820

atctaatcaa agcttactag atcagctggc taccaatctg tctctgattg ccatgcattc 14880

tgtgaaggag ggcggggtcg tgattatcaa agtactgtat gcaatgggat attacttcca 14940

tctactcatg aacttgttta ctccatgttc cacgaaagga tatattctct ctaatggcta 15000

tgcctgtaga ggggatatgg agtgttacct gatatttgtc atgggctacc taggcgggcc 15060

tacatttgta catgaggtgg tgaggatggc aaaaacgcta gtacagcggc atggcacact 15120

tttgtctaag tcagatgaga ttacactgac taggttattt acttcacagc aacagcgtgt 15180

aacagacatc ctatccagcc ctttaccgag actaatgaag tacttgagag agaatattga 15240

tactgcactg attgaagccg ggggacagcc tgtccgtcca ttctgtgcag agagtttagt 15300

gagcacacta acagacatga ctcagacaac ccagatcatc gccagccaca ttgatacagt 15360

cattcgatct gtgatctata tggaagcaga gggtgatctt gctgacacag tattcttatt 15420

taccccttac aacctctcta tggacgggaa aaagagaaca tcacttaaac agtgcacaag 15480

acagatctta gaggtcacaa tactgggtct cagagtcaaa aatctcaata aagtaggtga 15540

tgtaatcagc ctagtactca gaggtatgat ttctctggag gaccttctcc cactgagaac 15600

ctacttgaag tgtagtacct gccctaagta tttgaaggct gtcctaggta ttaccaaact 15660

caaagaaatg ttcacagacg cctctttatt atacttgact cgtgctcaac aaaaattcta 15720

catgaaaact ataggcaatg cagtcaaggg atactatagt aactgtgact cttaaaggca 15780

accacatatc aataggccct ctttctagcc gatcgtattc ttgttgactt cattatacca 15840

tattagaaaa aaattgaatt ccgacccttt aagactcgta ttcggattca aataattatc 15900

tcagaaaaaa agtgcacgta gttgttcttg attatagtcc cgtcattcac caagtctttg 15960

tttggt 15966

<210> 14
<211> 17436
<212> DNA
<213> Newcastle disease virus

<220>
<223> Mut HN (F277L)/M(G165W)/F(F117S)/F(F190L)/L(V757I)/L(F1551S)/L(R1
      700L)-Atezolizumab

<400> 14
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgttccgc ggcgtgcagt 3240

gcgaggtgca gctggtggag agcggcggcg gcctggtgca gcccggcggc agcctgaggc 3300

tgagctgcgc cgccagcggc ttcaccttca gcgacagctg gatccactgg gtgaggcagg 3360

cccccggcaa gggcctggag tgggtggcct ggatcagccc ctacggcggc agcacctact 3420

acgccgacag cgtgaagggc aggttcacca tcagcgccga caccagcaag aacaccgcct 3480

acctgcagat gaacagcctg agggccgagg acaccgccgt gtactactgc gccaggaggc 3540

actggcccgg cggcttcgac tactggggcc agggcaccct ggtgaccgtg agcagcgcca 3600

gcaccaaggg ccccagcgtg ttccccctgg cccccagcag caagagcacc agcggcggca 3660

ccgccgccct gggctgcctg gtgaaggact acttccccga gcccgtgacc gtgagctgga 3720

acagcggcgc cctgaccagc ggcgtgcaca ccttccccgc cgtgctgcag agcagcggcc 3780

tgtacagcct gagcagcgtg gtgaccgtgc ccagcagcag cctgggcacc cagacctaca 3840

tctgcaacgt gaaccacaag cccagcaaca ccaaggtgga caagaaggtg gagcccaaga 3900

gctgcgacaa gacccacacc tgccccccct gccccgcccc cgagctgctg ggcggcccca 3960

gcgtgttcct gttccccccc aagcccaagg acaccctgat gatcagcagg acccccgagg 4020

tgacctgcgt ggtggtggac gtgagccacg aggaccccga ggtgaagttc aactggtacg 4080

tggacggcgt ggaggtgcac aacgccaaga ccaagcccag ggaggagcag tacgccagca 4140

cctacagggt ggtgagcgtg ctgaccgtgc tgcaccagga ctggctgaac ggcaaggagt 4200

acaagtgcaa ggtgagcaac aaggccctgc ccgcccccat cgagaagacc atcagcaagg 4260

ccaagggcca gcccagggag ccccaggtgt acaccctgcc ccccagcagg gaggagatga 4320

ccaagaacca ggtgagcctg acctgcctgg tgaagggctt ctaccccagc gacatcgccg 4380

tggagtggga gagcaacggc cagcccgaga acaactacaa gaccaccccc cccgtgctgg 4440

acagcgacgg cagcttcttc ctgtacagca agctgaccgt ggacaagagc aggtggcagc 4500

agggcaacgt gttcagctgc agcgtgatgc acgaggccct gcacaaccac tacacccaga 4560

agagcctgag cctgagcccc ggcaagcgcc gcaagcgcgg ctccggcgag ggccgcggct 4620

ccctgctgac ctgcggcgac gtggaggaga accccggccc catgaagtac ctgctgccca 4680

ccgccgccgc cggcctgctg ctgctggccg cccagcccgc catggccgac atccagatga 4740

cccagagccc cagcagcctg agcgccagcg tgggcgacag ggtgaccatc acctgcaggg 4800

ccagccagga cgtgagcacc gccgtggcct ggtaccagca gaagcccggc aaggccccca 4860

agctgctgat ctacagcgcc agcttcctgt acagcggcgt gcccagcagg ttcagcggca 4920

gcggcagcgg caccgacttc accctgacca tcagcagcct gcagcccgag gacttcgcca 4980

cctactactg ccagcagtac ctgtaccacc ccgccacctt cggccagggc accaaggtgg 5040

agatcaagag gaccgtggcc gcccccagcg tgttcatctt cccccccagc gacgagcagc 5100

tgaagagcgg caccgccagc gtggtgtgcc tgctgaacaa cttctacccc agggaggcca 5160

aggtgcagtg gaaggtggac aacgccctgc agagcggcaa cagccaggag agcgtgaccg 5220

agcaggacag caaggacagc acctacagcc tgagcagcac cctgaccctg agcaaggccg 5280

actacgagaa gcacaaggtg tacgcctgcg aggtgaccca ccagggcctg agcagccccg 5340

tgaccaagag cttcaacagg ggcgagtgct gataagctga ccgagccctc ctctataaac 5400

ccaagattca aggctccaag cgataacctt ctcccgcctc ttccatccca ctgaatgatc 5460

gcgcagctgc aattaatcta gcaacattaa agattaagaa aaaatacggg tagaatcgga 5520

gtgcaccgat tgtgccaaga tggactcatc taggacaatt gggttatact ttgattctgc 5580

ccttccttct agcaacctgt tagcattccc gattgtctta cgagatgtag gagatggaaa 5640

gaagcaaatt actccgcaat ataggatccg gcgtcttgac tcgtggacag acagtaaaga 5700

agactcggta ttcatcacta cctacggatt catcttccag gttgggaatg aagaagtcac 5760

tgtcggcatg atcaatgata atcccaagcg agagttactc tccgctgcga tgctctgcct 5820

aggaagcgtc ccgaatgttg gagatcctgt tgagctggca agggcctgcc tcactatggt 5880

ggtaacatgc aagaagagtg caactaatac tgagagaatg gtcttctcag tagtgcaggc 5940

accccgggtg ctgcaaagct gtagggtcgt ggcagacaaa tactcgtcag tgaatgcagt 6000

taagcacgtg aaagcaccag agaagatccc ttggagtgaa accctagagt acaaggtgaa 6060

ttttgtctct ttgactgtgg tgccgaagaa ggatgtctac aagatcccaa ccgcagtatt 6120

gaaggtatcc ggctcgagcc tgtataatct tgcgctcaat gtcactattg atgtggaggt 6180

agacccaaag agcccgttag tcaagtccct ttctaggtcc gacagtggat actatgctaa 6240

tctcttctta catatcggac ttatgtccac tgtagataag aggggaaaga aagtgacatt 6300

tgaccagctg gagaggaaga taaggagact tgatctatct gtcgggctca gtgatgtgct 6360

cggaccttct gtgcttgtga aggcgagagg tgcacggact aggctgctgg cacctttctt 6420

ctctaacagc gggacagcct gctaccctat agcaaatgcc tctcctcagg tagctaagat 6480

actctggagt cagaccgcgt gcctgcggag tgtaaaaatc attattcaag cgggcaccca 6540

acgcgctgtc gcagtgactg ctgaccatga ggttacctct actaagatag agaagaggca 6600

taccatcgct aaatacaatc ccttcaagaa ataggccgta tccctgagac tgcaattcac 6660

ccgccttccc aaaacaccat gacaccagat aatgatctgt cttgattact tacagttagt 6720

ttccctgtct atcaaattag aaaaaacacg ggtagaagag ttcggatccc ggccggcgca 6780

ccaaaagcgc aagatgggcc ccagatcttc taccaggatc ccagtacctc taatgctgac 6840

catacggatc acgctggcac tgagttatgt ccgtctgaca agttctcttg atggcaggcc 6900

tcttgcagcc gcagggatcg tggtaacagg ggataaagca gttaacatat acacctcatc 6960

ccagacaggg tcaatcatag tcaagttact cccaaatatg cccaaggaca aagaggcatg 7020

tgcaaaagcc ccattggagg cttacaacag gacactgact actttgctta ccccccttgg 7080

tgattctatc cgcaggatac aagagtctgt gactacatcc ggaggaagga gacagagacg 7140

ctctataggt gccattattg gcagtgtagc tctaggggtt gcaacagctg cacagataac 7200

ggcagcctcg gctctgatac aagccaacca gaatgctgct aacatcctcc ggcttaagga 7260

gagcattgct gcaaccaatg aagctgtaca cgaggtcact ggcggattgt cacagttagc 7320

agtggcagtt ggaaagatgc agcaatttgt taatgaccag cttaataaca cagctcaaga 7380

gttggactgt ataaaaatta cacagcaggt tggtgtagaa ctcaacttat acttaactga 7440

attgactaca gtgttcgggc cacaaatcac ttcccctgcc ttaactcagt tgactgttca 7500

ggctctttac aatctggctg gtggtaatgt agattacttg ttgactaagt taggtgtagg 7560

gaacaaccag ctcagctcat tgattggtag cggcttgatc accggtaacc ctatttttta 7620

cgactcacag actcaactct tgggcataca ggtgacttta ccctcagtcg ggaacctaaa 7680

taatatgcgt gccacctact tggagacctt gtctgtaagc acaacaaagg gatttgcctc 7740

agcacttgtc ccaaaagtag tgacacaggt cggttctgtg atagaagagc ttgacacctc 7800

atactgtata gaagctgatt tggatttata ttgtacaaga atagtgacat tccctatgtc 7860

ccctggtatt tattcctgtt tgagcggcaa tacatcggct tgcatgtatt caaggactga 7920

aggcgcactt actacaccat acatgactct caaaggctca gttgttgcca attgccagat 7980

gacaacatgt agatgtgcag acccccgggg tatcatatca caaaattatg gagaggctgt 8040

gtctctaata gataagcact catgcaatgt cgtatcctta gacgggataa ctttgaggct 8100

cagtggggaa tttgatgcaa cttatcaaaa gaatatctca atattagatt ctcaagtact 8160

agtgacaggc aatctcgata tctcaactga gcttgggaat gtcaaccact caataagtaa 8220

tgctttggat aagttagagg aaagcaacag caaactagac aaagtcaatg tcaaactgac 8280

cagcacatct gctctcatta cctatattgt tttaactgtc atatctcttg ttcttggtat 8340

gcttagcctg gttctagcat gctatctgat gtacaagcaa aaagcgcaac gaaagacctt 8400

gttgtggctt ggaaataata ccctagatca gatgagagct actacaaaaa tgtgaatgca 8460

gatgagaggc agaggtatcc ccaatagcaa tctgtgtgtc aattctggca gcctgttaat 8520

cagaagaatt aagaaaaaac taccggatgt aggtgaacaa aagggaatat acgggtagaa 8580

cggcctgaga ggccacccct caatcgggag ccaggcccca ctacgtccgc tctaccgcat 8640

caccaacagc agtcttcagt catggacagc gcagttagcc aagttgcgct agagaatgat 8700

agaagagaag cgaataatac atggcgcttg gttttccgga tcgcagcctt acttttactg 8760

gtaataacct tagccgtctc tgcagtcgcc ctggcatata gtatggaggc tagcacacct 8820

ggcgaccttg taagcatacc gactgcgatc tctagggcag aggaaaggat tacatctgca 8880

ctcggttcca atcaagatgt agtagatagg atatacaagc aagtggccct tgagtctcca 8940

ttggcactgc taaataccga atctataatt atgaatgcaa taacgtctct ctcttatcaa 9000

atcaatggag ccacaaataa tagcgggtgt ggggcacctg ttcatgaccc agattacatc 9060

ggggggatag gtcaagaact tattgtagat gatacgagtg acgtcacatc attttatccc 9120

tctgcattcc aagaacacct gaattttatc ccggcgccca ctacaggatc aggctgcact 9180

cggataccct cattcgacat gagtgctacc cattactgtt acactcacaa tgtgatattg 9240

tctggctgca gagatcactc acactcacat cagtatttag cacttggtgt gcttcggaca 9300

tctgcaacag ggagggtatt cttttctact ctgcgttcca tcaacttgga tgacgcccaa 9360

aatcggaagt cttgcagtgt gagtgcaact cctttaggtt gtgatatgct gtgctctaaa 9420

atcacagaga ctgaggaaga ggattataaa tcagttatcc ccacatcgat ggtacatgga 9480

aggttagggc ttgacggcca ataccatgag aaggacctag acgtcacaac actatttagg 9540

gactgggtgg caaattaccc aggagtagga ggtgggtctt ttattaacaa tcgcgtatgg 9600

ttcccggtct acggagggct aaaacccagc tcgcctagtg acactgcaca agaagggaga 9660

tacgtaatat acaagcgata caatgacaca tgcccggatg agcaagatta ccagattcgg 9720

atggctaagt catcgtataa gcctaggcgg tttggtggaa aacgcgtaca gcaggccatc 9780

ctatccatca aggtgtcaac atccttgggt gaggacccgg tgttgactgt accgcctaat 9840

acggtcgcac tcatgggggc cgaaggcaga gttctcacag tggggacatc tcatttctta 9900

tatcagcgag ggtcatcata cttctctccc gctttgttat accctatgac agtcaacaac 9960

aaaacagcca ctcttcataa tccttataca ttcaatgctt tcactcggcc aggtagtgtc 10020

ccttgccagg cttcagcaag atgccccaac tcatgtgtta ccggagtcta tactgatcca 10080

tatcccttag tcttccatag gaaccacacc ttgcgagggg tattcggaac aatgcttgat 10140

gacgaacaag caagactcaa ccctgtatct gcagtatttg ataacatatc ccgcagtcgc 10200

ataactcggg taagttcaag cagtaccagg gcagcataca cgacatcaac gtgttttaaa 10260

gttgtcaaga ccaataaaac ctattgcctc agcattgcag aaatatccaa taccctcttc 10320

ggggaattca gaattgtccc cttactagtt gagattctca aggatggtgg ggtttaagaa 10380

gctaggaatg gtcggttgag tcaaccgtga gaaggcgggg aagatgatat tgcatcatct 10440

atcttctgta acaccaagaa tcaaatcgaa taccagtgcg agctcgaatc ctacgctgcc 10500

agtcagccat aatcggctgg tgctaatgtg gttagcctga atcttgtcga tagtcacttg 10560

attaagaaaa aatgtggacg gtagtgaaat ataaggcaaa gcagctcaca gaggacagca 10620

cgggtaggac atggcgggct ccggacccga aagggcagag catcagatta tcctaccaga 10680

gtcacatctg tcctcaccat tggtcaagca taaattgctt tattactgga aattaactgg 10740

gctaccgctt cctgacgaat gtgacttcga ccatcttatt atcagccgac aatggaagaa 10800

ggtacttgaa tcggccaccc ctgacattga gagaatgata aaactagggc gggcagtaca 10860

ccagactctc aaccacaatt ccaggataac cggagtactc catccccggt gtttagagga 10920

actggctagt attgaggttc ctgattcaac caacaaattt cggaagatcg aaaaaaagat 10980

ccagattcac aacacaaggt atggagaact gttcacaaga ttatgcacgc atgtagaaaa 11040

gaaactattg gggtcatctt ggtctaacaa tgtcccacgg tcagaggaat tcaacagcat 11100

ccgtacagat ccggcatttt ggtttcactc aaaatggtcc acagccaaat ttgcatggct 11160

ccatataaaa caggtccaga ggcatctgat tgtagcagca agaacaaggt ccgcagtcaa 11220

caaattagtg acgctgaccc ataaggtagg ccaaatcttt gttactcctg agcttgtcat 11280

tgtgacacat acagatgaga acaagttcac gtgtcttacc caggaacttg tgttgatgta 11340

tgcagatatg atggagggca gagatatggt cagcataata tcatccacgg cggcacatct 11400

taggagctta tcagagaaaa ttgatgatat tctgcggtta gtagatgctc tggcaagaga 11460

tttgggcaat caagtctacg atgttgtagc actaatggag ggattcgcat acggcgctgt 11520

tcagctgctt gaaccgtcag gtacatttgc gggggatttc ttcgcattca acctgcagga 11580

gctcaaagat actctaaccg gactcctccc caaggatatc gcagaatctg tgactcacgc 11640

aatcgcaacc atattctctg gcttagaaca aaatcaagca gctgagatgt tgtgcctgtt 11700

gcgtctgtgg ggtcacccac tactcgagtc ccgtattgca gcaaaagcag ttaggagtca 11760

aatgtgcgca ccaaaaatgg tagactttga tatgatcctc caggtattat ctttctttaa 11820

aggaacaatc atcaatggat atagaaagaa gaatgcaggc gtgtggccac gtgtcaagat 11880

agatacgata tacgggaagg tcatagggca gctacacgca gattctgcag agatttcaca 11940

tgatgtcatg ttgagggaat acaagagttt atctgcactt gaattcgagc catgtataga 12000

gtatgaccct gtcaccaatc tgagcatgtt tttaaaagac aaggcaatcg cacacccgaa 12060

agacaactgg ctcgcttcgt ttaggcgaaa ccttctctct gaggaccaga agaaacatgt 12120
aagagaggca acctcaacta accgcctctt gatagagttc ttagagtcaa atgattttga 12180
tccatataaa gagatggaat atctgacgac ccttgagtac ctaagagatg ataatgtggc 12240
agtatcatac tcactcaaag aaaaagaggt gaaagttaat gggcggattt tcgcaaagct 12300
aacaaataga ttaaggaatt gtcaggtaat ggcagaaggg atcctagctg accagattgc 12360
acctttcttc cagggaaatg gggtcattca ggatagcata tctttgacta aaagtatgct 12420
agcgatgagt caactgtctt ttcaacagca caagaaacgt attactgact gcaaagaaag 12480
agtatcctca aaccgcaatc atgatccgaa gagcaagaat cgtcggagag ttgccacttt 12540
tgtaactacc gacctgcaaa agtattgtct caattggaga tatcagacag tcaagctgtt 12600
cgctcatgcc atcaatcagc tgatgggctt acctcacttc ttcgagtgga ttcatcttag 12660
actaatggat actacgatgt ttgtagggga ccctttcaat cctccaagtg accctacgga 12720
ctgtgatcta tcaagagtcc caaatgatga catatatatt gtcagtgcta ggggaggcat 12780
tgagggatta tgccagaagc tatggacaat gatctcaatt gctgcaatcc agcttgctgc 12840
agcaagatca cattgtcgcg ttgcctgtat ggtacaaggc gacaatcaag taatagctat 12900
aacgagagag gtaagatcag atgactcccc agagatggtg ttaacacaat tgcatcaagc 12960
cagtgataat ttcttcaagg aattgattca tgtcaatcat ttgatcggcc ataatttgaa 13020
ggatcgtgaa accatcaggt cagacacgtt cttcatatac agcaaacgaa tattcaaaga 13080
tggagcaata ctcagtcagg tcctcaaaaa ctcatctaaa ttagtgctaa tatcaggcga 13140
ccttagtgaa aacactgtaa tgtcttgtgc caacattgca tctactgtag cacggttatg 13200
cgagaacggg cttcctaagg atttctgtta ttacttaaac tacttaatga gttgcgtgca 13260
gacatacttc gattccgaat tttccatcac caacaactcg caactcgatt ctaaccagtc 13320
gtggatagag gacatttctt ttgtgcactc atatgtcctg acccctgctc aactgggggg 13380
actgagtaac cttcaatact caaggctcta cacaaggaac atcggcgacc cgggaaccac 13440
tgctttcgca gagatcaaga gattagaggc agtggggtta ctgagtccta gcattatgac 13500
taacatctta actaggccgc ctggaaatgg agattgggcc agtctgtgca acgatccata 13560
ctcctttaat tttgagactg tcgcaagccc aaatattgtc cttaagaaac atacacaaag 13620
agtcctattt gaaacttgct cgaatccctt attatctgga gtgcacacag aggataatga 13680
ggcagaagag aaggcattgg ctgaattctt gctcaatcaa gaaatgattc atccacgtgt 13740
cgcgcatgct atcatggaag caagctctgt aggtaggaga aagcaaattc aggggcttgt 13800
tgacacaaca aacaccgtga ttaagattgc actgactagg aggccactcg gcatcaagag 13860
gctgatgcgg atagtcaatt actcgagcat gcatgcaatg ctatttagag atgatgtttt 13920
ctcgtctaat agatccaacc atcccttagt ctcttctgat atgtgttctc agacactggc 13980

agactatgca cggaacagaa gctggtcacc tttgacaggg ggtagaaaaa tactgggtgt 14040

atctaatcct gataccatag aacttgtaga gggtgagatc cttagtgtca gtggagggtg 14100

cactagatgt gacagtgggg atgaacagtt tacttggttc catcttccaa gcaatataga 14160

gctaaacgat gacaccagca agaatcctcc aatgagagtg ccatatctcg ggtcaaagac 14220

tcaagagagg agagccgcct cgcttgcgaa aatagctcat atgtcaccac acgtgaaggc 14280

ggctctaagg gcgtcatctg tgttaatctg ggcttatggg gacaacgaaa taaactggac 14340

tgctgccctt aagattgcaa ggtctcggtg caacataagc tcagagtatc ttcgactatt 14400

gtcacccttg cctacagctg ggaatctcca acatagattg gacgacggca taactcagat 14460

gacattcacc cctgcatctc tatacagggt gtcaccttac attcatatat ctaatgattc 14520

tcaaaggcta tttactgaag aaggagtcaa agaggggaat gtggtttatc agcaaatcat 14580

gctcttgggt ttgtccttaa ttgagtcact cttcccaatg acaacaacca agacatatga 14640

tgaaatcaca ttgcacctcc acagtaaatt tagctgctgt atcagggaag cacctgttgc 14700

agttcctttc gagctactcg ggtggcaccc ggaactaagg gcagtaacct caaataagtt 14760

tatgtatgat cctagccctg tatcagaggg agactttgcg agacttgact tagctatctt 14820

taagagttat gaacttaatt tagagtcata ttccacaata gagctaatga acgttctttc 14880

aatatctagt gggaagttga ttggccagtc tgtggtttct tatgatgaag atacctccat 14940

aaagaatgac gctataatag tgtatgacaa cacacggaat tggatcagcg aagctcagaa 15000

ttcagatgtg gtccgcctat tcgagtatgc ggcactcgaa gtgctcctcg actgttctta 15060

tcaactctac tatctgagag taagaggcct aaacaatatc gtcctgtaca tgagtgattt 15120

atacaagaat atgccaggaa ttctactctc taatattgca gccacaatat ctcaccctgt 15180

catccattca aggttgaatg cagtaggtct ggtcaaccat gacgggtcac accaacttgc 15240

agacacagat ttcattgaaa tgtctgcaaa gctgctagtc tcttgcactc gacgcgtggt 15300

ctcaggttta catgcaggga ataagtatga cctgctgttt ccatctgtct tggatgataa 15360

cctaagtgag aagatgcttc agttgatatc ccaattatgc tgtctgtata cggtgctctt 15420

tgctacaaca agagaaatcc cgaaaataag aggcttatct gcagaagaga aatgttcagt 15480

acttactgag tacctactgt cagatgctgt gaaaccgtta cttgggtccg agcaagtgag 15540

ctctatcatg tctcccaaca tagttacgtt cccagccaat ctgtattaca tgtctaggaa 15600

gagccttaat ttgatcaggg agagagagga caggatactt atcttggcat tgttgttccc 15660

tcaagaaccg ctgctcgagt ttcctctggt acgagatatt ggtgctcgtg taaaagatcc 15720

atttacccta caacctgcgg cgtttttaca agagttagat ttgagtgctc cggcaaggta 15780

tgacgcattc acaatcagtc aggcgcattc tgaacacata ttgccaaacc cagaggaaga 15840

tcacttagta cgatacttgt tcagaggaat agggactgcg tcctcctctt ggtataaggc 15900

atcccatctt ctttctgtac ccgaggtcag atgtgcaagg catgggaact ccttatattt 15960

agcagaagga agtggagcca tcatgagtct tctcgaattg catataccac acgaaactat 16020

ctattacaat acacttttct cgaacgagat gaacccccca cagcgacatt tcggaccaac 16080

tccaacacag tttctgaatt cggttgtttt taggaattta caggcggaag taccatgcaa 16140

ggatggattt gtccaggagt tccgtccgtt atggagagag aatacagaag aaagcgatct 16200

gacctcagat aaagcagtgg gatatatcac atctgtagtg ccgtacaggt ctgtatcatt 16260

gctgcattgt gacattgaaa tccctccagg atctaatcaa agcttactag atcagctggc 16320

taccaatctg tctctgattg ccatgcattc tgtgaaggag ggcggggtcg tgattatcaa 16380

agtactgtat gcaatgggat attacttcca tctactcatg aacttgttta ctccatgttc 16440

cacgaaagga tatattctct ctaatggcta tgcctgtaga ggggatatgg agtgttacct 16500

gatatttgtc atgggctacc taggcgggcc tacatttgta catgaggtgg tgaggatggc 16560

aaaaacgcta gtacagcggc atggcacact tttgtctaag tcagatgaga ttacactgac 16620

taggttattt acttcacagc aacagcgtgt aacagacatc ctatccagcc ctttaccgag 16680

actaatgaag tacttgagag agaatattga tactgcactg attgaagccg ggggacagcc 16740

tgtccgtcca ttctgtgcag agagtttagt gagcacacta acagacatga ctcagacaac 16800

ccagatcatc gccagccaca ttgatacagt cattcgatct gtgatctata tggaagcaga 16860

gggtgatctt gctgacacag tattcttatt taccccttac aacctctcta tggacgggaa 16920

aaagagaaca tcacttaaac agtgcacaag acagatctta gaggtcacaa tactgggtct 16980

cagagtcaaa aatctcaata aagtaggtga tgtaatcagc ctagtactca gaggtatgat 17040

ttctctggag gaccttctcc cactgagaac ctacttgaag tgtagtacct gccctaagta 17100

tttgaaggct gtcctaggta ttaccaaact caaagaaatg ttcacagacg cctctttatt 17160

atacttgact cgtgctcaac aaaaattcta catgaaaact ataggcaatg cagtcaaggg 17220

atactatagt aactgtgact cttaaaggca accacatatc aataggccct ctttctagcc 17280

gatcgtattc ttgttgactt cattatacca tattagaaaa aaattgaatt ccgacccttt 17340

aagactcgta ttcggattca aataattatc tcagaaaaaa agtgcacgta gttgttcttg 17400

attatagtcc cgtcattcac caagtctttg tttggt 17436

<210> 15
<211> 17448
<212> DNA
<213> Newcastle disease virus

<220>

<223> Mut HN (F277L)/M(G165W)/F(F117S)/F(F190L)/L(V757I)/L(F1551S)/L(R1
      700L)-Bevacizumab

<400> 15
accaaacaga gaatccgtga gatacgataa aaggcgaagg agcaatcgaa gtcgtacggg      60

tagaaggtgt gaatctcgag tgcgagaccg aagcttaaac tcgagagagc cttctgccaa     120

catgtcttcc gtatttgacg agtatgaaca gctcctcgcg gctcagactc gccctaatgg     180

agctcacgga ggaggagaga aggggagcac cttaaaagtt gaagtcccgg tattcactct     240

taacagtgac gacccagaag atagatggaa ctttgcagta ttctgtctta ggattgctgt     300

tagcgaagat gccaacaaac cactcagaca aggtgctctc atatctctct tatgctccca     360

ttctcaagtg atgaggaacc atgtcgccct tgcagggaaa cagaatgagg ccacactggc     420

tgttcttgag atcgatggtt tcaccaacag cgtgcctcaa tttaacaaca ggagtggagt     480

gtctgaggag agagcacaga gattcttgat gatagcaggg tccctccctc gggcatgcag     540

caacggtacc ccgttcatca cagctggggt tgaagatgat gcaccagaag acatcactga     600

tactctggaa agaatcatct ctatccaggc tcaagtatgg gtcacggtag caaaggccat     660

gactgcatat gagacagcgg atgagtcgga aacaagaaga atcaataagt atatgcagca     720

aggcagagtc caaaagaagt acatcctcca ccccgtatgt aggagcgcaa ttcaactcac     780

gattagacaa tctctggcag tccgcatttt cttggttagc gagcttaaga gaggccgcaa     840

cacggcaggt ggaagttcca cttattatag cttggtaggg gacatagact catacatcag     900

gaacaccggg cttactgcat tcttcctgac actcaggtat ggaattaaca ccaagacatc     960

agcccttgca ctcagcagcc tcgcaggcga tatccaaaaa atgaagcagc tcatgcgtct    1020

atatcggatg aaaggagata atgcgccgta catgacactg ctcggtgaca gtgaccagat    1080

gagctttgca ccggctgagt atgcacaact ctactctttt gcaatgggta tggcatccgt    1140

cctagacaaa ggaactggca agtaccagtt cgccagagac tttatgagca catcattctg    1200

gagacttgga gtagagtatg ctcgggctca gggaagcagc atcaatgagg atatggctgc    1260

cgagctaaag ctgaccccag cggcaaggag gggcctggca gctgctgccc aacgagcgtc    1320

cgaggagacc ggcagcatgg acattcccac tcaacaagct ggggtcctca cagggctcag     1380

cgacggaggc cctcaagccc cacaaggtgg actgaacagg tcgcaagggc aaccggacgc    1440

cggagatggg gagacccaat ttttagattt gatgagagca gtggcaaata gtatgagaga    1500

agcgccaaac tctgtgcaga acaccactca gcaggagcct ccatctactc ctgggccatc    1560

tcaagacaac gacaccgact ggggatactg accgacaaca ctcagcctgc ctccatcgaa    1620

ttatctcgat tcttctgcct gtaacctaac ccctggtcca cagtctcaca cggccaaacc    1680

cgcaaacgag cccccccctc tctccccccc cctccagcca cacggctcca accgcccaaa    1740

acaacacggg cacaactcga ctcactcata atctacacag agccgaagat cttagaaaaa    1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgttccgc ggcgtgcagt 3240

gcgaggtgca gctggtggag agcggcggcg gcctggtgca gcccggcggc agcctgaggc 3300

tgagctgcgc cgccagcggc tacaccttca ccaactacgg catgaactgg gtgaggcagg 3360

cccccggcaa gggcctggag tgggtgggct ggatcaacac ctacaccggc gagcccacct 3420

acgccgccga cttcaagagg aggttcacct tcagcctgga caccagcaag agcaccgcct 3480

acctgcagat gaacagcctg agggccgagg acaccgccgt gtactactgc gccaagtacc 3540

cccactacta cggcagcagc cactggtact tcgacgtgtg gggccagggc accctggtga 3600

ccgtgagcag cgccagcacc aagggcccca gcgtgttccc cctggccccc agcagcaaga 3660

gcaccagcgg cggcaccgcc gccctgggct gcctggtgaa ggactacttc cccgagcccg 3720
tgaccgtgag ctggaacagc ggcgccctga ccagcggcgt gcacaccttc cccgccgtgc 3780
tgcagagcag cggcctgtac agcctgagca gcgtggtgac cgtgcccagc agcagcctgg 3840
gcacccagac ctacatctgc aacgtgaacc acaagcccag caacaccaag gtggacaaga 3900
aggtggagcc caagagctgc gacaagaccc acacctgccc cccctgcccc gcccccgagc 3960
tgctgggcgg ccccagcgtg ttcctgttcc cccccaagcc caaggacacc ctgatgatca 4020
gcaggacccc cgaggtgacc tgcgtggtgg tggacgtgag ccacgaggac cccgaggtga 4080
agttcaactg gtacgtggac ggcgtggagg tgcacaacgc caagaccaag cccagggagg 4140
agcagtacaa cagcacctac agggtggtga gcgtgctgac cgtgctgcac caggactggc 4200
tgaacggcaa ggagtacaag tgcaaggtga gcaacaaggc cctgcccgcc cccatcgaga 4260
agaccatcag caaggccaag ggccagccca gggagcccca ggtgtacacc ctgcccccca 4320
gcagggagga gatgaccaag aaccaggtga gcctgacctg cctggtgaag ggcttctacc 4380
ccagcgacat cgccgtggag tgggagagca acggccagcc cgagaacaac tacaagacca 4440
ccccccccgt gctggacagc gacggcagct tcttcctgta cagcaagctg accgtggaca 4500
agagcaggtg gcagcagggc aacgtgttca gctgcagcgt gatgcacgag gccctgcaca 4560
accactacac ccagaagagc ctgagcctga gccccggcaa gcgccgcaag cgcggctccg 4620
gcgagggccg cggctccctg ctgacctgcg gcgacgtgga ggagaacccc ggccccatga 4680
agtacctgct gcccaccgcc gccgccggcc tgctgctgct ggccgcccag cccgccatgg 4740
ccgacatcca gatgacccag agccccagca gcctgagcgc cagcgtgggc gacagggtga 4800
ccatcacctg cagcgccagc caggacatca gcaactacct gaactggtac cagcagaagc 4860
ccggcaaggc ccccaaggtg ctgatctact tcaccagcag cctgcacagc ggcgtgccca 4920
gcaggttcag cggcagcggc agcggcaccg acttcaccct gaccatcagc agcctgcagc 4980
ccgaggactt cgccacctac tactgccagc agtacagcac cgtgccctgg accttcggcc 5040
agggcaccaa ggtggagatc aagaggaccg tggccgcccc cagcgtgttc atcttccccc 5100
ccagcgacga gcagctgaag agcggcaccg ccagcgtggt gtgcctgctg aacaacttct 5160
accccaggga ggccaaggtg cagtggaagg tggacaacgc cctgcagagc ggcaacagcc 5220
aggagagcgt gaccgagcag gacagcaagg acagcaccta cagcctgagc agcaccctga 5280
ccctgagcaa ggccgactac gagaagcaca aggtgtacgc ctgcgaggtg acccaccagg 5340
gcctgagcag ccccgtgacc aagagcttca acaggggcga gtgctgagct gaccgagccc 5400
tcctctataa acccaagatt caaggctcca agcgataacc ttctcccgcc tcttccatcc 5460
cactgaatga tcgcgcagct gcaattaatc tagcaacatt aaagattaag aaaaaatacg 5520
ggtagaatcg gagtgcaccg attgtgccaa gatggactca tctaggacaa ttgggttata 5580

ctttgattct gcccttcctt ctagcaacct gttagcattc ccgattgtct tacgagatgt 5640

aggagatgga aagaagcaaa ttactccgca atataggatc cggcgtcttg actcgtggac 5700

agacagtaaa gaagactcgg tattcatcac tacctacgga ttcatcttcc aggttgggaa 5760

tgaagaagtc actgtcggca tgatcaatga taatcccaag cgagagttac tctccgctgc 5820

gatgctctgc ctaggaagcg tcccgaatgt tggagatcct gttgagctgg caagggcctg 5880

cctcactatg gtggtaacat gcaagaagag tgcaactaat actgagagaa tggtcttctc 5940

agtagtgcag gcaccccggg tgctgcaaag ctgtagggtc gtggcagaca aatactcgtc 6000

agtgaatgca gttaagcacg tgaaagcacc agagaagatc ccttggagtg aaaccctaga 6060

gtacaaggtg aattttgtct ctttgactgt ggtgccgaag aaggatgtct acaagatccc 6120

aaccgcagta ttgaaggtat ccggctcgag cctgtataat cttgcgctca atgtcactat 6180

tgatgtggag gtagacccaa agagcccgtt agtcaagtcc ctttctaggt ccgacagtgg 6240

atactatgct aatctcttct tacatatcgg acttatgtcc actgtagata agaggggaaa 6300

gaaagtgaca tttgaccagc tggagaggaa gataaggaga cttgatctat ctgtcgggct 6360

cagtgatgtg ctcggacctt ctgtgcttgt gaaggcgaga ggtgcacgga ctaggctgct 6420

ggcacctttc ttctctaaca gcgggacagc ctgctaccct atagcaaatg cctctcctca 6480

ggtagctaag atactctgga gtcagaccgc gtgcctgcgg agtgtaaaaa tcattattca 6540

agcgggcacc caacgcgctg tcgcagtgac tgctgaccat gaggttacct ctactaagat 6600

agagaagagg cataccatcg ctaaatacaa tcccttcaag aaataggccg tatccctgag 6660

actgcaattc acccgccttc ccaaaacacc atgacaccag ataatgatct gtcttgatta 6720

cttacagtta gtttccctgt ctatcaaatt agaaaaaaca cgggtagaag agttcggatc 6780

ccggccggcg caccaaaagc gcaagatggg ccccagatct tctaccagga tcccagtacc 6840

tctaatgctg accatacgga tcacgctggc actgagttat gtccgtctga caagttctct 6900

tgatggcagg cctcttgcag ccgcagggat cgtggtaaca ggggataaag cagttaacat 6960

atacacctca tcccagacag ggtcaatcat agtcaagtta ctcccaaata tgcccaagga 7020

caaagaggca tgtgcaaaag ccccattgga ggcttacaac aggacactga ctactttgct 7080

tacccccctt ggtgattcta tccgcaggat acaagagtct gtgactacat ccggaggaag 7140

gagacagaga cgctctatag gtgccattat tggcagtgta gctctagggg ttgcaacagc 7200

tgcacagata acggcagcct cggctctgat acaagccaac cagaatgctg ctaacatcct 7260

ccggcttaag gagagcattg ctgcaaccaa tgaagctgta cacgaggtca ctggcggatt 7320

gtcacagtta gcagtggcag ttggaaagat gcagcaattt gttaatgacc agcttaataa 7380

cacagctcaa gagttggact gtataaaaat tacacagcag gttggtgtag aactcaactt 7440

atacttaact gaattgacta cagtgttcgg gccacaaatc acttcccctg ccttaactca 7500

gttgactgtt caggctcttt acaatctggc tggtggtaat gtagattact tgttgactaa 7560

gttaggtgta gggaacaacc agctcagctc attgattggt agcggcttga tcaccggtaa 7620

ccctattttt tacgactcac agactcaact cttgggcata caggtgactt taccctcagt 7680

cgggaaccta aataatatgc gtgccaccta cttggagacc ttgtctgtaa gcacaacaaa 7740

gggatttgcc tcagcacttg tcccaaaagt agtgacacag gtcggttctg tgatagaaga 7800

gcttgacacc tcatactgta tagaagctga tttggattta tattgtacaa gaatagtgac 7860

attccctatg tcccctggta tttattcctg tttgagcggc aatacatcgg cttgcatgta 7920

ttcaaggact gaaggcgcac ttactacacc atacatgact ctcaaaggct cagttgttgc 7980

caattgccag atgacaacat gtagatgtgc agaccccccg ggtatcatat cacaaaatta 8040

tggagaggct gtgtctctaa tagataagca ctcatgcaat gtcgtatcct tagacgggat 8100

aactttgagg ctcagtgggg aatttgatgc aacttatcaa aagaatatct caatattaga 8160

ttctcaagta ctagtgacag gcaatctcga tatctcaact gagcttggga atgtcaacca 8220

ctcaataagt aatgctttgg ataagttaga ggaaagcaac agcaaactag acaaagtcaa 8280

tgtcaaactg accagcacat ctgctctcat tacctatatt gttttaactg tcatatctct 8340

tgttcttggt atgcttagcc tggttctagc atgctatctg atgtacaagc aaaaagcgca 8400

acgaaagacc ttgttgtggc ttggaaataa taccctagat cagatgagag ctactacaaa 8460

aatgtgaatg cagatgagag gcagaggtat ccccaatagc aatctgtgtg tcaattctgg 8520

cagcctgtta atcagaagaa ttaagaaaaa actaccggat gtaggtgaac aaaaaggaat 8580

atacgggtag aacggcctga gaggccaccc ctcaatcggg agccaggccc cactacgtcc 8640

gctctaccgc atcaccaaca gcagtcttca gtcatggaca gcgcagttag ccaagttgcg 8700

ctagagaatg atagaagaga agcgaataat acatggcgct tggttttccg gatcgcagcc 8760

ttacttttac tggtaataac cttagccgtc tctgcagtcg ccctggcata tagtatggag 8820

gctagcacac ctggcgacct tgtaagcata ccgactgcga tctctagggc agaggaaagg 8880

attacatctg cactcggttc caatcaagat gtagtagata ggatatacaa gcaagtggcc 8940

cttgagtctc cattggcact gctaaatacc gaatctataa ttatgaatgc aataacgtct 9000

ctctcttatc aaatcaatgg agccacaaat aatagcgggt gtggggcacc tgttcatgac 9060

ccagattaca tcggggggat aggtcaagaa cttattgtag atgatacgag tgacgtcaca 9120

tcattttatc cctctgcatt ccaagaacac ctgaatttta tcccggcgcc cactacagga 9180

tcaggctgca ctcggatacc ctcattcgac atgagtgcta cccattactg ttacactcac 9240

aatgtgatat tgtctggctg cagagatcac tcacactcac atcagtattt agcacttggt 9300

gtgcttcgga catctgcaac agggagggta ttcttttcta ctctgcgttc catcaacttg 9360

gatgacgccc aaaatcggaa gtcttgcagt gtgagtgcaa ctcctttagg ttgtgatatg 9420

ctgtgctcta aaatcacaga gactgaggaa gaggattata aatcagttat ccccacatcg 9480

atggtacatg gaaggttagg gcttgacggc caataccatg agaaggacct agacgtcaca 9540

acactattta gggactgggt ggcaaattac ccaggagtag gaggtgggtc ttttattaac 9600

aatcgcgtat ggttcccggt ctacggaggg ctaaaaccca gctcgcctag tgacactgca 9660

caagaaggga gatacgtaat atacaagcga tacaatgaca catgcccgga tgagcaagat 9720

taccagattc ggatggctaa gtcatcgtat aagcctaggc ggtttggtgg aaaacgcgta 9780

cagcaggcca tcctatccat caaggtgtca acatccttgg gtgaggaccc ggtgttgact 9840

gtaccgccta atacggtcgc actcatgggg gccgaaggca gagttctcac agtggggaca 9900

tctcatttct tatatcagcg agggtcatca tacttctctc ccgctttgtt ataccctatg 9960

acagtcaaca acaaaacagc cactcttcat aatccttata cattcaatgc tttcactcgg 10020

ccaggtagtg tcccttgcca ggcttcagca agatgcccca actcatgtgt taccggagtc 10080

tatactgatc catatccctt agtcttccat aggaaccaca ccttgcgagg ggtattcgga 10140

acaatgcttg atgacgaaca agcaagactc aaccctgtat ctgcagtatt tgataacata 10200

tcccgcagtc gcataactcg ggtaagttca agcagtacca gggcagcata cacgacatca 10260

acgtgtttta aagttgtcaa gaccaataaa acctattgcc tcagcattgc agaaatatcc 10320

aataccctct tcggggaatt cagaattgtc cccttactag ttgagattct caaggatggt 10380

ggggtttaag aagctaggaa tggtcggttg agtcaaccgt gagaaggcgg ggaagatgat 10440

attgcatcat ctatcttctg taacaccaag aatcaaatcg aataccagtg cgagctcgaa 10500

tcctacgctg ccagtcagcc ataatcggct ggtgctaatg tggttagcct gaatcttgtc 10560

gatagtcact tgattaagaa aaaatgtgga cggtagtgaa atataaggca aagcagctca 10620

cagaggacag cacgggtagg acatggcggg ctccggaccc gaaagggcag agcatcagat 10680

tatcctacca gagtcacatc tgtcctcacc attggtcaag cataaattgc tttattactg 10740

gaaattaact gggctaccgc ttcctgacga atgtgacttc gaccatctta ttatcagccg 10800

acaatggaag aaggtacttg aatcggccac ccctgacatt gagagaatga taaaactagg 10860

gcgggcagta caccagactc tcaaccacaa ttccaggata accggagtac tccatccccg 10920

gtgtttagag gaactggcta gtattgaggt tcctgattca accaacaaat ttcggaagat 10980

cgaaaaaaag atccagattc acaacacaag gtatggagaa ctgttcacaa gattatgcac 11040

gcatgtagaa aagaaactat tggggtcatc ttggtctaac aatgtcccac ggtcagagga 11100

attcaacagc atccgtacag atccggcatt ttggtttcac tcaaaatggt ccacagccaa 11160

atttgcatgg ctccatataa aacaggtcca gaggcatctg attgtagcag caagaacaag 11220

gtccgcagtc aacaaattag tgacgctgac ccataaggta ggccaaatct ttgttactcc 11280

tgagcttgtc attgtgacac atacagatga gaacaagttc acgtgtctta cccaggaact 11340

tgtgttgatg tatgcagata tgatggaggg cagagatatg gtcagcataa tatcatccac 11400

ggcggcacat cttaggagct tatcagagaa aattgatgat attctgcggt tagtagatgc 11460

tctggcaaga gatttgggca atcaagtcta cgatgttgta gcactaatgg agggattcgc 11520

atacggcgct gttcagctgc ttgaaccgtc aggtacattt gcgggggatt tcttcgcatt 11580

caacctgcag gagctcaaag atactctaac cggactcctc cccaaggata tcgcagaatc 11640

tgtgactcac gcaatcgcaa ccatattctc tggcttagaa caaaatcaag cagctgagat 11700

gttgtgcctg ttgcgtctgt ggggtcaccc actactcgag tcccgtattg cagcaaaagc 11760

agttaggagt caaatgtgcg caccaaaaat ggtagacttt gatatgatcc tccaggtatt 11820

atctttcttt aaaggaacaa tcatcaatgg atatagaaag aagaatgcag gcgtgtggcc 11880

acgtgtcaag atagatacga tatacgggaa ggtcataggg cagctacacg cagattctgc 11940

agagatttca catgatgtca tgttgaggga atacaagagt ttatctgcac ttgaattcga 12000

gccatgtata gagtatgacc ctgtcaccaa tctgagcatg tttttaaaag acaaggcaat 12060

cgcacacccg aaagacaact ggctcgcttc gtttaggcga aaccttctct ctgaggacca 12120

gaagaaacat gtaagagagg caacctcaac taaccgcctc ttgatagagt tcttagagtc 12180

aaatgatttt gatccatata aagagatgga atatctgacg acccttgagt acctaagaga 12240

tgataatgtg gcagtatcat actcactcaa agaaaaagag gtgaaagtta atgggcggat 12300

tttcgcaaag ctaacaaata gattaaggaa ttgtcaggta atggcagaag ggatcctagc 12360

tgaccagatt gcacctttct tccagggaaa tggggtcatt caggatagca tatctttgac 12420

taaaagtatg ctagcgatga gtcaactgtc tttcaacagc aacaagaaac gtattactga 12480

ctgcaaagaa agagtatcct caaaccgcaa tcatgatccg aagagcaaga atcgtcggag 12540

agttgccact tttgtaacta ccgacctgca aaagtattgt ctcaattgga gatatcagac 12600

agtcaagctg ttcgctcatg ccatcaatca gctgatgggc ttacctcact tcttcgagtg 12660

gattcatctt agactaatgg atactacgat gtttgtaggg gaccctttca atcctccaag 12720

tgaccctacg gactgtgatc tatcaagagt cccaaatgat gacatatata ttgtcagtgc 12780

taggggaggc attgagggat tatgccagaa gctatggaca atgatctcaa ttgctgcaat 12840

ccagcttgct gcagcaagat cacattgtcg cgttgcctgt atggtacaag gcgacaatca 12900

agtaatagct ataacgagag aggtaagatc agatgactcc ccagagatgg tgttaacaca 12960

attgcatcaa gccagtgata atttcttcaa ggaattgatt catgtcaatc atttgatcgg 13020

ccataatttg aaggatcgtg aaaccatcag gtcagacacg ttcttcatat acagcaaacg 13080

aatattcaaa gatggagcaa tactcagtca ggtcctcaaa aactcatcta aattagtgct 13140

aatatcaggc gaccttagtg aaaacactgt aatgtcttgt gccaacattg catctactgt 13200

agcacggtta tgcgagaacg ggcttcctaa ggatttctgt tattacttaa actacttaat 13260

gagttgcgtg cagacatact tcgattccga attttccatc accaacaact cgcaactcga 13320

ttctaaccag tcgtggatag aggacatttc ttttgtgcac tcatatgtcc tgacccctgc 13380

tcaactgggg ggactgagta accttcaata ctcaaggctc tacacaagga acatcggcga 13440

cccgggaacc actgctttcg cagagatcaa gagattagag gcagtggggt tactgagtcc 13500

tagcattatg actaacatct taactaggcc gcctggaaat ggagattggg ccagtctgtg 13560

caacgatcca tactccttta attttgagac tgtcgcaagc ccaaatattg tccttaagaa 13620

acatacacaa agagtcctat ttgaaacttg ctcgaatccc ttattatctg gagtgcacac 13680

agaggataat gaggcagaag agaaggcatt ggctgaattc ttgctcaatc aagaaatgat 13740

tcatccacgt gtcgcgcatg ctatcatgga agcaagctct gtaggtagga gaaagcaaat 13800

tcaggggctt gttgacacaa caaacaccgt gattaagatt gcactgacta ggaggccact 13860

cggcatcaag aggctgatgc ggatagtcaa ttactcgagc atgcatgcaa tgctatttag 13920

agatgatgtt ttctcgtcta atagatccaa ccatccctta gtctcttctg atatgtgttc 13980

tcagacactg gcagactatg cacggaacag aagctggtca cctttgacag ggggtagaaa 14040

aatactgggt gtatctaatc ctgataccat agaacttgta gagggtgaga tccttagtgt 14100

cagtggaggg tgcactagat gtgacagtgg ggatgaacag tttacttggt tccatcttcc 14160

aagcaatata gagctaaacg atgacaccag caagaatcct ccaatgagag tgccatatct 14220

cgggtcaaag actcaagaga ggagagccgc ctcgcttgcg aaaatagctc atatgtcacc 14280

acacgtgaag gcggctctaa gggcgtcatc tgtgttaatc tgggcttatg gggacaacga 14340

aataaactgg actgctgccc ttaagattgc aaggtctcgg tgcaacataa gctcagagta 14400

tcttcgacta ttgtcaccct tgcctacagc tgggaatctc caacatagat tggacgacgg 14460

cataactcag atgacattca cccctgcatc tctatacagg gtgtcacctt acattcatat 14520

atctaatgat tctcaaaggc tatttactga agaaggagtc aaagagggga atgtggttta 14580

tcagcaaatc atgctcttgg gtttgtcctt aattgagtca ctcttcccaa tgacaacaac 14640

caagacatat gatgaaatca cattgcacct ccacagtaaa tttagctgct gtatcaggga 14700

agcacctgtt gcagttcctt tcgagctact cggggtggca ccggaactaa gggcagtaac 14760

ctcaaataag tttatgtatg atcctagccc tgtatcagag ggagactttg cgagacttga 14820

cttagctatc tttaagagtt atgaacttaa tttagagtca tattccacaa tagagctaat 14880

gaacgttctt tcaatatcta gtgggaagtt gattggccag tctgtggttt cttatgatga 14940

agatacctcc ataaagaatg acgctataat agtgtatgac aacacacgga attggatcag 15000

cgaagctcag aattcagatg tggtccgcct attcgagtat gcggcactcg aagtgctcct 15060

cgactgttct tatcaactct actatctgag agtaagaggc ctaaacaata tcgtcctgta 15120

catgagtgat ttatacaaga atatgccagg aattctactc tctaatattg cagccacaat 15180

atctcaccct gtcatccatt caaggttgaa tgcagtaggt ctggtcaacc atgacgggtc 15240

acaccaactt gcagacacag atttcattga aatgtctgca aagctgctag tctcttgcac 15300

tcgacgcgtg gtctcaggtt tacatgcagg gaataagtat gacctgctgt ttccatctgt 15360

cttggatgat aacctaagtg agaagatgct tcagttgata tcccaattat gctgtctgta 15420

tacggtgctc tttgctacaa caagagaaat cccgaaaata agaggcttat ctgcagaaga 15480

gaaatgttca gtacttactg agtacctact gtcagatgct gtgaaaccgt tacttgggtc 15540

cgagcaagtg agctctatca tgtctcccaa catagttacg ttcccagcca atctgtatta 15600

catgtctagg aagagcctta atttgatcag ggagagagag gacagggata ctatcttggc 15660

attgttgttc cctcaagaac cgctgctcga gtttcctctg gtacgagata ttggtgctcg 15720

tgtaaaagat ccatttaccc tacaacctgc ggcgttttta caagagttag atttgagtgc 15780

tccggcaagg tatgacgcat tcacaatcag tcaggcgcat tctgaacaca tattgccaaa 15840

cccagaggaa gatcacttag tacgatactt gttcagagga atagggactg cgtcctcctc 15900

ttggtataag gcatcccatc ttctttctgt acccgaggtc agatgtgcaa ggcatgggaa 15960

ctccttatat ttagcagaag gaagtggagc catcatgagt cttctcgaat tgcatatacc 16020

acacgaaact atctattaca atacactttt ctcgaacgag atgaaccccc cacagcgaca 16080

tttcggacca actccaacac agtttctgaa ttcggttgtt tttaggaatt tacaggcgga 16140

agtaccatgc aaggatggat ttgtccagga gttccgtccg ttatggagag agaatacaga 16200

agaaagcgat ctgacctcag ataaagcagt gggatatatc acatctgtag tgccgtacag 16260

gtctgtatca ttgctgcatt gtgacattga aatccctcca ggatctaatc aaagcttact 16320

agatcagctg gctaccaatc tgtctctgat tgccatgcat tctgtgaagg agggcggggt 16380

cgtgattatc aaagtactgt atgcaatggg atattacttc catctactca tgaacttgtt 16440

tactccatgt tccacgaaag gatatattct ctctaatggc tatgcctgta gaggggatat 16500

ggagtgttac ctgatatttg tcatgggcta cctaggcggg cctacatttg tacatgaggt 16560

ggtgaggatg gcaaaaacgc tagtacagcg gcatggcaca cttttgtcta agtcagatga 16620

gattacactg actaggttat ttacttcaca gcaacagcgt gtaacagaca tcctatccag 16680

ccctttaccg agactaatga agtacttgag agagaatatt gatactgcac tgattgaagc 16740

cgggggacag cctgtccgtc cattctgtgc agagagttta gtgagcacac taacagacat 16800

gactcagaca acccagatca tcgccagcca cattgataca gtcattcgat ctgtgatcta 16860

tatggaagca gagggtgatc ttgctgacac agtattctta tttacccctt acaacctctc 16920

tatggacggg aaaaagagaa catcacttaa acagtgcaca agacagatct tagaggtcac 16980

aatactgggt ctcagagtca aaaatctcaa taaagtaggt gatgtaatca gcctagtact 17040

cagaggtatg atttctctgg aggaccttct cccactgaga acctacttga agtgtagtac 17100

ctgccctaag tatttgaagg ctgtcctagg tattaccaaa ctcaaagaaa tgttcacaga 17160

cgcctcttta ttatacttga ctcgtgctca acaaaaattc tacatgaaaa ctataggcaa 17220

tgcagtcaag ggatactata gtaactgtga ctcttaaagg caaccacata tcaataggcc 17280

ctctttctag ccgatcgtat tcttgttgac ttcattatac catattagaa aaaaattgaa 17340

ttccgaccct ttaagactcg tattcggatt caaataatta tctcagaaaa aaagtgcacg 17400

tagttgttct tgattatagt cccgtcattc accaagtctt tgtttggt 17448

<210> 16
<211> 17442
<212> DNA
<213> Newcastle disease virus

<220>
<223> Mut HN (F277L)/M(G165W)/F(F117S)/F(F190L)/L(V757I)/L(F1551S)/L(R1
      700L)-Lirilumab

<400> 16
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat tttagatttt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gcccccaccc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgttccgc ggcgtgcagt 3240

gccaagtgca actggtgcag agcggtgcag aggtgaagaa gccaggatcc tctgtgaaag 3300

tctcatgcaa agccagtgga ggtacgttca gcttttacgc aatcagctgg gtgagacagg 3360

ctccgggcca aggtctggaa tggatgggcg gattcattcc aattttcggc gcagcaaact 3420

acgcacagaa gttccagggg cgggtcacga ttactgctga tgagtcgaca agcacagcat 3480

acatggagct cagttctttg agaagcgatg atacagcagt ttattactgt gctagaattc 3540

caagcggttc ttattactat gactacgata tggatgtctg gggacaggga acaacagtca 3600

ccgtatcttc cgcgagcacc aagggacctt ccgtgtttcc cctggcccct tgctccagat 3660

caacctcaga gtcgactgct gctctcggtt gcttggtgaa agattatttt ccagaacccg 3720

ttaccgtgtc ttggaactcc ggtgccctta cttcaggggt tcataccttc cccgcagtcc 3780

tacagtcatc tggtctgtat tctctcagct ccgtggtcac cgtcccaagc agctcactgg 3840

gcaccaagac ctatacctgc aacgtcgacc ataagcctag taatacaaag gtcgacaagc 3900

gagtcgaatc caaatacgga cctccttgtc caccttgccc cgctcctgaa ttcctgggtg 3960

gaccaagcgt ctttctgttt ccaccaaaac ctaaagacac acttatgatt tcccgcacac 4020

ccgaagtcac ctgcgtggtg gtggatgtca gccaggaaga ccctgaggtg caattcaact 4080

ggtacgttga tggcgtagaa gtccacaacg ccaagacaaa acccagagag gagcagttta 4140

atagtaccta tagggtggtg tccgtcctga ccgttctcca ccaggattgg ttgaatggca 4200

aggaatacaa gtgtaaagtg agcaacaaag gcctaccttc gagcatagaa aagactatct 4260

ccaaggcaaa gggacagcca agggagcccc aggtgtacac gctgccaccc tctcaggagg 4320

agatgactaa gaaccaggtg tccttgacct gcctcgtaaa ggggttttac ccctccgaca 4380

tagccgtcga atgggagagt aacggacaac ccgagaacaa ttataaaact acacctcccg 4440

tgctcgactc tgacggatcc ttcttcctgt actctaggct gacagtggat aaatcccgtt 4500

ggcaggaagg aaatgtattt tcctgtagtg tgatgcacga ggctctgcac aatcattata 4560

cacagaaatc actgtcctta agcctcggca aacgccgcaa gcgcggctcc ggcgagggcc 4620

gcggctccct gctgacctgc ggcgacgtgg aggagaaccc cggccccatg aagtacctgc 4680

tgcccaccgc cgccgccggc ctgctgctgc tggccgccca gcccgccatg gccgaaatcg 4740

tcctcacaca gagcccggtt acgctttcat tgtctccagg cgagcgcgct accctcagct 4800

gtagggcgtc tcagtccgtt tccagctatt tagcttggta ccagcaaaaa cctggccagg 4860

cccctagact gcttatttac gacgcctcta accgagcgac tggtataccc gcaaggttca 4920

gcggctcggg ctcaggcacc gactttactc tcaccatttc atcactggag cctgaggact 4980

tcgccgtgta ttactgtcag cagaggagta actggatgta caccttcggc caagggacca 5040

aattggaaat caaaaggaca gtggccgcgc caagcgtgtt tatatttccc ccctccgacg 5100

aacagttaaa gagtggaacc gcatctgtgg tatgtctgct taacaatttt taccctcgcg 5160

aggcaaaggt tcaatggaaa gtcgacaacg ccttacagtc tggtaacagt caagagtctg 5220

ttacggagca agacagcaag gactccacct attcgctgtc ttcaacacta accctcagca 5280

aggctgatta cgagaagcac aaagtttacg cctgtgaggt cacccatcag ggcctttcta 5340

gccctgtcac aaagtcgttc aacaggggag agtgctaatg agctgaccga gccctcctct 5400

ataaacccaa gattcaaggc tccaagcgat aaccttctcc cgcctcttcc atcccactga 5460

atgatcgcgc agctgcaatt aatctagcaa cattaaagat taagaaaaaa tacgggtaga 5520

atcggagtgc accgattgtg ccaagatgga ctcatctagg acaattgggt tatactttga 5580

ttctgccctt ccttctagca acctgttagc attcccgatt gtcttacgag atgtaggaga 5640

tggaaagaag caaattactc cgcaatatag gatccggcgt cttgactcgt ggacagacag 5700

taaagaagac tcggtattca tcactaccta cggattcatc ttccaggttg ggaatgaaga 5760

agtcactgtc ggcatgatca atgataatcc caagcgagag ttactctccg ctgcgatgct 5820

ctgcctagga agcgtcccga atgttggaga tcctgttgag ctggcaaggg cctgcctcac 5880

tatggtggta acatgcaaga agagtgcaac taatactgag agaatggtct tctcagtagt 5940

gcaggcaccc cgggtgctgc aaagctgtag ggtcgtggca gacaaatact cgtcagtgaa 6000

tgcagttaag cacgtgaaag caccagagaa gatcccttgg agtgaaaccc tagagtacaa 6060

ggtgaatttt gtctctttga ctgtggtgcc gaagaaggat gtctacaaga tcccaaccgc 6120

agtattgaag gtatccggct cgagcctgta taatcttgcg ctcaatgtca ctattgatgt 6180

ggaggtagac ccaaagagcc cgttagtcaa gtccctttct aggtccgaca gtggatacta 6240

tgctaatctc ttcttacata tcggacttat gtccactgta gataagaggg gaaagaaagt 6300

gacatttgac cagctggaga ggaagataag gagacttgat ctatctgtcg ggctcagtga 6360

tgtgctcgga ccttctgtgc ttgtgaaggc gagaggtgca cggactaggc tgctggcacc 6420

tttcttctct aacagcggga cagcctgcta ccctatagca aatgcctctc ctcaggtagc 6480

taagatactc tggagtcaga ccgcgtgcct gcggagtgta aaaatcatta ttcaagcggg 6540

cacccaacgc gctgtcgcag tgactgctga ccatgaggtt acctctacta agatagagaa 6600

gaggcatacc atcgctaaat acaatccctt caagaaatag gccgtatccc tgagactgca 6660

attcacccgc cttcccaaaa caccatgaca ccagataatg atctgtcttg attacttaca 6720

gttagtttcc ctgtctatca aattagaaaa aacacgggta gaagagttcg gatcccggcc 6780

ggcgcaccaa aagcgcaaga tgggccccag atcttctacc aggatcccag tacctctaat 6840

gctgaccata cggatcacgc tggcactgag ttatgtccgt ctgacaagtt ctcttgatgg 6900

caggcctctt gcagccgcag ggatcgtggt aacaggggat aaagcagtta acatatacac 6960

ctcatcccag acagggtcaa tcatagtcaa gttactccca aatatgccca aggacaaaga 7020

ggcatgtgca aaagccccat tggaggctta caacaggaca ctgactactt tgcttacccc 7080

ccttggtgat tctatccgca ggatacaaga gtctgtgact acatccggag gaaggagaca 7140

gagacgctct ataggtgcca ttattggcag tgtagctcta ggggttgcaa cagctgcaca 7200

gataacggca gcctcggctc tgatacaagc caaccagaat gctgctaaca tcctccggct 7260

taaggagagc attgctgcaa ccaatgaagc tgtacacgag gtcactggcg gattgtcaca 7320

gttagcagtg gcagttggaa agatgcagca atttgttaat gaccagctta ataacacagc 7380

tcaagagttg gactgtataa aaattacaca gcaggttggt gtagaactca acttatactt 7440

aactgaattg actacagtgt tcgggccaca aatcacttcc cctgccttaa ctcagttgac 7500

tgttcaggct ctttacaatc tggctggtgg taatgtagat tacttgttga ctaagttagg 7560

tgtagggaac aaccagctca gctcattgat tggtagcggc ttgatcaccg gtaaccctat 7620

tttttacgac tcacagactc aactcttggg catacaggtg actttaccct cagtcgggaa 7680

cctaaataat atgcgtgcca cctacttgga gaccttgtct gtaagcacaa caaagggatt 7740

tgcctcagca cttgtcccaa aagtagtgac acaggtcggt tctgtgatag aagagcttga 7800

cacctcatac tgtatagaag ctgatttgga tttatattgt acaagaatag tgacattccc 7860

tatgtcccct ggtatttatt cctgtttgag cggcaataca tcggcttgca tgtattcaag 7920

gactgaaggc gcacttacta caccatacat gactctcaaa ggctcagttg ttgccaattg 7980

ccagatgaca acatgtagat gtgcagaccc cccgggtatc atatcacaaa attatggaga 8040

ggctgtgtct ctaatagata agcactcatg caatgtcgta tccttagacg ggataacttt 8100

gaggctcagt ggggaatttg atgcaactta tcaaaagaat atctcaatat tagattctca 8160

agtactagtg acaggcaatc tcgatatctc aactgagctt gggaatgtca accactcaat 8220

aagtaatgct ttggataagt tagaggaaag caacagcaaa ctagacaaag tcaatgtcaa 8280

actgaccagc acatctgctc tcattaccta tattgtttta actgtcatat ctcttgttct 8340

tggtatgctt agcctggttc tagcatgcta tctgatgtac aagcaaaaag cgcaacgaaa 8400

gaccttgttg tggcttggaa ataataccct agatcagatg agagctacta caaaaatgtg 8460

aatgcagatg agaggcagag gtatccccaa tagcaatctg tgtgtcaatt ctggcagcct 8520

gttaatcaga agaattaaga aaaaactacc ggatgtaggt gaacaaaagg gaatatacgg 8580

gtagaacggc ctgagaggcc acccctcaat cgggagccag gccccactac gtccgctcta 8640

ccgcatcacc aacagcagtc ttcagtcatg gacagcgcag ttagccaagt tgcgctagag 8700

aatgatagaa gagaagcgaa taatacatgg cgcttggttt tccggatcgc agccttactt 8760

ttactggtaa taaccttagc cgtctctgca gtcgccctgg catatagtat ggaggctagc 8820

acacctggcg accttgtaag cataccgact gcgatctcta gggcagagga aaggattaca 8880

tctgcactcg gttccaatca agatgtagta gataggatat acaagcaagt ggcccttgag 8940

tctccattgg cactgctaaa taccgaatct ataattatga atgcaataac gtctctctct 9000

tatcaaatca atggagccac aaataatagc gggtgtgggg cacctgttca tgacccagat 9060

tacatcgggg ggataggtca agaacttatt gtagatgata cgagtgacgt cacatcattt 9120

tatccctctg cattccaaga acacctgaat tttatcccgg cgcccactac aggatcaggc 9180

tgcactcgga taccctcatt cgacatgagt gctacccatt actgttacac tcacaatgtg 9240

atattgtctg gctgcagaga tcactcacac tcacatcagt atttagcact tggtgtgctt 9300

cggacatctg caacagggag ggtattcttt tctactctgc gttccatcaa cttggatgac 9360

gcccaaaatc ggaagtcttg cagtgtgagt gcaactcctt taggttgtga tatgctgtgc 9420

tctaaaatca cagagactga ggaagaggat tataaatcag ttatccccac atcgatggta 9480

catggaaggt tagggcttga cggccaatac catgagaagg acctagacgt cacaacacta 9540

tttagggact gggtggcaaa ttacccagga gtaggaggtg ggtcttttat taacaatcgc 9600

gtatggttcc cggtctacgg agggctaaaa cccagctcgc ctagtgacac tgcacaagaa 9660

gggagatacg taatatacaa gcgatacaat gacacatgcc cggatgagca agattaccag 9720

attcggatgg ctaagtcatc gtataagcct aggcggtttg gtggaaaacg cgtacagcag 9780

gccatcctat ccatcaaggt gtcaacatcc ttgggtgagg acccggtgtt gactgtaccg 9840

cctaatacgg tcgcactcat gggggccgaa ggcagagttc tcacagtggg gacatctcat 9900

ttcttatatc agcgagggtc atcatacttc tctcccgctt tgttataccc tatgacagtc 9960

aacaacaaaa cagccactct tcataatcct tatacattca atgctttcac tcggccaggt 10020

agtgtccctt gccaggcttc agcaagatgc cccaactcat gtgttaccgg agtctatact 10080

gatccatatc ccttagtctt ccataggaac cacaccttgc gaggggtatt cggaacaatg 10140

cttgatgacg aacaagcaag actcaaccct gtatctgcag tatttgataa catatcccgc 10200

agtcgcataa ctcgggtaag ttcaagcagt accagggcag catacacgac atcaacgtgt 10260

tttaaagttg tcaagaccaa taaaacctat tgcctcagca ttgcagaaat atccaatacc 10320

ctcttcgggg aattcagaat tgtcccctta ctagttgaga ttctcaagga tggtggggtt 10380

taagaagcta ggaatggtcg gttgagtcaa ccgtgagaag gcggggaaga tgatattgca 10440

tcatctatct tctgtaacac caagaatcaa atcgaatacc agtgcgagct cgaatcctac 10500

gctgccagtc agccataatc ggctggtgct aatgtggtta gcctgaatct tgtcgatagt 10560

cacttgatta agaaaaaatg tggacggtag tgaaatataa ggcaaagcag ctcacagagg 10620

acagcacggg taggacatgg cgggctccgg acccgaaagg gcagagcatc agattatcct 10680

accagagtca catctgtcct caccattggt caagcataaa ttgctttatt actggaaatt 10740

aactgggcta ccgcttcctg acgaatgtga cttcgaccat cttattatca gccgacaatg 10800

gaagaaggta cttgaatcgg ccacccctga cattgagaga atgataaaac tagggcgggc 10860

agtacaccag actctcaacc acaattccag gataaccgga gtactccatc cccggtgttt 10920

agaggaactg gctagtattg aggttcctga ttcaaccaac aaatttcgga agatcgaaaa 10980

aaagatccag attcacaaca caaggtatgg agaactgttc acaagattat gcacgcatgt 11040

agaaaagaaa ctattggggt catcttggtc taacaatgtc ccacggtcag aggaattcaa 11100

cagcatccgt acagatccgg cattttggtt tcactcaaaa tggtccacag ccaaatttgc 11160

atggctccat ataaaacagg tccagaggca tctgattgta gcagcaagaa caaggtccgc 11220

agtcaacaaa ttagtgacgc tgacccataa ggtaggccaa atctttgtta ctcctgagct 11280

tgtcattgtg acacatacag atgagaacaa gttcacgtgt cttacccagg aacttgtgtt 11340

gatgtatgca gatatgatgg agggcagaga tatggtcagc ataatatcat ccacggcggc 11400

acatcttagg agcttatcag agaaaattga tgatattctg cggttagtag atgctctggc 11460

aagagatttg ggcaatcaag tctacgatgt tgtagcacta atggagggat tcgcatacgg 11520

cgctgttcag ctgcttgaac cgtcaggtac atttgcgggg gatttcttcg cattcaacct 11580

gcaggagctc aaagatactc taaccggact cctccccaag gatatcgcag aatctgtgac 11640

tcacgcaatc gcaaccatat tctctggctt agaacaaaat caagcagctg agatgttgtg 11700

cctgttgcgt ctgtggggtc acccactact cgagtcccgt attgcagcaa aagcagttag 11760

gagtcaaatg tgcgcaccaa aaatggtaga ctttgatatg atcctccagg tattatcttt 11820

ctttaaagga acaatcatca atggatatag aaagaagaat gcaggcgtgt ggccacgtgt 11880

caagatagat acgatatacg ggaaggtcat agggcagcta cacgcagatt ctgcagagat 11940

ttcacatgat gtcatgttga gggaatacaa gagtttatct gcacttgaat tcgagccatg 12000

tatagagtat gaccctgtca ccaatctgag catgttttta aaagacaagg caatcgcaca 12060

cccgaaagac aactggctcg cttcgtttag gcgaaacctt ctctctgagg accagaagaa 12120

acatgtaaga gaggcaacct caactaaccg cctcttgata gagttcttag agtcaaatga 12180

ttttgatcca tataaagaga tggaatatct gacgaccctt gagtacctaa gagatgataa 12240

tgtggcagta tcatactcac tcaaagaaaa agaggtgaaa gttaatgggc ggattttcgc 12300

aaagctaaca aatagattaa ggaattgtca ggtaatggca gaagggatcc tagctgacca 12360
gattgcacct ttcttccagg gaaatggggt cattcaggat agcatatctt tgactaaaag 12420
tatgctagcg atgagtcaac tgtctttcaa cagcaacaag aaacgtatta ctgactgcaa 12480
agaaagagta tcctcaaacc gcaatcatga tccgaagagc aagaatcgtc ggagagttgc 12540
cacttttgta actaccgacc tgcaaaagta ttgtctcaat tggagatatc agacagtcaa 12600
gctgttcgct catgccatca atcagctgat gggcttacct cacttcttcg agtggattca 12660
tcttagacta atggatacta cgatgtttgt aggggaccct ttcaatcctc caagtgaccc 12720
tacggactgt gatctatcaa gagtcccaaa tgatgacata tatattgtca gtgctagggg 12780
aggcattgag ggattatgcc agaagctatg gacaatgatc tcaattgctg caatccagct 12840
tgctgcagca agatcacatt gtcgcgttgc ctgtatggta caaggcgaca atcaagtaat 12900
agctataacg agagaggtaa gatcagatga ctccccagag atggtgttaa cacaattgca 12960
tcaagccagt gataatttct tcaaggaatt gattcatgtc aatcatttga tcggccataa 13020
tttgaaggat cgtgaaacca tcaggtcaga cacgttcttc atatacagca aacgaatatt 13080
caaagatgga gcaatactca gtcaggtcct caaaaactca tctaaattag tgctaatatc 13140
aggcgacctt agtgaaaaca ctgtaatgtc ttgtgccaac attgcatcta ctgtagcacg 13200
gttatgcgag aacgggcttc ctaaggattt ctgttattac ttaaactact taatgagttg 13260
cgtgcagaca tacttcgatt ccgaattttc catcaccaac aactcgcaac tcgattctaa 13320
ccagtcgtgg atagaggaca tttcttttgt gcactcatat gtcctgaccc ctgctcaact 13380
ggggggactg agtaaccttc aatactcaag gctctacaca aggaacatcg gcgacccggg 13440
aaccactgct ttcgcagaga tcaagagatt agaggcagtg gggttactga gtcctagcat 13500
tatgactaac atcttaacta ggccgcctgg aaatggagat tgggccagtc tgtgcaacga 13560
tccatactcc tttaattttg agactgtcgc aagcccaaat attgtcctta agaaacatac 13620
acaaagagtc ctatttgaaa cttgctcgaa tcccttatta tctggagtgc acacagagga 13680
taatgaggca gaagagaagg cattggctga attcttgctc aatcaagaaa tgattcatcc 13740
acgtgtcgcg catgctatca tggaagcaag ctctgtaggt aggagaaagc aaattcaggg 13800
gcttgttgac acaacaaaca ccgtgattaa gattgcactg actaggaggc cactcggcat 13860
caagaggctg atgcggatag tcaattactc gagcatgcat gcaatgctat ttagagatga 13920
tgttttctcg tctaatagat ccaaccatcc cttagtctct tctgatatgt gttctcagac 13980
actggcagac tatgcacgga acagaagctg gtcacctttg acagggggta gaaaaatact 14040
gggtgtatct aatcctgata ccatagaact tgtagagggt gagatcctta gtgtcagtgg 14100
agggtgcact agatgtgaca gtggggatga acagtttact tggttccatc ttccaagcaa 14160

tatagagcta aacgatgaca ccagcaagaa tcctccaatg agagtgccat atctcgggtc 14220

aaagactcaa gagaggagag ccgcctcgct tgcgaaaata gctcatatgt caccacacgt 14280

gaaggcggct ctaagggcgt catctgtgtt aatctgggct tatggggaca acgaaataaa 14340

ctggactgct gcccttaaga ttgcaaggtc tcggtgcaac ataagctcag agtatcttcg 14400

actattgtca cccttgccta cagctgggaa tctccaacat agattggacg acggcataac 14460

tcagatgaca ttcacccctg catctctata cagggtgtca ccttacattc atatatctaa 14520

tgattctcaa aggctattta ctgaagaagg agtcaaagag gggaatgtgg tttatcagca 14580

aatcatgctc ttgggtttgt ccttaattga gtcactcttc ccaatgacaa caaccaagac 14640

atatgatgaa atcacattgc acctccacag taaatttagc tgctgtatca gggaagcacc 14700

tgttgcagtt cctttcgagc tactcggggt ggcaccggaa ctaagggcag taacctcaaa 14760

taagtttatg tatgatccta gccctgtatc agagggagac tttgcgagac ttgacttagc 14820

tatctttaag agttatgaac ttaatttaga gtcatattcc acaatagagc taatgaacgt 14880

tctttcaata tctagtggga agttgattgg ccagtctgtg gtttcttatg atgaagatac 14940

ctccataaag aatgacgcta taatagtgta tgacaacaca cggaattgga tcagcgaagc 15000

tcagaattca gatgtggtcc gcctattcga gtatgcggca ctcgaagtgc tcctcgactg 15060

ttcttatcaa ctctactatc tgagagtaag aggcctaaac aatatcgtcc tgtacatgag 15120

tgatttatac aagaatatgc caggaattct actctctaat attgcagcca caatatctca 15180

ccctgtcatc cattcaaggt tgaatgcagt aggtctggtc aaccatgacg ggtcacacca 15240

acttgcagac acagatttca ttgaaatgtc tgcaaagctg ctagtctctt gcactcgacg 15300

cgtggtctca ggtttacatg cagggaataa gtatgacctg ctgtttccat ctgtcttgga 15360

tgataaccta agtgagaaga tgcttcagtt gatatcccaa ttatgctgtc tgtatacggt 15420

gctctttgct acaacaagag aaatcccgaa aataagaggc ttatctgcag aagagaaatg 15480

ttcagtactt actgagtacc tactgtcaga tgctgtgaaa ccgttacttg ggtccgagca 15540

agtgagctct atcatgtctc ccaacatagt tacgttccca gccaatctgt attacatgtc 15600

taggaagagc cttaatttga tcagggagag agaggacagg gatactatct tggcattgtt 15660

gttccctcaa gaaccgctgc tcgagtttcc tctggtacga gatattggtg ctcgtgtaaa 15720

agatccattt accctacaac ctgcggcgtt tttacaagag ttagatttga gtgctccggc 15780

aaggtatgac gcattcacaa tcagtcaggc gcattctgaa cacatattgc caaacccaga 15840

ggaagatcac ttagtacgat acttgttcag aggaataggg actgcgtcct cctcttggta 15900

taaggcatcc catcttcttt ctgtacccga ggtcagatgt gcaaggcatg ggaactcctt 15960

atatttagca gaaggaagtg gagccatcat gagtcttctc gaattgcata taccacacga 16020

aactatctat tacaatacac ttttctcgaa cgagatgaac cccccacagc gacatttcgg 16080

accaactcca acacagtttc tgaattcggt tgtttttagg aatttacagg cggaagtacc 16140

atgcaaggat ggatttgtcc aggagttccg tccgttatgg agagagaata cagaagaaag 16200

cgatctgacc tcagataaag cagtgggata tatcacatct gtagtgccgt acaggtctgt 16260

atcattgctg cattgtgaca ttgaaatccc tccaggatct aatcaaagct tactagatca 16320

gctggctacc aatctgtctc tgattgccat gcattctgtg aaggagggcg gggtcgtgat 16380

tatcaaagta ctgtatgcaa tgggatatta cttccatcta ctcatgaact tgtttactcc 16440

atgttccacg aaaggatata ttctctctaa tggctatgcc tgtagagggg atatggagtg 16500

ttacctgata tttgtcatgg gctacctagg cgggcctaca tttgtacatg aggtggtgag 16560

gatggcaaaa acgctagtac agcggcatgg cacacttttg tctaagtcag atgagattac 16620

actgactagg ttatttactt cacagcaaca gcgtgtaaca gacatcctat ccagcccttt 16680

accgagacta atgaagtact tgagagagaa tattgatact gcactgattg aagccggggg 16740

acagcctgtc cgtccattct gtgcagagag tttagtgagc acactaacag acatgactca 16800

gacaacccag atcatcgcca gccacattga tacagtcatt cgatctgtga tctatatgga 16860

agcagagggt gatcttgctg acacagtatt cttatttacc ccttacaacc tctctatgga 16920

cgggaaaaag agaacatcac ttaaacagtg cacaagacag atcttagagg tcacaatact 16980

gggtctcaga gtcaaaaatc tcaataaagt aggtgatgta atcagcctag tactcagagg 17040

tatgatttct ctggaggacc ttctcccact gagaacctac ttgaagtgta gtacctgccc 17100

taagtatttg aaggctgtcc taggtattac caaactcaaa gaaatgttca cagacgcctc 17160

tttattatac ttgactcgtg ctcaacaaaa attctacatg aaaactatag gcaatgcagt 17220

caagggatac tatagtaact gtgactctta aaggcaacca catatcaata ggccctcttt 17280

ctagccgatc gtattcttgt tgacttcatt ataccatatt agaaaaaaat tgaattccga 17340

ccctttaaga ctcgtattcg gattcaaata attatctcag aaaaaaagtg cacgtagttg 17400

ttcttgatta tagtcccgtc attcaccaag tctttgtttg gt 17442

<210> 17
<211> 17430
<212> DNA
<213> Newcastle disease virus

<220>
<223> Mut HN (F277L)/M(G165W)/F(F117S)/F(F190L)/L(V757I)/L(F1551S)/L(R1
      700L)-Relatlimab

<400> 17
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgttccgc ggcgtgcagt 3240

gccaggtgca actgcagcag tggggggccg gactcctgaa accatccgaa actctctcat 3300

tgacttgcgc cgtatacggc gggtcatttt ccgattacta ctggaattgg atccgtcagc 3360

ctccaggtaa aggacttgaa tggataggtg aaataaatca tcgtggaagt actaacagca 3420

atcctagtct gaaatcaaga gtaacgctga gtttggacac atctaagaac cagttttccc 3480

ttaaacttag aagtgttacc gctgctgaca cagctgtata ctactgtgct ttcggttact 3540

ccgattatga gtacaattgg ttcgaccctt ggggacaggg cacactagtg acagtgtcca 3600

gtgcttcaac caagggccca agcgtgtttc ccttggcccc ctgttcacgc tccactagcg 3660

aaagcacagc cgcgttaggc tgtctggtga aagactattt tccagagcca gtcacggtgt 3720

cttggaactc gggtgccctg acatcaggtg ttcatacttt ccccgctgtc ctccagtcca 3780

gtgggctgta ctcattatct tcggtggtga ctgtcccctc ctcatcgctg gggacgaaga 3840

cctatacctg taacgtggac cacaaaccaa gcaacactaa ggtggacaag cgcgtggaat 3900

ccaaatacgg gcccccatgc ccaccgtgcc ccgcacctga gttcctgggc ggtcctagcg 3960

tctttttgtt cccaccgaaa ccaaaggata ccctcatgat ttccaggacg ccggaggtca 4020

cctgcgtggt cgtggacgtg agtcaggagg accccgaagt gcaatttaac tggtatgtgg 4080

acggtgtgga agtccataat gcaaagacca agcctagaga ggaacaattt aatagcacgt 4140

accgggtggt tagtgtcctt accgtcttgc accaggattg gctgaacggg aaggagtata 4200

agtgcaaggt gtcaaacaag ggactgccct catcaatcga gaagacaata agtaaggcga 4260

agggccagcc acgggagcct caggtctaca cattgccgcc aagtcaagag gagatgacca 4320

aaaaccaggt gtctttgact tgcctcgtga agggttttta cccaagtgac atcgccgtag 4380

agtgggagtc taacggccag ccagagaata actataaaac gacacctcca gtgcttgatt 4440

ctgatggatc ttttttcttg tactcaaggt tgaccgtgga taagagcagg tggcaagaag 4500

gcaacgtttt tagttgttcc gttatgcacg aggccttgca taatcactat actcagaagt 4560

ccctatccct gtctctcggg cgccgcaagc gcggctccgg cgagggccgc ggctccctgc 4620

tgacctgcgg cgacgtggag gagaaccccg gccccatgaa gtacctgctg cccaccgccg 4680

ccgccggcct gctgctgctg gccgcccagc ccgccatggc cgaaattgtg ctgacacaat 4740

cgccagccac tctgtctctg agccctggtg agcgggccac tctgtcctgc agggccagtc 4800

agtctatttc cagctacttg gcttggtatc agcaaaaacc cggccaagct cctagattgc 4860

ttatttacga cgcgagcaat agggccaccg gtattccagc tcgctttagc ggctccggaa 4920

gtggtactga tttcacgctg acaatttcaa gcttggaacc cgaggatttt gcagtgtatt 4980

actgccagca gagaagcaat tggccgctga ccttcggcca ggggactaat cttgaaataa 5040

aacgcacggt ggctgccccc tccgtcttca tctttccacc ttctgacgag caattgaaga 5100

gcggtaccgc tagcgtagtg tgtcttctga acaatttcta cccacgcgag gccaaagtgc 5160

aatggaaagt cgacaatgct ctgcagtcag gaaactccca ggagagcgtc acagagcaag 5220

attctaaaga ttccacttac agtctgtcat cgacattgac actgagcaag gccgattacg 5280

aaaagcataa agtgtacgct tgtgaggtca ctcaccaagg actgagctca ccagttacca 5340

aatcgtttaa tagaggtgag tgttaatgag ctgaccgagc cctcctctat aaacccaaga 5400

ttcaaggctc caagcgataa ccttctcccg cctcttccat cccactgaat gatcgcgcag 5460

ctgcaattaa tctagcaaca ttaaagatta agaaaaaata cgggtagaat cggagtgcac 5520

cgattgtgcc aagatggact catctaggac aattgggtta tactttgatt ctgcccttcc 5580

ttctagcaac ctgttagcat tcccgattgt cttacgagat gtaggagatg gaaagaagca 5640

aattactccg caatatagga tccggcgtct tgactcgtgg acagacagta aagaagactc 5700

ggtattcatc actacctacg gattcatctt ccaggttggg aatgaagaag tcactgtcgg 5760

catgatcaat gataatccca agcgagagtt actctccgct gcgatgctct gcctaggaag 5820

cgtcccgaat gttggagatc ctgttgagct ggcaagggcc tgcctcacta tggtggtaac 5880

atgcaagaag agtgcaacta atactgagag aatggtcttc tcagtagtgc aggcaccccg 5940

ggtgctgcaa agctgtaggg tcgtggcaga caaatactcg tcagtgaatg cagttaagca 6000

cgtgaaagca ccagagaaga tcccttggag tgaaacccta gagtacaagg tgaattttgt 6060

ctctttgact gtggtgccga agaaggatgt ctacaagatc ccaaccgcag tattgaaggt 6120

atccggctcg agcctgtata atcttgcgct caatgtcact attgatgtgg aggtagaccc 6180

aaagagcccg ttagtcaagt ccctttctag gtccgacagt ggatactatg ctaatctctt 6240

cttacatatc ggacttatgt ccactgtaga taagagggga aagaaagtga catttgacca 6300

gctggagagg aagataagga gacttgatct atctgtcggg ctcagtgatg tgctcggacc 6360

ttctgtgctt gtgaaggcga gaggtgcacg gactaggctg ctggcacctt tcttctctaa 6420

cagcgggaca gcctgctacc ctatagcaaa tgcctctcct caggtagcta agatactctg 6480

gagtcagacc gcgtgcctgc ggagtgtaaa aatcattatt caagcgggca cccaacgcgc 6540

tgtcgcagtg actgctgacc atgaggttac ctctactaag atagagaaga ggcataccat 6600

cgctaaatac aatcccttca agaaataggc cgtatccctg agactgcaat tcacccgcct 6660

tcccaaaaca ccatgacacc agataatgat ctgtcttgat tacttacagt tagtttccct 6720

gtctatcaaa ttagaaaaaa cacgggtaga agagttcgga tcccggccgg cgcaccaaaa 6780

gcgcaagatg ggccccagat cttctaccag gatcccagta cctctaatgc tgaccatacg 6840

gatcacgctg gcactgagtt atgtccgtct gacaagttct cttgatggca ggcctcttgc 6900

agccgcaggg atcgtggtaa caggggataa agcagttaac atatacacct catcccagac 6960

agggtcaatc atagtcaagt tactcccaaa tatgcccaag gacaaagagg catgtgcaaa 7020

agccccattg gaggcttaca acaggacact gactactttg cttacccccc ttggtgattc 7080

tatccgcagg atacaagagt ctgtgactac atccggagga aggagacaga gacgctctat 7140

aggtgccatt attggcagtg tagctctagg ggttgcaaca gctgcacaga taacggcagc 7200

ctcggctctg atacaagcca accagaatgc tgctaacatc ctccggctta aggagagcat 7260

tgctgcaacc aatgaagctg tacacgaggt cactggcgga ttgtcacagt tagcagtggc 7320

agttggaaag atgcagcaat ttgttaatga ccagcttaat aacacagctc aagagttgga 7380

ctgtataaaa attacacagc aggttggtgt agaactcaac ttatacttaa ctgaattgac 7440

tacagtgttc gggccacaaa tcacttcccc tgccttaact cagttgactg ttcaggctct 7500

ttacaatctg gctggtggta atgtagatta cttgttgact aagttaggtg tagggaacaa 7560

ccagctcagc tcattgattg gtagcggctt gatcaccggt aaccctattt tttacgactc 7620

acagactcaa ctcttgggca tacaggtgac tttaccctca gtcgggaacc taaataatat 7680

gcgtgccacc tacttggaga ccttgtctgt aagcacaaca aagggatttg cctcagcact 7740

tgtcccaaaa gtagtgacac aggtcggttc tgtgatagaa gagcttgaca cctcatactg 7800

tatagaagct gatttggatt tatattgtac aagaatagtg acattcccta tgtcccctgg 7860

tatttattcc tgtttgagcg gcaatacatc ggcttgcatg tattcaagga ctgaaggcgc 7920

acttactaca ccatacatga ctctcaaagg ctcagttgtt gccaattgcc agatgacaac 7980

atgtagatgt gcagaccccc cgggtatcat atcacaaaat tatggagagg ctgtgtctct 8040

aatagataag cactcatgca atgtcgtatc cttagacggg ataactttga ggctcagtgg 8100

ggaatttgat gcaacttatc aaaagaatat ctcaatatta gattctcaag tactagtgac 8160

aggcaatctc gatatctcaa ctgagcttgg gaatgtcaac cactcaataa gtaatgcttt 8220

ggataagtta gaggaaagca acagcaaact agacaaagtc aatgtcaaac tgaccagcac 8280

atctgctctc attacctata ttgttttaac tgtcatatct cttgttcttg gtatgcttag 8340

cctggttcta gcatgctatc tgatgtacaa gcaaaaagcg caacgaaaga ccttgttgtg 8400

gcttggaaat aataccctag atcagatgag agctactaca aaaatgtgaa tgcagatgag 8460

aggcagaggt atccccaata gcaatctgtg tgtcaattct ggcagcctgt taatcagaag 8520

aattaagaaa aaactaccgg atgtaggtga acaaaaggga atatacgggt agaacggcct 8580

gagaggccac ccctcaatcg ggagccaggc cccactacgt ccgctctacc gcatcaccaa 8640

cagcagtctt cagtcatgga cagcgcagtt agccaagttg cgctagagaa tgatagaaga 8700

gaagcgaata atacatggcg cttggttttc cggatcgcag ccttactttt actggtaata 8760

accttagccg tctctgcagt cgccctggca tatagtatgg aggctagcac acctggcgac 8820

cttgtaagca taccgactgc gatctctagg gcagaggaaa ggattacatc tgcactcggt 8880

tccaatcaag atgtagtaga taggatatac aagcaagtgg cccttgagtc tccattggca 8940

ctgctaaata ccgaatctat aattatgaat gcaataacgt ctctctctta tcaaatcaat 9000

ggagccacaa ataatagcgg gtgtggggca cctgttcatg acccagatta catcgggggg 9060

ataggtcaag aacttattgt agatgatacg agtgacgtca catcatttta tccctctgca 9120

ttccaagaac acctgaattt tatcccggcg cccactacag gatcaggctg cactcggata 9180

ccctcattcg acatgagtgc tacccattac tgttacactc acaatgtgat attgtctggc 9240

tgcagagatc actcacactc acatcagtat ttagcacttg gtgtgcttcg gacatctgca 9300

acagggaggg tattcttttc tactctgcgt tccatcaact tggatgacgc ccaaaatcgg 9360

aagtcttgca gtgtgagtgc aactccttta ggttgtgata tgctgtgctc taaaatcaca 9420

gagactgagg aagaggatta taaatcagtt atccccacat cgatggtaca tggaaggtta 9480

gggcttgacg gccaatacca tgagaaggac ctagacgtca caacactatt tagggactgg 9540

gtggcaaatt acccaggagt aggaggtggg tcttttatta acaatcgcgt atggttcccg 9600

gtctacggag ggctaaaacc cagctcgcct agtgacactg cacaagaagg gagatacgta 9660

atatacaagc gatacaatga cacatgcccg gatgagcaag attaccagat tcggatggct 9720

aagtcatcgt ataagcctag gcggtttggt ggaaaacgcg tacagcaggc catcctatcc 9780

atcaaggtgt caacatcctt gggtgaggac ccggtgttga ctgtaccgcc taatacggtc 9840

gcactcatgg gggccgaagg cagagttctc acagtgggga catctcattt cttatatcag 9900

cgagggtcat catacttctc tcccgctttg ttatacccta tgacagtcaa caacaaaaca 9960

gccactcttc ataatcctta tacattcaat gctttcactc ggccaggtag tgtcccttgc 10020

caggcttcag caagatgccc caactcatgt gttaccggag tctatactga tccatatccc 10080

ttagtcttcc ataggaacca caccttgcga ggggtattcg gaacaatgct tgatgacgaa 10140

caagcaagac tcaaccctgt atctgcagta tttgataaca tatcccgcag tcgcataact 10200

cgggtaagtt caagcagtac cagggcagca tacacgacat caacgtgttt taaagttgtc 10260

aagaccaata aaacctattg cctcagcatt gcagaaatat ccaataccct cttcggggaa 10320

ttcagaattg tccccttact agttgagatt ctcaaggatg gtggggttta agaagctagg 10380

aatggtcggt tgagtcaacc gtgagaaggc ggggaagatg atattgcatc atctatcttc 10440

tgtaacacca agaatcaaat cgaataccag tgcgagctcg aatcctacgc tgccagtcag 10500

ccataatcgg ctggtgctaa tgtggttagc ctgaatcttg tcgatagtca cttgattaag 10560

aaaaaatgtg gacggtagtg aaatataagg caaagcagct cacagaggac agcacgggta 10620

ggacatggcg ggctccggac ccgaaagggc agagcatcag attatcctac cagagtcaca 10680

tctgtcctca ccattggtca agcataaatt gctttattac tggaaattaa ctgggctacc 10740

gcttcctgac gaatgtgact tcgaccatct tattatcagc cgacaatgga agaaggtact 10800

tgaatcggcc acccctgaca ttgagagaat gataaaacta gggcgggcag tacaccagac 10860

tctcaaccac aattccagga taaccggagt actccatccc cggtgtttag aggaactggc 10920

tagtattgag gttcctgatt caaccaacaa atttcggaag atcgaaaaaa agatccagat 10980

tcacaacaca aggtatggag aactgttcac aagattatgc acgcatgtag aaaagaaact 11040

attggggtca tcttggtcta acaatgtccc acggtcagag gaattcaaca gcatccgtac 11100

agatccggca ttttggtttc actcaaaatg gtccacagcc aaatttgcat ggctccatat 11160

aaaacaggtc cagaggcatc tgattgtagc agcaagaaca aggtccgcag tcaacaaatt 11220

agtgacgctg acccataagg taggccaaat ctttgttact cctgagcttg tcattgtgac 11280

acatacagat gagaacaagt tcacgtgtct tacccaggaa cttgtgttga tgtatgcaga 11340

tatgatggag ggcagagata tggtcagcat aatatcatcc acggcggcac atcttaggag 11400

cttatcagag aaaattgatg atattctgcg gttagtagat gctctggcaa gagatttggg 11460

caatcaagtc tacgatgttg tagcactaat ggagggattc gcatacggcg ctgttcagct 11520

gcttgaaccg tcaggtacat ttgcggggga tttcttcgca ttcaacctgc aggagctcaa 11580

agatactcta accggactcc tccccaagga tatcgcagaa tctgtgactc acgcaatcgc 11640

aaccatattc tctggcttag aacaaaatca agcagctgag atgttgtgcc tgttgcgtct 11700

gtggggtcac ccactactcg agtcccgtat tgcagcaaaa gcagttagga gtcaaatgtg 11760

cgcaccaaaa atggtagact ttgatatgat cctccaggta ttatctttct ttaaaggaac 11820

aatcatcaat ggatatagaa agaagaatgc aggcgtgtgg ccacgtgtca agatagatac 11880

gatatacggg aaggtcatag ggcagctaca cgcagattct gcagagattt cacatgatgt 11940

catgttgagg gaatacaaga gtttatctgc acttgaattc gagccatgta tagagtatga 12000

ccctgtcacc aatctgagca tgtttttaaa agacaaggca atcgcacacc cgaaagacaa 12060

ctggctcgct tcgtttaggc gaaaccttct ctctgaggac cagaagaaac atgtaagaga 12120

ggcaacctca actaaccgcc tcttgataga gttcttagag tcaaatgatt ttgatccata 12180

taaagagatg gaatatctga cgacccttga gtacctaaga gatgataatg tggcagtatc 12240

atactcactc aaagaaaaag aggtgaaagt taatgggcgg attttcgcaa agctaacaaa 12300

tagattaagg aattgtcagg taatggcaga agggatccta gctgaccaga ttgcaccttt 12360

cttccaggga aatggggtca ttcaggatag catatctttg actaaaagta tgctagcgat 12420

gagtcaactg tctttcaaca gcaacaagaa acgtattact gactgcaaag aaagagtatc 12480

ctcaaaccgc aatcatgatc cgaagagcaa gaatcgtcgg agagttgcca cttttgtaac 12540

taccgacctg caaaagtatt gtctcaattg gagatatcag acagtcaagc tgttcgctca 12600

tgccatcaat cagctgatgg gcttacctca cttcttcgag tggattcatc ttagactaat 12660

ggatactacg atgtttgtag gggacccttt caatcctcca agtgacccta cggactgtga 12720

tctatcaaga gtcccaaatg atgacatata tattgtcagt gctaggggag gcattgaggg 12780

attatgccag aagctatgga caatgatctc aattgctgca atccagcttg ctgcagcaag 12840

atcacattgt cgcgttgcct gtatggtaca aggcgacaat caagtaatag ctataacgag 12900

agaggtaaga tcagatgact ccccagagat ggtgttaaca caattgcatc aagccagtga 12960

taatttcttc aaggaattga ttcatgtcaa tcatttgatc ggccataatt tgaaggatcg 13020

tgaaaccatc aggtcagaca cgttcttcat atacagcaaa cgaatattca aagatggagc 13080

aatactcagt caggtcctca aaaactcatc taaattagtg ctaatatcag gcgaccttag 13140

tgaaaacact gtaatgtctt gtgccaacat tgcatctact gtagcacggt tatgcgagaa 13200

cgggcttcct aaggatttct gttattactt aaactactta atgagttgcg tgcagacata 13260

cttcgattcc gaattttcca tcaccaacaa ctcgcaactc gattctaacc agtcgtggat 13320

agaggacatt tcttttgtgc actcatatgt cctgacccct gctcaactgg ggggactgag 13380

taaccttcaa tactcaaggc tctacacaag gaacatcggc gacccgggaa ccactgcttt 13440

cgcagagatc aagagattag aggcagtggg gttactgagt cctagcatta tgactaacat 13500

cttaactagg ccgcctggaa atggagattg ggccagtctg tgcaacgatc catactcctt 13560

taattttgag actgtcgcaa gcccaaatat tgtccttaag aaacatacac aaagagtcct 13620

atttgaaact tgctcgaatc ccttattatc tggagtgcac acagaggata atgaggcaga 13680

agagaaggca ttggctgaat tcttgctcaa tcaagaaatg attcatccac gtgtcgcgca 13740

tgctatcatg gaagcaagct ctgtaggtag gagaaagcaa attcaggggc ttgttgacac 13800

aacaaacacc gtgattaaga ttgcactgac taggaggcca ctcggcatca agaggctgat 13860

gcggatagtc aattactcga gcatgcatgc aatgctattt agagatgatg ttttctcgtc 13920

taatagatcc aaccatccct tagtctcttc tgatatgtgt tctcagacac tggcagacta 13980

tgcacggaac agaagctggt cacctttgac agggggtaga aaaatactgg gtgtatctaa 14040

tcctgatacc atagaacttg tagagggtga gatccttagt gtcagtggag ggtgcactag 14100

atgtgacagt ggggatgaac agtttacttg gttccatctt ccaagcaata tagagctaaa 14160

cgatgacacc agcaagaatc ctccaatgag agtgccatat ctcgggtcaa agactcaaga 14220

gaggagagcc gcctcgcttg cgaaaatagc tcatatgtca ccacacgtga aggcggctct 14280

aagggcgtca tctgtgttaa tctgggctta tggggacaac gaaataaact ggactgctgc 14340

ccttaagatt gcaaggtctc ggtgcaacat aagctcagag tatcttcgac tattgtcacc 14400

cttgcctaca gctgggaatc tccaacatag attggacgac ggcataactc agatgacatt 14460

cacccctgca tctctataca gggtgtcacc ttacattcat atatctaatg attctcaaag 14520

gctatttact gaagaaggag tcaaagaggg gaatgtggtt tatcagcaaa tcatgctctt 14580

gggtttgtcc ttaattgagt cactcttccc aatgacaaca accaagacat atgatgaaat 14640

cacattgcac ctccacagta aatttagctg ctgtatcagg gaagcacctg ttgcagttcc 14700

tttcgagcta ctcggggtgg caccggaact aagggcagta acctcaaata agtttatgta 14760

tgatcctagc cctgtatcag agggagactt tgcgagactt gacttagcta tctttaagag 14820

ttatgaactt aatttagagt catattccac aatagagcta atgaacgttc tttcaatatc 14880

tagtgggaag ttgattggcc agtctgtggt ttcttatgat gaagatacct ccataaagaa 14940

tgacgctata atagtgtatg acaacacacg gaattggatc agcgaagctc agaattcaga 15000

tgtggtccgc ctattcgagt atgcggcact cgaagtgctc ctcgactgtt cttatcaact 15060

ctactatctg agagtaagag gcctaaacaa tatcgtcctg tacatgagtg atttatacaa 15120

gaatatgcca ggaattctac tctctaatat tgcagccaca atatctcacc ctgtcatcca 15180

ttcaaggttg aatgcagtag gtctggtcaa ccatgacggg tcacaccaac ttgcagacac 15240

agatttcatt gaaatgtctg caaagctgct agtctcttgc actcgacgcg tggtctcagg 15300

tttacatgca gggaataagt atgacctgct gtttccatct gtcttggatg ataacctaag 15360

tgagaagatg cttcagttga tatcccaatt atgctgtctg tatacggtgc tctttgctac 15420

aacaagagaa atcccgaaaa taagaggctt atctgcagaa gagaaatgtt cagtacttac 15480

tgagtaccta ctgtcagatg ctgtgaaacc gttacttggg tccgagcaag tgagctctat 15540

catgtctccc aacatagtta cgttcccagc caatctgtat tacatgtcta ggaagagcct 15600

taatttgatc agggagagag aggacaggga tactatcttg gcattgttgt tccctcaaga 15660

accgctgctc gagtttcctc tggtacgaga tattggtgct cgtgtaaaag atccatttac 15720

cctacaacct gcggcgtttt tacaagagtt agatttgagt gctccggcaa ggtatgacgc 15780

attcacaatc agtcaggcgc attctgaaca catattgcca aacccagagg aagatcactt 15840

agtacgatac ttgttcagag gaatagggac tgcgtcctcc tcttggtata aggcatccca 15900

tcttctttct gtacccgagg tcagatgtgc aaggcatggg aactccttat atttagcaga 15960

aggaagtgga gccatcatga gtcttctcga attgcatata ccacacgaaa ctatctatta 16020

caatacactt ttctcgaacg agatgaaccc cccacagcga catttcggac caactccaac 16080

acagtttctg aattcggttg tttttaggaa tttacaggcg gaagtaccat gcaaggatgg 16140

atttgtccag gagttccgtc cgttatggag agagaataca gaagaaagcg atctgacctc 16200

agataaagca gtgggatata tcacatctgt agtgccgtac aggtctgtat cattgctgca 16260

ttgtgacatt gaaatccctc caggatctaa tcaaagctta ctagatcagc tggctaccaa 16320

tctgtctctg attgccatgc attctgtgaa ggagggcggg gtcgtgatta tcaaagtact 16380

gtatgcaatg ggatattact tccatctact catgaacttg tttactccat gttccacgaa 16440

aggatatatt ctctctaatg gctatgcctg tagaggggat atggagtgtt acctgatatt 16500

tgtcatgggc tacctaggcg ggcctacatt tgtacatgag gtggtgagga tggcaaaaac 16560

gctagtacag cggcatggca cacttttgtc taagtcagat gagattacac tgactaggtt 16620

atttacttca cagcaacagc gtgtaacaga catcctatcc agccctttac cgagactaat 16680

gaagtacttg agagagaata ttgatactgc actgattgaa gccgggggac agcctgtccg 16740

tccattctgt gcagagagtt tagtgagcac actaacagac atgactcaga caacccagat 16800

catcgccagc cacattgata cagtcattcg atctgtgatc tatatggaag cagagggtga 16860

tcttgctgac acagtattct tatttacccc ttacaacctc tctatggacg ggaaaaagag 16920

aacatcactt aaacagtgca caagacagat cttagaggtc acaatactgg gtctcagagt 16980

caaaaatctc aataaagtag gtgatgtaat cagcctagta ctcagaggta tgatttctct 17040

ggaggacctt ctcccactga gaacctactt gaagtgtagt acctgcccta agtatttgaa 17100

ggctgtccta ggtattacca aactcaaaga aatgttcaca gacgcctctt tattatactt 17160

gactcgtgct caacaaaaat tctacatgaa aactataggc aatgcagtca agggatacta 17220

tagtaactgt gactcttaaa ggcaaccaca tatcaatagg ccctctttct agccgatcgt 17280

attcttgttg acttcattat accatattag aaaaaaattg aattccgacc ctttaagact 17340

cgtattcgga ttcaaataat tatctcagaa aaaaagtgca cgtagttgtt cttgattata 17400

gtcccgtcat tcaccaagtc tttgtttggt 17430

<210> 18
<211> 17448
<212> DNA
<213> Newcastle disease virus

<220>
<223> Mut HN (F277L)/M(G165W)/F(F117S)/F(F190L)/L(V757I)/L(F1551S)/L(R1
      700L)-Monalizumab

<400> 18
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200
gagacttgga gtagagtatg ctcgggctca gggaagcagc atcaatgagg atatggctgc 1260
cgagctaaag ctgaccccag cggcaaggag gggcctggca gctgctgccc aacgagcgtc 1320
cgaggagacc ggcagcatgg acattcccac tcaacaagct ggggtcctca cagggctcag 1380
cgacggaggc cctcaagccc cacaaggtgg actgaacagg tcgcaagggc aaccggacgc 1440
cggagatggg gagacccaat ttttagattt gatgagagca gtggcaaata gtatgagaga 1500
agcgccaaac tctgtgcaga acaccactca gcaggagcct ccatctactc ctgggccatc 1560
tcaagacaac gacaccgact ggggatactg accgacaaca ctcagcctgc ctccatcgaa 1620
ttatctcgat tcttctgcct gtaacctaac ccctggtcca cagtctcaca cggccaaacc 1680
cgcaaacgag cccccccctc tctccccccc cctccagcca cacggctcca accgcccaaa 1740
acaacacggg cacaactcga ctcactcata atctacacag agccgaagat cttagaaaaa 1800
aatacgggta gaagagagac attcagagat caggacaaat catcagggtc tccgctctcc 1860
cctctaccca gcagaccaga gtgaagatgg ccaccttcac ggatgcagag atcgacgatc 1920
tatttgaaac cagtggaact gtcattgaca gcataattac ggcccaaggc aacccagtag 1980
agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gccccccacc caggccgcag gcgaagccgg tgacacacag ctcaagactg 2220
gagcaagcaa ctccctcctg tcgatgctcg acaagcttag caataaatcg tccaatgcta 2280
aaaagggccc acggtcgagt ccccaggaag ggcaccacca acctccgacc cagcagcaag 2340
ggagtcaaca gagccgcgga aacagtcagg agagatcgca aaaccaggtc aaggccgcct 2400
ctggagacca gggcacagac gcgaacacag catatcatgg acaatggaag gagtcacaac 2460
catcagctgg tgcaatccct catgctctcc ggtcagggca gagccaagcc aatactcctg 2520
catctgtgga tcatgtccag ctacctgtcg actttgtgca ggcgatgatg tctatgatgg 2580
aggcgatatc acagaaggta aataaagtcg actatcagct agaccttgtc tcaaagcaga 2640
catcctccat ccccatgatg cgatctgaaa tccaacagct gaaaacatcc gttgcggtca 2700
tggaagccaa cctgggcatg atgaagattc tggaccctgg ttgtgctaac gtttcatctt 2760
taagtgacct acgggcggtc gcccgctctc acccagtttt agtttcaggc cccggagacc 2820
catctcctta tgtgacacaa gggggtgaat tgatactcaa taaactctcg caaccagtgc 2880
aacatccttc tgagttgatt aaacctgcca cgataagcgg gcctgatata ggagtggaga 2940
aggacactgt ccgtgcattg atcacctcac gcccaatgca tccgagctct tcagctaagc 3000
tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgttccgc ggcgtgcagt 3240

gccaggttca gttggtacag tctggcgcgg aggtgaaaaa acctggagca tctgtcaagg 3300

taagttgtaa agcttcgggc tataccttta cctcatattg gatgaactgg gtgcggcagg 3360

cccccggtca agggcttgaa tggatgggca gaattgatcc ttatgattcc gaaacacatt 3420

acgctcagaa gctgcagggc agggttacca tgacgacaga caccagcact tccactgctt 3480

acatggagct gcgatctctg cggtcagatg acacggctgt gtactattgc gccagaggcg 3540

gatacgattt tgacgtgggg actctgtatt ggttctttga cgtctggggg cagggaacca 3600

ctgtgacagt ctcatcggcg tcaactaaag ggccaagcgt gttccccttg gcaccatgta 3660

gtcggtccac cagtgaaagt accgctgcac tcggttgtct ggtaaaagat tattttccag 3720

agcccgttac tgtaagctgg aacagtgggg ccttgacgtc tggagtccat acctttccgg 3780

cggtccttca gtcatctggg ctgtacagcc tttccagcgt tgtcactgtt ccaagctcct 3840

ccctgggcac gaagacctat acatgtaatg tcgaccataa gccttcaaat acaaaagtag 3900

acaaacgtgt ggaatcaaaa tacggacccc catgcccgcc ctgtccagcg cctgaattct 3960

tgggaggccc gtctgtcttt ttattccccc cgaaaccgaa ggacaccctc atgatctcaa 4020

ggacaccaga ggtgacctgc gtggtggtgg atgttagtca agaggatcca gaggtgcagt 4080

ttaactggta tgtcgatggc gtcgaagtcc acaacgctaa aaccaagcca cgggaggagc 4140

agtttaactc tacctatcga gtggtgtccg tactgactgt gttgcaccag gactggctta 4200

atgggaaaga atacaagtgt aaagtttcaa acaaaggcct gccttcaagt atcgaaaaga 4260

ctattagcaa agctaaaggg cagccaagag aaccacaggt gtacactttg cccccttccc 4320

aagaggagat gactaagaat caagtttctc tgacatgtct ggtaaaaggc ttctaccctt 4380

ctgatatagc agtggagtgg gagtctaacg gacagcccga aaacaattac aagacgacac 4440

cccctgtgct agattcagac ggctcttttt ttctttacag tcggctcaca gtggataaat 4500

cacgctggca ggaagggaat gtctttagct gcagcgtcat gcatgaggct ttgcacaacc 4560

attacactca gaaatctctg agcctctcac ttggtaaacg ccgcaagcgc ggctccggcg 4620

agggccgcgg ctccctgctg acctgcggcg acgtggagga gaaccccggc cccatgaagt 4680

acctgctgcc caccgccgcc gccggcctgc tgctgctggc cgcccagccc gccatggccg 4740

atatccagat gacccagagc ccctccagcc ttagcgcttc agtcggggat agagtgacaa 4800

ttacctgcag agcgtctgaa aatatctaca gctacttggc ctggtaccaa cagaaaccag 4860

gcaaagctcc taagctcctg atttataacg caaaaacctt ggctgagggg gtgcccagtc 4920

gcttctcggg ctctggctca ggaaccgact ttacactcac catttcttca ctgcagcctg 4980

aggacttcgc tacgtactac tgccagcatc actatggtac cccacgcact tttggagggg 5040

ggactaaggt ggaaatcaag aggaccgtgg ccgccccatc cgtatttatc ttccccccat 5100

ccgatgagca gttaaaatca gggaccgcct cggttgtgtg tctgcttaac aacttttacc 5160

cccgcgaagc taaggtgcag tggaaggttg ataacgcctt gcagtctgga aacagccagg 5220

agagcgtcac agaacaagat tcaaaagaca gtacttattc cctgagtagt acgctgaccc 5280

tgtccaaagc cgattacgaa aagcacaaag tatacgcgtg tgaggtcact caccagggac 5340

tatcaagccc ggtcactaaa tcattcaaca ggggggagtg ctaatgagct gaccgagccc 5400

tcctctataa acccaagatt caaggctcca agcgataacc ttctcccgcc tcttccatcc 5460

cactgaatga tcgcgcagct gcaattaatc tagcaacatt aaagattaag aaaaaatacg 5520

ggtagaatcg gagtgcaccg attgtgccaa gatggactca tctaggacaa ttgggttata 5580

ctttgattct gcccttcctt ctagcaacct gttagcattc ccgattgtct tacgagatgt 5640

aggagatgga aagaagcaaa ttactccgca atataggatc cggcgtcttg actcgtggac 5700

agacagtaaa gaagactcgg tattcatcac tacctacgga ttcatcttcc aggttgggaa 5760

tgaagaagtc actgtcggca tgatcaatga taatcccaag cgagagttac tctccgctgc 5820

gatgctctgc ctaggaagcg tcccgaatgt tggagatcct gttgagctgg caagggcctg 5880

cctcactatg gtggtaacat gcaagaagag tgcaactaat actgagagaa tggtcttctc 5940

agtagtgcag gcaccccggg tgctgcaaag ctgtagggtc gtggcagaca aatactcgtc 6000

agtgaatgca gttaagcacg tgaaagcacc agagaagatc ccttggagtg aaaccctaga 6060

gtacaaggtg aattttgtct ctttgactgt ggtgccgaag aaggatgtct acaagatccc 6120

aaccgcagta ttgaaggtat ccggctcgag cctgtataat cttgcgctca atgtcactat 6180

tgatgtggag gtagacccaa agagcccgtt agtcaagtcc ctttctaggt ccgacagtgg 6240

atactatgct aatctcttct tacatatcgg acttatgtcc actgtagata agaggggaaa 6300

gaaagtgaca tttgaccagc tggagaggaa gataaggaga cttgatctat ctgtcgggct 6360

cagtgatgtg ctcggacctt ctgtgcttgt gaaggcgaga ggtgcacgga ctaggctgct 6420

ggcacctttc ttctctaaca gcgggacagc ctgctaccct atagcaaatg cctctcctca 6480

ggtagctaag atactctgga gtcagaccgc gtgcctgcgg agtgtaaaaa tcattattca 6540

agcgggcacc caacgcgctg tcgcagtgac tgctgaccat gaggttacct ctactaagat 6600

agagaagagg cataccatcg ctaaatacaa tcccttcaag aaataggccg tatccctgag 6660

actgcaattc acccgccttc ccaaaacacc atgacaccag ataatgatct gtcttgatta 6720

cttacagtta gtttccctgt ctatcaaatt agaaaaaaca cgggtagaag agttcggatc 6780

ccggccggcg caccaaaagc gcaagatggg ccccagatct tctaccagga tcccagtacc 6840

tctaatgctg accatacgga tcacgctggc actgagttat gtccgtctga caagttctct 6900
tgatggcagg cctcttgcag ccgcagggat cgtggtaaca ggggataaag cagttaacat 6960
atacacctca tcccagacag ggtcaatcat agtcaagtta ctcccaaata tgcccaagga 7020
caaagaggca tgtgcaaaag ccccattgga ggcttacaac aggacactga ctactttgct 7080
tacccccctt ggtgattcta tccgcaggat acaagagtct gtgactacat ccggaggaag 7140
gagacagaga cgctctatag gtgccattat tggcagtgta gctctagggg ttgcaacagc 7200
tgcacagata acggcagcct cggctctgat acaagccaac cagaatgctg ctaacatcct 7260
ccggcttaag gagagcattg ctgcaaccaa tgaagctgta cacgaggtca ctggcggatt 7320
gtcacagtta gcagtggcag ttggaaagat gcagcaattt gttaatgacc agcttaataa 7380
cacagctcaa gagttggact gtataaaaat tacacagcag gttggtgtag aactcaactt 7440
atacttaact gaattgacta cagtgttcgg gccacaaatc acttcccctg ccttaactca 7500
gttgactgtt caggctcttt acaatctggc tggtggtaat gtagattact tgttgactaa 7560
gttaggtgta gggaacaacc agctcagctc attgattggt agcggcttga tcaccggtaa 7620
ccctattttt tacgactcac agactcaact cttgggcata caggtgactt taccctcagt 7680
cgggaaccta aataatatgc gtgccaccta cttggagacc ttgtctgtaa gcacaacaaa 7740
gggatttgcc tcagcacttg tcccaaaagt agtgacacag gtcggttctg tgatagaaga 7800
gcttgacacc tcatactgta tagaagctga tttggattta tattgtacaa gaatagtgac 7860
attccctatg tcccctggta tttattcctg tttgagcggc aatacatcgg cttgcatgta 7920
ttcaaggact gaaggcgcac ttactacacc atacatgact ctcaaaggct cagttgttgc 7980
caattgccag atgacaacat gtagatgtgc agaccccccg ggtatcatat cacaaaatta 8040
tggagaggct gtgtctctaa tagataagca ctcatgcaat gtcgtatcct tagacgggat 8100
aactttgagg ctcagtgggg aatttgatgc aacttatcaa aagaatatct caatattaga 8160
ttctcaagta ctagtgacag gcaatctcga tatctcaact gagcttggga atgtcaacca 8220
ctcaataagt aatgctttgg ataagttaga ggaaagcaac agcaaactag acaaagtcaa 8280
tgtcaaactg accagcacat ctgctctcat tacctatatt gttttaactg tcatatctct 8340
tgttcttggt atgcttagcc tggttctagc atgctatctg atgtacaagc aaaaagcgca 8400
acgaaagacc ttgttgtggc ttggaaataa taccctagat cagatgagag ctactacaaa 8460
aatgtgaatg cagatgagag gcagaggtat ccccaatagc aatctgtgtg tcaattctgg 8520
cagcctgtta atcagaagaa ttaagaaaaa actaccggat gtaggtgaac aaaagggaat 8580
atacgggtag aacggcctga gaggccaccc ctcaatcggg agccaggccc cactacgtcc 8640
gctctaccgc atcaccaaca gcagtcttca gtcatggaca gcgcagttag ccaagttgcg 8700

ctagagaatg atagaagaga agcgaataat acatggcgct tggttttccg gatcgcagcc 8760

ttacttttac tggtaataac cttagccgtc tctgcagtcg ccctggcata tagtatggag 8820

gctagcacac ctggcgacct tgtaagcata ccgactgcga tctctagggc agaggaaagg 8880

attacatctg cactcggttc caatcaagat gtagtagata ggatatacaa gcaagtggcc 8940

cttgagtctc cattggcact gctaaatacc gaatctataa ttatgaatgc aataacgtct 9000

ctctcttatc aaatcaatgg agccacaaat aatagcgggt gtggggcacc tgttcatgac 9060

ccagattaca tcggggggat aggtcaagaa cttattgtag atgatacgag tgacgtcaca 9120

tcattttatc cctctgcatt ccaagaacac ctgaatttta tcccggcgcc cactacagga 9180

tcaggctgca ctcggatacc ctcattcgac atgagtgcta cccattactg ttacactcac 9240

aatgtgatat tgtctggctg cagagatcac tcacactcac atcagtattt agcacttggt 9300

gtgcttcgga catctgcaac agggagggta ttcttttcta ctctgcgttc catcaacttg 9360

gatgacgccc aaaatcggaa gtcttgcagt gtgagtgcaa ctcctttagg ttgtgatatg 9420

ctgtgctcta aaatcacaga gactgaggaa gaggattata aatcagttat ccccacatcg 9480

atggtacatg gaaggttagg gcttgacggc caataccatg agaaggacct agacgtcaca 9540

acactattta gggactgggt ggcaaattac ccaggagtag gaggtgggtc ttttattaac 9600

aatcgcgtat ggttcccggt ctacggaggg ctaaaaccca gctcgcctag tgacactgca 9660

caagaaggga gatacgtaat atacaagcga tacaatgaca catgcccgga tgagcaagat 9720

taccagattc ggatggctaa gtcatcgtat aagcctaggc ggtttggtgg aaaacgcgta 9780

cagcaggcca tcctatccat caaggtgtca acatccttgg gtgaggaccc ggtgttgact 9840

gtaccgccta atacggtcgc actcatgggg gccgaaggca gagttctcac agtggggaca 9900

tctcatttct tatatcagcg agggtcatca tacttctctc ccgctttgtt ataccctatg 9960

acagtcaaca acaaaacagc cactcttcat aatccttata cattcaatgc tttcactcgg 10020

ccaggtagtg tcccttgcca ggcttcagca agatgcccca actcatgtgt taccggagtc 10080

tatactgatc catatccctt agtcttccat aggaaccaca ccttgcgagg ggtattcgga 10140

acaatgcttg atgacgaaca agcaagactc aaccctgtat ctgcagtatt tgataacata 10200

tcccgcagtc gcataactcg ggtaagttca agcagtacca gggcagcata cacgacatca 10260

acgtgtttta aagttgtcaa gaccaataaa acctattgcc tcagcattgc agaaatatcc 10320

aataccctct tcggggaatt cagaattgtc cccttactag ttgagattct caaggatggt 10380

ggggtttaag aagctaggaa tggtcggttg agtcaaccgt gagaaggcgg ggaagatgat 10440

attgcatcat ctatcttctg taacaccaag aatcaaatcg aataccagtg cgagctcgaa 10500

tcctacgctg ccagtcagcc ataatcggct ggtgctaatg tggttagcct gaatcttgtc 10560

gatagtcact tgattaagaa aaaatgtgga cggtagtgaa atataaggca aagcagctca 10620

cagaggacag cacgggtagg acatggcggg ctccggaccc gaaagggcag agcatcagat 10680

tatcctacca gagtcacatc tgtcctcacc attggtcaag cataaattgc tttattactg 10740

gaaattaact gggctaccgc ttcctgacga atgtgacttc gaccatctta ttatcagccg 10800

acaatggaag aaggtacttg aatcggccac ccctgacatt gagagaatga taaaactagg 10860

gcgggcagta caccagactc tcaaccacaa ttccaggata accggagtac tccatccccg 10920

gtgtttagag gaactggcta gtattgaggt tcctgattca accaacaaat ttcggaagat 10980

cgaaaaaaag atccagattc acaacacaag gtatggagaa ctgttcacaa gattatgcac 11040

gcatgtagaa aagaaactat tggggtcatc ttggtctaac aatgtcccac ggtcagagga 11100

attcaacagc atccgtacag atccggcatt ttggtttcac tcaaaatggt ccacagccaa 11160

atttgcatgg ctccatataa aacaggtcca gaggcatctg attgtagcag caagaacaag 11220

gtccgcagtc aacaaattag tgacgctgac ccataaggta ggccaaatct ttgttactcc 11280

tgagcttgtc attgtgacac atacagatga gaacaagttc acgtgtctta cccaggaact 11340

tgtgttgatg tatgcagata tgatggaggg cagagatatg gtcagcataa tatcatccac 11400

ggcggcacat cttaggagct tatcagagaa aattgatgat attctgcggt tagtagatgc 11460

tctggcaaga gatttgggca atcaagtcta cgatgttgta gcactaatgg agggattcgc 11520

atacggcgct gttcagctgc ttgaaccgtc aggtacattt gcgggggatt tcttcgcatt 11580

caacctgcag gagctcaaag atactctaac cggactcctc cccaaggata tcgcagaatc 11640

tgtgactcac gcaatcgcaa ccatattctc tggcttagaa caaaatcaag cagctgagat 11700

gttgtgcctg ttgcgtctgt ggggtcaccc actactcgag tcccgtattg cagcaaaagc 11760

agttaggagt caaatgtgcg caccaaaaat ggtagacttt gatatgatcc tccaggtatt 11820

atctttcttt aaaggaacaa tcatcaatgg atatagaaag aagaatgcag gcgtgtggcc 11880

acgtgtcaag atagatacga tatacgggaa ggtcataggg cagctacacg cagattctgc 11940

agagatttca catgatgtca tgttgaggga atacaagagt ttatctgcac ttgaattcga 12000

gccatgtata gagtatgacc ctgtcaccaa tctgagcatg tttttaaaag acaaggcaat 12060

cgcacacccg aaagacaact ggctcgcttc gtttaggcga aaccttctct ctgaggacca 12120

gaagaaacat gtaagagagg caacctcaac taaccgcctc ttgatagagt tcttagagtc 12180

aaatgatttt gatccatata aagagatgga atatctgacg acccttgagt acctaagaga 12240

tgataatgtg gcagtatcat actcactcaa agaaaaagag gtgaaagtta atgggcggat 12300

tttcgcaaag ctaacaaata gattaaggaa ttgtcaggta atggcagaag ggatcctagc 12360

tgaccagatt gcacctttct tccagggaaa tggggtcatt caggatagca tatctttgac 12420

taaaagtatg ctagcgatga gtcaactgtc tttcaacagc aacaagaaac gtattactga 12480

ctgcaaagaa agagtatcct caaaccgcaa tcatgatccg aagagcaaga atcgtcggag 12540

agttgccact tttgtaacta ccgacctgca aaagtattgt ctcaattgga gatatcagac 12600

agtcaagctg ttcgctcatg ccatcaatca gctgatgggc ttacctcact tcttcgagtg 12660

gattcatctt agactaatgg atactacgat gtttgtaggg gaccctttca atcctccaag 12720

tgaccctacg gactgtgatc tatcaagagt cccaaatgat gacatatata ttgtcagtgc 12780

taggggaggc attgagggat tatgccagaa gctatggaca atgatctcaa ttgctgcaat 12840

ccagcttgct gcagcaagat cacattgtcg cgttgcctgt atggtacaag gcgacaatca 12900

agtaatagct ataacgagag aggtaagatc agatgactcc ccagagatgg tgttaacaca 12960

attgcatcaa gccagtgata atttcttcaa ggaattgatt catgtcaatc atttgatcgg 13020

ccataatttg aaggatcgtg aaaccatcag gtcagacacg ttcttcatat acagcaaacg 13080

aatattcaaa gatggagcaa tactcagtca ggtcctcaaa aactcatcta aattagtgct 13140

aatatcaggc gaccttagtg aaaacactgt aatgtcttgt gccaacattg catctactgt 13200

agcacggtta tgcgagaacg ggcttcctaa ggatttctgt tattacttaa actacttaat 13260

gagttgcgtg cagacatact tcgattccga attttccatc accaacaact cgcaactcga 13320

ttctaaccag tcgtggatag aggacatttc ttttgtgcac tcatatgtcc tgacccctgc 13380

tcaactgggg ggactgagta accttcaata ctcaaggctc tacacaagga acatcggcga 13440

cccgggaacc actgctttcg cagagatcaa gagattagag gcagtggggt tactgagtcc 13500

tagcattatg actaacatct taactaggcc gcctggaaat ggagattggg ccagtctgtg 13560

caacgatcca tactccttta attttgagac tgtcgcaagc ccaaatattg tccttaagaa 13620

acatacacaa agagtcctat ttgaaacttg ctcgaatccc ttattatctg gagtgcacac 13680

agaggataat gaggcagaag agaaggcatt ggctgaattc ttgctcaatc aagaaatgat 13740

tcatccacgt gtcgcgcatg ctatcatgga agcaagctct gtaggtagga gaaagcaaat 13800

tcaggggctt gttgacacaa caaacaccgt gattaagatt gcactgacta ggaggccact 13860

cggcatcaag aggctgatgc ggatagtcaa ttactcgagc atgcatgcaa tgctatttag 13920

agatgatgtt ttctcgtcta atagatccaa ccatccctta gtctcttctg atatgtgttc 13980

tcagacactg gcagactatg cacggaacag aagctggtca cctttgacag ggggtagaaa 14040

aatactgggt gtatctaatc ctgataccat agaacttgta gagggtgaga tccttagtgt 14100

cagtggaggg tgcactagat gtgacagtgg ggatgaacag tttacttggt tccatcttcc 14160

aagcaatata gagctaaacg atgacaccag caagaatcct ccaatgagag tgccatatct 14220

cgggtcaaag actcaagaga ggagagccgc ctcgcttgcg aaaatagctc atatgtcacc 14280

acacgtgaag gcggctctaa gggcgtcatc tgtgttaatc tgggcttatg gggacaacga 14340

aataaactgg actgctgccc ttaagattgc aaggtctcgg tgcaacataa gctcagagta 14400

tcttcgacta ttgtcaccct tgcctacagc tgggaatctc caacatagat tggacgacgg 14460

cataactcag atgacattca cccctgcatc tctatacagg gtgtcacctt acattcatat 14520

atctaatgat tctcaaaggc tatttactga agaaggagtc aaagagggga atgtggttta 14580

tcagcaaatc atgctcttgg gtttgtcctt aattgagtca ctcttcccaa tgacaacaac 14640

caagacatat gatgaaatca cattgcacct ccacagtaaa tttagctgct gtatcaggga 14700

agcacctgtt gcagttcctt tcgagctact cggggtggca ccggaactaa gggcagtaac 14760

ctcaaataag tttatgtatg atcctagccc tgtatcagag ggagactttg cgagacttga 14820

cttagctatc tttaagagtt atgaacttaa tttagagtca tattccacaa tagagctaat 14880

gaacgttctt tcaatatcta gtgggaagtt gattggccag tctgtggttt cttatgatga 14940

agatacctcc ataaagaatg acgctataat agtgtatgac aacacacgga attggatcag 15000

cgaagctcag aattcagatg tggtccgcct attcgagtat gcggcactcg aagtgctcct 15060

cgactgttct tatcaactct actatctgag agtaagaggc ctaaacaata tcgtcctgta 15120

catgagtgat ttatacaaga atatgccagg aattctactc tctaatattg cagccacaat 15180

atctcaccct gtcatccatt caaggttgaa tgcagtaggt ctggtcaacc atgacgggtc 15240

acaccaactt gcagacacag atttcattga aatgtctgca aagctgctag tctcttgcac 15300

tcgacgcgtg gtctcaggtt tacatgcagg gaataagtat gacctgctgt ttccatctgt 15360

cttggatgat aacctaagtg agaagatgct tcagttgata tcccaattat gctgtctgta 15420

tacggtgctc tttgctacaa caagagaaat cccgaaaata agaggcttat ctgcagaaga 15480

gaaatgttca gtacttactg agtacctact gtcagatgct gtgaaaccgt tacttgggtc 15540

cgagcaagtg agctctatca tgtctcccaa catagttacg ttcccagcca atctgtatta 15600

catgtctagg aagagcctta atttgatcag ggagagagag gacagggata ctatcttggc 15660

attgttgttc cctcaagaac cgctgctcga gtttcctctg gtacgagata ttggtgctcg 15720

tgtaaaagat ccatttaccc tacaacctgc ggcgttttta caagagttag atttgagtgc 15780

tccggcaagg tatgacgcat tcacaatcag tcaggcgcat tctgaacaca tattgccaaa 15840

cccagaggaa gatcacttag tacgatactt gttcagagga atagggactg cgtcctcctc 15900

ttggtataag gcatcccatc ttctttctgt acccgaggtc agatgtgcaa ggcatgggaa 15960

ctccttatat ttagcagaag gaagtggagc catcatgagt cttctcgaat tgcatatacc 16020

acacgaaact atctattaca atacactttt ctcgaacgag atgaaccccc cacagcgaca 16080

tttcggacca actccaacac agtttctgaa ttcggttgtt tttaggaatt tacaggcgga 16140

agtaccatgc aaggatggat ttgtccagga gttccgtccg ttatggagag agaatacaga 16200

agaaagcgat ctgacctcag ataaagcagt gggatatatc acatctgtag tgccgtacag 16260

gtctgtatca ttgctgcatt gtgacattga aatccctcca ggatctaatc aaagcttact 16320

agatcagctg gctaccaatc tgtctctgat tgccatgcat tctgtgaagg agggcggggt 16380

cgtgattatc aaagtactgt atgcaatggg atattacttc catctactca tgaacttgtt 16440

tactccatgt tccacgaaag gatatattct ctctaatggc tatgcctgta gaggggatat 16500

ggagtgttac ctgatatttg tcatgggcta cctaggcggg cctacatttg tacatgaggt 16560

ggtgaggatg gcaaaaacgc tagtacagcg gcatggcaca cttttgtcta agtcagatga 16620

gattacactg actaggttat ttacttcaca gcaacagcgt gtaacagaca tcctatccag 16680

ccctttaccg agactaatga agtacttgag agagaatatt gatactgcac tgattgaagc 16740

cgggggacag cctgtccgtc cattctgtgc agagagttta gtgagcacac taacagacat 16800

gactcagaca acccagatca tcgccagcca cattgataca gtcattcgat ctgtgatcta 16860

tatggaagca gagggtgatc ttgctgacac agtattctta tttacccctt acaacctctc 16920

tatggacggg aaaaagagaa catcacttaa acagtgcaca agacagatct tagaggtcac 16980

aatactgggt ctcagagtca aaaatctcaa taaagtaggt gatgtaatca gcctagtact 17040

cagaggtatg atttctctgg aggaccttct cccactgaga acctacttga agtgtagtac 17100

ctgccctaag tatttgaagg ctgtcctagg tattaccaaa ctcaaagaaa tgttcacaga 17160

cgcctcttta ttatacttga ctcgtgctca acaaaaattc tacatgaaaa ctataggcaa 17220

tgcagtcaag ggatactata gtaactgtga ctcttaaagg caaccacata tcaataggcc 17280

ctctttctag ccgatcgtat tcttgttgac ttcattatac catattagaa aaaaattgaa 17340

ttccgaccct ttaagactcg tattcggatt caaataatta tctcagaaaa aaagtgcacg 17400

tagttgttct tgattatagt cccgtcattc accaagtctt tgtttggt 17448

<210> 19
<211> 17436
<212> DNA
<213> Newcastle disease virus

<220>
<223> Mut HN (F277L)/M(G165W)/F(F117S)/F(F190L)/L(V757I)/L(F1551S)/L(R1
      700L)-TRX518

<400> 19
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360
ttctcaagtg atgaggaacc atgtcgccct tgcagggaaa cagaatgagg ccacactggc 420
tgttcttgag atcgatggtt tcaccaacag cgtgcctcaa tttaacaaca ggagtggagt 480
gtctgaggag agagcacaga gattcttgat gatagcaggg tccctccctc gggcatgcag 540
caacggtacc ccgttcatca cagctggggt tgaagatgat gcaccagaag acatcactga 600
tactctggaa agaatcatct ctatccaggc tcaagtatgg gtcacggtag caaaggccat 660
gactgcatat gagacagcgg atgagtcgga aacaagaaga atcaataagt atatgcagca 720
aggcagagtc caaaagaagt acatcctcca ccccgtatgt aggagcgcaa ttcaactcac 780
gattagacaa tctctggcag tccgcatttt cttggttagc gagcttaaga gaggccgcaa 840
cacggcaggt ggaagttcca cttattatag cttggtaggg gacatagact catacatcag 900
gaacaccggg cttactgcat tcttcctgac actcaggtat ggaattaaca ccaagacatc 960
agcccttgca ctcagcagcc tcgcaggcga tatccaaaaa atgaagcagc tcatgcgtct 1020
atatcggatg aaaggagata atgcgccgta catgacactg ctcggtgaca gtgaccagat 1080
gagctttgca ccggctgagt atgcacaact ctactctttt gcaatgggta tggcatccgt 1140
cctagacaaa ggaactggca agtaccagtt cgccagagac tttatgagca catcattctg 1200
gagacttgga gtagagtatg ctcgggctca gggaagcagc atcaatgagg atatggctgc 1260
cgagctaaag ctgaccccag cggcaaggag gggcctggca gctgctgccc aacgagcgtc 1320
cgaggagacc ggcagcatgg acattcccac tcaacaagct ggggtcctca cagggctcag 1380
cgacggaggc cctcaagccc cacaaggtgg actgaacagg tcgcaagggc aaccggacgc 1440
cggagatggg gagacccaat ttttagattt gatgagagca gtggcaaata gtatgagaga 1500
agcgccaaac tctgtgcaga acaccactca gcaggagcct ccatctactc ctgggccatc 1560
tcaagacaac gacaccgact ggggatactg accgacaaca ctcagcctgc ctccatcgaa 1620
ttatctcgat tcttctgcct gtaacctaac ccctggtcca cagtctcaca cggccaaacc 1680
cgcaaacgag cccccccctc tctccccccc cctccagcca cacggctcca accgcccaaa 1740
acaacacggg cacaactcga ctcactcata atctacacag agccgaagat cttagaaaaa 1800
aatacgggta gaagagagac attcagagat caggacaaat catcagggtc tccgctctcc 1860
cctctaccca gcagaccaga gtgaagatgg ccaccttcac ggatgcagag atcgacgatc 1920
tatttgaaac cagtggaact gtcattgaca gcataattac ggcccaaggc aacccagtag 1980
agactgttgg aaggagcgca atcccacaag gcaaaactaa agcattgagt gcagcatggg 2040
agaaacatgg aagcacccaa ccgccggccg gccaagacac ccctgatcga atggacagat 2100
cagacaaaca accatcccca cccgaacagg caaccacaca caacagcccg ccagccacat 2160
ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgttccgc ggcgtgcagt 3240

gccaagtgac cttacgggaa tcaggtccag cgctggtaaa gcccacccaa actctaacat 3300

tgacctgtac tttcagcggc ttttcccttt cgacaagtgg catgggggtg ggatggatcc 3360

ggcaacctcc tgggaaggcc ctcgaatggc tggcccacat ttggtgggac gatgacaagt 3420

actacaaccc ctcactgaag tcacggctga cgatttcaaa ggacaccagc aaaaatcaag 3480

tggtactgac gatgaccaat atggatcccg tcgacactgc tacatactac tgcgctcgca 3540

caaggcgata ctttcccttc gcctattggg gacagggaac tctggttacc gtgagttccg 3600

catccactaa gggcccaagc gtcttcccgc tggccccttc cagtaagagc acctccggag 3660

ggaccgctgc acttggatgc ctggttaaag actattttcc tgaacctgtt acagtcagtt 3720

ggaactcggg ggcactaacc tctggagtgc ataccttccc agctgtactc caatcatctg 3780

gactatactc actttcgtct gtcgtgactg tgccctcttc ttcacttggg acccagacat 3840

atatttgcaa cgtcaatcat aagccatcga acaccaaggt ggataagaag gtggagccga 3900

aatcttgtga taagacccac acatgtcctc catgcccagc ccctgagctg ttgggaggtc 3960

cctctgtgtt cctgttcccc ccaaaaccaa aagacaccct catgatctcc agaacgcctg 4020

aggtcacctg tgtcgttgtg gacgtttctc acgaagaccc agaggtcaaa ttcaattggt 4080

atgtcgacgg ggtagaggtc cacaatgcta agaccaagcc tcgagaagag caatatgctt 4140

ccacttacag agtggtgtct gttctgaccg tcctgcacca agactggcta aatggaaaag 4200

agtataaatg caaggtcagc aacaaagctc tgcccgcccc aattgaaaag actatcagca 4260

aagcgaaagg ccagccacgg gaacctcagg tctatacgct gcccccaagc cgtgatgagc 4320

tcacaaagaa ccaggtgagt ctgacttgtc tagtgaaagg cttttacccc agtgacattg 4380

cagtggagtg ggagtccaac ggacaacccg aaaacaacta taaaactacc ccccccgttc 4440

tcgattctga tggcagcttc ttcttataca gcaaacttac ggtggataaa tcgcggtggc 4500

agcagggaaa tgtgttttcc tgctctgtca tgcatgaggc cctacataat cactatacac 4560

aaaagtctct ctctttgtcc cccggaaaac gccgcaagcg cggctccggc gagggccgcg 4620

gctccctgct gacctgcggc gacgtggagg agaaccccgg ccccatgaag tacctgctgc 4680

ccaccgccgc cgccggcctg ctgctgctgg ccgcccagcc cgccatggcc gagatagtga 4740

tgactcagtc ccccgccact ctctcagtaa gcccaggaga gagagctacg ctgagctgca 4800

aggcctccca gaacgtgggg accaacgtag cctggtacca acaaaagccc ggtcaggccc 4860

cccggctgct catttattcc gcaagttata gatattcagg cattccggcc agatttagtg 4920

gatccggctc aggcacggag tttaccttga ccattagctc tctccaatct gaggatttcg 4980

cggtgtatta ttgtcagcag tataacacag accccttgac cttcggcggt ggcacaaaag 5040

tcgaaattaa gaggacagtc gccgcccctt ccgtgttcat tttccccccc tcagacgaac 5100

agctgaaatc tggcacagct tcgtggtgtt tactgaacaa cttctatccc agagaagcaa 5160

aggtccagtg gaaggtcgat aatgccctgc agtctggtaa tagtcaggag tccgtaactg 5220

agcaggacag caaggactcc acctacagcc tgtcctctac cctgacactg agcaaagcag 5280

actacgaaaa acacaaagtg tacgcctgtg aggtcactca ccaggggctg agctctccag 5340

tgactaagtc ttttaatcgc ggcgagtgct aatgagctga ccgagccctc ctctataaac 5400

ccaagattca aggctccaag cgataacctt ctcccgcctc ttccatccca ctgaatgatc 5460

gcgcagctgc aattaatcta gcaacattaa agattaagaa aaatacgggg tagaatcgga 5520

gtgcaccgat tgtgccaaga tggactcatc taggacaatt gggttatact ttgattctgc 5580

ccttccttct agcaacctgt tagcattccc gattgtctta cgagatgtag gagatggaaa 5640

gaagcaaatt actccgcaat ataggatccg gcgtcttgac tcgtggacag acagtaaaga 5700

agactcggta ttcatcacta cctacggatt catcttccag gttgggaatg aagaagtcac 5760

tgtcggcatg atcaatgata atcccaagcg agagttactc tccgctgcga tgctctgcct 5820

aggaagcgtc ccgaatgttg gagatcctgt tgagctggca agggcctgcc tcactatggt 5880

ggtaacatgc aagaagagtg caactaatac tgagagaatg gtcttctcag tagtgcaggc 5940

accccgggtg ctgcaaagct gtagggtcgt ggcagacaaa tactcgtcag tgaatgcagt 6000

taagcacgtg aaagcaccag agaagatccc ttggagtgaa accctagagt acaaggtgaa 6060

ttttgtctct ttgactgtgg tgccgaagaa ggatgtctac aagatcccaa ccgcagtatt 6120

gaaggtatcc ggctcgagcc tgtataatct tgcgctcaat gtcactattg atgtggaggt 6180

agacccaaag agcccgttag tcaagtccct ttctaggtcc gacagtggat actatgctaa 6240

tctcttctta catatcggac ttatgtccac tgtagataag aggggaaaga aagtgacatt 6300

tgaccagctg gagaggaaga taaggagact tgatctatct gtcgggctca gtgatgtgct 6360

cggaccttct gtgcttgtga aggcgagagg tgcacggact aggctgctgg cacctttctt 6420

ctctaacagc gggacagcct gctaccctat agcaaatgcc tctcctcagg tagctaagat 6480

actctggagt cagaccgcgt gcctgcggag tgtaaaaatc attattcaag cgggcaccca 6540

acgcgctgtc gcagtgactg ctgaccatga ggttacctct actaagatag agaagaggca 6600

taccatcgct aaatacaatc ccttcaagaa ataggccgta tccctgagac tgcaattcac 6660

ccgccttccc aaaacaccat gacaccagat aatgatctgt cttgattact tacagttagt 6720

ttccctgtct atcaaattag aaaaaacacg ggtagaagag ttcggatccc ggccggcgca 6780

ccaaaagcgc aagatgggcc ccagatcttc taccaggatc ccagtacctc taatgctgac 6840

catacggatc acgctggcac tgagttatgt ccgtctgaca agttctcttg atggcaggcc 6900

tcttgcagcc gcagggatcg tggtaacagg ggataaagca gttaacatat acacctcatc 6960

ccagacaggg tcaatcatag tcaagttact cccaaatatg cccaaggaca aagaggcatg 7020

tgcaaaagcc ccattggagg cttacaacag gacactgact actttgctta ccccccttgg 7080

tgattctatc cgcaggatac aagagtctgt gactacatcc ggaggaagga gacagagacg 7140

ctctataggt gccattattg gcagtgtagc tctaggggtt gcaacagctg cacagataac 7200

ggcagcctcg gctctgatac aagccaacca gaatgctgct aacatcctcc ggcttaagga 7260

gagcattgct gcaaccaatg aagctgtaca cgaggtcact ggcggattgt cacagttagc 7320

agtggcagtt ggaaagatgc agcaatttgt taatgaccag cttaataaca cagctcaaga 7380

gttggactgt ataaaaatta cacagcaggt tggtgtagaa ctcaacttat acttaactga 7440

attgactaca gtgttcgggc cacaaatcac ttcccctgcc ttaactcagt tgactgttca 7500

ggctctttac aatctggctg gtggtaatgt agattacttg ttgactaagt taggtgtagg 7560

gaacaaccag ctcagctcat tgattggtag cggcttgatc accggtaacc ctatttttta 7620

cgactcacag actcaactct tgggcataca ggtgacttta ccctcagtcg ggaacctaaa 7680

taatatgcgt gccacctact tggagacctt gtctgtaagc acaacaaagg gatttgcctc 7740

agcacttgtc ccaaaagtag tgacacaggt cggttctgtg atagaagagc ttgacacctc 7800

atactgtata gaagctgatt tggatttata ttgtacaaga atagtgacat tccctatgtc 7860

ccctggtatt tattcctgtt tgagcggcaa tacatcggct tgcatgtatt caaggactga 7920
aggcgcactt actacaccat acatgactct caaaggctca gttgttgcca attgccagat 7980
gacaacatgt agatgtgcag accccccggg tatcatatca caaaattatg gagaggctgt 8040
gtctctaata gataagcact catgcaatgt cgtatcctta gacgggataa ctttgaggct 8100
cagtggggaa tttgatgcaa cttatcaaaa gaatatctca atattagatt ctcaagtact 8160
agtgacaggc aatctcgata tctcaactga gcttgggaat gtcaaccact caataagtaa 8220
tgctttggat aagttagagg aaagcaacag caaactagac aaagtcaatg tcaaactgac 8280
cagcacatct gctctcatta cctatattgt tttaactgtc atatctcttg ttcttggtat 8340
gcttagcctg gttctagcat gctatctgat gtacaagcaa aaagcgcaac gaaagacctt 8400
gttgtggctt ggaaataata ccctagatca gatgagagct actacaaaaa tgtgaatgca 8460
gatgagaggc agaggtatcc ccaatagcaa tctgtgtgtc aattctggca gcctgttaat 8520
cagaagaatt aagaaaaaac taccggatgt aggtgaacaa aagggaatat acgggtagaa 8580
cggcctgaga ggccacccct caatcgggag ccaggcccca ctacgtccgc tctaccgcat 8640
caccaacagc agtcttcagt catggacagc gcagttagcc aagttgcgct agagaatgat 8700
agaagagaag cgaataatac atggcgcttg gttttccgga tcgcagcctt acttttactg 8760
gtaataacct tagccgtctc tgcagtcgcc ctggcatata gtatggaggc tagcacacct 8820
ggcgaccttg taagcatacc gactgcgatc tctagggcag aggaaaggat tacatctgca 8880
ctcggttcca atcaagatgt agtagatagg atatacaagc aagtggccct tgagtctcca 8940
ttggcactgc taaataccga atctataatt atgaatgcaa taacgtctct ctcttatcaa 9000
atcaatggag ccacaaataa tagcgggtgt ggggcacctg ttcatgaccc agattacatc 9060
ggggggatag gtcaagaact tattgtagat gatacgagtg acgtcacatc attttatccc 9120
tctgcattcc aagaacacct gaattttatc ccggcgccca ctacaggatc aggctgcact 9180
cggataccct cattcgacat gagtgctacc cattactgtt acactcacaa tgtgatattg 9240
tctggctgca gagatcactc acactcacat cagtatttag cacttggtgt gcttcggaca 9300
tctgcaacag ggagggtatt cttttctact ctgcgttcca tcaacttgga tgacgcccaa 9360
aatcggaagt cttgcagtgt gagtgcaact cctttaggtt gtgatatgct gtgctctaaa 9420
atcacagaga ctgaggaaga ggattataaa tcagttatcc ccacatcgat ggtacatgga 9480
aggttagggc ttgacggcca ataccatgag aaggacctag acgtcacaac actatttagg 9540
gactgggtgg caaattaccc aggagtagga ggtgggtctt ttattaacaa tcgcgtatgg 9600
ttcccggtct acggagggct aaaacccagc tcgcctagtg acactgcaca agaagggaga 9660
tacgtaatat acaagcgata caatgacaca tgcccggatg agcaagatta ccagattcgg 9720
atggctaagt catcgtataa gcctaggcgg tttggtggaa aacgcgtaca gcaggccatc 9780

ctatccatca aggtgtcaac atccttgggt gaggacccgg tgttgactgt accgcctaat 9840

acggtcgcac tcatgggggc cgaaggcaga gttctcacag tggggacatc tcatttctta 9900

tatcagcgag ggtcatcata cttctctccc gctttgttat accctatgac agtcaacaac 9960

aaaacagcca ctcttcataa tccttataca ttcaatgctt tcactcggcc aggtagtgtc 10020

ccttgccagg cttcagcaag atgccccaac tcatgtgtta ccggagtcta tactgatcca 10080

tatcccttag tcttccatag gaaccacacc ttgcgagggg tattcggaac aatgcttgat 10140

gacgaacaag caagactcaa ccctgtatct gcagtatttg ataacatatc ccgcagtcgc 10200

ataactcggg taagttcaag cagtaccagg gcagcataca cgacatcaac gtgttttaaa 10260

gttgtcaaga ccaataaaac ctattgcctc agcattgcag aaatatccaa taccctcttc 10320

ggggaattca gaattgtccc cttactagtt gagattctca aggatggtgg ggtttaagaa 10380

gctaggaatg gtcggttgag tcaaccgtga gaaggcgggg aagatgatat tgcatcatct 10440

atcttctgta acaccaagaa tcaaatcgaa taccagtgcg agctcgaatc ctacgctgcc 10500

agtcagccat aatcggctgg tgctaatgtg gttagcctga atcttgtcga tagtcacttg 10560

attaagaaaa aatgtggacg gtagtgaaat ataaggcaaa gcagctcaca gaggacagca 10620

cgggtaggac atggcgggct ccggacccga aagggcagag catcagatta tcctaccaga 10680

gtcacatctg tcctcaccat tggtcaagca taaattgctt tattactgga aattaactgg 10740

gctaccgctt cctgacgaat gtgacttcga ccatcttatt atcagccgac aatggaagaa 10800

ggtacttgaa tcggccaccc ctgacattga gagaatgata aaactagggc gggcagtaca 10860

ccagactctc aaccacaatt ccaggataac cggagtactc catccccggt gtttagagga 10920

actggctagt attgaggttc ctgattcaac caacaaattt cggaagatcg aaaaaaagat 10980

ccagattcac aacacaaggt atggagaact gttcacaaga ttatgcacgc atgtagaaaa 11040

gaaactattg gggtcatctt ggtctaacaa tgtcccacgg tcagaggaat tcaacagcat 11100

ccgtacagat ccggcatttt ggtttcactc aaaatggtcc acagccaaat ttgcatggct 11160

ccatataaaa caggtccaga ggcatctgat tgtagcagca agaacaaggt ccgcagtcaa 11220

caaattagtg acgctgaccc ataaggtagg ccaaatcttt gttactcctg agcttgtcat 11280

tgtgacacat acagatgaga acaagttcac gtgtcttacc caggaacttg tgttgatgta 11340

tgcagatatg atggagggca gagatatggt cagcataata tcatccacgg cggcacatct 11400

taggagctta tcagagaaaa ttgatgatat tctgcggtta gtagatgctc tggcaagaga 11460

tttgggcaat caagtctacg atgttgtagc actaatggag ggattcgcat acggcgctgt 11520

tcagctgctt gaaccgtcag gtacatttgc gggggatttc ttcgcattca acctgcagga 11580

gctcaaagat actctaaccg gactcctccc caaggatatc gcagaatctg tgactcacgc 11640

aatcgcaacc atattctctg gcttagaaca aaatcaagca gctgagatgt tgtgcctgtt 11700

gcgtctgtgg ggtcacccac tactcgagtc ccgtattgca gcaaaagcag ttaggagtca 11760

aatgtgcgca ccaaaaatgg tagactttga tatgatcctc caggtattat ctttctttaa 11820

aggaacaatc atcaatggat atagaaagaa gaatgcaggc gtgtggccac gtgtcaagat 11880

agatacgata tacgggaagg tcatagggca gctacacgca gattctgcag agatttcaca 11940

tgatgtcatg ttgagggaat acaagagttt atctgcactt gaattcgagc catgtataga 12000

gtatgaccct gtcaccaatc tgagcatgtt tttaaaagac aaggcaatcg cacacccgaa 12060

agacaactgg ctcgcttcgt ttaggcgaaa ccttctctct gaggaccaga agaaacatgt 12120

aagagaggca acctcaacta accgcctctt gatagagttc ttagagtcaa atgattttga 12180

tccatataaa gagatggaat atctgacgac ccttgagtac ctaagagatg ataatgtggc 12240

agtatcatac tcactcaaag aaaaagaggt gaaagttaat gggcggattt tcgcaaagct 12300

aacaaataga ttaaggaatt gtcaggtaat ggcagaaggg atcctagctg accagattgc 12360

acctttcttc cagggaaatg gggtcattca ggatagcata tctttgacta aaagtatgct 12420

agcgatgagt caactgtctt tcaacagcaa caagaaacgt attactgact gcaaagaaag 12480

agtatcctca aaccgcaatc atgatccgaa gagcaagaat cgtcggagag ttgccacttt 12540

tgtaactacc gacctgcaaa agtattgtct caattggaga tatcagacag tcaagctgtt 12600

cgctcatgcc atcaatcagc tgatgggctt acctcacttc ttcgagtgga ttcatcttag 12660

actaatggat actacgatgt ttgtagggga ccctttcaat cctccaagtg accctacgga 12720

ctgtgatcta tcaagagtcc caaatgatga catatatatt gtcagtgcta ggggaggcat 12780

tgagggatta tgccagaagc tatggacaat gatctcaatt gctgcaatcc agcttgctgc 12840

agcaagatca cattgtcgcg ttgcctgtat ggtacaaggc gacaatcaag taatagctat 12900

aacgagagag gtaagatcag atgactcccc agagatggtg ttaacacaat tgcatcaagc 12960

cagtgataat ttcttcaagg aattgattca tgtcaatcat ttgatcggcc ataatttgaa 13020

ggatcgtgaa accatcaggt cagacacgtt cttcatatac agcaaacgaa tattcaaaga 13080

tggagcaata ctcagtcagg tcctcaaaaa ctcatctaaa ttagtgctaa tatcaggcga 13140

ccttagtgaa aacactgtaa tgtcttgtgc caacattgca tctactgtag cacggttatg 13200

cgagaacggg cttcctaagg atttctgtta ttacttaaac tacttaatga gttgcgtgca 13260

gacatacttc gattccgaat tttccatcac caacaactcg caactcgatt ctaaccagtc 13320

gtggatagag gacatttctt ttgtgcactc atatgtcctg acccctgctc aactgggggg 13380

actgagtaac cttcaatact caaggctcta cacaaggaac atcggcgacc cgggaaccac 13440

tgctttcgca gagatcaaga gattagaggc agtggggtta ctgagtccta gcattatgac 13500

taacatctta actaggccgc ctggaaatgg agattgggcc agtctgtgca acgatccata 13560

ctcctttaat tttgagactg tcgcaagccc aaatattgtc cttaagaaac atacacaaag 13620

agtcctattt gaaacttgct cgaatccctt attatctgga gtgcacacag aggataatga 13680

ggcagaagag aaggcattgg ctgaattctt gctcaatcaa gaaatgattc atccacgtgt 13740

cgcgcatgct atcatggaag caagctctgt aggtaggaga aagcaaattc aggggcttgt 13800

tgacacaaca aacaccgtga ttaagattgc actgactagg aggccactcg gcatcaagag 13860

gctgatgcgg atagtcaatt actcgagcat gcatgcaatg ctatttagag atgatgtttt 13920

ctcgtctaat agatccaacc atcccttagt ctcttctgat atgtgttctc agacactggc 13980

agactatgca cggaacagaa gctggtcacc tttgacaggg ggtagaaaaa tactgggtgt 14040

atctaatcct gataccatag aacttgtaga gggtgagatc cttagtgtca gtggagggtg 14100

cactagatgt gacagtgggg atgaacagtt tacttggttc catcttccaa gcaatataga 14160

gctaaacgat gacaccagca agaatcctcc aatgagagtg ccatatctcg ggtcaaagac 14220

tcaagagagg agagccgcct cgcttgcgaa aatagctcat atgtcaccac acgtgaaggc 14280

ggctctaagg gcgtcatctg tgttaatctg ggcttatggg gacaacgaaa taaactggac 14340

tgctgccctt aagattgcaa ggtctcggtg caacataagc tcagagtatc ttcgactatt 14400

gtcacccttg cctacagctg ggaatctcca acatagattg gacgacggca taactcagat 14460

gacattcacc cctgcatctc tatacagggt gtcaccttac attcatatat ctaatgattc 14520

tcaaaggcta tttactgaag aaggagtcaa agaggggaat gtggtttatc agcaaatcat 14580

gctcttgggt ttgtccttaa ttgagtcact cttcccaatg acaacaacca agacatatga 14640

tgaaatcaca ttgcacctcc acagtaaatt tagctgctgt atcagggaag cacctgttgc 14700

agttcctttc gagctactcg gggtggcacc ggaactaagg gcagtaacct caaataagtt 14760

tatgtatgat cctagccctg tatcagaggg agactttgcg agacttgact tagctatctt 14820

taagagttat gaacttaatt tagagtcata ttccacaata gagctaatga acgttctttc 14880

aatatctagt gggaagttga ttggccagtc tgtggtttct tatgatgaag atacctccat 14940

aaagaatgac gctataatag tgtatgacaa cacacggaat tggatcagcg aagctcagaa 15000

ttcagatgtg gtccgcctat tcgagtatgc ggcactcgaa gtgctcctcg actgttctta 15060

tcaactctac tatctgagag taagaggcct aaacaatatc gtcctgtaca tgagtgattt 15120

atacaagaat atgccaggaa ttctactctc taatattgca gccacaatat ctcaccctgt 15180

catccattca aggttgaatg cagtaggtct ggtcaaccat gacgggtcac accaacttgc 15240

agacacagat ttcattgaaa tgtctgcaaa gctgctagtc tcttgcactc gacgcgtggt 15300

ctcaggttta catgcaggga ataagtatga cctgctgttt ccatctgtct tggatgataa 15360

cctaagtgag aagatgcttc agttgatatc ccaattatgc tgtctgtata cggtgctctt 15420

tgctacaaca agagaaatcc cgaaaataag aggcttatct gcagaagaga aatgttcagt 15480

acttactgag tacctactgt cagatgctgt gaaaccgtta cttgggtccg agcaagtgag 15540

ctctatcatg tctcccaaca tagttacgtt cccagccaat ctgtattaca tgtctaggaa 15600

gagccttaat ttgatcaggg agagagagga cagggatact atcttggcat tgttgttccc 15660

tcaagaaccg ctgctcgagt ttcctctggt acgagatatt ggtgctcgtg taaaagatcc 15720

atttacccta caacctgcgg cgtttttaca agagttagat ttgagtgctc cggcaaggta 15780

tgacgcattc acaatcagtc aggcgcattc tgaacacata ttgccaaacc cagaggaaga 15840

tcacttagta cgatacttgt tcagaggaat agggactgcg tcctcctctt ggtataaggc 15900

atcccatctt ctttctgtac ccgaggtcag atgtgcaagg catgggaact ccttatattt 15960

agcagaagga agtggagcca tcatgagtct tctcgaattg catataccac acgaaactat 16020

ctattacaat acacttttct cgaacgagat gaacccccca cagcgacatt tcggaccaac 16080

tccaacacag tttctgaatt cggttgtttt taggaattta caggcggaag taccatgcaa 16140

ggatggattt gtccaggagt tccgtccgtt atggagagag aatacagaag aaagcgatct 16200

gacctcagat aaagcagtgg gatatatcac atctgtagtg ccgtacaggt ctgtatcatt 16260

gctgcattgt gacattgaaa tccctccagg atctaatcaa agcttactag atcagctggc 16320

taccaatctg tctctgattg ccatgcattc tgtgaaggag ggcggggtcg tgattatcaa 16380

agtactgtat gcaatgggat attacttcca tctactcatg aacttgttta ctccatgttc 16440

cacgaaagga tatattctct ctaatggcta tgcctgtaga ggggatatgg agtgttacct 16500

gatatttgtc atgggctacc taggcgggcc tacatttgta catgaggtgg tgaggatggc 16560

aaaaacgcta gtacagcggc atggcacact tttgtctaag tcagatgaga ttacactgac 16620

taggttattt acttcacagc aacagcgtgt aacagacatc ctatccagcc ctttaccgag 16680

actaatgaag tacttgagag agaatattga tactgcactg attgaagccg ggggacagcc 16740

tgtccgtcca ttctgtgcag agagtttagt gagcacacta acagacatga ctcagacaac 16800

ccagatcatc gccagccaca ttgatacagt cattcgatct gtgatctata tggaagcaga 16860

gggtgatctt gctgacacag tattcttatt taccccttac aacctctcta tggacgggaa 16920

aaagagaaca tcacttaaac agtgcacaag acagatctta gaggtcacaa tactgggtct 16980

cagagtcaaa aatctcaata aagtaggtga tgtaatcagc ctagtactca gaggtatgat 17040

ttctctggag gaccttctcc cactgagaac ctacttgaag tgtagtacct gccctaagta 17100

tttgaaggct gtcctaggta ttaccaaact caaagaaatg ttcacagacg cctctttatt 17160

atacttgact cgtgctcaac aaaaattcta catgaaaact ataggcaatg cagtcaaggg 17220

atactatagt aactgtgact cttaaaggca accacatatc aataggccct ctttctagcc 17280

gatcgtattc ttgttgactt cattatacca tattagaaaa aaattgaatt ccgacccttt 17340

aagactcgta ttcggattca aataattatc tcagaaaaaa agtgcacgta gttgttcttg 17400

attatagtcc cgtcattcac caagtctttg tttggt 17436

<210> 20
<211> 17430
<212> DNA
<213> Newcastle disease virus

<220>
<223> Mut HN (F277L)/M(G165W)/F(F117S)/F(F190L)/L(V757I)/L(F1551S)/L(R1
      700L)-BMS 986178

<400> 20
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat ttttagattt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaag cagtttgaga gctccgttcg 3180

ccaccatgga gttcggcctg tcctgggtgt tcctggtggc cctgttccgc ggcgtgcagt 3240

gcgaagtgca gctagtgcag agtgggggcg ggctagtgca gccagggggg tctcttcgct 3300

tgtcttgcgc cggtagcggt tttacctttt catcgtatgc aatgtactgg gtgaggcaag 3360

cccctggcaa gggattggag tgggtgagcg cgatagacac tgacgccggt accttctatg 3420

cggatagtgt aagggggcgc ttcaccatta gtcgcgacaa cgccaagaac tctctttatc 3480

tccagatgaa ttcactccga gctgaggaca ccgcagtcta cttttgtgct aggctggggg 3540

aaggctactt ctttgattac tggggacagg gaaccttggt gactgtgagc tctgcctcca 3600

cgaaaggtcc ttctgttttc cccttgcccc cctcctcaaa atctacctct ggaggtaccg 3660

ccgctctagg ctgcctagta aaggattact tccctgaacc cgtgacggtg tcttggaaca 3720

gtggtgctct tacaagtggc gtgcacacct ttcctgccgt gctccaaagt tctgggcttt 3780

attctctgtc ctccgtggtg accgtgccca gcagctctct tggtacccag acttacatct 3840

gtaatgttaa ccacaaaccc tcaaatacaa aggtggacaa gagagtggag ccaaagagct 3900

gcgataaaac ccacacctgt cccccctgtc ccgcacctga gcttcttggg ggccctagtg 3960

tgtttctgtt cccacctaag cccaaagaca ccctgatgat ctctaggacc ccggaggtta 4020

cctgcgtagt tgtggacgtc tcccacgagg atcccgaggt gaaattcaat tggtacgtcg 4080

acggcgttga agtccataac gccaagacca agcctcgcga agaacagtat aactctacct 4140

atcgcgtggt gtcggtcctg actgtgcttc accaggactg gcttaacggg aaggaatata 4200

aatgcaaggt cagcaataag gccctccccg ctccaattga gaagaccatt tccaaagcta 4260

aaggccagcc acgggagccc caggtctaca ctctaccacc ctcccgcgag gagatgacca 4320

aaaaccaggt gtccctgacc tgtttggtga agggatttta cccgtccgat atcgctgtgg 4380

agtgggaaag taatggccaa cctgaaaaca actataagac aaccccgcca gtcctagata 4440

gcgatggatc cttcttcctg tatagtaaac ttacagtgga taaaagtagg tggcagcagg 4500

gtaacgtttt ttcgtgtagt gtgatgcatg aagcactcca caatcactat acccaaaaaa 4560

gcctttcgct gtcccctggc cgccgcaagc gcggctccgg cgagggccgc ggctccctgc 4620

tgacctgcgg cgacgtggag gagaaccccg gccccatgaa gtacctgctg cccaccgccg 4680

ccgccggcct gctgctgctg gccgcccagc ccgccatggc cgagattgtg ctgacgcaga 4740

gcccagcaac tttgagtctc tcgcccggcg agcgcgccac actttcctgt cgagcctccc 4800

aatccgtgag cagttacctg gcatggtacc aacagaagcc tgggcaagcc cccagattgc 4860

tgatttatga tgcctccaat cgagcaacag gaataccagc tcggttcagt gggagcggaa 4920

gcggtacaga tttcactctg acaatcagct cgctggagcc agaggacttc gctgtttatt 4980

actgccagca gcgttcgaac tggcccccaa cgtttggagg aggcaccaaa gtggaaataa 5040

aacgaacagt agcagcccca tctgtcttca tcttcccccc cagcgatgaa cagctgaagt 5100

caggcactgc ttccgtcgtc tgcctactga acaattttta tcctagagag gccaaggtcc 5160

aatggaaggt agacaacgca ctccaatcag ggaactcaca agagtctgtc accgagcagg 5220

actctaagga ctcgacatac tcattatcta gtaccctgac actgagcaag gccgactacg 5280

aaaaacataa agtctacgcc tgtgaagtga cgcaccaggg actcagctct cccgtcacta 5340

agagcttcaa tcggggggag tgttaatgag ctgaccgagc cctcctctat aaacccaaga 5400

ttcaaggctc caagcgataa ccttctcccg cctcttccat cccactgaat gatcgcgcag 5460

ctgcaattaa tctagcaaca ttaaagatta agaaaaaata cgggtagaat cggagtgcac 5520

cgattgtgcc aagatggact catctaggac aattgggtta tactttgatt ctgcccttcc 5580

ttctagcaac ctgttagcat tcccgattgt cttacgagat gtaggagatg gaaagaagca 5640

aattactccg caatatagga tccggcgtct tgactcgtgg acagacagta aagaagactc 5700

ggtattcatc actacctacg gattcatctt ccaggttggg aatgaagaag tcactgtcgg 5760

catgatcaat gataatccca agcgagagtt actctccgct gcgatgctct gcctaggaag 5820

cgtcccgaat gttggagatc ctgttgagct ggcaagggcc tgcctcacta tggtggtaac 5880

atgcaagaag agtgcaacta atactgagag aatggtcttc tcagtagtgc aggcaccccg 5940

ggtgctgcaa agctgtaggg tcgtggcaga caaatactcg tcagtgaatg cagttaagca 6000

cgtgaaagca ccagagaaga tcccttggag tgaaacccta gagtacaagg tgaattttgt 6060

ctctttgact gtggtgccga agaaggatgt ctacaagatc ccaaccgcag tattgaaggt 6120

atccggctcg agcctgtata atcttgcgct caatgtcact attgatgtgg aggtagaccc 6180

aaagagcccg ttagtcaagt ccctttctag gtccgacagt ggatactatg ctaatctctt 6240

cttacatatc ggacttatgt ccactgtaga taagagggga aagaaagtga catttgacca 6300

gctggagagg aagataagga gacttgatct atctgtcggg ctcagtgatg tgctcggacc 6360

ttctgtgctt gtgaaggcga gaggtgcacg gactaggctg ctggcacctt tcttctctaa 6420

cagcgggaca gcctgctacc ctatagcaaa tgcctctcct caggtagcta agatactctg 6480

gagtcagacc gcgtgcctgc ggagtgtaaa aatcattatt caagcgggca cccaacgcgc 6540

tgtcgcagtg actgctgacc atgaggttac ctctactaag atagagaaga ggcataccat 6600

cgctaaatac aatcccttca agaaataggc cgtatccctg agactgcaat tcacccgcct 6660

tcccaaaaca ccatgacacc agataatgat ctgtcttgat tacttacagt tagtttccct 6720

gtctatcaaa ttagaaaaaa cacgggtaga agagttcgga tcccggccgg cgcaccaaaa 6780

gcgcaagatg ggccccagat cttctaccag gatcccagta cctctaatgc tgaccatacg 6840

gatcacgctg gcactgagtt atgtccgtct gacaagttct cttgatggca ggcctcttgc 6900

agccgcaggg atcgtggtaa caggggataa agcagttaac atatacacct catcccagac 6960

agggtcaatc atagtcaagt tactcccaaa tatgcccaag gacaaagagg catgtgcaaa 7020

agccccattg gaggcttaca acaggacact gactactttg cttacccccc ttggtgattc 7080

tatccgcagg atacaagagt ctgtgactac atccggagga aggagacaga gacgctctat 7140

aggtgccatt attggcagtg tagctctagg ggttgcaaca gctgcacaga taacggcagc 7200

ctcggctctg atacaagcca accagaatgc tgctaacatc ctccggctta aggagagcat 7260

tgctgcaacc aatgaagctg tacacgaggt cactggcgga ttgtcacagt tagcagtggc 7320

agttggaaag atgcagcaat ttgttaatga ccagcttaat aacacagctc aagagttgga 7380

ctgtataaaa attacacagc aggttggtgt agaactcaac ttatacttaa ctgaattgac 7440

tacagtgttc gggccacaaa tcacttcccc tgccttaact cagttgactg ttcaggctct 7500

ttacaatctg gctggtggta atgtagatta cttgttgact aagttaggtg tagggaacaa 7560

ccagctcagc tcattgattg gtagcggctt gatcaccggt aaccctattt tttacgactc 7620

acagactcaa ctcttgggca tacaggtgac tttaccctca gtcgggaacc taaataatat 7680

gcgtgccacc tacttggaga ccttgtctgt aagcacaaca aagggatttg cctcagcact 7740

tgtcccaaaa gtagtgacac aggtcggttc tgtgatagaa gagcttgaca cctcatactg 7800

tatagaagct gatttggatt tatattgtac aagaatagtg acattcccta tgtcccctgg 7860

tatttattcc tgtttgagcg gcaatacatc ggcttgcatg tattcaagga ctgaaggcgc 7920

acttactaca ccatacatga ctctcaaagg ctcagttgtt gccaattgcc agatgacaac 7980

atgtagatgt gcagaccccc cgggtatcat atcacaaaat tatggagagg ctgtgtctct 8040

aatagataag cactcatgca atgtcgtatc cttagacggg ataactttga ggctcagtgg 8100

ggaatttgat gcaacttatc aaaagaatat ctcaatatta gattctcaag tactagtgac 8160

aggcaatctc gatatctcaa ctgagcttgg gaatgtcaac cactcaataa gtaatgcttt 8220

ggataagtta gaggaaagca acagcaaact agacaaagtc aatgtcaaac tgaccagcac 8280

atctgctctc attacctata ttgttttaac tgtcatatct cttgttcttg gtatgcttag 8340

cctggttcta gcatgctatc tgatgtacaa gcaaaaagcg caacgaaaga ccttgttgtg 8400

gcttggaaat aataccctag atcagatgag agctactaca aaaatgtgaa tgcagatgag 8460

aggcagaggt atccccaata gcaatctgtg tgtcaattct ggcagcctgt taatcagaag 8520

aattaagaaa aaactaccgg atgtaggtga acaaaaggga atatacgggt agaacggcct 8580

gagaggccac ccctcaatcg ggagccaggc cccactacgt ccgctctacc gcatcaccaa 8640

cagcagtctt cagtcatgga cagcgcagtt agccaagttg cgctagagaa tgatagaaga 8700

gaagcgaata atacatggcg cttggttttc cggatcgcag ccttactttt actggtaata 8760

accttagccg tctctgcagt cgccctggca tatagtatgg aggctagcac acctggcgac 8820

cttgtaagca taccgactgc gatctctagg gcagaggaaa ggattacatc tgcactcggt 8880

tccaatcaag atgtagtaga taggatatac aagcaagtgg cccttgagtc tccattggca 8940

ctgctaaata ccgaatctat aattatgaat gcaataacgt ctctctctta tcaaatcaat 9000

ggagccacaa ataatagcgg gtgtggggca cctgttcatg acccagatta catcgggggg 9060

ataggtcaag aacttattgt agatgatacg agtgacgtca catcatttta tccctctgca 9120

ttccaagaac acctgaattt tatcccggcg cccactacag gatcaggctg cactcggata 9180

ccctcattcg acatgagtgc tacccattac tgttacactc acaatgtgat attgtctggc 9240

tgcagagatc actcacactc acatcagtat ttagcacttg gtgtgcttcg gacatctgca 9300

acagggaggg tattcttttc tactctgcgt tccatcaact tggatgacgc ccaaaatcgg 9360

aagtcttgca gtgtgagtgc aactccttta ggttgtgata tgctgtgctc taaaatcaca 9420

gagactgagg aagaggatta taaatcagtt atccccacat cgatggtaca tggaaggtta 9480

gggcttgacg gccaatacca tgagaaggac ctagacgtca caacactatt tagggactgg 9540

gtggcaaatt acccaggagt aggaggtggg tcttttatta acaatcgcgt atggttcccg 9600

gtctacggag ggctaaaacc cagctcgcct agtgacactg cacaagaagg gagatacgta 9660

atatacaagc gatacaatga cacatgcccg gatgagcaag attaccagat tcggatggct 9720

aagtcatcgt ataagcctag gcggtttggt ggaaaacgcg tacagcaggc catcctatcc 9780

atcaaggtgt caacatcctt gggtgaggac ccggtgttga ctgtaccgcc taatacggtc 9840

gcactcatgg gggccgaagg cagagttctc acagtgggga catctcattt cttatatcag 9900

cgagggtcat catacttctc tcccgctttg ttatacccta tgacagtcaa caacaaaaca 9960

gccactcttc ataatcctta tacattcaat gctttcactc ggccaggtag tgtcccttgc 10020

caggcttcag caagatgccc caactcatgt gttaccggag tctatactga tccatatccc 10080

ttagtcttcc ataggaacca caccttgcga ggggtattcg gaacaatgct tgatgacgaa 10140

caagcaagac tcaaccctgt atctgcagta tttgataaca tatcccgcag tcgcataact 10200

cgggtaagtt caagcagtac cagggcagca tacacgacat caacgtgttt taaagttgtc 10260

aagaccaata aaacctattg cctcagcatt gcagaaatat ccaataccct cttcggggaa 10320

ttcagaattg tccccttact agttgagatt ctcaaggatg gtggggttta agaagctagg 10380

aatggtcggt tgagtcaacc gtgagaaggc ggggaagatg atattgcatc atctatcttc 10440

tgtaacacca agaatcaaat cgaataccag tgcgagctcg aatcctacgc tgccagtcag 10500

ccataatcgg ctggtgctaa tgtggttagc ctgaatcttg tcgatagtca cttgattaag 10560

aaaaaatgtg gacggtagtg aaatataagg caaagcagct cacagaggac agcacgggta 10620

ggacatggcg ggctccggac ccgaaagggc agagcatcag attatcctac cagagtcaca 10680

tctgtcctca ccattggtca agcataaatt gctttattac tggaaattaa ctgggctacc 10740

gcttcctgac gaatgtgact tcgaccatct tattatcagc cgacaatgga agaaggtact 10800

tgaatcggcc acccctgaca ttgagagaat gataaaacta gggcgggcag tacaccagac 10860

tctcaaccac aattccagga taaccggagt actccatccc cggtgtttag aggaactggc 10920

tagtattgag gttcctgatt caaccaacaa atttcggaag atcgaaaaaa agatccagat 10980

tcacaacaca aggtatggag aactgttcac aagattatgc acgcatgtag aaaagaaact 11040

attggggtca tcttggtcta acaatgtccc acggtcagag gaattcaaca gcatccgtac 11100

agatccggca ttttggtttc actcaaaatg gtccacagcc aaatttgcat ggctccatat 11160

aaaacaggtc cagaggcatc tgattgtagc agcaagaaca aggtccgcag tcaacaaatt 11220

agtgacgctg acccataagg taggccaaat ctttgttact cctgagcttg tcattgtgac 11280

acatacagat gagaacaagt tcacgtgtct tacccaggaa cttgtgttga tgtatgcaga 11340

tatgatggag ggcagagata tggtcagcat aatatcatcc acggcggcac atcttaggag 11400

cttatcagag aaaattgatg atattctgcg gttagtagat gctctggcaa gagatttggg 11460

caatcaagtc tacgatgttg tagcactaat ggagggattc gcatacggcg ctgttcagct 11520

gcttgaaccg tcaggtacat ttgcggggga tttcttcgca ttcaacctgc aggagctcaa 11580

agatactcta accggactcc tccccaagga tatcgcagaa tctgtgactc acgcaatcgc 11640

aaccatattc tctggcttag aacaaaatca agcagctgag atgttgtgcc tgttgcgtct 11700

gtggggtcac ccactactcg agtcccgtat tgcagcaaaa gcagttagga gtcaaatgtg 11760

cgcaccaaaa atggtagact ttgatatgat cctccaggta ttatctttct ttaaaggaac 11820

aatcatcaat ggatatagaa agaagaatgc aggcgtgtgg ccacgtgtca agatagatac 11880

gatatacggg aaggtcatag ggcagctaca cgcagattct gcagagattt cacatgatgt 11940

catgttgagg gaatacaaga gtttatctgc acttgaattc gagccatgta tagagtatga 12000

ccctgtcacc aatctgagca tgtttttaaa agacaaggca atcgcacacc cgaaagacaa 12060

ctggctcgct tcgtttaggc gaaaccttct ctctgaggac cagaagaaac atgtaagaga 12120

ggcaacctca actaaccgcc tcttgataga gttcttagag tcaaatgatt ttgatccata 12180

taaagagatg gaatatctga cgacccttga gtacctaaga gatgataatg tggcagtatc 12240

atactcactc aaagaaaaag aggtgaaagt taatgggcgg attttcgcaa agctaacaaa 12300

tagattaagg aattgtcagg taatggcaga agggatccta gctgaccaga ttgcaccttt 12360

cttccaggga aatggggtca ttcaggatag catatctttg actaaaagta tgctagcgat 12420

gagtcaactg tctttcaaca gcaacaagaa acgtattact gactgcaaag aaagagtatc 12480

ctcaaaccgc aatcatgatc cgaagagcaa gaatcgtcgg agagttgcca cttttgtaac 12540

taccgacctg caaaagtatt gtctcaattg gagatatcag acagtcaagc tgttcgctca 12600

tgccatcaat cagctgatgg gcttacctca cttcttcgag tggattcatc ttagactaat 12660

ggatactacg atgtttgtag gggacccttt caatcctcca agtgacccta cggactgtga 12720

tctatcaaga gtcccaaatg atgacatata tattgtcagt gctaggggag gcattgaggg 12780

attatgccag aagctatgga caatgatctc aattgctgca atccagcttg ctgcagcaag 12840

atcacattgt cgcgttgcct gtatggtaca aggcgacaat caagtaatag ctataacgag 12900

agaggtaaga tcagatgact ccccagagat ggtgttaaca caattgcatc aagccagtga 12960

taatttcttc aaggaattga ttcatgtcaa tcatttgatc ggccataatt tgaaggatcg 13020

tgaaaccatc aggtcagaca cgttcttcat atacagcaaa cgaatattca aagatggagc 13080

aatactcagt caggtcctca aaaactcatc taaattagtg ctaatatcag gcgaccttag 13140

tgaaaacact gtaatgtctt gtgccaacat tgcatctact gtagcacggt tatgcgagaa 13200

cgggcttcct aaggatttct gttattactt aaactactta atgagttgcg tgcagacata 13260

cttcgattcc gaattttcca tcaccaacaa ctcgcaactc gattctaacc agtcgtggat 13320

agaggacatt tcttttgtgc actcatatgt cctgacccct gctcaactgg ggggactgag 13380

taaccttcaa tactcaaggc tctacacaag gaacatcggc gacccgggaa ccactgcttt 13440

cgcagagatc aagagattag aggcagtggg gttactgagt cctagcatta tgactaacat 13500

cttaactagg ccgcctggaa atggagattg ggccagtctg tgcaacgatc catactcctt 13560

taattttgag actgtcgcaa gcccaaatat tgtccttaag aaacatacac aaagagtcct 13620

atttgaaact tgctcgaatc ccttattatc tggagtgcac acagaggata atgaggcaga 13680

agagaaggca ttggctgaat tcttgctcaa tcaagaaatg attcatccac gtgtcgcgca 13740

tgctatcatg gaagcaagct ctgtaggtag gagaaagcaa attcaggggc ttgttgacac 13800

aacaaacacc gtgattaaga ttgcactgac taggaggcca ctcggcatca agaggctgat 13860

gcggatagtc aattactcga gcatgcatgc aatgctattt agagatgatg ttttctcgtc 13920

taatagatcc aaccatccct tagtctcttc tgatatgtgt tctcagacac tggcagacta 13980

tgcacggaac agaagctggt cacctttgac agggggtaga aaaatactgg gtgtatctaa 14040

tcctgatacc atagaacttg tagagggtga gatccttagt gtcagtggag ggtgcactag 14100

atgtgacagt ggggatgaac agtttacttg gttccatctt ccaagcaata tagagctaaa 14160

cgatgacacc agcaagaatc ctccaatgag agtgccatat ctcgggtcaa agactcaaga 14220

gaggagagcc gcctcgcttg cgaaaatagc tcatatgtca ccacacgtga aggcggctct 14280

aagggcgtca tctgtgttaa tctgggctta tggggacaac gaaataaact ggactgctgc 14340

ccttaagatt gcaaggtctc ggtgcaacat aagctcagag tatcttcgac tattgtcacc 14400

cttgcctaca gctgggaatc tccaacatag attggacgac ggcataactc agatgacatt 14460

cacccctgca tctctataca gggtgtcacc ttacattcat atatctaatg attctcaaag 14520

gctatttact gaagaaggag tcaaagaggg gaatgtggtt tatcagcaaa tcatgctctt 14580

gggtttgtcc ttaattgagt cactcttccc aatgacaaca accaagacat atgatgaaat 14640
cacattgcac ctccacagta aatttagctg ctgtatcagg gaagcacctg ttgcagttcc 14700
tttcgagcta ctcggggtgg caccggaact aagggcagta acctcaaata agtttatgta 14760
tgatcctagc cctgtatcag agggagactt tgcgagactt gacttagcta tctttaagag 14820
ttatgaactt aatttagagt catattccac aatagagcta atgaacgttc tttcaatatc 14880
tagtgggaag ttgattggcc agtctgtggt ttcttatgat gaagatacct ccataaagaa 14940
tgacgctata atagtgtatg acaacacacg gaattggatc agcgaagctc agaattcaga 15000
tgtggtccgc ctattcgagt atgcggcact cgaagtgctc ctcgactgtt cttatcaact 15060
ctactatctg agagtaagag gcctaaacaa tatcgtcctg tacatgagtg atttatacaa 15120
gaatatgcca ggaattctac tctctaatat tgcagccaca atatctcacc ctgtcatcca 15180
ttcaaggttg aatgcagtag gtctggtcaa ccatgacggg tcacaccaac ttgcagacac 15240
agatttcatt gaaatgtctg caaagctgct agtctcttgc actcgacgcg tggtctcagg 15300
tttacatgca gggaataagt atgacctgct gtttccatct gtcttggatg ataacctaag 15360
tgagaagatg cttcagttga tatcccaatt atgctgtctg tatacggtgc tctttgctac 15420
aacaagagaa atcccgaaaa taagaggctt atctgcagaa gagaaatgtt cagtacttac 15480
tgagtaccta ctgtcagatg ctgtgaaacc gttacttggg tccgagcaag tgagctctat 15540
catgtctccc aacatagtta cgttcccagc caatctgtat tacatgtcta ggaagagcct 15600
taatttgatc agggagagag aggacaggga tactatcttg gcattgttgt tccctcaaga 15660
accgctgctc gagtttcctc tggtacgaga tattggtgct cgtgtaaaag atccatttac 15720
cctacaacct gcggcgtttt tacaagagtt agatttgagt gctccggcaa ggtatgacgc 15780
attcacaatc agtcaggcgc attctgaaca catattgcca aacccagagg aagatcactt 15840
agtacgatac ttgttcagag gaatagggac tgcgtcctcc tcttggtata aggcatccca 15900
tcttctttct gtacccgagg tcagatgtgc aaggcatggg aactccttat atttagcaga 15960
aggaagtgga gccatcatga gtcttctcga attgcatata ccacacgaaa ctatctatta 16020
caatacactt ttctcgaacg agatgaaccc cccacagcga catttcggac caactccaac 16080
acagtttctg aattcggttg tttttaggaa tttacaggcg gaagtaccat gcaaggatgg 16140
atttgtccag gagttccgtc cgttatggag agagaataca gaagaaagcg atctgacctc 16200
agataaagca gtgggatata tcacatctgt agtgccgtac aggtctgtat cattgctgca 16260
ttgtgacatt gaaatccctc caggatctaa tcaaagctta ctagatcagc tggctaccaa 16320
tctgtctctg attgccatgc attctgtgaa ggagggcggg gtcgtgatta tcaaagtact 16380
gtatgcaatg ggatattact tccatctact catgaacttg tttactccat gttccacgaa 16440
aggatatatt ctctctaatg gctatgcctg tagaggggat atggagtgtt acctgatatt 16500

tgtcatgggc tacctaggcg ggcctacatt tgtacatgag gtggtgagga tggcaaaaac 16560

gctagtacag cggcatggca cacttttgtc taagtcagat gagattacac tgactaggtt 16620

atttacttca cagcaacagc gtgtaacaga catcctatcc agccctttac cgagactaat 16680

gaagtacttg agagagaata ttgatactgc actgattgaa gccgggggac agcctgtccg 16740

tccattctgt gcagagagtt tagtgagcac actaacagac atgactcaga caacccagat 16800

catcgccagc cacattgata cagtcattcg atctgtgatc tatatggaag cagagggtga 16860

tcttgctgac acagtattct tatttacccc ttacaacctc tctatggacg ggaaaaagag 16920

aacatcactt aaacagtgca caagacagat cttagaggtc acaatactgg gtctcagagt 16980

caaaaatctc aataaagtag gtgatgtaat cagcctagta ctcagaggta tgatttctct 17040

ggaggacctt ctcccactga gaacctactt gaagtgtagt acctgcccta agtatttgaa 17100

ggctgtccta ggtattacca aactcaaaga aatgttcaca gacgcctctt tattatactt 17160

gactcgtgct caacaaaaat tctacatgaa aactataggc aatgcagtca agggatacta 17220

tagtaactgt gactcttaaa ggcaaccaca tatcaatagg ccctctttct agccgatcgt 17280

attcttgttg acttcattat accatattag aaaaaaattg aattccgacc ctttaagact 17340

cgtattcgga ttcaaataat tatctcagaa aaaaagtgca cgtagttgtt cttgattata 17400

gtcccgtcat tcaccaagtc tttgtttggt 17430

<210> 21
<211> 16122
<212> DNA
<213> Newcastle disease virus

<220>
<223> Mut HN (F277L)/M(G165W)/F(F117S)/F(F190L)/L(V757I)/L(F1551S)/L(R1
700L)-CD80

<400> 21
   accaaacaga gaatccgtga gatacgataa aaggcgaagg agcaatcgaa gtcgtacggg 60
   tagaaggtgt gaatctcgag tgcgagaccg aagcttaaac tcgagagagc cttctgccaa 120
   catgtcttcc gtatttgacg agtatgaaca gctcctcgcg gctcagactc gccctaatgg 180
   agctcacgga ggaggagaga aggggagcac cttaaaagtt gaagtcccgg tattcactct 240
   taacagtgac gacccagaag atagatggaa ctttgcagta ttctgtctta ggattgctgt 300
   tagcgaagat gccaacaaac cactcagaca aggtgctctc atatctctct tatgctccca 360
   ttctcaagtg atgaggaacc atgtcgccct tgcagggaaa cagaatgagg ccacactggc 420
   tgttcttgag atcgatggtt tcaccaacag cgtgcctcaa tttaacaaca ggagtggagt 480
   gtctgaggag agagcacaga gattcttgat gatagcaggg tccctccctc gggcatgcag 540
   caacggtacc ccgttcatca cagctggggt tgaagatgat gcaccagaag acatcactga 600
   tactctggaa agaatcatct ctatccaggc tcaagtatgg gtcacggtag caaaggccat 660
   gactgcatat gagacagcgg atgagtcgga aacaagaaga atcaataagt atatgcagca 720
   aggcagagtc caaaagaagt acatcctcca ccccgtatgt aggagcgcaa ttcaactcac 780
   gattagacaa tctctggcag tccgcatttt cttggttagc gagcttaaga gaggccgcaa 840
   cacggcaggt ggaagttcca cttattatag cttggtaggg gacatagact catacatcag 900
   gaacaccggg cttactgcat tcttcctgac actcaggtat ggaattaaca ccaagacatc 960
   agcccttgca ctcagcagcc tcgcaggcga tatccaaaaa atgaagcagc tcatgcgtct 1020
   atatcggatg aaaggagata atgcgccgta catgacactg ctcggtgaca gtgaccagat 1080
   gagctttgca ccggctgagt atgcacaact ctactctttt gcaatgggta tggcatccgt 1140
   cctagacaaa ggaactggca agtaccagtt cgccagagac tttatgagca catcattctg 1200
   gagacttgga gtagagtatg ctcgggctca gggaagcagc atcaatgagg atatggctgc 1260
   cgagctaaag ctgaccccag cggcaaggag gggcctggca gctgctgccc aacgagcgtc 1320
   cgaggagacc ggcagcatgg acattcccac tcaacaagct ggggtcctca cagggctcag 1380
   cgacggaggc cctcaagccc cacaaggtgg actgaacagg tcgcaagggc aaccggacgc 1440
   cggagatggg gagacccaat tttagatttt gatgagagca gtggcaaata gtatgagaga 1500
   agcgccaaac tctgtgcaga acaccactca gcaggagcct ccatctactc ctgggccatc 1560
   tcaagacaac gacaccgact ggggatactg accgacaaca ctcagcctgc ctccatcgaa 1620
   ttatctcgat tcttctgcct gtaacctaac ccctggtcca cagtctcaca cggccaaacc 1680
   cgcaaacgag cccccccctc tctccccccc cctccagcca cacggctcca accgcccaaa 1740
   acaacacggg cacaactcga ctcactcata atctacacag agccgaagat cttagaaaaa 1800
   aatacgggta gaagagagac attcagagat caggacaaat catcagggtc tccgctctcc 1860
   cctctaccca gcagaccaga gtgaagatgg ccaccttcac ggatgcagag atcgacgatc 1920
   tatttgaaac cagtggaact gtcattgaca gcataattac ggcccaaggc aacccagtag 1980
   agactgttgg aaggagcgca atcccacaag gcaaaactaa agcattgagt gcagcatggg 2040
   agaaacatgg aagcacccaa ccgccggccg gccaagacac ccctgatcga atggacagat 2100
   cagacaaaca accatcccca cccgaacagg caaccacaca caacagcccg ccagccacat 2160
   ccaccgacca gcccccaccc caggccgcag gcgaagccgg tgacacacag ctcaagactg 2220
   gagcaagcaa ctccctcctg tcgatgctcg acaagcttag caataaatcg tccaatgcta 2280
   aaaagggccc acggtcgagt ccccaggaag ggcaccacca acctccgacc cagcagcaag 2340
   ggagtcaaca gagccgcgga aacagtcagg agagatcgca aaaccaggtc aaggccgcct 2400
   ctggagacca gggcacagac gcgaacacag catatcatgg acaatggaag gagtcacaac 2460
   catcagctgg tgcaatccct catgctctcc ggtcagggca gagccaagcc aatactcctg 2520
   catctgtgga tcatgtccag ctacctgtcg actttgtgca ggcgatgatg tctatgatgg 2580
   aggcgatatc acagaaggta aataaagtcg actatcagct agaccttgtc tcaaagcaga 2640
   catcctccat ccccatgatg cgatctgaaa tccaacagct gaaaacatcc gttgcggtca 2700
   tggaagccaa cctgggcatg atgaagattc tggaccctgg ttgtgctaac gtttcatctt 2760
   taagtgacct acgggcggtc gcccgctctc acccagtttt agtttcaggc cccggagacc 2820
   catctcctta tgtgacacaa gggggtgaat tgatactcaa taaactctcg caaccagtgc 2880
   aacatccttc tgagttgatt aaacctgcca cgataagcgg gcctgatata ggagtggaga 2940
   aggacactgt ccgtgcattg atcacctcac gcccaatgca tccgagctct tcagctaagc 3000
   tcctgagtaa gctggatgca gccgggtcga tagaagaaat taggaaaatc aaacgccttg 3060
   cgctgaatgg ctgatcacta ctacaacccg caacgggttc ccgtttatct attgtcacaa 3120
   ggaacccgcc ccgaagctta agaaaaaata cgggtagaag tggctttcac ttttgaccct 3180
   aagcatctgc caccatgggc cacacacgga ggcagggaac atcaccatcc aagtgtccat 3240
   acctcaattt ctttcagctc ttggtgctgg ctggtctttc tcacttctgt tcaggtgtta 3300
   tccacgtgac caaggaagtg aaagaagtgg caacgctgtc ctgtggtcac aatgtttctg 3360
   ttgaagagct ggcacaaact cgcatctact ggcaaaagga gaagaaaatg gtgctgacta 3420
   tgatgtctgg ggacatgaat atatggcccg agtacaagaa ccggaccatc tttgatatca 3480
   ctaataacct ctccattgtg atcctggctc tgcgcccatc tgacgagggc acatacgagt 3540
   gtgttgttct gaagtatgaa aaagacgctt tcaagcggga acacctggct gaagtgacgt 3600
   tatcagtcaa agctgacttc cctacaccta gtatatctga ctttgaaatt ccaacttcta 3660
   atattagaag gataatttgc tcaacctctg gaggttttcc agagcctcac ctctcctggt 3720
   tggaaaatgg agaagaatta aatgccatca acacaacagt ttcccaagat cctgaaactg 3780
   agctctatgc tgttagcagc aaactggatt tcaatatgac aaccaaccac agcttcatgt 3840
   gtctcatcaa gtatggacat ttaagagtga atcagacctt caactggaat acaaccaagc 3900
   aagagcattt tcctgataac ctgctcccat cctgggccat taccttaatc tcagtaaatg 3960
   gaatttttgt gatatgctgc ctgacctact gctttgcccc aagatgcaga gagagaagga 4020
   ggaatgagag attgagaagg gaaagtgtac gccctgtata agctgaccga gccctcctct 4080
   ataaacccaa gattcaaggc tccaagcgat aaccttctcc cgcctcttcc atcccactga 4140
   atgatcgcgc agctgcaatt aatctagcaa cattaaagat taagaaaaaa tacgggtaga 4200
   atcggagtgc accgattgtg ccaagatgga ctcatctagg acaattgggt tatactttga 4260
   ttctgccctt ccttctagca acctgttagc attcccgatt gtcttacgag atgtaggaga 4320
   tggaaagaag caaattactc cgcaatatag gatccggcgt cttgactcgt ggacagacag 4380
   taaagaagac tcggtattca tcactaccta cggattcatc ttccaggttg ggaatgaaga 4440
   agtcactgtc ggcatgatca atgataatcc caagcgagag ttactctccg ctgcgatgct 4500
   ctgcctagga agcgtcccga atgttggaga tcctgttgag ctggcaaggg cctgcctcac 4560
   tatggtggta acatgcaaga agagtgcaac taatactgag agaatggtct tctcagtagt 4620
   gcaggcaccc cgggtgctgc aaagctgtag ggtcgtggca gacaaatact cgtcagtgaa 4680
   tgcagttaag cacgtgaaag caccagagaa gatcccttgg agtgaaaccc tagagtacaa 4740
   ggtgaatttt gtctctttga ctgtggtgcc gaagaaggat gtctacaaga tcccaaccgc 4800
   agtattgaag gtatccggct cgagcctgta taatcttgcg ctcaatgtca ctattgatgt 4860
   ggaggtagac ccaaagagcc cgttagtcaa gtccctttct aggtccgaca gtggatacta 4920
   tgctaatctc ttcttacata tcggacttat gtccactgta gataagaggg gaaagaaagt 4980
   gacatttgac cagctggaga ggaagataag gagacttgat ctatctgtcg ggctcagtga 5040
   tgtgctcgga ccttctgtgc ttgtgaaggc gagaggtgca cggactaggc tgctggcacc 5100
   tttcttctct aacagcggga cagcctgcta ccctatagca aatgcctctc ctcaggtagc 5160
   taagatactc tggagtcaga ccgcgtgcct gcggagtgta aaaatcatta ttcaagcggg 5220
   cacccaacgc gctgtcgcag tgactgctga ccatgaggtt acctctacta agatagagaa 5280
   gaggcatacc atcgctaaat acaatccctt caagaaatag gccgtatccc tgagactgca 5340
   attcacccgc cttcccaaaa caccatgaca ccagataatg atctgtcttg attacttaca 5400
   gttagtttcc ctgtctatca aattagaaaa aacacgggta gaagagttcg gatcccggcc 5460
   ggcgcaccaa aagcgcaaga tgggccccag atcttctacc aggatcccag tacctctaat 5520
   gctgaccata cggatcacgc tggcactgag ttatgtccgt ctgacaagtt ctcttgatgg 5580
   caggcctctt gcagccgcag ggatcgtggt aacaggggat aaagcagtta acatatacac 5640
   ctcatcccag acagggtcaa tcatagtcaa gttactccca aatatgccca aggacaaaga 5700
   ggcatgtgca aaagccccat tggaggctta caacaggaca ctgactactt tgcttacccc 5760
   ccttggtgat tctatccgca ggatacaaga gtctgtgact acatccggag gaaggagaca 5820
   gagacgctct ataggtgcca ttattggcag tgtagctcta ggggttgcaa cagctgcaca 5880
   gataacggca gcctcggctc tgatacaagc caaccagaat gctgctaaca tcctccggct 5940
   taaggagagc attgctgcaa ccaatgaagc tgtacacgag gtcactggcg gattgtcaca 6000
   gttagcagtg gcagttggaa agatgcagca atttgttaat gaccagctta ataacacagc 6060
   tcaagagttg gactgtataa aaattacaca gcaggttggt gtagaactca acttatactt 6120
   aactgaattg actacagtgt tcgggccaca aatcacttcc cctgccttaa ctcagttgac 6180
   tgttcaggct ctttacaatc tggctggtgg taatgtagat tacttgttga ctaagttagg 6240
   tgtagggaac aaccagctca gctcattgat tggtagcggc ttgatcaccg gtaaccctat 6300
   tttttacgac tcacagactc aactcttggg catacaggtg actttaccct cagtcgggaa 6360
   cctaaataat atgcgtgcca cctacttgga gaccttgtct gtaagcacaa caaagggatt 6420
   tgcctcagca cttgtcccaa aagtagtgac acaggtcggt tctgtgatag aagagcttga 6480
   cacctcatac tgtatagaag ctgatttgga tttatattgt acaagaatag tgacattccc 6540
   tatgtcccct ggtatttatt cctgtttgag cggcaataca tcggcttgca tgtattcaag 6600
   gactgaaggc gcacttacta caccatacat gactctcaaa ggctcagttg ttgccaattg 6660
   ccagatgaca acatgtagat gtgcagaccc cccgggtatc atatcacaaa attatggaga 6720
   ggctgtgtct ctaatagata agcactcatg caatgtcgta tccttagacg ggataacttt 6780
   gaggctcagt ggggaatttg atgcaactta tcaaaagaat atctcaatat tagattctca 6840
   agtactagtg acaggcaatc tcgatatctc aactgagctt gggaatgtca accactcaat 6900
   aagtaatgct ttggataagt tagaggaaag caacagcaaa ctagacaaag tcaatgtcaa 6960
   actgaccagc acatctgctc tcattaccta tattgtttta actgtcatat ctcttgttct 7020
   tggtatgctt agcctggttc tagcatgcta tctgatgtac aagcaaaaag cgcaacgaaa 7080
   gaccttgttg tggcttggaa ataataccct agatcagatg agagctacta caaaaatgtg 7140
   aatgcagatg agaggcagag gtatccccaa tagcaatctg tgtgtcaatt ctggcagcct 7200
   gttaatcaga agaattaaga aaaaactacc ggatgtaggt gaacaaaagg gaatatacgg 7260
   gtagaacggc ctgagaggcc acccctcaat cgggagccag gccccactac gtccgctcta 7320
   ccgcatcacc aacagcagtc ttcagtcatg gacagcgcag ttagccaagt tgcgctagag 7380
   aatgatagaa gagaagcgaa taatacatgg cgcttggttt tccggatcgc agccttactt 7440
   ttactggtaa taaccttagc cgtctctgca gtcgccctgg catatagtat ggaggctagc 7500
   acacctggcg accttgtaag cataccgact gcgatctcta gggcagagga aaggattaca 7560
   tctgcactcg gttccaatca agatgtagta gataggatat acaagcaagt ggcccttgag 7620
   tctccattgg cactgctaaa taccgaatct ataattatga atgcaataac gtctctctct 7680
   tatcaaatca atggagccac aaataatagc gggtgtgggg cacctgttca tgacccagat 7740
   tacatcgggg ggataggtca agaacttatt gtagatgata cgagtgacgt cacatcattt 7800
   tatccctctg cattccaaga acacctgaat tttatcccgg cgcccactac aggatcaggc 7860
   tgcactcgga taccctcatt cgacatgagt gctacccatt actgttacac tcacaatgtg 7920
   atattgtctg gctgcagaga tcactcacac tcacatcagt atttagcact tggtgtgctt 7980
   cggacatctg caacagggag ggtattcttt tctactctgc gttccatcaa cttggatgac 8040
   gcccaaaatc ggaagtcttg cagtgtgagt gcaactcctt taggttgtga tatgctgtgc 8100
   tctaaaatca cagagactga ggaagaggat tataaatcag ttatccccac atcgatggta 8160
   catggaaggt tagggcttga cggccaatac catgagaagg acctagacgt cacaacacta 8220
   tttagggact gggtggcaaa ttacccagga gtaggaggtg ggtcttttat taacaatcgc 8280
   gtatggttcc cggtctacgg agggctaaaa cccagctcgc ctagtgacac tgcacaagaa 8340
   gggagatacg taatatacaa gcgatacaat gacacatgcc cggatgagca agattaccag 8400
   attcggatgg ctaagtcatc gtataagcct aggcggtttg gtggaaaacg cgtacagcag 8460
   gccatcctat ccatcaaggt gtcaacatcc ttgggtgagg acccggtgtt gactgtaccg 8520
   cctaatacgg tcgcactcat gggggccgaa ggcagagttc tcacagtggg gacatctcat 8580
   ttcttatatc agcgagggtc atcatacttc tctcccgctt tgttataccc tatgacagtc 8640
   aacaacaaaa cagccactct tcataatcct tatacattca atgctttcac tcggccaggt 8700
   agtgtccctt gccaggcttc agcaagatgc cccaactcat gtgttaccgg agtctatact 8760
   gatccatatc ccttagtctt ccataggaac cacaccttgc gaggggtatt cggaacaatg 8820
   cttgatgacg aacaagcaag actcaaccct gtatctgcag tatttgataa catatcccgc 8880
   agtcgcataa ctcgggtaag ttcaagcagt accagggcag catacacgac atcaacgtgt 8940
   tttaaagttg tcaagaccaa taaaacctat tgcctcagca ttgcagaaat atccaatacc 9000
   ctcttcgggg aattcagaat tgtcccctta ctagttgaga ttctcaagga tggtggggtt 9060
   taagaagcta ggaatggtcg gttgagtcaa ccgtgagaag gcggggaaga tgatattgca 9120
   tcatctatct tctgtaacac caagaatcaa atcgaatacc agtgcgagct cgaatcctac 9180
   gctgccagtc agccataatc ggctggtgct aatgtggtta gcctgaatct tgtcgatagt 9240
   cacttgatta agaaaaaatg tggacggtag tgaaatataa ggcaaagcag ctcacagagg 9300
   acagcacggg taggacatgg cgggctccgg acccgaaagg gcagagcatc agattatcct 9360
   accagagtca catctgtcct caccattggt caagcataaa ttgctttatt actggaaatt 9420
   aactgggcta ccgcttcctg acgaatgtga cttcgaccat cttattatca gccgacaatg 9480
   gaagaaggta cttgaatcgg ccacccctga cattgagaga atgataaaac tagggcgggc 9540
   agtacaccag actctcaacc acaattccag gataaccgga gtactccatc cccggtgttt 9600
   agaggaactg gctagtattg aggttcctga ttcaaccaac aaatttcgga agatcgaaaa 9660
   aaagatccag attcacaaca caaggtatgg agaactgttc acaagattat gcacgcatgt 9720
   agaaaagaaa ctattggggt catcttggtc taacaatgtc ccacggtcag aggaattcaa 9780
   cagcatccgt acagatccgg cattttggtt tcactcaaaa tggtccacag ccaaatttgc 9840
   atggctccat ataaaacagg tccagaggca tctgattgta gcagcaagaa caaggtccgc 9900
   agtcaacaaa ttagtgacgc tgacccataa ggtaggccaa atctttgtta ctcctgagct 9960
   tgtcattgtg acacatacag atgagaacaa gttcacgtgt cttacccagg aacttgtgtt 10020
   gatgtatgca gatatgatgg agggcagaga tatggtcagc ataatatcat ccacggcggc 10080
   acatcttagg agcttatcag agaaaattga tgatattctg cggttagtag atgctctggc 10140
   aagagatttg ggcaatcaag tctacgatgt tgtagcacta atggagggat tcgcatacgg 10200
   cgctgttcag ctgcttgaac cgtcaggtac atttgcgggg gatttcttcg cattcaacct 10260
   gcaggagctc aaagatactc taaccggact cctccccaag gatatcgcag aatctgtgac 10320
   tcacgcaatc gcaaccatat tctctggctt agaacaaaat caagcagctg agatgttgtg 10380
   cctgttgcgt ctgtggggtc acccactact cgagtcccgt attgcagcaa aagcagttag 10440
   gagtcaaatg tgcgcaccaa aaatggtaga ctttgatatg atcctccagg tattatcttt 10500
   ctttaaagga acaatcatca atggatatag aaagaagaat gcaggcgtgt ggccacgtgt 10560
   caagatagat acgatatacg ggaaggtcat agggcagcta cacgcagatt ctgcagagat 10620
   ttcacatgat gtcatgttga gggaatacaa gagtttatct gcacttgaat tcgagccatg 10680
   tatagagtat gaccctgtca ccaatctgag catgttttta aaagacaagg caatcgcaca 10740
   cccgaaagac aactggctcg cttcgtttag gcgaaacctt ctctctgagg accagaagaa 10800
   acatgtaaga gaggcaacct caactaaccg cctcttgata gagttcttag agtcaaatga 10860
   ttttgatcca tataaagaga tggaatatct gacgaccctt gagtacctaa gagatgataa 10920
   tgtggcagta tcatactcac tcaaagaaaa agaggtgaaa gttaatgggc ggattttcgc 10980
   aaagctaaca aatagattaa ggaattgtca ggtaatggca gaagggatcc tagctgacca 11040
   gattgcacct ttcttccagg gaaatggggt cattcaggat agcatatctt tgactaaaag 11100
   tatgctagcg atgagtcaac tgtctttcaa cagcaacaag aaacgtatta ctgactgcaa 11160
   agaaagagta tcctcaaacc gcaatcatga tccgaagagc aagaatcgtc ggagagttgc 11220
   cacttttgta actaccgacc tgcaaaagta ttgtctcaat tggagatatc agacagtcaa 11280
   gctgttcgct catgccatca atcagctgat gggcttacct cacttcttcg agtggattca 11340
   tcttagacta atggatacta cgatgtttgt aggggaccct ttcaatcctc caagtgaccc 11400
   tacggactgt gatctatcaa gagtcccaaa tgatgacata tatattgtca gtgctagggg 11460
   aggcattgag ggattatgcc agaagctatg gacaatgatc tcaattgctg caatccagct 11520
   tgctgcagca agatcacatt gtcgcgttgc ctgtatggta caaggcgaca atcaagtaat 11580
   agctataacg agagaggtaa gatcagatga ctccccagag atggtgttaa cacaattgca 11640
   tcaagccagt gataatttct tcaaggaatt gattcatgtc aatcatttga tcggccataa 11700
   tttgaaggat cgtgaaacca tcaggtcaga cacgttcttc atatacagca aacgaatatt 11760
   caaagatgga gcaatactca gtcaggtcct caaaaactca tctaaattag tgctaatatc 11820
   aggcgacctt agtgaaaaca ctgtaatgtc ttgtgccaac attgcatcta ctgtagcacg 11880
   gttatgcgag aacgggcttc ctaaggattt ctgttattac ttaaactact taatgagttg 11940
   cgtgcagaca tacttcgatt ccgaattttc catcaccaac aactcgcaac tcgattctaa 12000
   ccagtcgtgg atagaggaca tttcttttgt gcactcatat gtcctgaccc ctgctcaact 12060
   ggggggactg agtaaccttc aatactcaag gctctacaca aggaacatcg gcgacccggg 12120
   aaccactgct ttcgcagaga tcaagagatt agaggcagtg gggttactga gtcctagcat 12180
   tatgactaac atcttaacta ggccgcctgg aaatggagat tgggccagtc tgtgcaacga 12240
   tccatactcc tttaattttg agactgtcgc aagcccaaat attgtcctta agaaacatac 12300
   acaaagagtc ctatttgaaa cttgctcgaa tcccttatta tctggagtgc acacagagga 12360
   taatgaggca gaagagaagg cattggctga attcttgctc aatcaagaaa tgattcatcc 12420
   acgtgtcgcg catgctatca tggaagcaag ctctgtaggt aggagaaagc aaattcaggg 12480
   gcttgttgac acaacaaaca ccgtgattaa gattgcactg actaggaggc cactcggcat 12540
   caagaggctg atgcggatag tcaattactc gagcatgcat gcaatgctat ttagagatga 12600
   tgttttctcg tctaatagat ccaaccatcc cttagtctct tctgatatgt gttctcagac 12660
   actggcagac tatgcacgga acagaagctg gtcacctttg acagggggta gaaaaatact 12720
   gggtgtatct aatcctgata ccatagaact tgtagagggt gagatcctta gtgtcagtgg 12780
   agggtgcact agatgtgaca gtggggatga acagtttact tggttccatc ttccaagcaa 12840
   tatagagcta aacgatgaca ccagcaagaa tcctccaatg agagtgccat atctcgggtc 12900
   aaagactcaa gagaggagag ccgcctcgct tgcgaaaata gctcatatgt caccacacgt 12960
   gaaggcggct ctaagggcgt catctgtgtt aatctgggct tatggggaca acgaaataaa 13020
   ctggactgct gcccttaaga ttgcaaggtc tcggtgcaac ataagctcag agtatcttcg 13080
   actattgtca cccttgccta cagctgggaa tctccaacat agattggacg acggcataac 13140
   tcagatgaca ttcacccctg catctctata cagggtgtca ccttacattc atatatctaa 13200
   tgattctcaa aggctattta ctgaagaagg agtcaaagag gggaatgtgg tttatcagca 13260
   aatcatgctc ttgggtttgt ccttaattga gtcactcttc ccaatgacaa caaccaagac 13320
   atatgatgaa atcacattgc acctccacag taaatttagc tgctgtatca gggaagcacc 13380
   tgttgcagtt cctttcgagc tactcggggt ggcaccggaa ctaagggcag taacctcaaa 13440
   taagtttatg tatgatccta gccctgtatc agagggagac tttgcgagac ttgacttagc 13500
   tatctttaag agttatgaac ttaatttaga gtcatattcc acaatagagc taatgaacgt 13560
   tctttcaata tctagtggga agttgattgg ccagtctgtg gtttcttatg atgaagatac 13620
   ctccataaag aatgacgcta taatagtgta tgacaacaca cggaattgga tcagcgaagc 13680
   tcagaattca gatgtggtcc gcctattcga gtatgcggca ctcgaagtgc tcctcgactg 13740
   ttcttatcaa ctctactatc tgagagtaag aggcctaaac aatatcgtcc tgtacatgag 13800
   tgatttatac aagaatatgc caggaattct actctctaat attgcagcca caatatctca 13860
   ccctgtcatc cattcaaggt tgaatgcagt aggtctggtc aaccatgacg ggtcacacca 13920
   acttgcagac acagatttca ttgaaatgtc tgcaaagctg ctagtctctt gcactcgacg 13980
   cgtggtctca ggtttacatg cagggaataa gtatgacctg ctgtttccat ctgtcttgga 14040
   tgataaccta agtgagaaga tgcttcagtt gatatcccaa ttatgctgtc tgtatacggt 14100
   gctctttgct acaacaagag aaatcccgaa aataagaggc ttatctgcag aagagaaatg 14160
   ttcagtactt actgagtacc tactgtcaga tgctgtgaaa ccgttacttg ggtccgagca 14220
   agtgagctct atcatgtctc ccaacatagt tacgttccca gccaatctgt attacatgtc 14280
   taggaagagc cttaatttga tcagggagag agaggacagg gatactatct tggcattgtt 14340
   gttccctcaa gaaccgctgc tcgagtttcc tctggtacga gatattggtg ctcgtgtaaa 14400
   agatccattt accctacaac ctgcggcgtt tttacaagag ttagatttga gtgctccggc 14460
   aaggtatgac gcattcacaa tcagtcaggc gcattctgaa cacatattgc caaacccaga 14520
   ggaagatcac ttagtacgat acttgttcag aggaataggg actgcgtcct cctcttggta 14580
   taaggcatcc catcttcttt ctgtacccga ggtcagatgt gcaaggcatg ggaactcctt 14640
   atatttagca gaaggaagtg gagccatcat gagtcttctc gaattgcata taccacacga 14700
   aactatctat tacaatacac ttttctcgaa cgagatgaac cccccacagc gacatttcgg 14760
   accaactcca acacagtttc tgaattcggt tgtttttagg aatttacagg cggaagtacc 14820
   atgcaaggat ggatttgtcc aggagttccg tccgttatgg agagagaata cagaagaaag 14880
   cgatctgacc tcagataaag cagtgggata tatcacatct gtagtgccgt acaggtctgt 14940
   atcattgctg cattgtgaca ttgaaatccc tccaggatct aatcaaagct tactagatca 15000
   gctggctacc aatctgtctc tgattgccat gcattctgtg aaggagggcg gggtcgtgat 15060
   tatcaaagta ctgtatgcaa tgggatatta cttccatcta ctcatgaact tgtttactcc 15120
   atgttccacg aaaggatata ttctctctaa tggctatgcc tgtagagggg atatggagtg 15180
   ttacctgata tttgtcatgg gctacctagg cgggcctaca tttgtacatg aggtggtgag 15240
   gatggcaaaa acgctagtac agcggcatgg cacacttttg tctaagtcag atgagattac 15300
   actgactagg ttatttactt cacagcaaca gcgtgtaaca gacatcctat ccagcccttt 15360
   accgagacta atgaagtact tgagagagaa tattgatact gcactgattg aagccggggg 15420
   acagcctgtc cgtccattct gtgcagagag tttagtgagc acactaacag acatgactca 15480
   gacaacccag atcatcgcca gccacattga tacagtcatt cgatctgtga tctatatgga 15540
   agcagagggt gatcttgctg acacagtatt cttatttacc ccttacaacc tctctatgga 15600
   cgggaaaaag agaacatcac ttaaacagtg cacaagacag atcttagagg tcacaatact 15660
   gggtctcaga gtcaaaaatc tcaataaagt aggtgatgta atcagcctag tactcagagg 15720
   tatgatttct ctggaggacc ttctcccact gagaacctac ttgaagtgta gtacctgccc 15780
   taagtatttg aaggctgtcc taggtattac caaactcaaa gaaatgttca cagacgcctc 15840
   tttattatac ttgactcgtg ctcaacaaaa attctacatg aaaactatag gcaatgcagt 15900
   caagggatac tatagtaact gtgactctta aaggcaacca catatcaata ggccctcttt 15960
   ctagccgatc gtattcttgt tgacttcatt ataccatatt agaaaaaaat tgaattccga 16020
   ccctttaaga ctcgtattcg gattcaaata attatctcag aaaaaaagtg cacgtagttg 16080
   ttcttgatta tagtcccgtc attcaccaag tctttgtttg gt 16122

<210> 22
<211> 16824
<212> DNA
<213> Newcastle disease virus

<220>
<223> Mut HN (F277L)/M(G165W)/F(F117S)/F(F190L)/L(V757I)/L(F1551S)/L(R1
      700L)-(ns)hIL-12

<400> 22
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat tttagatttt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gcccccaccc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940
aggacactgt ccgtgcattg atcacctcac gcccaatgca tccgagctct tcagctaagc 3000
tcctgagtaa gctggatgca gccgggtcga tagaagaaat taggaaaatc aaacgccttg 3060
cgctgaatgg ctgatcacta ctacaacccg caacgggttc ccgtttatct attgtcacaa 3120
ggaacccgcc ccgaagctta agaaaaaata cgggtagaag cagtttgaga gctccgttcg 3180
ccaccatgag aaacctcccc gtggccactc cagacccagg aatgttccca tgccttcacc 3240
actcccaaaa cctgctgagg gccgtcagca acatgctcca gaaggccaga caaactctag 3300
aattttaccc ttgcacttct gaagagattg atcatgaaga tatcacaaaa gataaaacca 3360
gcacagtgga ggcctgttta ccattggaat taaccaagaa tgagagttgc ctaaattcca 3420
gagagacctc tttcataact aatgggagtt gcctggcctc cagaaagacc tcttttatga 3480
tggccctgtg ccttagtagt atttatgaag acttgaagat gtaccaggtg gagttcaaga 3540
ccatgaatgc aaagctgctg atggatccta agaggcagat ctttctagat caaaacatgc 3600
tggcagttat tgatgagctg atgcaggccc tgaatttcaa cagtgagact gtgccacaaa 3660
aatcctccct tgaagaaccg gatttttata aaactaaaat caagctctgc atacttcttc 3720
atgctttcag aattcgggca gtgactattg atagagtgat gagctatctg aatgcttccg 3780
gctccggcga gggccgcggc tccctgctga cctgcggcga cgtggaggag aaccccggcc 3840
ccatatggga actgaagaaa gatgtttatg tcgtagaatt ggattggtat ccggatgccc 3900
ctggagaaat ggtggtcctc acctgtgaca cccctgaaga agatggtatc acctggacct 3960
tggaccagag cagtgaggtc ttaggctctg gcaaaaccct gaccatccaa gtcaaagagt 4020
ttggagatgc tggccagtac acctgtcaca aaggaggcga ggttctaagc cattcgctcc 4080
tgctgcttca caaaaaggaa gatggaattt ggtccactga tattttaaag gaccagaaag 4140
aacccaaaaa taagaccttt ctaagatgcg aggccaagaa ttattctgga cgtttcacct 4200
gctggtggct gacgacaatc agtactgatt tgacattcag tgtcaaaagc agcagaggct 4260
cttctgaccc ccaaggggtg acgtgcggag ctgctacact ctctgcagag agagtcagag 4320
gggacaacaa ggagtatgag tactcagtgg agtgccagga ggacagtgcc tgcccagctg 4380
ctgaggagag tctgcccatt gaggtcatgg tggatgccgt tcacaagctc aagtatgaaa 4440
actacaccag cagcttcttc atcagggaca tcatcaaacc tgacccaccc aagaacttgc 4500
agctgaagcc attaaagaat tctcggcagg tggaggtcag ctgggagtac cctgacacct 4560
ggagtactcc acattcctac ttctccctga cattctgcgt tcaggtccag ggcaagagca 4620
agagagaaaa gaaagataga gtcttcacgg acaagacctc agccacggtc atctgccgca 4680
aaaatgccag cattagcgtg cgggcccagg accgctacta tagctcatct tggagcgaat 4740
gggcatctgt gccctgcagt taagctgacc gagccctcct ctataaaccc aagattcaag 4800

gctccaagcg ataaccttct cccgcctctt ccatcccact gaatgatcgc gcagctgcaa 4860

ttaatctagc aacattaaag attaagaaaa aatacgggta gaatcggagt gcaccgattg 4920

tgccaagatg gactcatcta ggacaattgg gttatacttt gattctgccc ttccttctag 4980

caacctgtta gcattcccga ttgtcttacg agatgtagga gatggaaaga agcaaattac 5040

tccgcaatat aggatccggc gtcttgactc gtggacagac agtaaagaag actcggtatt 5100

catcactacc tacggattca tcttccaggt tgggaatgaa gaagtcactg tcggcatgat 5160

caatgataat cccaagcgag agttactctc cgctgcgatg ctctgcctag gaagcgtccc 5220

gaatgttgga gatcctgttg agctggcaag ggcctgcctc actatggtgg taacatgcaa 5280

gaagagtgca actaatactg agagaatggt cttctcagta gtgcaggcac cccgggtgct 5340

gcaaagctgt agggtcgtgg cagacaaata ctcgtcagtg aatgcagtta agcacgtgaa 5400

agcaccagag aagatccctt ggagtgaaac cctagagtac aaggtgaatt ttgtctcttt 5460

gactgtggtg ccgaagaagg atgtctacaa gatcccaacc gcagtattga aggtatccgg 5520

ctcgagcctg tataatcttg cgctcaatgt cactattgat gtggaggtag acccaaagag 5580

cccgttagtc aagtcccttt ctaggtccga cagtggatac tatgctaatc tcttcttaca 5640

tatcggactt atgtccactg tagataagag gggaaagaaa gtgacatttg accagctgga 5700

gaggaagata aggagacttg atctatctgt cgggctcagt gatgtgctcg gaccttctgt 5760

gcttgtgaag gcgagaggtg cacggactag gctgctggca cctttcttct ctaacagcgg 5820

gacagcctgc taccctatag caaatgcctc tcctcaggta gctaagatac tctggagtca 5880

gaccgcgtgc ctgcggagtg taaaaatcat tattcaagcg ggcacccaac gcgctgtcgc 5940

agtgactgct gaccatgagg ttacctctac taagatagag aagaggcata ccatcgctaa 6000

atacaatccc ttcaagaaat aggccgtatc cctgagactg caattcaccc gccttcccaa 6060

aacaccatga caccagataa tgatctgtct tgattactta cagttagttt ccctgtctat 6120

caaattagaa aaaacacggg tagaagagtt cggatcccgg ccggcgcacc aaaagcgcaa 6180

gatgggcccc agatcttcta ccaggatccc agtacctcta atgctgacca tacggatcac 6240

gctggcactg agttatgtcc gtctgacaag ttctcttgat ggcaggcctc ttgcagccgc 6300

agggatcgtg gtaacagggg ataaagcagt taacatatac acctcatccc agacagggtc 6360

aatcatagtc aagttactcc caaatatgcc caaggacaaa gaggcatgtg caaaagcccc 6420

attggaggct tacaacagga cactgactac tttgcttacc ccccttggtg attctatccg 6480

caggatacaa gagtctgtga ctacatccgg aggaaggaga cagagacgct ctataggtgc 6540

cattattggc agtgtagctc taggggttgc aacagctgca cagataacgg cagcctcggc 6600

tctgatacaa gccaaccaga atgctgctaa catcctccgg cttaaggaga gcattgctgc 6660

aaccaatgaa gctgtacacg aggtcactgg cggattgtca cagttagcag tggcagttgg 6720

aaagatgcag caatttgtta atgaccagct taataacaca gctcaagagt tggactgtat 6780

aaaaattaca cagcaggttg gtgtagaact caacttatac ttaactgaat tgactacagt 6840

gttcgggcca caaatcactt cccctgcctt aactcagttg actgttcagg ctctttacaa 6900

tctggctggt ggtaatgtag attacttgtt gactaagtta ggtgtaggga acaaccagct 6960

cagctcattg attggtagcg gcttgatcac cggtaaccct attttttacg actcacagac 7020

tcaactcttg ggcatacagg tgactttacc ctcagtcggg aacctaaata atatgcgtgc 7080

cacctacttg gagaccttgt ctgtaagcac aacaaaggga tttgcctcag cacttgtccc 7140

aaaagtagtg acacaggtcg gttctgtgat agaagagctt gacacctcat actgtataga 7200

agctgatttg gatttatatt gtacaagaat agtgacattc cctatgtccc ctggtattta 7260

ttcctgtttg agcggcaata catcggcttg catgtattca aggactgaag gcgcacttac 7320

tacaccatac atgactctca aaggctcagt tgttgccaat tgccagatga caacatgtag 7380

atgtgcagac cccccgggta tcatatcaca aaattatgga gaggctgtgt ctctaataga 7440

taagcactca tgcaatgtcg tatccttaga cgggataact ttgaggctca gtggggaatt 7500

tgatgcaact tatcaaaaga atatctcaat attagattct caagtactag tgacaggcaa 7560

tctcgatatc tcaactgagc ttgggaatgt caaccactca ataagtaatg ctttggataa 7620

gttagaggaa agcaacagca aactagacaa agtcaatgtc aaactgacca gcacatctgc 7680

tctcattacc tatattgttt taactgtcat atctcttgtt cttggtatgc ttagcctggt 7740

tctagcatgc tatctgatgt acaagcaaaa agcgcaacga aagaccttgt tgtggcttgg 7800

aaataatacc ctagatcaga tgagagctac tacaaaaatg tgaatgcaga tgagaggcag 7860

aggtatcccc aatagcaatc tgtgtgtcaa ttctggcagc ctgttaatca gaagaattaa 7920

gaaaaaacta ccggatgtag gtgaacaaaa gggaatatac gggtagaacg gcctgagagg 7980

ccacccctca atcgggagcc aggccccact acgtccgctc taccgcatca ccaacagcag 8040

tcttcagtca tggacagcgc agttagccaa gttgcgctag agaatgatag aagagaagcg 8100

aataatacat ggcgcttggt tttccggatc gcagccttac ttttactggt aataacctta 8160

gccgtctctg cagtcgccct ggcatatagt atggaggcta gcacacctgg cgaccttgta 8220

agcataccga ctgcgatctc tagggcagag gaaaggatta catctgcact cggttccaat 8280

caagatgtag tagataggat atacaagcaa gtggcccttg agtctccatt ggcactgcta 8340

aataccgaat ctataattat gaatgcaata acgtctctct cttatcaaat caatggagcc 8400

acaaataata gcgggtgtgg ggcacctgtt catgacccag attacatcgg ggggataggt 8460

caagaactta ttgtagatga tacgagtgac gtcacatcat tttatccctc tgcattccaa 8520

gaacacctga attttatccc ggcgcccact acaggatcag gctgcactcg gataccctca 8580

ttcgacatga gtgctaccca ttactgttac actcacaatg tgatattgtc tggctgcaga 8640

gatcactcac actcacatca gtatttagca cttggtgtgc ttcggacatc tgcaacaggg 8700

agggtattct tttctactct gcgttccatc aacttggatg acgcccaaaa tcggaagtct 8760

tgcagtgtga gtgcaactcc tttaggttgt gatatgctgt gctctaaaat cacagagact 8820

gaggaagagg attataaatc agttatcccc acatcgatgg tacatggaag gttagggctt 8880

gacggccaat accatgagaa ggacctagac gtcacaacac tatttaggga ctgggtggca 8940

aattacccag gagtaggagg tgggtctttt attaacaatc gcgtatggtt cccggtctac 9000

ggagggctaa aacccagctc gcctagtgac actgcacaag aagggagata cgtaatatac 9060

aagcgataca atgacacatg cccggatgag caagattacc agattcggat ggctaagtca 9120

tcgtataagc ctaggcggtt tggtggaaaa cgcgtacagc aggccatcct atccatcaag 9180

gtgtcaacat ccttgggtga ggacccggtg ttgactgtac cgcctaatac ggtcgcactc 9240

atgggggccg aaggcagagt tctcacagtg gggacatctc atttcttata tcagcgaggg 9300

tcatcatact tctctcccgc tttgttatac cctatgacag tcaacaacaa aacagccact 9360

cttcataatc cttatacatt caatgctttc actcggccag gtagtgtccc ttgccaggct 9420

tcagcaagat gccccaactc atgtgttacc ggagtctata ctgatccata tcccttagtc 9480

ttccatagga accacacctt gcgaggggta ttcggaacaa tgcttgatga cgaacaagca 9540

agactcaacc ctgtatctgc agtatttgat aacatatccc gcagtcgcat aactcgggta 9600

agttcaagca gtaccagggc agcatacacg acatcaacgt gttttaaagt tgtcaagacc 9660

aataaaacct attgcctcag cattgcagaa atatccaata ccctcttcgg ggaattcaga 9720

attgtcccct tactagttga gattctcaag gatggtgggg tttaagaagc taggaatggt 9780

cggttgagtc aaccgtgaga aggcggggaa gatgatattg catcatctat cttctgtaac 9840

accaagaatc aaatcgaata ccagtgcgag ctcgaatcct acgctgccag tcagccataa 9900

tcggctggtg ctaatgtggt tagcctgaat cttgtcgata gtcacttgat taagaaaaaa 9960

tgtggacggt agtgaaatat aaggcaaagc agctcacaga ggacagcacg ggtaggacat 10020

ggcgggctcc ggacccgaaa gggcagagca tcagattatc ctaccagagt cacatctgtc 10080

ctcaccattg gtcaagcata aattgcttta ttactggaaa ttaactgggc taccgcttcc 10140

tgacgaatgt gacttcgacc atcttattat cagccgacaa tggaagaagg tacttgaatc 10200

ggccacccct gacattgaga gaatgataaa actagggcgg gcagtacacc agactctcaa 10260

ccacaattcc aggataaccg gagtactcca tccccggtgt ttagaggaac tggctagtat 10320

tgaggttcct gattcaacca acaaatttcg gaagatcgaa aaaaagatcc agattcacaa 10380

cacaaggtat ggagaactgt tcacaagatt atgcacgcat gtagaaaaga aactattggg 10440

gtcatcttgg tctaacaatg tcccacggtc agaggaattc aacagcatcc gtacagatcc 10500

ggcattttgg tttcactcaa aatggtccac agccaaattt gcatggctcc atataaaaca 10560

ggtccagagg catctgattg tagcagcaag aacaaggtcc gcagtcaaca aattagtgac 10620

gctgacccat aaggtaggcc aaatctttgt tactcctgag cttgtcattg tgacacatac 10680

agatgagaac aagttcacgt gtcttaccca ggaacttgtg ttgatgtatg cagatatgat 10740

ggagggcaga gatatggtca gcataatatc atccacggcg gcacatctta ggagcttatc 10800

agagaaaatt gatgatattc tgcggttagt agatgctctg gcaagagatt tgggcaatca 10860

agtctacgat gttgtagcac taatggaggg attcgcatac ggcgctgttc agctgcttga 10920

accgtcaggt acatttgcgg gggatttctt cgcattcaac ctgcaggagc tcaaagatac 10980

tctaaccgga ctcctcccca aggatatcgc agaatctgtg actcacgcaa tcgcaaccat 11040

attctctggc ttagaacaaa atcaagcagc tgagatgttg tgcctgttgc gtctgtgggg 11100

tcacccacta ctcgagtccc gtattgcagc aaaagcagtt aggagtcaaa tgtgcgcacc 11160

aaaaatggta gactttgata tgatcctcca ggtattatct ttctttaaag gaacaatcat 11220

caatggatat agaaagaaga atgcaggcgt gtggccacgt gtcaagatag atacgatata 11280

cgggaaggtc atagggcagc tacacgcaga ttctgcagag atttcacatg atgtcatgtt 11340

gagggaatac aagagtttat ctgcacttga attcgagcca tgtatagagt atgaccctgt 11400

caccaatctg agcatgtttt taaaagacaa ggcaatcgca cacccgaaag acaactggct 11460

cgcttcgttt aggcgaaacc ttctctctga ggaccagaag aaacatgtaa gagaggcaac 11520

ctcaactaac cgcctcttga tagagttctt agagtcaaat gattttgatc catataaaga 11580

gatggaatat ctgacgaccc ttgagtacct aagagatgat aatgtggcag tatcatactc 11640

actcaaagaa aaagaggtga aagttaatgg gcggattttc gcaaagctaa caaatagatt 11700

aaggaattgt caggtaatgg cagaagggat cctagctgac cagattgcac ctttcttcca 11760

gggaaatggg gtcattcagg atagcatatc tttgactaaa agtatgctag cgatgagtca 11820

actgtctttc aacagcaaca agaaacgtat tactgactgc aaagaaagag tatcctcaaa 11880

ccgcaatcat gatccgaaga gcaagaatcg tcggagagtt gccacttttg taactaccga 11940

cctgcaaaag tattgtctca attggagata tcagacagtc aagctgttcg ctcatgccat 12000

caatcagctg atgggcttac ctcacttctt cgagtggatt catcttagac taatggatac 12060

tacgatgttt gtaggggacc ctttcaatcc tccaagtgac cctacggact gtgatctatc 12120

aagagtccca aatgatgaca tatatattgt cagtgctagg ggaggcattg agggattatg 12180

ccagaagcta tggacaatga tctcaattgc tgcaatccag cttgctgcag caagatcaca 12240

ttgtcgcgtt gcctgtatgg tacaaggcga caatcaagta atagctataa cgagagaggt 12300

aagatcagat gactccccag agatggtgtt aacacaattg catcaagcca gtgataattt 12360

cttcaaggaa ttgattcatg tcaatcattt gatcggccat aatttgaagg atcgtgaaac 12420

catcaggtca gacacgttct tcatatacag caaacgaata ttcaaagatg gagcaatact 12480

cagtcaggtc ctcaaaaact catctaaatt agtgctaata tcaggcgacc ttagtgaaaa 12540

cactgtaatg tcttgtgcca acattgcatc tactgtagca cggttatgcg agaacgggct 12600

tcctaaggat ttctgttatt acttaaacta cttaatgagt tgcgtgcaga catacttcga 12660

ttccgaattt tccatcacca acaactcgca actcgattct aaccagtcgt ggatagagga 12720

catttctttt gtgcactcat atgtcctgac ccctgctcaa ctggggggac tgagtaacct 12780

tcaatactca aggctctaca caaggaacat cggcgacccg ggaaccactg ctttcgcaga 12840

gatcaagaga ttagaggcag tggggttact gagtcctagc attatgacta acatcttaac 12900

taggccgcct ggaaatggag attgggccag tctgtgcaac gatccatact cctttaattt 12960

tgagactgtc gcaagcccaa atattgtcct taagaaacat acacaaagag tcctatttga 13020

aacttgctcg aatcccttat tatctggagt gcacacagag gataatgagg cagaagagaa 13080

ggcattggct gaattcttgc tcaatcaaga aatgattcat ccacgtgtcg cgcatgctat 13140

catggaagca agctctgtag gtaggagaaa gcaaattcag gggcttgttg acacaacaaa 13200

caccgtgatt aagattgcac tgactaggag gccactcggc atcaagaggc tgatgcggat 13260

agtcaattac tcgagcatgc atgcaatgct atttagagat gatgttttct cgtctaatag 13320

atccaaccat cccttagtct cttctgatat gtgttctcag acactggcag actatgcacg 13380

gaacagaagc tggtcacctt tgacaggggg tagaaaaata ctgggtgtat ctaatcctga 13440

taccatagaa cttgtagagg gtgagatcct tagtgtcagt ggagggtgca ctagatgtga 13500

cagtggggat gaacagttta cttggttcca tcttccaagc aatatagagc taaacgatga 13560

caccagcaag aatcctccaa tgagagtgcc atatctcggg tcaaagactc aagagaggag 13620

agccgcctcg cttgcgaaaa tagctcatat gtcaccacac gtgaaggcgg ctctaagggc 13680

gtcatctgtg ttaatctggg cttatgggga caacgaaata aactggactg ctgcccttaa 13740

gattgcaagg tctcggtgca acataagctc agagtatctt cgactattgt cacccttgcc 13800

tacagctggg aatctccaac atagattgga cgacggcata actcagatga cattcacccc 13860

tgcatctcta tacagggtgt caccttacat tcatatatct aatgattctc aaaggctatt 13920

tactgaagaa ggagtcaaag aggggaatgt ggtttatcag caaatcatgc tcttgggttt 13980

gtccttaatt gagtcactct tcccaatgac aacaaccaag acatatgatg aaatcacatt 14040

gcacctccac agtaaattta gctgctgtat cagggaagca cctgttgcag ttcctttcga 14100

gctactcggg gtggcaccgg aactaaggcc agtaacctca aataagttta tgtatgatcc 14160

tagccctgta tcagagggag actttgcgag acttgactta gctatcttta agagttatga 14220

acttaattta gagtcatatt ccacaataga gctaatgaac gttctttcaa tatctagtgg 14280

gaagttgatt ggccagtctg tggtttctta tgatgaagat acctccataa agaatgacgc 14340

tataatagtg tatgacaaca cacggaattg gatcagcgaa gctcagaatt cagatgtggt 14400

ccgcctattc gagtatgcgg cactcgaagt gctcctcgac tgttcttatc aactctacta 14460

tctgagagta agaggcctaa acaatatcgt cctgtacatg agtgatttat acaagaatat 14520

gccaggaatt ctactctcta atattgcagc cacaatatct caccctgtca tccattcaag 14580

gttgaatgca gtaggtctgg tcaaccatga cgggtcacac caacttgcag acacagattt 14640

cattgaaatg tctgcaaagc tgctagtctc ttgcactcga cgcgtggtct caggtttaca 14700

tgcagggaat aagtatgacc tgctgtttcc atctgtcttg gatgataacc taagtgagaa 14760

gatgcttcag ttgatatccc aattatgctg tctgtatacg gtgctctttg ctacaacaag 14820

agaaatcccg aaaataagag gcttatctgc agaagagaaa tgttcagtac ttactgagta 14880

cctactgtca gatgctgtga aaccgttact tgggtccgag caagtgagct ctatcatgtc 14940

tcccaacata gttacgttcc cagccaatct gtattacatg tctaggaaga gccttaattt 15000

gatcagggag agagaggaca gggatactat cttggcattg ttgttccctc aagaaccgct 15060

gctcgagttt cctctggtac gagatattgg tgctcgtgta aaagatccat ttaccctaca 15120

acctgcggcg tttttacaag agttagattt gagtgctccg gcaaggtatg acgcattcac 15180

aatcagtcag gcgcattctg aacacatatt gccaaaccca gaggaagatc acttagtacg 15240

atacttgttc agaggaatag ggactgcgtc ctcctcttgg tataaggcat cccatcttct 15300

ttctgtaccc gaggtcagat gtgcaaggca tgggaactcc ttatatttag cagaaggaag 15360

tggagccatc atgagtcttc tcgaattgca tataccacac gaaactatct attacaatac 15420

acttttctcg aacgagatga accccccaca gcgacatttc ggaccaactc caacacagtt 15480

tctgaattcg gttgttttta ggaatttaca ggcggaagta ccatgcaagg atggatttgt 15540

ccaggagttc cgtccgttat ggagagagaa tacagaagaa agcgatctga cctcagataa 15600

agcagtggga tatatcacat ctgtagtgcc gtacaggtct gtatcattgc tgcattgtga 15660

cattgaaatc cctccaggat ctaatcaaag cttactagat cagctggcta ccaatctgtc 15720

tctgattgcc atgcattctg tgaaggaggg cggggtcgtg attatcaaag tactgtatgc 15780

aatgggatat tacttccatc tactcatgaa cttgtttact ccatgttcca cgaaaggata 15840

tattctctct aatggctatg cctgtagagg ggatatggag tgttacctga tatttgtcat 15900

gggctaccta ggcgggccta catttgtaca tgaggtggtg aggatggcaa aaacgctagt 15960

acagcggcat ggcacacttt tgtctaagtc agatgagatt acactgacta ggttatttac 16020

ttcacagcaa cagcgtgtaa cagacatcct atccagccct ttaccgagac taatgaagta 16080

cttgagagag aatattgata ctgcactgat tgaagccggg ggacagcctg tccgtccatt 16140

ctgtgcagag agtttagtga gcacactaac agacatgact cagacaaccc agatcatcgc 16200

cagccacatt gatacagtca ttcgatctgt gatctatatg gaagcagagg gtgatcttgc 16260

tgacacagta ttcttattta ccccttacaa cctctctatg gacgggaaaa agagaacatc 16320

acttaaacag tgcacaagac agatcttaga ggtcacaata ctgggtctca gagtcaaaaa 16380

tctcaataaa gtaggtgatg taatcagcct agtactcaga ggtatgattt ctctggagga 16440

ccttctccca ctgagaacct acttgaagtg tagtacctgc cctaagtatt tgaaggctgt 16500

cctaggtatt accaaactca aagaaatgtt cacagacgcc tctttattat acttgactcg 16560

tgctcaacaa aaattctaca tgaaaactat aggcaatgca gtcaagggat actatagtaa 16620

ctgtgactct taaaggcaac cacatatcaa taggccctct ttctagccga tcgtattctt 16680

gttgacttca ttataccata ttagaaaaaa attgaattcc gaccctttaa gactcgtatt 16740

cggattcaaa taattatctc agaaaaaaag tgcacgtagt tgttcttgat tatagtcccg 16800

tcattcacca agtctttgtt tggt 16824

<210> 23
<211> 15966
<212> DNA
<213> Newcastle disease virus

<220>
<223> Mut HN (F277L)/M(G165W)/F(F117S)/F(F190L)/L(V757I)/L(F1551S)/L(R1
700L)-eGFP

<400> 23
accaaacaga gaatccgtga gatacgataa aaggcgaagg agcaatcgaa gtcgtacggg 60

tagaaggtgt gaatctcgag tgcgagaccg aagcttaaac tcgagagagc cttctgccaa 120

catgtcttcc gtatttgacg agtatgaaca gctcctcgcg gctcagactc gccctaatgg 180

agctcacgga ggaggagaga aggggagcac cttaaaagtt gaagtcccgg tattcactct 240

taacagtgac gacccagaag atagatggaa ctttgcagta ttctgtctta ggattgctgt 300

tagcgaagat gccaacaaac cactcagaca aggtgctctc atatctctct tatgctccca 360

ttctcaagtg atgaggaacc atgtcgccct tgcagggaaa cagaatgagg ccacactggc 420

tgttcttgag atcgatggtt tcaccaacag cgtgcctcaa tttaacaaca ggagtggagt 480

gtctgaggag agagcacaga gattcttgat gatagcaggg tccctccctc gggcatgcag 540

caacggtacc ccgttcatca cagctggggt tgaagatgat gcaccagaag acatcactga 600

tactctggaa agaatcatct ctatccaggc tcaagtatgg gtcacggtag caaaggccat 660

gactgcatat gagacagcgg atgagtcgga aacaagaaga atcaataagt atatgcagca 720

aggcagagtc caaaagaagt acatcctcca ccccgtatgt aggagcgcaa ttcaactcac 780

gattagacaa tctctggcag tccgcatttt cttggttagc gagcttaaga gaggccgcaa 840

cacggcaggt ggaagttcca cttattatag cttggtaggg gacatagact catacatcag 900

gaacaccggg cttactgcat tcttcctgac actcaggtat ggaattaaca ccaagacatc 960

agcccttgca ctcagcagcc tcgcaggcga tatccaaaaa atgaagcagc tcatgcgtct 1020

atatcggatg aaaggagata atgcgccgta catgacactg ctcggtgaca gtgaccagat 1080

gagctttgca ccggctgagt atgcacaact ctactctttt gcaatgggta tggcatccgt 1140

cctagacaaa ggaactggca agtaccagtt cgccagagac tttatgagca catcattctg 1200

gagacttgga gtagagtatg ctcgggctca gggaagcagc atcaatgagg atatggctgc 1260

cgagctaaag ctgaccccag cggcaaggag gggcctggca gctgctgccc aacgagcgtc 1320

cgaggagacc ggcagcatgg acattcccac tcaacaagct ggggtcctca cagggctcag 1380

cgacggaggc cctcaagccc cacaaggtgg actgaacagg tcgcaagggc aaccggacgc 1440

cggagatggg gagacccaat tttagatttt gatgagagca gtggcaaata gtatgagaga 1500

agcgccaaac tctgtgcaga acaccactca gcaggagcct ccatctactc ctgggccatc 1560

tcaagacaac gacaccgact ggggatactg accgacaaca ctcagcctgc ctccatcgaa 1620

ttatctcgat tcttctgcct gtaacctaac ccctggtcca cagtctcaca cggccaaacc 1680

cgcaaacgag cccccccctc tctccccccc cctccagcca cacggctcca accgcccaaa 1740

acaacacggg cacaactcga ctcactcata atctacacag agccgaagat cttagaaaaa 1800

aatacgggta gaagagagac attcagagat caggacaaat catcagggtc tccgctctcc 1860

cctctaccca gcagaccaga gtgaagatgg ccaccttcac ggatgcagag atcgacgatc 1920

tatttgaaac cagtggaact gtcattgaca gcataattac ggcccaaggc aacccagtag 1980

agactgttgg aaggagcgca atcccacaag gcaaaactaa agcattgagt gcagcatggg 2040

agaaacatgg aagcacccaa ccgccggccg gccaagacac ccctgatcga atggacagat 2100

cagacaaaca accatcccca cccgaacagg caaccacaca caacagcccg ccagccacat 2160

ccaccgacca gccccccacc caggccgcag gcgaagccgg tgacacacag ctcaagactg 2220

gagcaagcaa ctccctcctg tcgatgctcg acaagcttag caataaatcg tccaatgcta 2280

aaaagggccc acggtcgagt ccccaggaag ggcaccacca acctccgacc cagcagcaag 2340

ggagtcaaca gagccgcgga aacagtcagg agagatcgca aaaccaggtc aaggccgcct 2400

ctggagacca gggcacagac gcgaacacag catatcatgg acaatggaag gagtcacaac 2460

catcagctgg tgcaatccct catgctctcc ggtcagggca gagccaagcc aatactcctg 2520

catctgtgga tcatgtccag ctacctgtcg actttgtgca ggcgatgatg tctatgatgg 2580

aggcgatatc acagaaggta aataaagtcg actatcagct agaccttgtc tcaaagcaga 2640

catcctccat ccccatgatg cgatctgaaa tccaacagct gaaaacatcc gttgcggtca 2700

tggaagccaa cctgggcatg atgaagattc tggaccctgg ttgtgctaac gtttcatctt 2760

taagtgacct acgggcggtc gcccgctctc acccagtttt agtttcaggc cccggagacc 2820

catctcctta tgtgacacaa gggggtgaat tgatactcaa taaactctcg caaccagtgc 2880

aacatccttc tgagttgatt aaacctgcca cgataagcgg gcctgatata ggagtggaga 2940

aggacactgt ccgtgcattg atcacctcac gcccaatgca tccgagctct tcagctaagc 3000

tcctgagtaa gctggatgca gccgggtcga tagaagaaat taggaaaatc aaacgccttg 3060

cgctgaatgg ctgatcacta ctacaacccg caacgggttc ccgtttatct attgtcacaa 3120

ggaacccgcc ccgaagctta agaaaaaata cgggtagaat tggagcggcc gcattgtgcc 3180

aagatggtga gcaagggcga ggagctgttc accggggtgg tgcccatcct ggtcgagctg 3240

gacggcgacg taaacggcca caagttcagc gtgtccggcg agggcgaggg cgatgccacc 3300

tacggcaagc tgaccctgaa gttcatctgc accaccggca agctgcccgt gccctggccc 3360

accctcgtga ccaccctgac ctacggcgtg cagtgcttca gccgctaccc cgaccacatg 3420

aagcagcacg acttcttcaa gtccgccatg cccgaaggct acgtccagga gcgcaccatc 3480

ttcttcaagg acgacggcaa ctacaagacc cgcgccgagg tgaagttcga gggcgacacc 3540

ctggtgaacc gcatcgagct gaagggcatc gacttcaagg aggacggcaa catcctgggg 3600

cacaagctgg agtacaacta caacagccac aacgtctata tcatggccga caagcagaag 3660

aacggcatca aggtgaactt caagatccgc cacaacatcg aggacggcag cgtgcagctc 3720

gccgaccact accagcagaa cacccccatc ggcgacggcc ccgtgctgct gcccgacaac 3780

cactacctga gcacccagtc cgccctgagc aaagacccca acgagaagcg cgatcacatg 3840

gtcctgctgg agttcgtgac cgccgccggg atcactctcg gcatggacga gctgtacaag 3900

taagagctga ccgagccctc ctctataaac ccaagattca aggctccaag cgataacctt 3960

ctcccgcctc ttccatccca ctgaatgatc gcgcagctgc aattaatcta gcaacattaa 4020

agattaagaa aaaatacggg tagaatcgga gtgcaccgat tgtgccaaga tggactcatc 4080

taggacaatt gggttatact ttgattctgc ccttccttct agcaacctgt tagcattccc 4140

gattgtctta cgagatgtag gagatggaaa gaagcaaatt actccgcaat ataggatccg 4200

gcgtcttgac tcgtggacag acagtaaaga agactcggta ttcatcacta cctacggatt 4260

catcttccag gttgggaatg aagaagtcac tgtcggcatg atcaatgata atcccaagcg 4320

agagttactc tccgctgcga tgctctgcct aggaagcgtc ccgaatgttg gagatcctgt 4380

tgagctggca agggcctgcc tcactatggt ggtaacatgc aagaagagtg caactaatac 4440

tgagagaatg gtcttctcag tagtgcaggc accccgggtg ctgcaaagct gtagggtcgt 4500

ggcagacaaa tactcgtcag tgaatgcagt taagcacgtg aaagcaccag agaagatccc 4560

ttggagtgaa accctagagt acaaggtgaa ttttgtctct ttgactgtgg tgccgaagaa 4620

ggatgtctac aagatcccaa ccgcagtatt gaaggtatcc ggctcgagcc tgtataatct 4680

tgcgctcaat gtcactattg atgtggaggt agacccaaag agcccgttag tcaagtccct 4740

ttctaggtcc gacagtggat actatgctaa tctcttctta catatcggac ttatgtccac 4800

tgtagataag aggggaaaga aagtgacatt tgaccagctg gagaggaaga taaggagact 4860

tgatctatct gtcgggctca gtgatgtgct cggaccttct gtgcttgtga aggcgagagg 4920

tgcacggact aggctgctgg cacctttctt ctctaacagc gggacagcct gctaccctat 4980

agcaaatgcc tctcctcagg tagctaagat actctggagt cagaccgcgt gcctgcggag 5040

tgtaaaaatc attattcaag cgggcaccca acgcgctgtc gcagtgactg ctgaccatga 5100

ggttacctct actaagatag agaagaggca taccatcgct aaatacaatc ccttcaagaa 5160

ataggccgta tccctgagac tgcaattcac ccgccttccc aaaacaccat gacaccagat 5220

aatgatctgt cttgattact tacagttagt ttccctgtct atcaaattag aaaaaacacg 5280

ggtagaagag ttcggatccc ggccggcgca ccaaaagcgc aagatgggcc ccagatcttc 5340

taccaggatc ccagtacctc taatgctgac catacggatc acgctggcac tgagttatgt 5400

ccgtctgaca agttctcttg atggcaggcc tcttgcagcc gcagggatcg tggtaacagg 5460

ggataaagca gttaacatat acacctcatc ccagacaggg tcaatcatag tcaagttact 5520

cccaaatatg cccaaggaca aagaggcatg tgcaaaagcc ccattggagg cttacaacag 5580

gacactgact actttgctta ccccccttgg tgattctatc cgcaggatac aagagtctgt 5640

gactacatcc ggaggaagga gacagagacg ctctataggt gccattattg gcagtgtagc 5700

tctaggggtt gcaacagctg cacagataac ggcagcctcg gctctgatac aagccaacca 5760

gaatgctgct aacatcctcc ggcttaagga gagcattgct gcaaccaatg aagctgtaca 5820

cgaggtcact ggcggattgt cacagttagc agtggcagtt ggaaagatgc agcaatttgt 5880

taatgaccag cttaataaca cagctcaaga gttggactgt ataaaaatta cacagcaggt 5940

tggtgtagaa ctcaacttat acttaactga attgactaca gtgttcgggc cacaaatcac 6000

ttcccctgcc ttaactcagt tgactgttca ggctctttac aatctggctg gtggtaatgt 6060

agattacttg ttgactaagt taggtgtagg gaacaaccag ctcagctcat tgattggtag 6120

cggcttgatc accggtaacc ctatttttta cgactcacag actcaactct tgggcataca 6180

ggtgacttta ccctcagtcg ggaacctaaa taatatgcgt gccacctact tggagacctt 6240

gtctgtaagc acaacaaagg gatttgcctc agcacttgtc ccaaaagtag tgacacaggt 6300

cggttctgtg atagaagagc ttgacacctc atactgtata gaagctgatt tggatttata 6360

ttgtacaaga atagtgacat tccctatgtc ccctggtatt tattcctgtt tgagcggcaa 6420

tacatcggct tgcatgtatt caaggactga aggcgcactt actacaccat acatgactct 6480

caaaggctca gttgttgcca attgccagat gacaacatgt agatgtgcag accccccggg 6540

tatcatatca caaaattatg gagaggctgt gtctctaata gataagcact catgcaatgt 6600

cgtatcctta gacgggataa ctttgaggct cagtggggaa tttgatgcaa cttatcaaaa 6660

gaatatctca atattagatt ctcaagtact agtgacaggc aatctcgata tctcaactga 6720

gcttgggaat gtcaaccact caataagtaa tgctttggat aagttagagg aaagcaacag 6780

caaactagac aaagtcaatg tcaaactgac cagcacatct gctctcatta cctatattgt 6840

tttaactgtc atatctcttg ttcttggtat gcttagcctg gttctagcat gctatctgat 6900

gtacaagcaa aaagcgcaac gaaagacctt gttgtggctt ggaaataata ccctagatca 6960

gatgagagct actacaaaaa tgtgaatgca gatgagaggc agaggtatcc ccaatagcaa 7020

tctgtgtgtc aattctggca gcctgttaat cagaagaatt aagaaaaaac taccggatgt 7080

aggtgaacaa aagggaatat acgggtagaa cggcctgaga ggccacccct caatcgggag 7140

ccaggcccca ctacgtccgc tctaccgcat caccaacagc agtcttcagt catggacagc 7200

gcagttagcc aagttgcgct agagaatgat agaagagaag cgaataatac atggcgcttg 7260

gttttccgga tcgcagcctt acttttactg gtaataacct tagccgtctc tgcagtcgcc 7320

ctggcatata gtatggaggc tagcacacct ggcgaccttg taagcatacc gactgcgatc 7380

tctagggcag aggaaaggat tacatctgca ctcggttcca atcaagatgt agtagatagg 7440

atatacaagc aagtggccct tgagtctcca ttggcactgc taaataccga atctataatt 7500

atgaatgcaa taacgtctct ctcttatcaa atcaatggag ccacaaataa tagcgggtgt 7560

ggggcacctg ttcatgaccc agattacatc ggggggatag gtcaagaact tattgtagat 7620

gatacgagtg acgtcacatc attttatccc tctgcattcc aagaacacct gaattttatc 7680

ccggcgccca ctacaggatc aggctgcact cggataccct cattcgacat gagtgctacc 7740

cattactgtt acactcacaa tgtgatattg tctggctgca gagatcactc acactcacat 7800

cagtatttag cacttggtgt gcttcggaca tctgcaacag ggagggtatt cttttctact 7860

ctgcgttcca tcaacttgga tgacgcccaa aatcggaagt cttgcagtgt gagtgcaact 7920

cctttaggtt gtgatatgct gtgctctaaa atcacagaga ctgaggaaga ggattataaa 7980

tcagttatcc ccacatcgat ggtacatgga aggttagggc ttgacggcca ataccatgag 8040

aaggacctag acgtcacaac actatttagg gactgggtgg caaattaccc aggagtagga 8100

ggtgggtctt ttattaacaa tcgcgtatgg ttcccggtct acggagggct aaaacccagc 8160

tcgcctagtg acactgcaca agaagggaga tacgtaatat acaagcgata caatgacaca 8220

tgcccggatg agcaagatta ccagattcgg atggctaagt catcgtataa gcctaggcgg 8280

tttggtggaa aacgcgtaca gcaggccatc ctatccatca aggtgtcaac atccttgggt 8340

gaggacccgg tgttgactgt accgcctaat acggtcgcac tcatgggggc cgaaggcaga 8400

gttctcacag tggggacatc tcatttctta tatcagcgag ggtcatcata cttctctccc 8460

gctttgttat accctatgac agtcaacaac aaaacagcca ctcttcataa tccttataca 8520

ttcaatgctt tcactcggcc aggtagtgtc ccttgccagg cttcagcaag atgccccaac 8580

tcatgtgtta ccggagtcta tactgatcca tatcccttag tcttccatag gaaccacacc 8640

ttgcgagggg tattcggaac aatgcttgat gacgaacaag caagactcaa ccctgtatct 8700

gcagtatttg ataacatatc ccgcagtcgc ataactcggg taagttcaag cagtaccagg 8760

gcagcataca cgacatcaac gtgttttaaa gttgtcaaga ccaataaaac ctattgcctc 8820

agcattgcag aaatatccaa taccctcttc ggggaattca gaattgtccc cttactagtt 8880

gagattctca aggatggtgg ggtttaagaa gctaggaatg gtcggttgag tcaaccgtga 8940

gaaggcgggg aagatgatat tgcatcatct atcttctgta acaccaagaa tcaaatcgaa 9000

taccagtgcg agctcgaatc ctacgctgcc agtcagccat aatcggctgg tgctaatgtg 9060

gttagcctga atcttgtcga tagtcacttg attaagaaaa aatgtggacg gtagtgaaat 9120

ataaggcaaa gcagctcaca gaggacagca cgggtaggac atggcgggct ccggacccga 9180

aagggcagag catcagatta tcctaccaga gtcacatctg tcctcaccat tggtcaagca 9240

taaattgctt tattactgga aattaactgg gctaccgctt cctgacgaat gtgacttcga 9300

ccatcttatt atcagccgac aatggaagaa ggtacttgaa tcggccaccc ctgacattga 9360

gagaatgata aaactagggc gggcagtaca ccagactctc aaccacaatt ccaggataac 9420

cggagtactc catccccggt gtttagagga actggctagt attgaggttc ctgattcaac 9480

caacaaattt cggaagatcg aaaaaaagat ccagattcac aacacaaggt atggagaact 9540

gttcacaaga ttatgcacgc atgtagaaaa gaaactattg gggtcatctt ggtctaacaa 9600

tgtcccacgg tcagaggaat tcaacagcat ccgtacagat ccggcatttt ggtttcactc 9660

aaaatggtcc acagccaaat ttgcatggct ccatataaaa caggtccaga ggcatctgat 9720

tgtagcagca agaacaaggt ccgcagtcaa caaattagtg acgctgaccc ataaggtagg 9780

ccaaatcttt gttactcctg agcttgtcat tgtgacacat acagatgaga acaagttcac 9840

gtgtcttacc caggaacttg tgttgatgta tgcagatatg atggagggca gagatatggt 9900

cagcataata tcatccacgg cggcacatct taggagctta tcagagaaaa ttgatgatat 9960

tctgcggtta gtagatgctc tggcaagaga tttgggcaat caagtctacg atgttgtagc 10020

actaatggag ggattcgcat acggcgctgt tcagctgctt gaaccgtcag gtacatttgc 10080

gggggatttc ttcgcattca acctgcagga gctcaaagat actctaaccg gactcctccc 10140

caaggatatc gcagaatctg tgactcacgc aatcgcaacc atattctctg gcttagaaca 10200

aaatcaagca gctgagatgt tgtgcctgtt gcgtctgtgg ggtcacccac tactcgagtc 10260

ccgtattgca gcaaaagcag ttaggagtca aatgtgcgca ccaaaaatgg tagactttga 10320

tatgatcctc caggtattat ctttctttaa aggaacaatc atcaatggat atagaaagaa 10380

gaatgcaggc gtgtggccac gtgtcaagat agatacgata tacgggaagg tcatagggca 10440

gctacacgca gattctgcag agatttcaca tgatgtcatg ttgagggaat acaagagttt 10500

atctgcactt gaattcgagc catgtataga gtatgaccct gtcaccaatc tgagcatgtt 10560

tttaaaagac aaggcaatcg cacacccgaa agacaactgg ctcgcttcgt ttaggcgaaa 10620

ccttctctct gaggaccaga agaaacatgt aagagaggca acctcaacta accgcctctt 10680

gatagagttc ttagagtcaa atgattttga tccatataaa gagatggaat atctgacgac 10740

ccttgagtac ctaagagatg ataatgtggc agtatcatac tcactcaaag aaaaagaggt 10800

gaaagttaat gggcggattt tcgcaaagct aacaaataga ttaaggaatt gtcaggtaat 10860

ggcagaaggg atcctagctg accagattgc acctttcttc cagggaaatg gggtcattca 10920

ggatagcata tctttgacta aaagtatgct agcgatgagt caactgtctt ttaacagcaa 10980

caagaaacgt attactgact gcaaagaaag agtatcctca aaccgcaatc atgatccgaa 11040

gagcaagaat cgtcggagag ttgccacttt tgtaactacc gacctgcaaa agtattgtct 11100

caattggaga tatcagacag tcaagctgtt cgctcatgcc atcaatcagc tgatgggctt 11160

acctcacttc ttcgagtgga ttcatcttag actaatggat actacgatgt ttgtagggga 11220

ccctttcaat cctccaagtg accctacgga ctgtgatcta tcaagagtcc caaatgatga 11280

catatatatt gtcagtgcta ggggaggcat tgagggatta tgccagaagc tatggacaat 11340

gatctcaatt gctgcaatcc agcttgctgc agcaagatca cattgtcgcg ttgcctgtat 11400

ggtacaaggc gacaatcaag taatagctat aacgagagag gtaagatcag atgactcccc 11460

agagatggtg ttaacacaat tgcatcaagc cagtgataat ttcttcaagg aattgattca 11520

tgtcaatcat ttgatcggcc ataatttgaa ggatcgtgaa accatcaggt cagacacgtt 11580

cttcatatac agcaaacgaa tattcaaaga tggagcaata ctcagtcagg tcctcaaaaa 11640

ctcatctaaa ttagtgctaa tatcaggcga ccttagtgaa aacactgtaa tgtcttgtgc 11700

caacattgca tctactgtag cacggttatg cgagaacggg cttcctaagg atttctgtta 11760

ttacttaaac tacttaatga gttgcgtgca gacatacttc gattccgaat tttccatcac 11820

caacaactcg caactcgatt ctaaccagtc gtggatagag gacatttctt ttgtgcactc 11880

atatgtcctg acccctgctc aactgggggg actgagtaac cttcaatact caaggctcta 11940

cacaaggaac atcggcgacc cgggaaccac tgctttcgca gagatcaaga gattagaggc 12000

agtggggtta ctgagtccta gcattatgac taacatctta actaggccgc ctggaaatgg 12060

agattgggcc agtctgtgca acgatccata ctcctttaat tttgagactg tcgcaagccc 12120

aaatattgtc cttaagaaac atacacaaag agtcctattt gaaacttgct cgaatccctt 12180

attatctgga gtgcacacag aggataatga ggcagaagag aaggcattgg ctgaattctt 12240

gctcaatcaa gaaatgattc atccacgtgt cgcgcatgct atcatggaag caagctctgt 12300

aggtaggaga aagcaaattc aggggcttgt tgacacaaca aacaccgtga ttaagattgc 12360

actgactagg aggccactcg gcatcaagag gctgatgcgg atagtcaatt actcgagcat 12420

gcatgcaatg ctatttagag atgatgtttt ctcgtctaat agatccaacc atcccttagt 12480

ctcttctgat atgtgttctc agacactggc agactatgca cggaacagaa gctggtcacc 12540

tttgacaggg ggtagaaaaa tactgggtgt atctaatcct gataccatag aacttgtaga 12600

gggtgagatc cttagtgtca gtggagggtg cactagatgt gacagtgggg atgaacagtt 12660

tacttggttc catcttccaa gcaatataga gctaaacgat gacaccagca agaatcctcc 12720

aatgagagtg ccatatctcg ggtcaaagac tcaagagagg agagccgcct cgcttgcgaa 12780

aatagctcat atgtcaccac acgtgaaggc ggctctaagg gcgtcatctg tgttaatctg 12840

ggcttatggg gacaacgaaa taaactggac tgctgccctt aagattgcaa ggtctcggtg 12900

caacataagc tcagagtatc ttcgactatt gtcacccttg cctacagctg ggaatctcca 12960

acatagattg gacgacggca taactcagat gacattcacc cctgcatctc tatacagggt 13020

gtcaccttac attcatatat ctaatgattc tcaaaggcta tttactgaag aaggagtcaa 13080

agaggggaat gtggtttatc agcaaatcat gctcttgggt ttgtccttaa ttgagtcact 13140

cttcccaatg acaacaacca agacatatga tgaaatcaca ttgcacctcc acagtaaatt 13200

tagctgctgt atcagggaag cacctgttgc agttcctttc gagctactcg gggtggcacc 13260

ggaactaagg gcagtaacct caaataagtt tatgtatgat cctagccctg tatcagaggg 13320

agactttgcg agacttgact tagctatctt taagagttat gaacttaatt tagagtcata 13380

ttccacaata gagctaatga acgttctttc aatatctagt gggaagttga ttggccagtc 13440

tgtggtttct tatgatgaag atacctccat aaagaatgac gctataatag tgtatgacaa 13500

cacacggaat tggatcagcg aagctcagaa ttcagatgtg gtccgcctat tcgagtatgc 13560

ggcactcgaa gtgctcctcg actgttctta tcaactctac tatctgagag taagaggcct 13620

aaacaatatc gtcctgtaca tgagtgattt atacaagaat atgccaggaa ttctactctc 13680

taatattgca gccacaatat ctcaccctgt catccattca aggttgaatg cagtaggtct 13740

ggtcaaccat gacgggtcac accaacttgc agacacagat ttcattgaaa tgtctgcaaa 13800

gctgctagtc tcttgcactc gacgcgtggt ctcaggttta catgcaggga ataagtatga 13860

cctgctgttt ccatctgtct tggatgataa cctaagtgag aagatgcttc agttgatatc 13920

ccaattatgc tgtctgtata cggtgctctt tgctacaaca agagaaatcc cgaaaataag 13980

aggcttatct gcagaagaga aatgttcagt acttactgag tacctactgt cagatgctgt 14040

gaaaccgtta cttgggtccg agcaagtgag ctctatcatg tctcccaaca tagttacgtt 14100

cccagccaat ctgtattaca tgtctaggaa gagccttaat ttgatcaggg agagagagga 14160

cagggatact atcttggcat tgttgttccc tcaagaaccg ctgctcgagt ttcctctggt 14220

acgagatatt ggtgctcgtg taaaagatcc atttacccta caacctgcgg cgtttttaca 14280

agagttagat ttgagtgctc cggcaaggta tgacgcattc acaatcagtc aggcgcattc 14340

tgaacacata ttgccaaacc cagaggaaga tcacttagta cgatacttgt tcagaggaat 14400

agggactgcg tcctcctctt ggtataaggc atcccatctt ctttctgtac ccgaggtcag 14460

atgtgcaagg catgggaact ccttatattt agcagaagga agtggagcca tcatgagtct 14520

tctcgaattg catataccac acgaaactat ctattacaat acacttttct cgaacgagat 14580

gaacccccca cagcgacatt tcggaccaac tccaacacag tttctgaatt cggttgtttt 14640

taggaattta caggcggaag taccatgcaa ggatggattt gtccaggagt tccgtccgtt 14700

atggagagag aatacagaag aaagcgatct gacctcagat aaagcagtgg gatatatcac 14760

atctgtagtg ccgtacaggt ctgtatcatt gctgcattgt gacattgaaa tccctccagg 14820

atctaatcaa agcttactag atcagctggc taccaatctg tctctgattg ccatgcattc 14880

tgtgaaggag ggcggggtcg tgattatcaa agtactgtat gcaatgggat attacttcca 14940

tctactcatg aacttgttta ctccatgttc cacgaaagga tatattctct ctaatggcta 15000

tgcctgtaga ggggatatgg agtgttacct gatatttgtc atgggctacc taggcgggcc 15060

tacatttgta catgaggtgg tgaggatggc aaaaacgcta gtacagcggc atggcacact 15120

tttgtctaag tcagatgaga ttacactgac taggttattt acttcacagc aacagcgtgt 15180

aacagacatc ctatccagcc ctttaccgag actaatgaag tacttgagag agaatattga 15240

tactgcactg attgaagccg ggggacagcc tgtccgtcca ttctgtgcag agagtttagt 15300

gagcacacta acagacatga ctcagacaac ccagatcatc gccagccaca ttgatacagt 15360

cattcgatct gtgatctata tggaagcaga gggtgatctt gctgacacag tattcttatt 15420

taccccttac aacctctcta tggacgggaa aaagagaaca tcacttaaac agtgcacaag 15480

acagatctta gaggtcacaa tactgggtct cagagtcaaa aatctcaata aagtaggtga 15540

tgtaatcagc ctagtactca gaggtatgat ttctctggag gaccttctcc cactgagaac 15600

ctacttgaag tgtagtacct gccctaagta tttgaaggct gtcctaggta ttaccaaact 15660

caaagaaatg ttcacagacg cctctttatt atacttgact cgtgctcaac aaaaattcta 15720

catgaaaact ataggcaatg cagtcaaggg atactatagt aactgtgact cttaaaggca 15780

accacatatc aataggccct ctttctagcc gatcgtattc ttgttgactt cattatacca 15840

tattagaaaa aaattgaatt ccgacccttt aagactcgta ttcggattca aataattatc 15900

tcagaaaaaa agtgcacgta gttgttcttg attatagtcc cgtcattcac caagtctttg    15960

tttggt                                                              15966

<210> 24
<211> 571
<212> PRT
<213> Newcastle disease virus

<220>
<223> HN protein

<400> 24
Met Asp Ser Ala Val Ser Gln Val Ala Leu Glu Asn Asp Arg Arg Glu
1               5                   10                  15
Ala Asn Asn Thr Trp Arg Leu Val Phe Arg Ile Ala Ala Leu Leu Leu
            20                  25                  30
Leu Val Ile Thr Leu Ala Val Ser Ala Val Ala Leu Ala Tyr Ser Met
        35                  40                  45
Glu Ala Ser Thr Pro Gly Asp Leu Val Ser Ile Pro Thr Ala Ile Ser
    50                  55                  60
Arg Ala Glu Glu Arg Ile Thr Ser Ala Leu Gly Ser Asn Gln Asp Val
65                  70                  75                  80
Val Asp Arg Ile Tyr Lys Gln Val Ala Leu Glu Ser Pro Leu Ala Leu
                85                  90                  95
Leu Asn Thr Glu Ser Ile Ile Met Asn Ala Ile Thr Ser Leu Ser Tyr
            100                 105                 110
Gln Ile Asn Gly Ala Thr Asn Asn Ser Gly Cys Gly Ala Pro Val His
        115                 120                 125
Asp Pro Asp Tyr Ile Gly Gly Ile Gly Gln Glu Leu Ile Val Asp Asp
    130                 135                 140
Thr Ser Asp Val Thr Ser Phe Tyr Pro Ser Ala Phe Gln Glu His Leu
145                 150                 155                 160
Asn Phe Ile Pro Ala Pro Thr Thr Gly Ser Gly Cys Thr Arg Ile Pro
                165                 170                 175
Ser Phe Asp Met Ser Ala Thr His Tyr Cys Tyr Thr His Asn Val Ile
            180                 185                 190
Leu Ser Gly Cys Arg Asp His Ser His Ser His Gln Tyr Leu Ala Leu
        195                 200                 205
Gly Val Leu Arg Thr Ser Ala Thr Gly Arg Val Phe Phe Ser Thr Leu
    210                 215                 220
Arg Ser Ile Asn Leu Asp Asp Ala Gln Asn Arg Lys Ser Cys Ser Val
225                 230                 235                 240
Ser Ala Thr Pro Leu Gly Cys Asp Met Leu Cys Ser Lys Ile Thr Glu
                245                 250                 255
Thr Glu Glu Glu Asp Tyr Lys Ser Val Ile Pro Thr Ser Met Val His
            260                 265                 270
Gly Arg Leu Gly Leu Asp Gly Gln Tyr His Glu Lys Asp Leu Asp Val
        275                 280                 285
Thr Thr Leu Phe Arg Asp Trp Val Ala Asn Tyr Pro Gly Val Gly Gly
    290                 295                 300
Gly Ser Phe Ile Asn Asn Arg Val Trp Phe Pro Val Tyr Gly Gly Leu
305                 310                 315                 320
Lys Pro Ser Ser Pro Ser Asp Thr Ala Gln Glu Gly Arg Tyr Val Ile
                325                 330                 335
Tyr Lys Arg Tyr Asn Asp Thr Cys Pro Asp Glu Gln Asp Tyr Gln Ile
            340                 345                 350
Arg Met Ala Lys Ser Ser Tyr Lys Pro Arg Arg Phe Gly Gly Lys Arg
        355                 360                 365

Val Gln Gln Ala Ile Leu Ser Ile Lys Val Ser Thr Ser Leu Gly Glu
    370                 375                 380
Asp Pro Val Leu Thr Val Pro Pro Asn Thr Val Ala Leu Met Gly Ala
385                 390                 395                 400
Glu Gly Arg Val Leu Thr Val Gly Thr Ser His Phe Leu Tyr Gln Arg
                405                 410                 415
Gly Ser Ser Tyr Phe Ser Pro Ala Leu Leu Tyr Pro Met Thr Val Asn
            420                 425                 430
Asn Lys Thr Ala Thr Leu His Asn Pro Tyr Thr Phe Asn Ala Phe Thr
        435                 440                 445
Arg Pro Gly Ser Val Pro Cys Gln Ala Ser Ala Arg Cys Pro Asn Ser
    450                 455                 460
Cys Val Thr Gly Val Tyr Thr Asp Pro Tyr Pro Leu Val Phe His Arg
465                 470                 475                 480
Asn His Thr Leu Arg Gly Val Phe Gly Thr Met Leu Asp Asp Glu Gln
                485                 490                 495
Ala Arg Leu Asn Pro Val Ser Ala Val Phe Asp Asn Ile Ser Arg Ser
            500                 505                 510
Arg Ile Thr Arg Val Ser Ser Ser Ser Thr Arg Ala Ala Tyr Thr Thr
        515                 520                 525
Ser Thr Cys Phe Lys Val Val Lys Thr Asn Lys Thr Tyr Cys Leu Ser
    530                 535                 540
Ile Ala Glu Ile Ser Asn Thr Leu Phe Gly Glu Phe Arg Ile Val Pro
545                 550                 555                 560
Leu Leu Val Glu Ile Leu Lys Asp Gly Gly Val
                565                 570

<210> 25
<211> 364
<212> PRT
<213> Newcastle disease virus

<220>
<223> M protein

<400> 25
Met Asp Ser Ser Arg Thr Ile Gly Leu Tyr Phe Asp Ser Ala Leu Pro
1               5                   10                  15
Ser Ser Asn Leu Leu Ala Phe Pro Ile Val Leu Arg Asp Val Gly Asp
            20                  25                  30
Gly Lys Lys Gln Ile Thr Pro Gln Tyr Arg Ile Arg Arg Leu Asp Ser
        35                  40                  45
Trp Thr Asp Ser Lys Glu Asp Ser Val Phe Ile Thr Thr Tyr Gly Phe
    50                  55                  60
Ile Phe Gln Val Gly Asn Glu Glu Val Thr Val Gly Met Ile Asn Asp
65                  70                  75                  80
Asn Pro Lys Arg Glu Leu Leu Ser Ala Ala Met Leu Cys Leu Gly Ser
                85                  90                  95
Val Pro Asn Val Gly Asp Pro Val Glu Leu Ala Arg Ala Cys Leu Thr
            100                 105                 110
Met Val Val Thr Cys Lys Lys Ser Ala Thr Asn Thr Glu Arg Met Val
        115                 120                 125
Phe Ser Val Val Gln Ala Pro Arg Val Leu Gln Ser Cys Arg Val Val
    130                 135                 140
Ala Asp Lys Tyr Ser Ser Val Asn Ala Val Lys His Val Lys Ala Pro
145                 150                 155                 160
Glu Lys Ile Pro Trp Ser Glu Thr Leu Glu Tyr Lys Val Asn Phe Val
                165                 170                 175
Ser Leu Thr Val Val Pro Lys Lys Asp Val Tyr Lys Ile Pro Thr Ala
            180                 185                 190

Val Leu Lys Val Ser Gly Ser Ser Leu Tyr Asn Leu Ala Leu Asn Val
        195                 200                 205
Thr Ile Asp Val Glu Val Asp Pro Lys Ser Pro Leu Val Lys Ser Leu
    210                 215                 220
Ser Arg Ser Asp Ser Gly Tyr Tyr Ala Asn Leu Phe Leu His Ile Gly
225                 230                 235                 240
Leu Met Ser Thr Val Asp Lys Arg Gly Lys Lys Val Thr Phe Asp Gln
                245                 250                 255
Leu Glu Arg Lys Ile Arg Arg Leu Asp Leu Ser Val Gly Leu Ser Asp
            260                 265                 270
Val Leu Gly Pro Ser Val Leu Val Lys Ala Arg Gly Ala Arg Thr Arg
        275                 280                 285
Leu Leu Ala Pro Phe Phe Ser Asn Ser Gly Thr Ala Cys Tyr Pro Ile
    290                 295                 300
Ala Asn Ala Ser Pro Gln Val Ala Lys Ile Leu Trp Ser Gln Thr Ala
305                 310                 315                 320
Cys Leu Arg Ser Val Lys Ile Ile Ile Gln Ala Gly Thr Gln Arg Ala
                325                 330                 335
Val Ala Val Thr Ala Asp His Glu Val Thr Ser Thr Lys Ile Glu Lys
            340                 345                 350
Arg His Thr Ile Ala Lys Tyr Asn Pro Phe Lys Lys
        355                 360

<210> 26
<211> 553
<212> PRT
<213> Newcastle disease virus

<220>
<223> F protein

<400> 26
Met Gly Pro Arg Ser Ser Thr Arg Ile Pro Val Pro Leu Met Leu Thr
1               5                   10                  15
Ile Arg Ile Thr Leu Ala Leu Ser Tyr Val Arg Leu Thr Ser Ser Leu
            20                  25                  30
Asp Gly Arg Pro Leu Ala Ala Ala Gly Ile Val Val Thr Gly Asp Lys
        35                  40                  45
Ala Val Asn Ile Tyr Thr Ser Ser Gln Thr Gly Ser Ile Ile Val Lys
    50                  55                  60
Leu Leu Pro Asn Met Pro Lys Asp Lys Glu Ala Cys Ala Lys Ala Pro
65                  70                  75                  80
Leu Glu Ala Tyr Asn Arg Thr Leu Thr Thr Leu Leu Thr Pro Leu Gly
                85                  90                  95
Asp Ser Ile Arg Arg Ile Gln Glu Ser Val Thr Thr Ser Gly Gly Arg
            100                 105                 110
Arg Gln Arg Arg Ser Ile Gly Ala Ile Ile Gly Ser Val Ala Leu Gly
        115                 120                 125
Val Ala Thr Ala Ala Gln Ile Thr Ala Ala Ser Ala Leu Ile Gln Ala
    130                 135                 140
Asn Gln Asn Ala Ala Asn Ile Leu Arg Leu Lys Glu Ser Ile Ala Ala
145                 150                 155                 160
Thr Asn Glu Ala Val His Glu Val Thr Gly Gly Leu Ser Gln Leu Ala
                165                 170                 175
Val Ala Val Gly Lys Met Gln Gln Phe Val Asn Asp Gln Leu Asn Asn
            180                 185                 190
Thr Ala Gln Glu Leu Asp Cys Ile Lys Ile Thr Gln Gln Val Gly Val
        195                 200                 205
Glu Leu Asn Leu Tyr Leu Thr Glu Leu Thr Thr Val Phe Gly Pro Gln
    210                 215                 220

Ile Thr Ser Pro Ala Leu Thr Gln Leu Thr Val Gln Ala Leu Tyr Asn
225                 230                 235                 240
Leu Ala Gly Gly Asn Val Asp Tyr Leu Leu Thr Lys Leu Gly Val Gly
                245                 250                 255
Asn Asn Gln Leu Ser Ser Leu Ile Gly Ser Gly Leu Ile Thr Gly Asn
            260                 265                 270
Pro Ile Phe Tyr Asp Ser Gln Thr Gln Leu Leu Gly Ile Gln Val Thr
        275                 280                 285
Leu Pro Ser Val Gly Asn Leu Asn Asn Met Arg Ala Thr Tyr Leu Glu
    290                 295                 300
Thr Leu Ser Val Ser Thr Thr Lys Gly Phe Ala Ser Ala Leu Val Pro
305                 310                 315                 320
Lys Val Val Thr Gln Val Gly Ser Val Ile Glu Glu Leu Asp Thr Ser
                325                 330                 335
Tyr Cys Ile Glu Ala Asp Leu Asp Leu Tyr Cys Thr Arg Ile Val Thr
            340                 345                 350
Phe Pro Met Ser Pro Gly Ile Tyr Ser Cys Leu Ser Gly Asn Thr Ser
        355                 360                 365
Ala Cys Met Tyr Ser Arg Thr Glu Gly Ala Leu Thr Thr Pro Tyr Met
    370                 375                 380
Thr Leu Lys Gly Ser Val Val Ala Asn Cys Gln Met Thr Thr Cys Arg
385                 390                 395                 400
Cys Ala Asp Pro Pro Gly Ile Ile Ser Gln Asn Tyr Gly Glu Ala Val
                405                 410                 415
Ser Leu Ile Asp Lys His Ser Cys Asn Val Val Ser Leu Asp Gly Ile
            420                 425                 430
Thr Leu Arg Leu Ser Gly Glu Phe Asp Ala Thr Tyr Gln Lys Asn Ile
        435                 440                 445
Ser Ile Leu Asp Ser Gln Val Leu Val Thr Gly Asn Leu Asp Ile Ser
    450                 455                 460
Thr Glu Leu Gly Asn Val Asn His Ser Ile Ser Asn Ala Leu Asp Lys
465                 470                 475                 480
Leu Glu Glu Ser Asn Ser Lys Leu Asp Lys Val Asn Val Lys Leu Thr
                485                 490                 495
Ser Thr Ser Ala Leu Ile Thr Tyr Ile Val Leu Thr Val Ile Ser Leu
            500                 505                 510
Val Leu Gly Met Leu Ser Leu Val Leu Ala Cys Tyr Leu Met Tyr Lys
        515                 520                 525
Gln Lys Ala Gln Arg Lys Thr Leu Leu Trp Leu Gly Asn Asn Thr Leu
    530                 535                 540
Asp Gln Met Arg Ala Thr Thr Lys Met
545                 550

<210> 27
<211> 2204
<212> PRT
<213> Newcastle disease virus

<220>
<223> L protein

<400> 27
Met Ala Gly Ser Gly Pro Glu Arg Ala Glu His Gln Ile Ile Leu Pro
1               5                   10                  15
Glu Ser His Leu Ser Ser Pro Leu Val Lys His Lys Leu Leu Tyr Tyr
            20                  25                  30
Trp Lys Leu Thr Gly Leu Pro Leu Pro Asp Glu Cys Asp Phe Asp His
        35                  40                  45
Leu Ile Ile Ser Arg Gln Trp Lys Lys Val Leu Glu Ser Ala Thr Pro
    50                  55                  60

Asp Ile Glu Arg Met Ile Lys Leu Gly Arg Ala Val His Gln Thr Leu
65                  70                  75                  80
Asn His Asn Ser Arg Ile Thr Gly Val Leu His Pro Arg Cys Leu Glu
                85                  90                  95
Glu Leu Ala Ser Ile Glu Val Pro Asp Ser Thr Asn Lys Phe Arg Lys
            100                 105                 110
Ile Glu Lys Lys Ile Gln Ile His Asn Thr Arg Tyr Gly Glu Leu Phe
        115                 120                 125
Thr Arg Leu Cys Thr His Val Glu Lys Lys Leu Leu Gly Ser Ser Trp
    130                 135                 140
Ser Asn Asn Val Pro Arg Ser Glu Glu Phe Asn Ser Ile Arg Thr Asp
145                 150                 155                 160
Pro Ala Phe Trp Phe His Ser Lys Trp Ser Thr Ala Lys Phe Ala Trp
                165                 170                 175
Leu His Ile Lys Gln Val Gln Arg His Leu Ile Val Ala Ala Arg Thr
            180                 185                 190
Arg Ser Ala Val Asn Lys Leu Val Thr Leu Thr His Lys Val Gly Gln
        195                 200                 205
Ile Phe Val Thr Pro Glu Leu Val Ile Val Thr His Thr Asp Glu Asn
    210                 215                 220
Lys Phe Thr Cys Leu Thr Gln Glu Leu Val Leu Met Tyr Ala Asp Met
225                 230                 235                 240
Met Glu Gly Arg Asp Met Val Ser Ile Ile Ser Ser Thr Ala Ala His
                245                 250                 255
Leu Arg Ser Leu Ser Glu Lys Ile Asp Asp Ile Leu Arg Leu Val Asp
            260                 265                 270
Ala Leu Ala Arg Asp Leu Gly Asn Gln Val Tyr Asp Val Val Ala Leu
        275                 280                 285
Met Glu Gly Phe Ala Tyr Gly Ala Val Gln Leu Leu Glu Pro Ser Gly
    290                 295                 300
Thr Phe Ala Gly Asp Phe Phe Ala Phe Asn Leu Gln Glu Leu Lys Asp
305                 310                 315                 320
Thr Leu Thr Gly Leu Leu Pro Lys Asp Ile Ala Glu Ser Val Thr His
                325                 330                 335
Ala Ile Ala Thr Ile Phe Ser Gly Leu Glu Gln Asn Gln Ala Ala Glu
            340                 345                 350
Met Leu Cys Leu Leu Arg Leu Trp Gly His Pro Leu Leu Glu Ser Arg
        355                 360                 365
Ile Ala Ala Lys Ala Val Arg Ser Gln Met Cys Ala Pro Lys Met Val
    370                 375                 380
Asp Phe Asp Met Ile Leu Gln Val Leu Ser Phe Phe Lys Gly Thr Ile
385                 390                 395                 400
Ile Asn Gly Tyr Arg Lys Lys Asn Ala Gly Val Trp Pro Arg Val Lys
                405                 410                 415
Ile Asp Thr Ile Tyr Gly Lys Val Ile Gly Gln Leu His Ala Asp Ser
            420                 425                 430
Ala Glu Ile Ser His Asp Val Met Leu Arg Glu Tyr Lys Ser Leu Ser
        435                 440                 445
Ala Leu Glu Phe Glu Pro Cys Ile Glu Tyr Asp Pro Val Thr Asn Leu
    450                 455                 460
Ser Met Phe Leu Lys Asp Lys Ala Ile Ala His Pro Lys Asp Asn Trp
465                 470                 475                 480
Leu Ala Ser Phe Arg Arg Asn Leu Leu Ser Glu Asp Gln Lys Lys His
                485                 490                 495
Val Arg Glu Ala Thr Ser Thr Asn Arg Leu Leu Ile Glu Phe Leu Glu
            500                 505                 510
Ser Asn Asp Phe Asp Pro Tyr Lys Glu Met Glu Tyr Leu Thr Thr Leu
        515                 520                 525
Glu Tyr Leu Arg Asp Asp Asn Val Ala Val Ser Tyr Ser Leu Lys Glu
    530                 535                 540
Lys Glu Val Lys Val Asn Gly Arg Ile Phe Ala Lys Leu Thr Asn Arg
545                 550                 555                 560
Leu Arg Asn Cys Gln Val Met Ala Glu Gly Ile Leu Ala Asp Gln Ile

                565                 570                 575
Ala Pro Phe Phe Gln Gly Asn Gly Val Ile Gln Asp Ser Ile Ser Leu
            580                 585                 590
Thr Lys Ser Met Leu Ala Met Ser Gln Leu Ser Phe Asn Ser Asn Lys
        595                 600                 605
Lys Arg Ile Thr Asp Cys Lys Glu Arg Val Ser Ser Asn Arg Asn His
    610                 615                 620
Asp Pro Lys Ser Lys Asn Arg Arg Arg Val Ala Thr Phe Val Thr Thr
625                 630                 635                 640
Asp Leu Gln Lys Tyr Cys Leu Asn Trp Arg Tyr Gln Thr Val Lys Leu
                645                 650                 655
Phe Ala His Ala Ile Asn Gln Leu Met Gly Leu Pro His Phe Phe Glu
            660                 665                 670
Trp Ile His Leu Arg Leu Met Asp Thr Thr Met Phe Val Gly Asp Pro
        675                 680                 685
Phe Asn Pro Pro Ser Asp Pro Thr Asp Cys Asp Leu Ser Arg Val Pro
    690                 695                 700
Asn Asp Asp Ile Tyr Ile Val Ser Ala Arg Gly Gly Ile Glu Gly Leu
705                 710                 715                 720
Cys Gln Lys Leu Trp Thr Met Ile Ser Ile Ala Ala Ile Gln Leu Ala
                725                 730                 735
Ala Ala Arg Ser His Cys Arg Val Ala Cys Met Val Gln Gly Asp Asn
            740                 745                 750
Gln Val Ile Ala Ile Thr Arg Glu Val Arg Ser Asp Asp Ser Pro Glu
        755                 760                 765
Met Val Leu Thr Gln Leu His Gln Ala Ser Asp Asn Phe Phe Lys Glu
    770                 775                 780
Leu Ile His Val Asn His Leu Ile Gly His Asn Leu Lys Asp Arg Glu
785                 790                 795                 800
Thr Ile Arg Ser Asp Thr Phe Phe Ile Tyr Ser Lys Arg Ile Phe Lys
                805                 810                 815
Asp Gly Ala Ile Leu Ser Gln Val Leu Lys Asn Ser Ser Lys Leu Val
            820                 825                 830
Leu Ile Ser Gly Asp Leu Ser Glu Asn Thr Val Met Ser Cys Ala Asn
        835                 840                 845
Ile Ala Ser Thr Val Ala Arg Leu Cys Glu Asn Gly Leu Pro Lys Asp
    850                 855                 860
Phe Cys Tyr Tyr Leu Asn Tyr Leu Met Ser Cys Val Gln Thr Tyr Phe
865                 870                 875                 880
Asp Ser Glu Phe Ser Ile Thr Asn Asn Ser Gln Leu Asp Ser Asn Gln
                885                 890                 895
Ser Trp Ile Glu Asp Ile Ser Phe Val His Ser Tyr Val Leu Thr Pro
            900                 905                 910
Ala Gln Leu Gly Gly Leu Ser Asn Leu Gln Tyr Ser Arg Leu Tyr Thr
        915                 920                 925
Arg Asn Ile Gly Asp Pro Gly Thr Thr Ala Phe Ala Glu Ile Lys Arg
    930                 935                 940
Leu Glu Ala Val Gly Leu Leu Ser Pro Ser Ile Met Thr Asn Ile Leu
945                 950                 955                 960
Thr Arg Pro Pro Gly Asn Gly Asp Trp Ala Ser Leu Cys Asn Asp Pro
                965                 970                 975
Tyr Ser Phe Asn Phe Glu Thr Val Ala Ser Pro Asn Ile Val Leu Lys
            980                 985                 990
Lys His Thr Gln Arg Val Leu Phe Glu Thr Cys Ser Asn Pro Leu Leu
        995                 1000                1005
Ser Gly Val His Thr Glu Asp Asn Glu Ala Glu Glu Lys Ala Leu Ala
    1010                1015                1020
Glu Phe Leu Leu Asn Gln Glu Met Ile His Pro Arg Val Ala His Ala
1025                1030                1035                1040
Ile Met Glu Ala Ser Ser Val Gly Arg Arg Lys Gln Ile Gln Gly Leu
                1045                1050                1055
Val Asp Thr Thr Asn Thr Val Ile Lys Ile Ala Leu Thr Arg Arg Pro
            1060                1065                1070

Leu Gly Ile Lys Arg Leu Met Arg Ile Val Asn Tyr Ser Ser Met His
        1075                1080                1085
Ala Met Leu Phe Arg Asp Asp Val Phe Ser Ser Asn Arg Ser Asn His
    1090                1095                1100
Pro Leu Val Ser Ser Asp Met Cys Ser Gln Thr Leu Ala Asp Tyr Ala
1105                1110                1115                1120
Arg Asn Arg Ser Trp Ser Pro Leu Thr Gly Gly Arg Lys Ile Leu Gly
                1125                1130                1135
Val Ser Asn Pro Asp Thr Ile Glu Leu Val Glu Gly Glu Ile Leu Ser
            1140                1145                1150
Val Ser Gly Gly Cys Thr Arg Cys Asp Ser Gly Asp Glu Gln Phe Thr
        1155                1160                1165
Trp Phe His Leu Pro Ser Asn Ile Glu Leu Asn Asp Asp Thr Ser Lys
    1170                1175                1180
Asn Pro Pro Met Arg Val Pro Tyr Leu Gly Ser Lys Thr Gln Glu Arg
1185                1190                1195                1200
Arg Ala Ala Ser Leu Ala Lys Ile Ala His Met Ser Pro His Val Lys
                1205                1210                1215
Ala Ala Leu Arg Ala Ser Ser Val Leu Ile Trp Ala Tyr Gly Asp Asn
            1220                1225                1230
Glu Ile Asn Trp Thr Ala Ala Leu Lys Ile Ala Arg Ser Arg Cys Asn
        1235                1240                1245
Ile Ser Ser Glu Tyr Leu Arg Leu Leu Ser Pro Leu Pro Thr Ala Gly
    1250                1255                1260
Asn Leu Gln His Arg Leu Asp Asp Gly Ile Thr Gln Met Thr Phe Thr
1265                1270                1275                1280
Pro Ala Ser Leu Tyr Arg Val Ser Pro Tyr Ile His Ile Ser Asn Asp
                1285                1290                1295
Ser Gln Arg Leu Phe Thr Glu Glu Gly Val Lys Glu Gly Asn Val Val
            1300                1305                1310
Tyr Gln Gln Ile Met Leu Leu Gly Leu Ser Leu Ile Glu Ser Leu Phe
        1315                1320                1325
Pro Met Thr Thr Thr Lys Thr Tyr Asp Glu Ile Thr Leu His Leu His
    1330                1335                1340
Ser Lys Phe Ser Cys Cys Ile Arg Glu Ala Pro Val Ala Val Pro Phe
1345                1350                1355                1360
Glu Leu Leu Gly Val Ala Pro Glu Leu Arg Ala Val Thr Ser Asn Lys
                1365                1370                1375
Phe Met Tyr Asp Pro Ser Pro Val Ser Glu Gly Asp Phe Ala Arg Leu
            1380                1385                1390
Asp Leu Ala Ile Phe Lys Ser Tyr Glu Leu Asn Leu Glu Ser Tyr Ser
        1395                1400                1405
Thr Ile Glu Leu Met Asn Val Leu Ser Ile Ser Ser Gly Lys Leu Ile
    1410                1415                1420
Gly Gln Ser Val Val Ser Tyr Asp Glu Asp Thr Ser Ile Lys Asn Asp
1425                1430                1435                1440
Ala Ile Ile Val Tyr Asp Asn Thr Arg Asn Trp Ile Ser Glu Ala Gln
                1445                1450                1455
Asn Ser Asp Val Val Arg Leu Phe Glu Tyr Ala Ala Leu Glu Val Leu
            1460                1465                1470
Leu Asp Cys Ser Tyr Gln Leu Tyr Tyr Leu Arg Val Arg Gly Leu Asn
        1475                1480                1485
Asn Ile Val Leu Tyr Met Ser Asp Leu Tyr Lys Asn Met Pro Gly Ile
    1490                1495                1500
Leu Leu Ser Asn Ile Ala Ala Thr Ile Ser His Pro Val Ile His Ser
1505                1510                1515                1520
Arg Leu Asn Ala Val Gly Leu Val Asn His Asp Gly Ser His Gln Leu
                1525                1530                1535
Ala Asp Thr Asp Phe Ile Glu Met Ser Ala Lys Leu Leu Val Ser Cys
            1540                1545                1550
Thr Arg Arg Val Val Ser Gly Leu His Ala Gly Asn Lys Tyr Asp Leu
        1555                1560                1565
Leu Phe Pro Ser Val Leu Asp Asp Asn Leu Ser Glu Lys Met Leu Gln

1570                    1575                    1580
Leu Ile Ser Gln Leu Cys Cys Leu Tyr Thr Val Leu Phe Ala Thr Thr
1585                    1590                    1595                    1600
Arg Glu Ile Pro Lys Ile Arg Gly Leu Ser Ala Glu Glu Lys Cys Ser
                1605                    1610                    1615
Val Leu Thr Glu Tyr Leu Leu Ser Asp Ala Val Lys Pro Leu Leu Gly
            1620                    1625                    1630
Ser Glu Gln Val Ser Ser Ile Met Ser Pro Asn Ile Val Thr Phe Pro
        1635                    1640                    1645
Ala Asn Leu Tyr Tyr Met Ser Arg Lys Ser Leu Asn Leu Ile Arg Glu
    1650                    1655                    1660
Arg Glu Asp Arg Asp Thr Ile Leu Ala Leu Leu Phe Pro Gln Glu Pro
1665                    1670                    1675                    1680
Leu Leu Glu Phe Pro Leu Val Arg Asp Ile Gly Ala Arg Val Lys Asp
                1685                    1690                    1695
Pro Phe Thr Leu Gln Pro Ala Ala Phe Leu Gln Glu Leu Asp Leu Ser
            1700                    1705                    1710
Ala Pro Ala Arg Tyr Asp Ala Phe Thr Ile Ser Gln Ala His Ser Glu
        1715                    1720                    1725
His Ile Leu Pro Asn Pro Glu Glu Asp His Leu Val Arg Tyr Leu Phe
    1730                    1735                    1740
Arg Gly Ile Gly Thr Ala Ser Ser Ser Trp Tyr Lys Ala Ser His Leu
1745                    1750                    1755                    1760
Leu Ser Val Pro Glu Val Arg Cys Ala Arg His Gly Asn Ser Leu Tyr
                1765                    1770                    1775
Leu Ala Glu Gly Ser Gly Ala Ile Met Ser Leu Leu Glu Leu His Ile
            1780                    1785                    1790
Pro His Glu Thr Ile Tyr Tyr Asn Thr Leu Phe Ser Asn Glu Met Asn
        1795                    1800                    1805
Pro Pro Gln Arg His Phe Gly Pro Thr Pro Thr Gln Phe Leu Asn Ser
    1810                    1815                    1820
Val Val Phe Arg Asn Leu Gln Ala Glu Val Pro Cys Lys Asp Gly Phe
1825                    1830                    1835                    1840
Val Gln Glu Phe Arg Pro Leu Trp Arg Glu Asn Thr Glu Glu Ser Asp
                1845                    1850                    1855
Leu Thr Ser Asp Lys Ala Val Gly Tyr Ile Thr Ser Val Val Pro Tyr
            1860                    1865                    1870
Arg Ser Val Ser Leu Leu His Cys Asp Ile Glu Ile Pro Pro Gly Ser
        1875                    1880                    1885
Asn Gln Ser Leu Leu Asp Gln Leu Ala Thr Asn Leu Ser Leu Ile Ala
    1890                    1895                    1900
Met His Ser Val Lys Glu Gly Gly Val Val Ile Ile Lys Val Leu Tyr
1905                    1910                    1915                    1920
Ala Met Gly Tyr Tyr Phe His Leu Leu Met Asn Leu Phe Thr Pro Cys
                1925                    1930                    1935
Ser Thr Lys Gly Tyr Ile Leu Ser Asn Gly Tyr Ala Cys Arg Gly Asp
            1940                    1945                    1950
Met Glu Cys Tyr Leu Ile Phe Val Met Gly Tyr Leu Gly Gly Pro Thr
        1955                    1960                    1965
Phe Val His Glu Val Val Arg Met Ala Lys Thr Leu Val Gln Arg His
    1970                    1975                    1980
Gly Thr Leu Leu Ser Lys Ser Asp Glu Ile Thr Leu Thr Arg Leu Phe
1985                    1990                    1995                    2000
Thr Ser Gln Gln Gln Arg Val Thr Asp Ile Leu Ser Ser Pro Leu Pro
                2005                    2010                    2015
Arg Leu Met Lys Tyr Leu Arg Glu Asn Ile Asp Thr Ala Leu Ile Glu
            2020                    2025                    2030
Ala Gly Gly Gln Pro Val Arg Pro Phe Cys Ala Glu Ser Leu Val Ser
        2035                    2040                    2045
Thr Leu Thr Asp Met Thr Gln Thr Thr Gln Ile Ile Ala Ser His Ile
    2050                    2055                    2060
Asp Thr Val Ile Arg Ser Val Ile Tyr Met Glu Ala Glu Gly Asp Leu
2065                    2070                    2075                    2080

Ala Asp Thr Val Phe Leu Phe Thr Pro Tyr Asn Leu Ser Met Asp Gly
                2085                2090                2095
Lys Lys Arg Thr Ser Leu Lys Gln Cys Thr Arg Gln Ile Leu Glu Val
            2100                2105                2110
Thr Ile Leu Gly Leu Arg Val Lys Asn Leu Asn Lys Val Gly Asp Val
        2115                2120                2125
Ile Ser Leu Val Leu Arg Gly Met Ile Ser Leu Glu Asp Leu Leu Pro
    2130                2135                2140
Leu Arg Thr Tyr Leu Lys Cys Ser Thr Cys Pro Lys Tyr Leu Lys Ala
2145                2150                2155                2160
Val Leu Gly Ile Thr Lys Leu Lys Glu Met Phe Thr Asp Ala Ser Leu
                2165                2170                2175
Leu Tyr Leu Thr Arg Ala Gln Gln Lys Phe Tyr Met Lys Thr Ile Gly
            2180                2185                2190
Asn Ala Val Lys Gly Tyr Tyr Ser Asn Cys Asp Ser
        2195                2200

<210> 28
<211> 36
<212> DNA
<213> Artificial Sequence

<220>
<223> Primer

<400> 28
acgactcact ataggaccaa acagagaatc cgtgag                                36

<210> 29
<211> 35
<212> DNA
<213> Artificial Sequence

<220>
<223> Primer

<400> 29
ccgggaagat ccagggcact cttcttgcat gttac                                 35

<210> 30
<211> 35
<212> DNA
<213> Artificial Sequence

<220>
<223> Primer

<400> 30
gggcctgcct cactatggtg gtaacatgca agaag                                 35

<210> 31
<211> 35

<212> DNA
<213> Artificial Sequence

<220>
<223> Primer

<400> 31
tgcatgttac caccaatgtg tcattgtatc gcttg                                35

<210> 32
<211> 37
<212> DNA
<213> Artificial Sequence

<220>
<223> Primer

<400> 32
caagaaggga gatacgtaat atacaagcga tacaatg                              37

<210> 33
<211> 35
<212> DNA
<213> Artificial Sequence

<220>
<223> Primer

<400> 33
tcgcttgtat attacttgtt gtagcaaaga gcacc                                35

<210> 34
<211> 38
<212> DNA
<213> Artificial Sequence

<220>
<223> Primer

<400> 34
ggcctggatc ttcccattat gctgtctgta tacggtgc                             38

<210> 35
<211> 38
<212> DNA
<213> Artificial Sequence

<220>
<223> Primer

<400> 35
atgccatgcc gacccaccaa acaaagactt ggtgaatg                                38

<210> 36
<211> 24
<212> DNA
<213> Artificial Sequence

<220>
<223> Primer

<400> 36
cctatagtga gtcgtattaa tttc                                              24

<210> 37
<211> 20
<212> DNA
<213> Artificial Sequence

<220>
<223> Primer

<400> 37
cctggatctt cccgggtcgg                                                   20

<210> 38
<211> 22
<212> DNA
<213> Artificial Sequence

<220>
<223> Primer

<400> 38
gggtcggcat ggcatctcca cc                                                22

<210> 39
<211> 22
<212> DNA
<213> Artificial Sequence

<220>
<223> Primer

<400> 39
gggaagatcc aggcctatag tg                                                22

<210> 40
<211> 35

<212> DNA
<213> Artificial Sequence

<220>
<223> Primer

<400> 40
ctctagagtc gacccttctg ccaacatgtc ttccg                                35

<210> 41
<211> 35
<212> DNA
<213> Artificial Sequence

<220>
<223> Primer

<400> 41
gggaagcggc cgcccgtcgg tcagtatccc cagtc                                35

<210> 42
<211> 37
<212> DNA
<213> Artificial Sequence

<220>
<223> Primer

<400> 42
ctctagagtc gaccccagag tgaagatggc caccttc                              37

<210> 43
<211> 35
<212> DNA
<213> Artificial Sequence

<220>
<223> Primer

<400> 43
gggaagcggc cgcccgtagt agtgatcagc cattc                                35

<210> 44
<211> 35
<212> DNA
<213> Artificial Sequence

<220>
<223> Primer

<400> 44
ctctagagtc gacccgggta ggacatggcg ggctc                                35

<210> 45
<211> 37
<212> DNA
<213> Artificial Sequence

<220>
<223> Primer

<400> 45
gggaagcggc cgccctgcct ttaagagtca cagttac                              37

## Description

This invention relates to recombinant Newcastle Disease Viruses (NDV), which have been demonstrated to possess significant oncolytic activity against mammalian cancers, especially selected from the specific cancers as described herein and/or mentioned in the claims. The invention provides the development of novel and improved oncolytic agents through the use of genetic engineering, including the transfer of genes across species boundaries in order to produce improved genetically modified viruses carrying these transgenes.

### Background

NDV is known as an oncolytic virus that is a virus for use in oncological treatment, preferably in the treatment of human subjects in need thereof. A number of RNA viruses, including NDV, reovirus, measles virus, and vesicular stomatitis virus (VSV), are members of this novel class of viruses being exploited as potential oncolytic agents. These oncolytic viruses are characterized in inherently replicating selectively within and killing tumor cells while leaving non-tumor cells unharmed. Accordingly these oncolytic viruses offer an attractive new tool for cancer therapy.

NDV derives its name from the site of the original outbreak in chickens at a farm near Newcastle-upon-Tyne in England in 1926. The virus is an economically important pathogen in multiple avian species and it is endemic in many countries. NDV is a member of the Avulavirus genus in the Paramyxoviridae family and is a non-segmented, negative-strand RNA virus, whose natural host range is limited to avian species; however, it is known to enter cells by binding to sialic acid residues present on a wide range of human and rodent cancer cells. The oncolytic property of NDV, i.e. to selectively replicate in and destroy tumor cells, while sparing normal cells, is believed to be based in part on defective antiviral responses in tumor cells. Normal cells, which are competent in launching an efficient antiviral response rapidly after infection, are able to inhibit viral replication before viral-mediated cell damage can be initiated. The sensitivity of NDV to interferon (IFN), coupled with defective IFN signalling pathways in tumor cells, provides one plausible mechanism whereby NDV can replicate exclusively within neoplastic tissue. The selective replication of NDV in human tumor cells may also be caused by several other mechanisms, including defects in activation of anti-viral signalling pathways, and activation of Ras signalling and/or expression of Rac1 (Schirrmacher, 2015, Expert Opin. Biol. Ther. 15:17 57-71).

Due to its tumor-specifity NDV is already under investigation as a safe clinical oncolytic virotherapy agent (Cassel et al., 1965, Cancer 1:863-888; Steiner et al., 2004, J. Clin. Oncol. 22:4272-4281; Schulze et al., 2009, Cancer Immunol. Immunother. 58:61-69; Csatary et al., 1999, Anticancer Res. 19:635-638; Pecora et al., 2002, J. Clin. Oncol. 20:2251-2266; Lorence et al., 2003, Curr. Opin. Mol. Ther. 5:618-624; Freeman et al., 2006, Mol. Ther. 13:221-228). The safety margin of NDV is a function of its inability to replicate and kill normal human cells.

Typically, an oncolytic NDV strain is defined as an NDV for use in oncological virotherapy, preferably in the treatment of human subjects in need thereof. Multiple preclinical model studies have shown significant anti-tumor activity of natural and recombinant oncolytic NDV strains after varying treatment modalities. Promising results were noted in preclinical models of glioblastoma multiforme, anaplastic astrocytoma, leukemia, lymphoma, melanoma, neuroblastoma, osteosarcoma, rhabdomyosarcoma, Ewing's sarcoma, fibrosarcoma, pheochromocytoma, colon carcinoma, lung carcinoma, prostate carcinoma, breast carcinoma, ovary carcinoma, gastric carcinoma, mesothelioma, renal cell carcinoma, and head and neck carcinoma. One typically useful oncolytic NDV that has been extensively explored for use in the treatment of humans, and for which an advantageous safety and efficacy profile has been established in human trials, is a strain named MTH-68/H (Csatary LK, et al., J Neurooncol. 2004 Mar-Apr;67(1-2):83-93). MTH-68/H therapy has been employed in a range of different tumors with success. MTH-68/H has been developed into a highly purified, lyophilized product, containing live, replication competent viral particles, grown to standardized titers. A Phase II clinical trial in humans was completed where the inhalatory mode of administration was used on patients suffering from a variety of advanced malignancies no longer responding to conventional cancer therapies. The study demonstrated relative efficacy, an overall improved quality of life, and a relatively benign side effect profile (Csatary LK, et al., Cancer detection and Prevention, 1993, 17(6):619-627). Success of treatment with MTH-68/H has also been shown in glioblastomas and anaplastic astrocytomas. Glioblastoma multiforme (GBM) is the most common primary brain tumor and by far the most aggressive form of glial tumors. This neoplasm has a poor prognosis, averaging six months to a year without therapy or about one-and-one-half years with conventional therapy. Four cases of advanced GBM and anaplastic astrocytoma were treated with the NDV viral product MTH-68/H after the conventional modalities of cancer therapy had failed. Results included survival rates of twelve and half to six years post-viral treatment for the surviving patients, and six years for one patient who has since died, after having discontinued treatment. Against all odds, these surviving patients returned to good quality of life and to a lifestyle that resemble their pre-morbid daily routines including giving birth to a healthy child and full professional activity. They have been able to enjoy good clinical health. These patients regularly received the MTH-68/H viral therapy for a number of years without interruption as a form of monotherapy once the classical modalities failed. The MRI and CT results have revealed an objective decrease in size of the tumors, in some cases the near total disappearance of the tumor mass.

As reviewed by Zamarin and Palese (Future Microbiol. 2012 Mar; 7(3): 347-367), when compared to other oncolytic agents in development such as poxvirus, HSV-1, adenovirus, measles, and reovirus vectors, NDV as an oncolytic virus exhibits several advantages over other viruses for use in oncological treatment.

The first advantage is that NDV is an avian pathogen. This avoids the problems of preexisting immunity that can neutralize virus infectivity and pathogenicity of the virus in humans. These potential problems exist when using vaccinia, HSV-1, adenovirus, and measles vectors. Based on serological studies it seems that about 96% of the human population is seronegative for NDV, which avoids the problem of pre-existing immunity. The actual level of seropositivity in today's population may be even lower due to low human exposure to NDV, as the outbreaks of which are primarily limited to farm settings. To attest for viral safety, administration of virulent strains to humans has been shown to result in only mild to moderate adverse effects, with mild conjunctivitis, laryngitis, and flu-like symptoms as the only reported side effects. Natural human infections with highly virulent (avian) strains have been reported in the literature in farmers and laboratory workers and have been limited to mild symptoms such as conjunctivitis and laryngitis.

The second advantage offered by using NDV as an oncolytic agent is that NDV, similarly to other oncolytic viruses, possesses strong immunostimulatory properties that are the basis for oncolytic therapy being considered an immunotherapy. These properties include the induction of type I IFN and chemokines, upregulation of MHC and cell adhesion molecules, and facilitation of adhesion of lymphocytes and antigen-presenting cells (APCs) through expression of viral glycoproteins on the surface of infected cells. These properties have been shown to generate effective anti-tumor immune responses, which may persist long after clearance of the primary viral infection.

A third advantage is that the NDV genome has the plasticity to enable the incorporation and stable expression of foreign genes of relatively large size. Since these viruses replicate in the cytoplasm of the cell and not in the nucleus, an unintended integration of the foreign gene into the host genome is avoided. Such an unintended integration of foreign genes carried by an oncolytic agent into the host genome could be a safety problem with some DNA oncolytic vectors. Moreover, the absence of homologous RNA recombination ensures that foreign genes incorporarted in and expressed from the NDV genome are stable for many serial passages in cell culture and in tumor cells.

Lastly, the ubiquitous nature of the NDV receptor allows for utilization of the virus against a wide variety of tumor types. The specificity of NDV for cancer cells due to their defects in antiviral pathways ensures viral safety.

Nevertheless, the full view of all clinical data generated to date in different trials of different NDV strains shows that there is much need for improvement in terms of percentage responders and magnitude of therapeutic effect. Also it is desired to further improve the viral safety. This need for improvement has spurred efforts to design improved recombinant NDV strains, which is the subject of the present patent application.

Similar to other paramyxoviruses in its family, the 15186, 15192 or 15198 nucleotide(nt)-long negative single-strand RNA genome of NDV encodes six genes including the nucleocapsid protein (NP), phosphoprotein (P), matrix protein (M), fusion protein (F), hemagglutinin-neuraminidase (HN), and RNA-dependent RNA polymerase (L) (Lamb R, Paramyxoviridae: the viruses and their replication. In: Knipe D, editor. Fields Virology, Lippincott Williams & Wilkins; 2007, pp. 1449-1496). The genes are separated by junction sequences that consist of three elements, known as gene start (GS), intergenic (IG), and gene-end (GE) motifs, which regulate mRNA transcription. In the P gene, a unique RNA editing mechanism adds non-templated G residues, resulting in the expression of V and W proteins that are colinear to the P protein in the amino-terminal end. The genomic RNA is bound in a ribonucleotide protein complex (RNP) consisting of NP, P, and L proteins and is surrounded by a lipid envelope containing three virus glycoprotein spikes, HN, M and F. An important factor that influences the extent of virulence of NDV in birds is the cleavage site of the F protein.

Pathogenic classification and virulence of NDV strains in birds generally correlates with their oncolytic properties in human cancer cells. Velogenic (high virulence) strains produce severe respiratory and nervous system signs, spread rapidly through chicken flocks, and can cause up to 90% mortality. Mesogenic (intermediate virulence) strains cause coughing, affect egg production and quality, and can result in up to 10% mortality. Lentogenic (low virulence) strains produce only mild symptoms with little if any mortality. Correspondingly, velogenic strains can efficiently carry out multicycle replication in multiple human cancer cells with effective and efficient cell lysis, lentogenic strains are more attenuated due to lack of activation of the F0 protein, and mesogenic strains convey intermediate effects. On the basis of these observations in human cancers, NDV strains have been classified as either lytic or non-lytic, with velogenic and mesogenic viruses being lytic (high and low respectively), and lentogenic viruses in general being non-lytic. Early studies demonstrated that the lytic abilities of lentogenic NDV strains could be enhanced by the introduction of a polybasic cleavage site into their F proteins (Peeters et al, J. Virol. 1999; 73:5001-5009). Other NDV proteins including NP, P, V, HN, and L have also been shown to be implicated in virulence in birds. A deletion (18nt) introduced in the NP gene (Mebatsion T et al., J Virol. 2002 Oct; 76 (20):10138-46) resulted in the deletion of NP residues 443-460. The resulting mutant NDV propagated in embryonated specific-pathogen-free (SPF) chicken eggs to a level fully comparable to that of the parent virus.

In addition, a B-cell epitope of the S2 glycoprotein of murine hepatitis virus (MHV) was inserted in-frame. Recombinant NDV viruses properly expressing the introduced MHV epitope were successfully generated, demonstrating that the NP can be used as the insertion point in the NDV genome to insert foreign or transgenic sequences to be co-expressed with NDV during virus infection.

Several studies showed that type I IFN antagonist activity of NDV V protein is associated with pathogenicity in birds. In recombinant viruses possessing attenuating mutations or deletions in the V protein or in viruses expressing no or lower levels of V protein decreased pathogenicity in birds was observed (Huang et al., 2003, J Virol. 77:8676-8685; Mebatsion et al., 2001, J Virol. 75:420-428; Qiu et al., 2016, PLoS ONE 11(2): e0148560. doi: 10.1371/journal.pone.0148560; Elankumaran et al., 2010, J Virol. 84:3835-3844; Jang et al., 2010, Appl Microbiol Biotechnol. 85:1509-1520; Alamares et al., 2010, Virus Res. 147:153-157; Park et al., 2003, J Virol.77:9522-9532.). It was found to be sufficient to introduce a small (6 nt) deletion in the P gene to abolish expression of the V protein (Mebatsion et al., 2001).

Recently, two studies showed the role of the polymerase complex in viral pathogenesis, with L protein contributing to pathogenicity of the mesogenic Beaudette C strain, and NP, P, and L proteins contributing to pathogenicity of the velogenic Herts strain (Rout & Samal, 2008, J Virol. 82:7828-7836; Dortmans et al., 2010, J Virol. 84:10113-10120). In addition, several studies noted the contribution of the HN protein to virulence in birds (Römer-Oberdörfer et al., 2006, Avian Dis. 50:259-263; Paldurai et al., 2014, J Virol. 88: 8579-8596.) The HN (hemagglutinin-neuraminidase) protein of NDV is a multifunctional protein with receptor-recognition, hemagglutinin (HA) and receptor-destroying neuraminidase (NA) activities associated with the virus. HN is thought to possess both the receptor recognition of sialic acid at the termini of host glycoconjugates and the neuraminidase activity to hydrolyze sialic acid from progeny virion particles in order to prevent viral self-aggregation. It also recognizes sialic acid-containing receptors on cell surfaces and promotes the fusion activity of the F protein, thereby allowing the virus to penetrate the cell surface. Thus, the HN protein plays a critical role in viral infection of birds.

A recombinant NDV vector virus can be provided by a reverse genetics (rg) system, which has been known since 1999 (Peeters et al., 1999, J. Virol. 73:5001-5009). This system allows desired modification and engineering of NDV genomes. Indeed, various reports have shown the successful use of recombinant NDV vectors engineered to express various transgenes, i.e. foreign genes incorporated in the genome of the virus, with the goal of improving viral oncolytic efficacy (Vigil et al. Cancer Res. 2007, 67:8285-8292; Janke et al., 2007, Gene Ther. 14:1639-1649). However, it should be noted that the replication capability of a recombinant NDV expressing a transgene may be hampered in comparison with its parental NDV strain not expressing the transgene. As discussed above, the viral F protein of NDV is responsible for viral fusion with the cell membrane and for viral spread from host cell to host cell via formation of syncytia. The presence of the multi-basic amino acid cleavage site in the F protein enables protein cleavage and activation by a broad range or proteases and is known to be a determinant of virulence in velogenic NDV strains. In order to increase the oncolytic potency of a highly attenuated lentogenic Hitchner B1 NDV strain, a polybasic cleavage site was introduced into the F protein to generate rNDV/F3aa with mutated F protein. Altomonte et al. (Mol Ther. 2010 18:275-84) reported an oncolytic NDV vector virus with the mutated F-protein rNDV/F(L289A), harboring an L289A mutation within the F protein, which is required for virus entry and cell-cell fusion. This rNDV/F3aa(L289A) virus with mutated F protein showed further enhanced fusion and cytotoxicity of hepatocellular carcinoma (HCC) cells in vitro, as compared with a rNDV/F3aa control virus. In vivo administration of rNDV/F3aa(L289A), via hepatic arterial infusion in immune-competent Buffalo rats bearing multifocal, orthotopic liver tumors resulted in tumor-specific syncytia formation and necrosis, with no evidence of toxicity to the neighboring hepatic parenchyma, which translated to a 20% prolongation of survival time relative to treatment with the original rNDV/F3aa virus.

Due to compelling preclinical data implicating oncolytic NDV as an ideal candidate for cancer therapy, phase I and II clinical trials have been initiated. Several trials have shown that NDV is a safe and potentially therapeutically useful therapeutic agent, with no reports of significant adverse effects in patients beyond conjunctivitis or mild flu-like symptoms (Csatary et al., 1999, Anticancer Res. 19:635-638; Pecora et al., 2002, J. Clin. Oncol. 20:2251-2266; Lorence et al., 2003, Curr. Opin. Mol. Ther. 5, 618-624; Freeman et al., 2006, Mol. Ther. 13:221-228). Although the results of these clinical trials have been encouraging, the extent of clinical responses has not been strong and robust enough to consider bringing one of these initial NDV strains into advanced clinical development. Thus, strategies for improving therapeutic effectiveness while maintaining an acceptable safety profile of oncolytic NDV strains are needed. Thus there remains a clear need for improvement in therapeutic outcome by providing improved NDV strains, in particular NDV strains with enhanced killing effect without affecting - or even improving - its safety toward normal cells.

### Description of the present invention

In a first aspect the present invention provides recombinant Newcastle disease virus or NDV strains. A recombinant NDV according to the present invention carries as a foreign gene at least one gene selected from:
- A gene encoding an antibody directed to the protein PD-L1 or an antigen-binding part directed to the protein PD-L1 (anti-PD-Ll), such as Atezolizumab or a variant or an antigen-binding part thereof.
- A gene encoding an antibody directed to the growth factor protein VEGF-A or an antigen-binding part directed to the growth factor protein VEGF-A (anti-VEGF-A), such as Bevacizumab or a variant or an antigen-binding part thereof.
- A gene encoding an antibody directed to the killer-cell immunoglobulin-like receptors (KIRs), in particular KIR2DL1/2L3, thereby regulating the killing function of the cells expressing the receptors, or an antigen-binding part directed to killer-cell immunoglobulin-like receptors (KIRs), in particular KIR2DL1/2L3, (anti-KIR).
- A gene encoding an antibody directed to and inhibiting LAG-3 or an antigen-binding part directed to directed to and inhibiting LAG-3 (anti-LAG-3). LAG-3 is a protein, which is present on the surface of T-cells. When the antibody or its antigen-binding part is bound to LAG-3 on T-cells, the T-cells are stimulated to attack cancer cells.
- A gene encoding an antibody directed to NKG2A or an antigen-binding part directed to protein NKG2A (anti- NKG2A). Binding of the antibody to NKG2a prevents the binding of NKG2A to its ligand human leukocyte antigen-E (HLA-E), which induces an immune response against the cancer cells leading to their destruction.
- A gene encoding an antibody directed to the glucocorticoid-induced TNF-superfamily receptor (GITR), thereby blocking the interaction of GITR with its ligand, or an antigen-binding part directed to the glucocorticoid-induced TNF-superfamily receptor (GITR) (anti-GITR).
- A gene encoding an antibody directed to and activating the protein OX40 (also known as CD134 or TNFRSF4), thereby increasing the number of T-cells, or an antigen-binding part directed to protein OX40 (anti-OX40).
- A gene encoding a membrane protein which is the receptor for the proteins CD28 and CTLA-4, and which membrane protein is involved in the costimulatory signal essential for T-lymphocyte activation, or a part thereof which is the receptor for the proteins CD28 and CTLA-4, and which membrane protein is involved in the costimulatory signal essential for T-lymphocyte activation.
- A gene encoding a human interleukin 12 (hIL-12) protein, or a part thereof.
- A gene encoding a green fluorescent protein, which can be used for the localization and monitoring of cells, in particular cancer cells, or a part thereof.
- Any combination of these genes, parts or variants of these genes or parts thereof.

The foreign gene or part or the respective variant is/are expressed in the recombinant NDV strain of the present invention, when the virus is replicating in a suitable host or host cell. An expression of a gene means that the nucleic acid information encoded by the gene is translated in a respective amino acid sequence, which is the primary sequence for building up the respective protein or gene product. By way of example, the gene product of the HN gene is the HN protein.

Within the present invention the foreign gene carried by the recombinant NDV encodes in one embodiment an antibody or an antigen-binding part of the antibody directed to the protein PD-L1 (anti-PD-L1). According to the present invention the antibody is in a particularly preferred embodiment Atezolizumab, an antigen-binding part of Atezolizumab, a variant of Atezolizumab or a variant of an antigen-binding part of Atezolizumab.

According to another embodiment of the present invention the foreign gene carried by the recombinant NDV encodes an antibody or an antigen-binding part of the antibody directed to the growth factor protein VEGF-A (anti-VEGF-A). VEGF-A is a growth factor protein that stimulates angiogenesis in a variety of diseases, especially in cancer. Thus the antibody is capable of blocking angiogenesis. According to the present invention the antibody is in a particularly preferred embodiment Bevacizumab, an antigen-binding part of Bevacizumab, a variant of Bevacizumab or a variant of an antigen-binding part of Bevacizumab.

According to another embodiment of the present invention the foreign gene carried by the recombinant NDV encodes an antibody directed to killer-cell immunoglobulin-like receptors (KIRs), in particular KIR2DL1/2L3, or an antigen-binding part directed to killer-cell immunoglobulin-like receptors (KIRs), in particular KIR2DL1/2L3. According to the present invention the antiboby or antigen-binding part thereof is in a particularly preferred embodiment Lirilumab, an antigen-binding part of Lirilumab, a variant of Lirilumab or a variant of an antigen-binding part of Lirilumab. They work by regulating the killing function of the cells expressing the receptors.

According to another embodiment of the present invention the foreign gene carried by the recombinant NDV encodes an antibody directed to and inhibiting LAG-3, a protein, which is present on the surface of T-cells, or an antigen-binding part directed to and inhibiting LAG-3. According to the present invention the antiboby or antigen-binding part thereof is in a particularly preferred embodiment Relatlimab, an antigen-binding part of Relatlimab, a variant of Relatlimab or a variant of an antigen-binding part of Relatlimab. When bound to LAG-3 on T-cells, the T-cells are stimulated to attack cancer cells.

According to another embodiment of the present invention the foreign gene carried by the recombinant NDV encodes an antibody directed to NKG2A, or an antigen-binding part directed to NKG2A. According to the present invention the antiboby or antigen-binding part thereof is in a particularly preferred embodiment Monalizumab, an antigen-binding part of Monalizumab, a variant of Monalizumab or a variant of an antigen-binding part of Monalizumab. Binding of the antibody or its antigen-binding part to NKG2A prevents the binding of NKG2A to its ligand human leukocyte antigen-E (HLA-E), which induces a immune response against the cancer cells leading to their destruction.

According to another embodiment of the present invention the foreign gene carried by the recombinant NDV encodes an antibody directed to the glucocorticoid-induced TNF-superfamily receptor (GITR), or an antigen-binding part directed to the glucocorticoid-induced TNF-superfamily receptor (GITR). According to the present invention the antiboby or antigen-binding part thereof is in a particularly preferred embodiment TRX518, an antigen-binding part of TRX518, a variant of TRX518 or a variant of an antigen-binding part of TRX518. The antibody or its antigen-binding part acts by blocking the interaction of GITR with its ligand.

According to another embodiment of the present invention the foreign gene carried by the recombinant NDV encodes an antibody directed to and activating the protein OX40, or an antigen-binding part directed to and activating the protein OX40. According to the present invention the antiboby or antigen-binding part thereof is in a particularly preferred embodiment BMS 986178, an antigen-binding part of BMS 986178, a variant of BMS 986178 or a variant of an antigen-binding part of BMS 986178. The antibody or its antigen-binding part is capable of increasing the number of T-cells.

According to another embodiment of the present invention the foreign gene carried by the recombinant NDV encodes a membrane protein which is the receptor for the proteins CD28 and CTLA-4, and which membrane protein is involved in the costimulatory signal essential for T-lymphocyte activation, or a part thereof which is the receptor for the proteins CD28 and CTLA-4, and which membrane protein is involved in the costimulatory signal essential for T-lymphocyte activation. According to the present invention the membrane proteine or part thereof is in a particularly preferred embodiment CD80 (cluster of differentiation 80), a part of CD80, a variant of CD80 or a variant of a part of CD80.

According to another embodiment of the present invention the foreign gene carried by the recombinant NDV encodes a human interleukin 12 (hIL-12), or a part thereof. According to the present invention the proteine or part thereof is in a particularly preferred embodiment (ns)hIL-12, a part of (ns)hIL-12, a variant of (ns)hIL-12 or a variant of a part of (ns)hIL-12. As used herein "(ns)" is intended to mean "non secreting". That is the interleukin is preferred to be a non secreting human interleukin 12. A non secreting interleukin may be advantageous over a normal interleukin, as it may prevent that there is an uncontrolled immune response, for example in the form of a massive cytokine secretion, triggered by administration of the recombinant NDV strain.

According to another embodiment of the present invention the foreign gene carried by the recombinant NDV encodes a green fluorescent protein, which can be used for the localization and monitoring of cells, in particular cancer cells, or a part thereof. According to the present invention the proteine or part thereof is in a particularly preferred embodiment enhanced green fluorescent protein (eGFP), a part of enhanced green fluorescent protein (eGFP), a variant of enhanced green fluorescent protein (eGFP) or a variant of a part of eGFP.

In another embodiment of the present invention a recombinant NDV comprises in its viral genome at least two foreign genes or parts or variants thereof, which foreign genes are selected from any one of the above mentioned foreign genes. In one embodiment the foreign genes are selected from a gene encoding an antibody or an antigen-binding part of the antibody directed to the protein PD-L1 or a part thereof, preferably Atezolizumab, an antigen-binding part thereof or a variant of Atezolizumab or its antigen-binding part, and a gene encoding an antibody or an antigen-binding part of the antibody directed to the growth factor protein VEGF-A or a part thereof, preferably Bevacizumab, an antigen-binding part thereof or a variant of Bevacizumab or its antigen-binding part.

The above mentioned group of foreign genes will also be referred to the following as "the foreign genes of the present invention". This term is intended to also include any parts of these genes as well as the particularly preferred foreign genes (encoding Atezolizumab, Bevacizumab, Lirilumab, Relatlimab, Monalizumab, TRX518, BMS 986178, CD80, (ns)hIL-12 or eGFP) and parts or variants therof. Even though in the following the present invention will be described in particular with reference to the foreign genes encoding Atezolizumab or Bevacizumab, it should be understood that the respective teaching and all disclosed preferred embodiments of such Atezolizumab or Bevacizumab carrying NDV strains also apply to the other foreign genes, parts or variants.

A "variant" of a starting nucleic acid is a nucleic acid that comprises a nucleic acid sequence different from that of the starting nucleic acid. Generally, a variant will possess at least 75 % sequence identity, preferably at least 90 % sequence identity, more preferably at least 95 % sequence identity, and most preferably at least 98 % sequence identity with the native nucleic acid. Variants of a nucleic acid may be prepared by introducing appropriate nucleotide changes into the nucleic acid. Such variants include, for example, deletions from, and/or insertions into and/or substitutions of residues within the nucleic acid sequence of the gene of interest. Any combination of deletion, insertion, and substitution is possibly made to arrive at the final construct, provided that the final construct possesses the desired characteristics. Methods for generating nucleic acid sequence variants are known to the skilled person.

A "variant" of a starting polypeptide or protein, e.g. an antibody or another protein, is a polypeptide or protein that comprises an amino acid sequence different from that of the starting polypeptide or protein. Generally, a variant will possess at least 75 % sequence identity, preferably at least 90 % sequence identity, more preferably at least 95 % sequence identity, and most preferably at least 98 % sequence identity with the native polypeptide or protein. Variants of a polypeptide or a proteine may be prepared by introducing appropriate nucleotide changes into the nucleic acid encoding the polypeptide or protein. Such variants include, for example, deletions from, and/or insertions into and/or substitutions of residues within the amino acid sequence of the polypeptide/proteine of interest. Any combination of deletion, insertion, and substitution is possibly made to arrive at the final construct, provided that the final construct possesses the desired characteristics. The amino acid changes also may alter post-translational processes of the polypeptide, such as changing the number or position of glycosylation sites. Methods for generating amino acid sequence variants of polypeptides are known to the skilled person.

Percentage sequence identity is determined after the best alignment of the sequence of interest with the respective reference sequence. The best alignment of the sequences may for example be produced by means of the Fitchet al., Proc. Natl. Acad. Sci. USA 80:1382-1386 (1983), version of the algorithm described by Needleman et al., J. Mol. Biol.48:443-453 (1970), after aligning the sequences to provide for best homology, by means of the similarity search method of Pearson and Lipman, Proc. Natl Acad. Sci. USA 85, 2444 (1988), or by means of computer programs which use these algorithms (in particular BLASTN in Wisconsin Genetics Software Package, Genetics Computer Group, 575 Science Drive, Madison, Wis.). The percentage identity is calculated by determining the number of identical positions between the two sequences being compared, dividing this number by the number of positions compared and multiplying the result obtained by 100 so as to obtain the percentage identity between these two sequences. Preferably, sequence identity of a nucleotide sequence is determined using BLASTN, preferably BLASTN in standard settings as provided by the website of the U.S. National Library of Medicine "https://blast.ncbi.nlm.nih.gov". Also preferably, sequence identity of an amino acid sequence is determined using BLASTP, preferably BLASTP in standard settings as provided by the website of the U.S. National Library of Medicine "https://blast.ncbi.nlm.nih.gov". It is further preferred that the sequence identity is calculated over the entire length of the respective sequence.

As used herein, the term "part" means a fragment of a starting nucleic acid or amino acid sequence, which fragment is a made up of a consecutive sequence of the nucleotides or amino acids of the starting sequence. The part or fragment preferably comprises at least 10, more preferably at least 20 and preferably up to 500 nucleotides or amino acids. In any case a part according to the present invention is a functional part. That is nucleic acid or proteine encoded by that nucleic acid must provide the desired function, e.g. an antigen-binding function or expression inhibiting function as specified herein, of the starting gene or protein.

In addition to carrying at least one foreign gene or a part or variant thereof, the recombinant NDV strains according to the present invention further comprise a mutation in the viral HN gene leading to a change in the amino acid sequence of the HN protein and providing the NDV with an increased replication capability in a cancer cell, preferably in a human cancer cell, as compared to an otherwise identical NDV not having the said mutation in the HN gene.

Preferably the mutation in the HN gene results in an unexpected beneficially increased replication capability of the recombinant NDV strain in cancer cells, preferably in human cancer cells, and more preferably results in a replication of said oncolytic NDV in a human cancer cell which is at least 2-fold higher, more preferably 2- to 10-fold higher, still more preferably 5- to 10-fold higher than an NDV parent strain not having said mutation in its viral HN gene. Preferably, said parent strain comprises or is an oncolytic NDV already having an advantageous safety and efficacy profile in humans, such as the NDV strain MTH-68/H.

According to a preferred embodiment the recombinant NDV strain comprises a mutated HN gene and the encoded hemagglutinin-neuramidase (HN proteine) with an amino acid substitution at position 277 to an amino acid with a hydrophobic side chain other than phenylalanine. Still more preferred is the amino acid phenylalanine (F) substituted to leucine (L) at amino acid position 277 of the HN gene. In a more preferred embodiment, the invention provides an oncolytic NDV derived from NDV strain MTH-68/H having this mutation in the HN gene and carrying at least one of the foreign genes or parts or functional analogs thereof as specified supra, said oncolytic NDV according to the invention having an unexpected beneficially increased replication capability allowing replication of said oncolytic NDV in a human cancer cell preferably up to at least 10-fold higher levels than the oncolytic NDV parent strain MTH-68/H, thereby maintaining the useful oncological safety and efficacy specifications of the parent virus and advantageously supplementing it with the increased replication capability of an oncolytic NDV and the ability to express within infected cancer cells at least one of the before mentioned foreign genes, which beneficialls influence the immunological response towards the cancer cells. Herein an oncolytic NDV having such a mutation is also identified as NDV^{F277L} to discern it from a parent NDV having a phenylalanine at amino acid position 277 of the HN gene.

A NDV strain derived from NDV strain MTH-68/H having a mutation in the HN gene product or HN protein at position 277, wherein the amino acid phenylalanine (F) is substituted to leucine (L) at position 277 is referred to herein also as NDV-Mut HN(F277L). The foreign gene or part or functional analog thereof carried by this strain is referred to as Atezolizumab, Bevacizumab, Lirilumab, Relatlimab, Monalizumab, TRX518, BMS 986178, CD80, (ns)hIL-12, or eGFP in this shortcut for the strain.

Within the shortcut for the recombinant NDV strains as used herein a reference to e.g. Atezolizumab is intended to comprise the complete antibody, antigen-binding parts, and variants of the antibody or of antigen-binding parts, but also encompasses the more general form of an anti-PD-L1-antibody and antigen-binding parts of this antibody. A reference to Bevacizumab is again intended to comprise the complete antibody, antigen-binding parts and variants of the antibody or antigen-binding parts, but also the more general form of an antibody to the growth factor protein VEGF-A, and antigen-binding parts of this antibody. A reference to Lirilumab is intended to comprise the complete antibody, antigen-binding parts, and variants of the antibody or of antigen-binding parts, but also encompasses the more general form of an anti-KIR-antibody and antigen-binding parts of this antibody. A reference to Relatlimab is intended to comprise the complete antibody, antigen-binding parts, and variants of the antibody or of antigen-binding parts, but also encompasses the more general form of an anti-LAG-3-antibody and antigen-binding parts of this antibody. A reference to Monalizumab is intended to comprise the complete antibody, antigen-binding parts, and variants of the antibody or of antigen-binding parts, but also encompasses the more general form of an anti-NKG2A-antibody and antigen-binding parts of this antibody. A reference to TRX518 is intended to comprise the complete antibody, antigen-binding parts, and variants of the antibody or of antigen-binding parts, but also encompasses the more general form of an anti-GITR-antibody and antigen-binding parts of this antibody. A reference to BMS 986178 is intended to comprise the complete antibody, antigen-binding parts, and variants of the antibody or of antigen-binding parts, but also encompasses the more general form of an anti-OX40-antibody and antigen-binding parts of this antibody.

A reference to CD80 is intended to comprise the complete protein, parts and variants of the protein, as well as the more general form of a membrane protein which is the receptor for the proteins CD28 and CTLA-4, and which membrane protein is involved in the costimulatory signal essential for T-lymphocyte activation, or a respective part thereof. A reference to (ns)hIL-12 is intended to comprise the complete protein, parts and variants of the protein, as well as the more general form of a human interleukin 12 (hIL-12), or a respective part thereof. A reference to eGFP is intended to comprise the complete protein, parts and variants of the protein, as well as the more general form of a green fluorescent protein, or a respective part thereof.

That is a recombinant NDV strain referred to as "NDV-Mut HN(F277L)-Atezolizumab" is a NDV strain derived from NDV strain MTH-68/H having a mutation in the HN gene resulting in an amino acid exchange at position 277, wherein the amino acid phenylalanine (F) is substituted to leucine (L) at position 277, and carrying as a foreign gene a gene encoding an antibody that is directed to the protein PD-L1, preferably Atezolizumab or a variant thereof. A recombinant NDV strain referred to as "NDV-Mut HN(F277L)-Bevacizumab" is a NDV strain derived from NDV strain MTH-68/H having a mutation in the HN gene resulting in an amino acid exchange at position 277, wherein the amino acid phenylalanine (F) is substituted to leucine (L) at position 277, and carrying as a foreign gene a gene encoding an antibody that is directed to the growth factor protein VEGF-A, preferably Bevacizumab or a variant thereof. The NDV strains NDV-Mut HN(F277L)-Atezolizumab and NDV-Mut HN(F277L)-Bevacizumab are provided by the present invention in a preferred embodiment. Beside these both NDV-strains the following NDV strains are provided by the present invention in a preferred embodiment:
- NDV-Mut HN(F277L)-Lirilumab
- NDV-Mut HN(F277L)-Relatlimab
- NDV-Mut HN(F277L)-Monalizumab
- NDV-Mut HN(F277L)-TRX518
- NDV-Mut HN(F277L)-BMS 986178
- NDV-Mut HN(F277L)-CD80
- NDV-Mut HN(F277L)-(ns)hIL-12
- NDV-Mut HN(F277L)-eGFP

Within the meaning of the present invention the term "Newcastle disease virus" or "NDV" includes all known velogenic, mesogenic and lentogenic NDV strains. According to the present invention mesogenic and lentogenic strains are preferred. Still more preferred is the NDV derived from NDV strain MTH-68/H.

Within the meaning of the present invention "derived from NDV strain MTH-68/H" is intended to mean that the derived NDV strain is encoded by a viral genome or comprises a viral genome having a nucleic acid sequence with a sequence identity of at least 75 %, particularly at least 90 %, more particularly at least 95 % to the nucleic acid sequence of the NDV strain MTH-68/H. In one embodiment the sequence identity is at least 99 % or 100 %. Any foreign genes comprised in the recombinant NDV strains are not considered in the sequence alignment, and the reference sequence for the sequence alignment is that of SEQ ID No. 1 of the sequence listing. A sequence variation is for example the substitution, insertion or deletion of at least one nucleotide, preferably the substitution, insertion or deletion of up to 20 nucleotides, still more preferably of up to 15 nucleotides. Any combination of substitution, insertion and deletion is also comprised by that definition, provided that the final construct possesses the desired characteristics. The variation may result in at least one amino acid substitution, wherein each amino acid substitution can be a conservative or a non-conservative amino acid substitution.

It has been surprisingly shown that the mutation in the HN gene results in a beneficially improved replication capability of the respective strain as compared to an otherwise identical NDV not having said mutation in the HN gene. The beneficially increased replication rate of the NDV not only results in higher number of virus particles capable of infecting and destroying cancer cells, but also results in a higher expression of the foreign gene carried by the said recombinant NDV strains. As explained earlier, the respective gene products of the foreign gene results in an improved immunological response and thus an improved destruction of cancer cells.

In a preferred embodiment of the present invention the recombinant and mutated NDV strains comprise in addition to the above described mutation in the HN gene, a mutation in the M gene and the thus encoded matrix protein. Still more preferred the mutated M gene encodes a matrix protein with an amino acid substitution at position 165 to an amino acid with an aromatic side chain, wherein still more preferred the amino acid glycine (G) at amino acid position 165 of the M gene is substituted to the amino acid tryptophane (W).

NDV strains comprising both a mutation in the HN gene and the M gene, as described supra, have been shown as being particularly suitable to infect cancer cells and to provide an enhanced replication rate within the cancer cells. Thus, according to one embodiment of the present invention a recombinant NDV strain comprises as a foreign gene at least one gene selected from the group of the foreign genes of the present invention, as defined above. In one embodiment of the present invention a recombinant NDV strain comprises as a foreign gene at least one a gene encoding an antibody directed to the protein PD-L1 or a part thereof, preferably Atezolizumab, a part thereof or a variant of Atezolizumab or its part, a gene encoding an antibody directed to the growth factor protein VEGF-A or a part thereof, preferably Bevacizumab, a part thereof or a variant of Bevacizumab or its part, and any combination of these genes or variants thereof, and has a mutation both in the HN gene, preferably resulting in a substitution of the amino acid phenylalanine (F) to leucine (L) at position 277 of the HN gene product, and a mutation in the M gene, preferably resulting in a substitution of the amino acid glycine (G) to the amino acid tryptophane (W) at position 165 of the M gene product. As explained supra, other substitutions at these two amino acid positions are also comprised within the present invention.

A NDV strain derived from NDV strain MTH-68/H having a mutation in the HN gene, wherein the amino acid phenylalanine (F) is substituted to leucine (L) at position 277 of the HN gene product, and having a mutation in the M gene, wherein the amino acid glycine (G) is substituted to the amino acid tryptophane (W) at position 165 of the M gene product is herein referred to also as NDV-Mut HN(F277L)/M(G165W) or MutHu or NDV-NoThaBene-1. The foreign gene or part or variant thereof carried by this strain is referred to as Atezolizumab, Bevacizumab, Lirilumab, Relatlimab, Monalizumab, TRX518, BMS 986178, CD80, (ns)hIL-12 or eGFP. Within these shortcuts for the recombinant NDV strains a reference to the respective foreign gene is intended to have the meaning as already defined above. The following NDV strains are provided by the present invention in a particularly preferred embodiment:
- NDV-Mut HN(F277L)/M(G165W)-Atezolizumab,
- NDV-Mut HN(F277L)/M(G165W)-Bevacizumab,
- NDV-Mut HN(F277L)/M(G165W)-Lirilumab,
- NDV-Mut HN(F277L)/M(G165W)-Relatlimab,
- NDV-Mut HN(F277L)/M(G165W)-Monalizumab,
- NDV-Mut HN(F277L)/M(G165W)-TRX518,
- NDV-Mut HN(F277L)/M(G165W)-BMS 986178,
- NDV-Mut HN(F277L)/M(G165W)-CD80,
- NDV-Mut HN(F277L)/M(G165W)-(ns)hIL-12,
- NDV-Mut HN(F277L)/M(G165W)-eGFP.

A recombinant NDV strain referred to as "NDV-Mut HN(F277L)/M(G165W)-Atezolizumab" is a NDV strain derived from NDV strain MTH-68/H having a mutation in the HN gene at amino acid position 277, wherein the amino acid phenylalanine (F) is substituted to leucine (L) at position 277, and having a mutation in the M gene, wherein the amino acid glycine (G) is substituted to the amino acid tryptophane (W) at position 165 of the M gene product, and carrying as a foreign gene a gene encoding an antibody that is directed to the protein PD-L1, preferably Atezolizumab, or a variant thereof. A recombinant NDV strain referred to as "NDV-Mut HN(F277L)/M(G165W)-Bevacizumab" is a NDV strain derived from NDV strain MTH-68/H having a mutation in the HN gene at amino acid position 277, wherein the amino acid phenylalanine (F) is substituted to leucine (L) at amino acid position 277, and having a mutation in the M gene, wherein the amino acid glycine (G) is substituted to the amino acid tryptophane (W) at position 165 of the M gene product, and carrying as a foreign gene a gene encoding an antibody directed to the growth factor protein VEGF-A or a part thereof, preferably Bevacizumab or a variant thereof. The NDV strains NDV-Mut HN(F277L)/M(G165W)-Atezolizumab and NDV-Mut HN(F277L)/M(G165W)-Bevacizumab are provided by the present invention in a preferred embodiment.

In a preferred embodiment of the present invention the recombinant and mutated NDV strains comprise in addition to the above described mutation in the HN gene, at least one mutation in the F gene and the thus encoded fusion protein. Still more preferred the mutated F gene encodes a fusion protein with an amino acid substitution at amino acid position 117 to an amino acid with a hydroxylated side chain, preferably where phenylalanine (F) is substituted to serine (S) at position 117 of the F gene product, and/or with an amino acid substitution at amino acid position 190 to an amino acid with an aliphatic amino acid side chain, preferably where phenylalanine (F) is substituted to leucine (L) at position 190 of the F gene product. The combination of both mutations at amino acid position 117 and 190 of the F gene is particularly preferred. These recombinant NDV strains may in addition also comprise the above described mutation in the M gene.

Thus, according to one embodiment of the present invention a recombinant NDV strain comprises as a foreign gene at least one gene selected from the group of the foreign genes of the present invention as defined. In one embodiment of the present invention there is provided a recombinant NDV strain comprises as a foreign gene a gene encoding an antibody directed to the protein PD-L1 or a part thereof, preferably Atezolizumab, an antigen-binding part thereof or a variant of Atezolizumab or its antigen-binding part, a gene encoding an antibody directed to the growth factor protein VEGF-A or a part thereof, preferably Bevacizumab, an antigen-binding part thereof or a variant of Bevacizumab or its antigen-binding part, and any combination of these genes or parts or variants thereof, and has mutations both in the HN gene, preferably resulting in a substitution of the amino acid phenylalanine (F) to leucine (L) at position 277 of the HN gene product, and at least one mutation in the F gene, preferably resulting in a substitution of the amino acid phenylalanine (F) to the amino acid serine (S) at position 117 of the F gene product, and/or resulting in a substitution of the amino acid phenylalanine (F) to the amino acid leucine (L) at position 190 of the F gene product. The combination of both mutations at amino acid position 117 and 190 of the F gene is particularly preferred. These recombinant NDV strains may in addition also comprise the above described mutation in the M gene, preferably resulting in a substitution of the amino acid glycine (G) to the amino acid tryptophane (W) at amino acid position 165 of the M gene. As explained supra, other substitutions at these amino acid positions are also comprised within the present invention.

In a preferred embodiment of the present invention the recombinant and mutated NDV strains comprise in addition to the above described mutation in the HN gene, at least one mutation in the L gene and the thus encoded RNA-dependent RNA polymerase protein, also referred to as L protein or L gene product herein. Still more preferred the mutated L gene encodes a RNA-dependent RNA polymerase protein with an amino acid substitution at position 757 to an amino acid with an aliphatic amino acid side chain other than valine (V), preferably where valine (V) is substituted to isoleucine (I) at position 757 of the L gene product, and/or with an amino acid substitution at position 1551 to an amino acid with a hydroxylated side chain, preferably where phenylalanine (F) is substituted to serine (S) at position 1551 of the L gene product, and/or with an amino acid substitution at position 1700 to an amino acid with an aliphatic side chain, preferably where arginine (R) is substituted to leucine (L) at position 1700 of the L gene product, preferably wherein the NDV comprises a mutated L gene and the encoded L protein with the amino acid substitution at position 757, position 1551 and position 1700. These recombinant NDV strains may in addition also comprise the above described mutation in the M gene and/or one or more of the above described mutations in the F gene.

Thus, according to one embodiment of the present invention a recombinant NDV strain comprises as a foreign gene at least one gene selected from the group of the foreign genes of the present invention as defined above. In one embodiment of the present invention a recombinant NDV strain comprises as a foreign gene at least one gene selected from a gene encoding an antibody directed to the protein PD-L1 or a part thereof, preferably Atezolizumab or an antigen-binding part thereof or a variant of Atezolizumab or its antigen-binding part, a gene encoding an antibody directed to the growth factor protein VEGF-A or a part thereof, preferably Bevacizumab, an antigen-binding part thereof or a variant of Bevacizumab or its antigen-binding part, and any combination of these genes or parts or variants thereof, and has mutations both in the HN gene, preferably resulting in a substitution of the amino acid phenylalanine (F) to leucine (L) at amino acid position 277 of the HN gene, and at least one mutation in the L gene, preferably one resulting at amino acid position 757 to an amino acid with an aliphatic amino acid side chain other than valine (V), preferably where valine (V) is substituted to isoleucine (I) at position 757 of the L gene product, and/or with an amino acid substitution at position 1551 to an amino acid with a hydroxylated side chain, preferably where phenylalanine (F) is substituted to serine (S) at position 1551 of the L gene product, and/or with an amino acid substitution at position 1700 to an amino acid with an aliphatic side chain, preferably where arginine (R) is substituted to leucine (L) at position 1700 of the L gene product, preferably wherein the NDV comprises a mutated L gene and the encoded L protein with the amino acid substitution at position 757, position 1551 and position 1700. These recombinant NDV strains may in addition also comprise the above described mutation in the M gene, preferably resulting in a substitution of the amino acid glycine (G) to the amino acid tryptophane (W) at position 165 of the M gene product, and/or one or more of the above described mutations in the F gene, preferably resulting in a substitution of the amino acid phenylalanine (F) to the amino acid serine (S) at position 117 of the gene product and/or resulting in a substitution of the amino acid phenylalanine (F) to the amino acid leucine (L) at position 190 of the F gene product. As explained supra, other substitutions at these amino acid positions are also comprised within the present invention.

A NDV strain derived from NDV strain MTH-68/H having a mutation in the HN gene, wherein the amino acid phenylalanine (F) is substituted to leucine (L) at amino acid position 277, having a mutation in the M gene, wherein the amino acid glycine (G) is substituted to the amino acid tryptophane (W) at position 165 of the M gene product, having two mutations in the F gene, wherein the amino acid phenylalanine (F) is substituted to the amino acid serine (W) at position 117 and the amino acid phenylalanine (F) is substituted to the amino acid leucine (L) at position 190 of the F gene product, and having three mutations in the L gene, wherein the amino acid valine (V) is substituted to the amino acid isoleucine (I) at position 757, the amino acid phenylalanine (F) is substituted to the amino acid serine (S) at position 1551, and the amino acid arginine (R) is substituted to the amino acid leucine (L) at position 1700 of the L gene product is herein referred to also as NDV-Mut HN(F277L)/M(G165W)/F(F117S)/F(F190L) /L(V757I)/L(F1551S)/L(R1700L) or MutHu2 or NDV-NoThaBene-2. The foreign gene or part or variant thereof carried by this strain is referred to as Atezolizumab, Bevacizumab, Lirilumab, Relatlimab, Monalizumab, TRX518, BMS 986178, CD80, (ns)hIL-12 or eGFP. Within these shortcuts for the recombinant NDV strains a reference to the respective foreign gene is intended to have the meaning as already defined above. The following NDV strains are provided by the present invention in a particularly preferred embodiment:
- NDV-Mut HN(F277L)/M(G165W)/F(F117S)/F(F190L)/L(V757I)/L(F1551S)/ L(R1700L)-Atezolizumab,
- NDV-Mut HN(F277L)/M(G165W)/F(F117S)/F(F190L)/L(V757I)/L(F1551S)/ L(R1700L)-Bevacizumab,
- NDV-Mut HN(F277L)/M(G165W)/F(F117S)/F(F190L)/L(V757I)/L(F1551S)/ L(R1700L)-Lirilumab,
- NDV-Mut HN(F277L)/M(G165W)/F(F117S)/F(F190L)/L(V757I)/L(F1551S)/ L(R1700L)-Relatlimab,
- NDV-Mut HN(F277L)/M(G165W)/F(F117S)/F(F190L)/L(V757I)/L(F1551S)/ L(R1700L)-Monalizumab,
- NDV-Mut HN(F277L)/M(G165W)/F(F117S)/F(F190L)/L(V757I)/L(F1551S)/ L(R1700L)-TRX518,
- NDV-Mut HN(F277L)/M(G165W)/F(F117S)/F(F190L)/L(V757I)/L(F1551S)/ L(R1700L)-BMS 986178,
- NDV-Mut HN(F277L)/M(G165W)/F(F117S)/F(F190L)/L(V757I)/L(F1551S)/ L(R1700L)-CD80,
- NDV-Mut HN(F277L)/M(G165W)/F(F117S)/F(F190L)/L(V757I)/L(F1551S)/ L(R1700L)-(ns)hIL-12,
- NDV-Mut HN(F277L)/M(G165W)/F(F117S)/F(F190L)/L(V757I)/L(F1551S)/ L(R1700L)-eGFP.

A recombinant NDV strain referred to as "NDV-Mut HN(F277L)/M(G165W)/F(F117S)/F(F190L)/L(V757I)/L(F1551S)/L(R1700L)-Atezolizumab" is a NDV strain derived from NDV strain MTH-68/H having a mutation in the HN gene product at position 277, wherein the amino acid phenylalanine (F) is substituted to leucine (L) at position 277, and having a mutation in the M gene, wherein the amino acid glycine (G) is substituted to the amino acid tryptophane (W) at position 165 of the M gene product, having two mutations in the F gene, wherein the amino acid phenylalanine (F) is substituted to the amino acid serine (S) at position 117 and the amino acid phenylalanine (F) is substituted to the amino acid leucine (L) at position 190 of the F gene product, and having three mutations in the L gene, wherein the amino acid valine (V) is substituted to the amino acid isoleucine (I) at position 757, the amino acid phenylalanine (F) is substituted to the amino acid serine (S) at position 1551, and the amino acid arginine (R) is substituted to the amino acid leucine (L) at position 1700 of the L gene product, and carrying as a foreign gene a gene encoding an antibody or an antigen-binding part directed to the protein PD-L1, preferably Atezolizumab or an antigen-binding part thereof or a variant of Atezolizumab or an antigen-binding part of Atezolizumab. A recombinant NDV strain referred to as "NDV-Mut HN(F277L)/M(G165W)/F(F117S)/F(F190L)/L(V757I)/L(F1551S)/ L(R1700L)-Bevacizumab" is a NDV strain derived from NDV strain MTH-68/H having a mutation in the HN gene at amino acid position 277 of the respective gene product, wherein the amino acid phenylalanine (F) is substituted to leucine (L) at position 277, having a mutation in the M gene, wherein the amino acid glycine (G) is substituted to the amino acid tryptophane (W) at position 165 of the M gene product, having two mutations in the F gene, wherein the amino acid phenylalanine (F) is substituted to the amino acid serine (S) at position 117 and the amino acid phenylalanine (F) is substituted to the amino acid leucine (L) at position 190 of the F gene product, and having three mutations in the L gene, wherein the amino acid valine (V) is substituted to the amino acid isoleucine (I) at position 757, the amino acid phenylalanine (F) is substituted to the amino acid serine (S) at position 1551, and the amino acid arginine (R) is substituted to the amino acid leucine (L) at position 1700 of the L gene product, and carrying as a foreign gene a gene encoding an antibody or an antigen-binding part thereof directed to the growth factor protein VEGF-A, preferably Bevacizumab or an antigen-binding part thereof or a variant of Bevacizumab or a variant of an antigen-binding part of Bevacizumab. The NDV strains NDV-Mut HN(F277L)/M(G165W)/F(F117S)/F(F190L)/L(V757I)/L(F1551S)/ L(R1700L)-Atezolizumab and NDV-Mut HN(F277L)/M(G165W)/F(F117S)/F(F190L)/ L(V757I)/ L(F1551S)/L(R1700L)-Bevacizumab are provided by the present invention in a preferred embodiment.

The two mutations in the F gene at position 117 and 190 of the amino acid sequence of the resulting F protein and the three mutations in the L gene at position 757, 1551 and 1700 of the amino acid sequence of the resulting L protein, either alone or in combination, have been shown to beneficially improve the oncolytic potential of the respective NDV strains by improving the safety profile of the viruses in humans. The combinations include any combination of the referenced mutations in the F gene and L gene, and the F protein and L protein, respectively. That is any combination from two to five mutations. Particularly preferred is a combination of both mutations at amino acid position 117 and 190 of the F gene (also referred to as "F2" herein) and a combination of all five mutations, that is mutations at positions 117 und 190 of the F gene product and at positions 757, 1551 and 1700 of the L gene product (also referred to as "F2L3" herein). The respective amino acid substitutions are as specified supra.

With regard to the viral safety profile, this in particular means that the intracerebral pathogenicity index (ICPI) is reduced by these mutations in the F gene, preferably F2, and/or the L gene, preferably F2L3. The ICPI value indicates the the pathogenicity of a Newcastle disease virus. Preferably, the ICPI is reduced by at least 10 %, more preferably by at least 20 % and still more preferred by at least 30 %, as compared to the identically determined ICPI value of a NDV strain not having the respective mutations in the F gene and/or the L gene. The ICPI can be determined according to the method set forth in World Organization for Animal Health (OIE) 2009, Chapter 2.3.14. Newcastle disease. pp. 576-589. In: Manual of Diagnostic Tests and Vaccines for Terrestrial Animals 2009. World Organization for Animal Health (OIE), Paris.

According to another embodiment of the present invention, in addition to the mutation at position 277 of the HN gene product and preferably one or more of the above specified mutations at position 165 of the M gene product, at positions 117 and/or 190 of F gene product, and at positions 757 and/or 1551 and/or 1700 of the L gene product, a recombinant NDV according to the present invention may also comprise a further mutation in the F gene, which mutation is a mutation which encodes a fusion protein having an amino acid substitution at position 289, and which mutation is capable of improving the oncolytic potential of the NDV strain. Still more preferred, the amino acid at position 289 of the F gene product is substituted from leucine (L) to alanine (A). According to another embodiment of the present invention, alternatively or in addition to the mutation at position 289 of the F protein, a recombinant NDV according to the present invention may also comprise at least one further mutation in the L gene and the encoded L protein having an amino acid substitution at position 1717 to an amino acid with aromatic side chain other than tyrosine (Y), preferably where tyrosine (Y) is substituted to histidine (H) at position 1717 of the L gene product, and/or at position 1910 to an amino acid with a basic side chain, preferably where glutamic acid (E) is substituted to lysine (K) at position 1910 of the L gene product.

Alternatively or in addition a recombinant NDV according to the present invention may comprise a mutation in the RNA-editing sequence of the P gene. More preferably, the mutation abolishes and/or decreases the expression of the V protein.

Table 1a shows a list of mutations for obtaining NDV variants with increased titer production and/or improved safety profile according to the present invention. The mutation in the HN gene is comprised in all NDV strains according to the present invention, while the mentioned mutations in the M gene, F gene and L gene may or may not be present. If present, any possible combination of these mutations is encompassed by the present invention.

**Table 1a:**

| Gene | Relevant amino acid |
|---|---|
| HN | F277L |
| M | G165W |
| F | F117S |
| F | F190L |
| F | L289A |
| L | V757I |
| L | F1551S |
| L | R1700L |
| L | Y1717H |
| L | E1910K |

Table 1b shows particulary preferred NDV variants with increased titer production and/or improved safety profile, and the parent NDV strain MTH-68/H. "+" means that at the mentioned amino acid position the original (first mentioned) amino acid is substituted by the last mentioned amino acid, while "-" means that there is no amino acid substitution at the mentioned position, i.e. the first mentioned amino acid is present. For example and with regard to the HN gene "+" means that at amino acid position 277 phenylalanine (F) is substituted to leucine (L).

**Table 1b:**

| Gene | Relevant amino acid | MTH-68/H | | | | | | | | | | | | |
|---|---|---|---|---|---|---|---|---|---|---|---|---|---|---|
| HN | F277L | - | + | + | + | + | + | + | + | + | + | + | + | + |
| M | G165W | - | - | + | - | + | - | + | - | + | - | + | - | + |
| F | F117S | - | - | - | - | - | + | + | + | + | + | + | + | + |
| F | F190L | - | - | - | - | - | + | + | + | + | + | + | + | + |
| F | L289A | - | - | - | + | + | - | - | + | + | - | - | + | + |
| L | V757I | - | - | - | - | - | - | - | - | - | + | + | + | + |
| L | F1551S | - | - | - | - | - | - | - | - | - | + | + | + | + |
| L | R1700L | - | - | - | - | - | - | - | - | - | + | + | + | + |
| L | Y1717H | - | - | - | - | - | - | - | - | - | - | - | + | + |
| L | E1910K | - | - | - | - | - | - | - | - | - | - | - | + | + |

Table 1c shows all mutations in the F gene encompassed by the present invention. "+" means that at the mentioned amino acid position the original (first mentioned) amino acid is substituted by the last mentioned amino acid, while "-" means that there is no amino acid substitution at the mentioned position, i.e. the first mentioned amino acid is present.

**Table 1c:**

| Gene | Relevant amino acid | 1 | 1 | 1 | 2 | 2 | 2 | 3 |
|---|---|---|---|---|---|---|---|---|
| F | F117S | + | - | - | + | + | - | + |
| F | F190L | - | + | - | + | - | + | + |
| F | L289A | - | - | + | - | + | + | + |

Table Id shows preferred mutations in the L gene encompassed by the present invention. "+" means that at the mentioned amino acid position the original (first mentioned) amino acid is substituted by the last mentioned amino acid, while "-" means that there is no amino acid substitution at the mentioned position, i.e. the first mentioned amino acid is present.

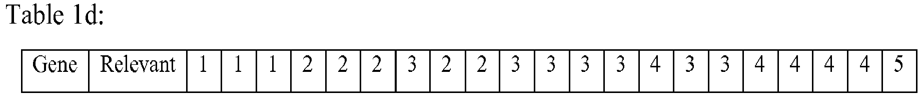

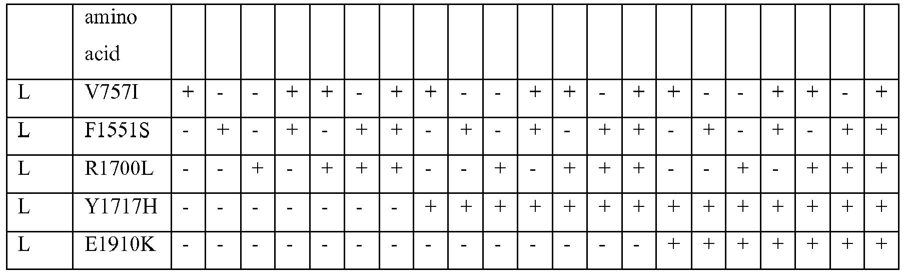

The NDV variants mentioned in tables 1a to Id carry as a foreign gene at least one gene selected from the group of the foreign genes of the present invention, as defined above. In one embodiment of the present invention the at least one gene is selected from the group consisting of a gene encoding an antibody or its antigen-binding part directed to the protein PD-L1, such as Atezolizumab, an antigen-binding part thereof or a variant of Atezolizumab or its antigen-binding part, a gene encoding an antibody or its antigen-binding part directed to the growth factor protein VEGF-A, such as Bevacizumab, an antigen-binding part thereof or a variant of Bevacizumab or its antigen-binding part, and any combination of these genes or parts or variants thereof.

In describing a protein or peptide formulation, structure and function herein, reference is made to amino acids. In the present specification, amino acid residues are expressed by using the following abbreviations. Also, unless explicitly otherwise indicated, the amino acid sequences of peptides and proteins are identified from N-terminal to C-terminal (left terminal to right terminal), the N-terminal being identified as a first residue. Amino acids are designated by their 3-letter abbreviation, 1-letter abbreviation, or full name, as follows. Ala : A : alanine; Asp : D : aspartic acid; Glu : E : glutamic acid ; Phe : F : phenylalanine; Gly : G : glycine; His : H : histidine; Ile : I : isoleucine; Lys : K : lysine; Leu : L : leucine; Met: M : methionine; Asn : N : asparagine; Pro : P: proline; Gln : Q : glutamine; Arg : R : arginine; Ser : S : serine; Thr : T : threonine; Val : V : valine; Trp : W : tryptophan; Tyr : Y : tyrosine; Cys : C : cysteine.

In a preferred embodiment the NDV and the recombinant NDV resulting thereof is encoded by and/or comprises at least one of the nucleic acids according to SEQ ID No. 1 to 23 or parts thereof. According to another preferred the recombinant NDV strains of the present invention have a sequence identity of at least 75 %, particularly of at least 90 %, more particularly of at least 95 % to any one of SEQ ID No. 1 to 23. The sequences given by these SEQ ID numbers are as follows:

| | |
|---|---|
| SEQ ID No. 1: | Nucleic acid (cDNA) sequence of the Newcastle disease virus (NDV) strain MTH-68/H genome; |
| SEQ ID No. 2: | Nucleic acid (cDNA) sequence of Newcastle disease virus strain Mut HN(F277L)/M(G165W) genome having a mutation in the HN gene and in the M gene (= NDV-NoThaBene-1 genome); |
| SEQ ID No. 3: | Nucleic acid (cDNA) sequence of Newcastle disease virus strain Mut HN(F277L)/M(G165W)/F(F117S)/F(F190L)/L(V757I)/L(F1 551S)/L(R1700L) genome having a mutation in the HN gene, a mutation in the M gene, two mutations in the F gene (F2) and three mutations in the L gene (L3) (= NDV-NoThaBene-2 genome); |
| SEQ ID No. 4: | Nucleic acid (cDNA) sequence of Newcastle disease virus strain Mut HN(F277L)/M(G165W)-Atezolizumab genome created by reverse genetics as disclosed herein, wherein the nucleic acid comprises a mutation in the HN gene and in the M gene, and additionally encodes Atezolizumab; |
| SEQ ID No. 5: | Nucleic acid (cDNA) sequence of Newcastle disease virus strain Mut HN(F277L)/M(G165W)-Bevacizumab genome created by reverse genetics as disclosed herein, wherein the nucleic acid comprises a mutation in the HN gene and in the M gene, and additionally encodes Bevacizumab; |
| SEQ ID No. 6: | Nucleic acid (cDNA) sequence of Newcastle disease virus strain Mut HN(F277L)/M(G165W)-Lirilumab genome created by reverse genetics as disclosed herein, wherein the nucleic acid comprises a mutation in the HN gene and in the M gene, and additionally encodes Lirilumab; |
| SEQ ID No. 7: | Nucleic acid (cDNA) sequence of Newcastle disease virus strain Mut HN(F277L)/M(G165W)-Relatlimab genome created by reverse genetics as disclosed herein, wherein the nucleic acid comprises a mutation in the HN gene and in the M gene, and additionally encodes Relatlimab; |
| SEQ ID No. 8: | Nucleic acid (cDNA) sequence of Newcastle disease virus strain Mut HN(F277L)/M(G165W)-Monalizumab genome created by reverse genetics as disclosed herein, wherein the nucleic acid comprises a mutation in the HN gene and in the M gene, and additionally encodes Monalizumab; |
| SEQ ID No. 9: | Nucleic acid (cDNA) sequence of Newcastle disease virus strain Mut HN(F277L)/M(G165W)-TRX518 genome created by reverse genetics as disclosed herein, wherein the nucleic acid comprises a mutation in the HN gene and in the M gene, and additionally encodes TRX518; |
| SEQ ID No. 10: | Nucleic acid (cDNA) sequence of Newcastle disease virus strain Mut HN(F277L)/M(G165W)-BMS 986178 genome created by reverse genetics as disclosed herein, wherein the nucleic acid comprises a mutation in the HN gene and in the M gene, and additionally encodes BMS 986178; |
| SEQ ID No. 11: | Nucleic acid (cDNA) sequence of Newcastle disease virus strain Mut HN(F277L)/M(G165W)-CD80 genome created by reverse genetics as disclosed herein, wherein the nucleic acid comprises a mutation in the HN gene and in the M gene, and additionally encodes CD80; |
| SEQ ID No. 12: | Nucleic acid (cDNA) sequence of Newcastle disease virus strain Mut HN(F277L)/M(G165W)-(ns)hIL-12 genome created by reverse genetics as disclosed herein, wherein the nucleic acid comprises a mutation in the HN gene and in the M gene, and additionally encodes (ns)hIL-12; |
| SEQ ID No. 13: | Nucleic acid (cDNA) sequence of Newcastle disease virus strain Mut HN(F277L)/M(G165W)-eGFP genome created by reverse genetics as disclosed herein, wherein the nucleic acid comprises a mutation in the HN gene and in the M gene, and additionally encodes eGFP; |
| SEQ ID No. 14: | Nucleic acid (cDNA) sequence of Newcastle disease virus strain Mut HN(F277L)/M(G165W)/F(F117S)/F(F190L)/L(V757I)/L(F1 551S)/L(R1700L)-Atezolizumab genome created by reverse genetics as disclosed herein, wherein the nucleic acid comprises a mutation in the HN gene, a mutation in the M gene, two mutations in the F gene (F2) and three mutations in the L gene (L3), and additionally encodes Atezolizumab; |
| SEQ ID No. 15: | Nucleic acid (cDNA) sequence of Newcastle disease virus strain Mut HN(F277L)/M(G165W)/F(F117S)/F(F190L)/L(V757I)/L(F1 551S)/L(R1700L)-Bevacizumab genome created by reverse genetics as disclosed herein, wherein the nucleic acid comprises a mutation in the HN gene, a mutation in the M gene, two mutations in the F gene (F2) and three mutations in the L gene (L3), and additionally encodes Bevacizumab; |
| SEQ ID No. 16: | Nucleic acid (cDNA) sequence of Newcastle disease virus strain Mut HN(F277L)/M(G165W)/F(F117S)/F(F190L)/L(V757I)/L(F1 551S)/L(R1700L)-Lirilumab genome created by reverse genetics as disclosed herein, wherein the nucleic acid comprises a mutation in the HN gene, a mutation in the M gene, two mutations in the F gene (F2) and three mutations in the L gene (L3), and additionally encodes Lirilumab; |
| SEQ ID No. 17: | Nucleic acid (cDNA) sequence of Newcastle disease virus strain Mut HN(F277L)/M(G165W)/F(F117S)/F(F190L)/L(V757I)/L(F1 551S)/L(R1700L)-Relatlimab genome created by reverse genetics as disclosed herein, wherein the nucleic acid comprises a mutation in the HN gene, a mutation in the M gene, two mutations in the F gene (F2) and three mutations in the L gene (L3), and additionally encodes Relatlimab; |
| SEQ ID No. 18: | Nucleic acid (cDNA) sequence of Newcastle disease virus strain Mut HN(F277L)/M(G165W)/F(F117S)/F(F190L)/L(V757I)/L(F1 551S)/L(R1700L)-Monalizumab genome created by reverse genetics as disclosed herein, wherein the nucleic acid comprises a mutation in the HN gene, a mutation in the M gene, two mutations in the F gene (F2) and three mutations in the L gene (L3), and additionally encodes Monalizumab; |
| SEQ ID No. 19: | Nucleic acid (cDNA) sequence of Newcastle disease virus strain Mut HN(F277L)/M(G165W)/F(F117S)/F(F190L)/L(V757I)/L(F1 551S)/L(R1700L)-TRX518 genome created by reverse genetics as disclosed herein, wherein the nucleic acid comprises a mutation in the HN gene, a mutation in the M gene, two mutations in the F gene (F2) and three mutations in the L gene (L3), and additionally encodes TRX518; |
| SEQ ID No. 20: | Nucleic acid (cDNA) sequence of Newcastle disease virus strain Mut HN(F277L)/M(G165W)/F(F117S)/F(F190L)/L(V757I)/L(F1 551S)/L(R1700L)-BMS 986178 genome created by reverse genetics as disclosed herein, wherein the nucleic acid comprises a mutation in the HN gene, a mutation in the M gene, two mutations in the F gene (F2) and three mutations in the L gene (L3), and additionally encodes BMS 986178; |
| SEQ ID No. 21: | Nucleic acid (cDNA) sequence of Newcastle disease virus strain Mut HN(F277L)/M(G165W)/F(F117S)/F(F190L)/L(V757I)/L(F1 551S)/L(R1700L)-CD80 genome created by reverse genetics as disclosed herein, wherein the nucleic acid comprises a mutation in the HN gene, a mutation in the M gene, two mutations in the F gene (F2) and three mutations in the L gene (L3), and additionally encodes CD80; |
| SEQ ID No. 22: | Nucleic acid (cDNA) sequence of Newcastle disease virus strain Mut HN(F277L)/M(G165W)/F(F117S)/F(F190L)/L(V757I)/L(F1 551S)/L(R1700L)-(ns)hIL-12 genome created by reverse genetics as disclosed herein, wherein the nucleic acid comprises a mutation in the HN gene, a mutation in the M gene, two mutations in the F gene (F2) and three mutations in the L gene (L3), and additionally encodes (ns)hIL-12; |
| SEQ ID No. 23: | Nucleic acid (cDNA) sequence of Newcastle disease virus strain Mut HN(F277L)/M(G165W)/F(F117S)/F(F190L)/L(V757I)/L(F1 551S)/L(R1700L)-eGFP genome created by reverse genetics as disclosed herein, wherein the nucleic acid comprises a mutation in the HN gene, a mutation in the M gene, two mutations in the F gene (F2) and three mutations in the L gene (L3), and additionally encodes eGFP. |

A person skilled in the art is well aware of the fact that the nucleobase thymine in the nucleic acid sequence of DNA is replaced by the nucleobase uracil in the nucleic acid sequence of RNA.

That is in a preferred embodiment of the present invention the viral HN gene product has an amino acid sequence as identified in SEQ ID No. 24, and/or the viral M gene product preferably has an amino acid sequence as identified in SEQ ID No. 25.

In a further preferred embodiment of the present inventionen, a NDV strain according to the present invention comprises beside the viral HN gene product having an amino acid sequence as identified in SEQ ID No. 24, and/or the viral M gene product preferably having an amino acid sequence as identified in SEQ ID No. 25, a viral F gene product having an amino acid sequence as identified in SEQ ID No. 26 and/or a viral L gene product having an amino acid sequence as identified in SEQ ID No. 27. Also encompassed by the present invention are NDV strains comprising variants of the gene products according to SEQ ID No. 24 to 27.

In another aspect the present invention also provides a recombinant nucleic acid comprising the nucleic acid sequence of the recombinant NDV strains of the present invention.

In describing nucleic acid formulation, structure and function herein, reference is made to any of a group of polymeric nucleotides such as DNA or RNA. Nucleic acid is composed of either one or two chains of repeating units called nucleotides, which nucleotides consist of a nitrogen base (a purine or pyrimidine) attached to a sugar phosphate. In the present specification, nucleotide residues are identified by using the following abbreviations. Adenine residue: A; guanine residue: G; thymine residue: T; cytosine residue: C; uracil residue: U. Also, unless explicitly otherwise indicated, the nucleotide sequences of nucleic acid are identified from 5'-terminal to 3'-terminal (left to right terminal), the 5'-terminal being identified as a first residue.

A nucleic acid according to the present invention comprises a transgenic construct. According to one embodiment the transgenic construct encodes an antibody directed to the protein PD-L1 or an antigen-binding part that is directed to the protein PD-L1. According to the present invention the encoded antibody is in a particularly preferred embodiment Atezolizumab, an antigen-binding part of Atezolizumab or a variant of Atezolizumab or its antigen-binding part. According to another embodiment the transgenic construct encodes an antibody directed to the growth factor protein VEGF-A or an antigen-binding part that is directed to the growth factor protein VEGF-A. According to the present invention the encoded antibody is in a particularly preferred embodiment Bevacizumab, an antigen-binding part of Bevacizumab or a variant of Bevacizumab or its antigen-binding part. According to another embodiment the transgenic construct encodes an antibody directed to the killer-cell immunoglobulin-like receptors (KIRs), in particular KIR2DL1/2L3, or an antigen-binding part that is directed to the killer-cell immunoglobulin-like receptors (KIRs), in particular KIR2DL1/2L3. According to the present invention the encoded antibody is in a particularly preferred embodiment Lirilumab, an antigen-binding part of Lirilumab or a variant of Lirilumab or its antigen-binding part. According to another embodiment the transgenic construct encodes an antibody directed to and inhibiting LAG-3, or an antigen-binding part that is directed to and inhibiting LAG-3. According to the present invention the encoded antibody is in a particularly preferred embodiment Relatlimab, an antigen-binding part of Relatlimab or a variant of Relatlimab or its antigen-binding part. According to another embodiment the transgenic construct encodes an antibody directed to NKG2A, or an antigen-binding part that is directed to NKG2A. According to the present invention the encoded antibody is in a particularly preferred embodiment Monalizumab, an antigen-binding part of Monalizumab or a variant of Monalizumab or its antigen-binding part. According to another embodiment the transgenic construct encodes an antibody directed to the glucocorticoid-induced TNF-superfamily receptor (GITR), or an antigen-binding part that is directed to the glucocorticoid-induced TNF-superfamily receptor (GITR). According to the present invention the encoded antibody is in a particularly preferred embodiment TRX518, an antigen-binding part of TRX518 or a variant of TRX518 or its antigen-binding part. According to another embodiment the transgenic construct encodes an antibody directed to and activating the protein OX40, or an antigen-binding part that is directed to and activating the protein OX40. According to the present invention the encoded antibody is in a particularly preferred embodiment BMS 986178, an antigen-binding part of BMS 986178 or a variant of BMS 986178 or its antigen-binding part. According to another embodiment the transgenic construct encodes a membrane protein which is the receptor for the proteins CD28 and CTLA-4, and which membrane protein is involved in the costimulatory signal essential for T-lymphocyte activation, or part thereof which is the receptor for the proteins CD28 and CTLA-4, and which membrane protein is involved in the costimulatory signal essential for T-lymphocyte activation. According to the present invention the encoded protein is in a particularly preferred embodiment CD80, a part of CD80 or a variant of CD80 or its part. According to another embodiment the transgenic construct encodes a human interleukin 12 (hIL-12), or part thereof. According to the present invention the encoded protein is in a particularly preferred embodiment (ns)hIL-12, a part of (ns)hIL-12 or a variant of (ns)hIL-12 or its part. According to another embodiment the transgenic construct encodes a green fluorescent protein, or part thereof which can be used for the localization and monitoring of cells, in particular cancer cells. According to the present invention the encoded protein is in a particularly preferred embodiment eGFP, a part of eGFP or a variant of eGFP or its part.

According to a further embodiment of the present invention, the transgenic construct comprised in the nucleic acid may be a combination of at least two of the foreign genes or parts or variants thereof as mentioned supra.

In addition to the transgenic construct a nucleic acid according to the present invention comprises a mutation in the HN gene, said mutation allowing replication of the NDV in a cancer cell to a higher level than replication of an otherwise identical NDV not having said the mutation in the HN gene.

In a preferred embodiment of the present invention the sequence encoding the recombinant NDV comprises a mutated HN gene encoding hemagglutinin-neuramidase with an amino acid substitution at position 277 to an amino acid with a hydrophobic side chain other than phenylalanine. Still more preferred the mutated HN gene encodes for a hemagglutinin-neuramidase referred to as HN^{F277L}. That is the amino acid phenylalanine (F) at position 277 of the hemagglutinin-neuramidase is substituted to leucine (L).

In a particular preferred embodiment the nucleic acid is derived from the NDV strain MTH-68/H.

In another embodiment of the present invention the sequence encoding the recombinant NDV may also comprises a mutation in the M gene. Preferably the mutated M gene encodes a matrix protein with an amino acid substitution at position 165 to an amino acid with an aromatic side chain. Most preferred the mutated M gene encodes M^{G165W}. That is the amino acid glycine (G) at position 165 of the M gene product is substituted to the amino acid tryptophane (W) at the respective position. In one embodiment of the present invention the mutation in the M gene is comprised in combination with the mutation in the HN gene as specified supra.

In a particular preferred embodiment the nucleic acid is derived from the NDV strain MTH-68/H and comprises a mutation in the HN gene resulting in an amino acid with a hydrophobic side chain other than phenylalanine at amino acid position 277, and comprising a mutation in the M gene resulting in a matrix protein with an amino acid substitution at position 165 to an amino acid with an aromatic side chain. Still more preferred the mutated HN gene encodes for a hemagglutinin-neuramidase in which the amino acid phenylalanine (F) at position 277 of the hemagglutinin-neuramidase is substituted to leucine (L), and the mutated M gene encodes for a matrix protein in which the amino acid glycine (G) at position 165 of the M protein is substituted to the amino acid tryptophane (W).

According to a further preferred embodiment of the present invention the sequence encoding the recombinant NDV comprises, in addition to the above described mutation in the HN gene, particulary the mutation resulting in an amino acid exchange or substitution at position 277 of the HN gene product as specified supra, optionally in combination with the above described mutation on the M gene, particulary the mutation resulting in an amino acid exchange at position 165 of the M gene product as specified supra, the nucleic acid sequence encoding the recombinant NDV also comprises at least one mutation in the F gene. Preferably the mutated F gene encodes a fusion protein with an amino acid substitution at position 117 and/or position 190. With regard to amino acid position 117 the amino acid is substituted to an amino acid with a hydroxylated side chain. Most preferred the mutated F gene encodes F^{F117S}. That is the amino acid phenylalanine (F) at position 117 of the F gene product is substituted to the amino acid serine (S) at the respective position. With regard to the amino acid substitution at position 190 the amino acid is changed to an amino acid with an aliphatic side chain. Most preferred the mutated F gene encodes F^{F190L} . That is the amino acid phenylalanine (F) at position 190 of the F gene product is substituted to the amino acid leucine (L) at the respective position. The combination of boths mutations at amino acid position 117 (F^{F117S}) and 190 (F^{F190L}) is particularly preferred. In one embodiment of the present invention the at least one mutation, preferably both mutations, in the F gene is comprised in combination with the mutation in the HN gene as specified supra and optionally, also in combination with the mutation in the M gene as specified supra.

In a particular preferred embodiment the nucleic acid is derived from the NDV strain MTH-68/H and comprises a mutation in the HN gene resulting in an amino acid with a hydrophobic side chain other than phenylalanine at amino acid position 277, a mutation in the M gene resulting in a matrix protein with an amino acid substitution at position 165 to an amino acid with an aromatic side chain, and at least one mutation in the F gene resulting in a fusion protein with an amino acid substitution at position 117 to an amino acid with a hydroxylated side chain and/or an amino acid substitution at position 190 to an amino acid with an aliphatic side chain. Still more preferred the mutated HN gene encodes for a hemagglutinin-neuramidase in which the amino acid phenylalanine (F) at position 277 of the hemagglutinin-neuramidase is substituted to leucine (L), the optionally mutated M gene encodes for a matrix protein in which the amino acid glycine (G) at position 165 is substituted to the amino acid tryptophane (W), and the mutated F gene encodes for a fusion protein in which the amino acid phenylalanine (F) at position 117 is substituted to the amino acid serine (S) and/or the amino acid phenylalanine (F) at position 190 is substituted to the amino acid leucine (L). The combination of boths mutations at amino acid position 117 (F^{F117S}) and 190 (F^{F190L}) of the F gene is particularly preferred.

According to a further preferred embodiment of the present invention the sequence encoding the recombinant NDV comprises, in addition to the above described mutation in the HN gene, particulary the mutation resulting in an amino acid substitution at position 277 of the HN gene product as specified supra, and in addition to the above described at least one mutation in the F gene, particulary the mutation resulting in an amino acid substitution at position 117 (F^{F117S}) and/or at position 190 (F^{F190L}) of the F gene product as specified supra, optionally in combination with the above described mutation on the M gene, particulary the mutation resulting in an amino acid exchange at position 165 of the M gene product as specified supra, also comprises at least one mutation in the L gene. Preferably the mutated L gene encodes for a L protein with at least one amino acid substitution at amino acid position 757, 1551 and/or 1700. At position 757 the amino acid may be changed to an amino acid with an aliphatic side chain other than valine (V). Most preferred the mutated L gene encodes L^{V757I}. That is the amino acid valine (V) at position 757 of the L gene product is substituted to the amino acid isoleucine (I) at the respective position. At position 1551 the amino acid may be changed to an amino acid with a hydroxylated side chain. Most preferred the mutated L gene encodes L^{F1551S}. That is the amino acid phenylalanine (F) at position 1551 of the L gene product is substituted to the amino acid serine (S) at the respective position. At position 1700 the amino acid may be changed to an amino acid with an aliphatic side chain. Most preferred the mutated L gene encodes L^{R1700L}. That is the amino acid arginine (R) at position 1700 of the L gene product is substituted to the amino acid leucine (L) at the respective position. The mutations at amino acid positions 757, 1551 and 1700 of the L gene may be present alone or in each possible combination, i.e. position 757 and/or position 1551 and/or position 1700. The combination of all three mutations in the L gene (L3) is particularly preferred. In one embodiment of the present invention the at least one mutation in the L gene, preferably all three mutation at the respective amino acid positions 757, 1551 and 1700, is/are comprised in combination with the mutation in the HN gene as specified supra, the one or two mutations in the F gene as specified supra, and optionally, also in combination with the mutation in the M gene as specified supra.

In a particular preferred embodiment the nucleic acid is derived from the NDV strain MTH-68/H and comprises a mutation in the HN gene resulting in an amino acid with a hydrophobic side chain other than phenylalanine at position 277 of the respective amino acid sequence, a mutation in the M gene resulting in a matrix protein with an amino acid substitution at position 165 to an amino acid with an aromatic side chain, at least one mutation of the F gene resulting in a fusion protein with an amino acid substitution at position 117 to an amino acid with a hydroxylated side chain and/or an amino acid substitution at position 190 to an amino acid with an aliphatic side chain, and at least one mutation in the L gene resulting in a L protein with an amino acid substitution at position 757 to an amino acid with an aliphatic side chain other than valine (V), and/or an amino acid substitution at position 1551 to an amino acid with a hydroxylated side chain, and/or an amino acid substitution at position 1700 to an amino acid with an aliphatic side chain. More preferred, the mutated L gene results in a L protein comprising the three amino acid substitutions at positions 757, 1551 and 1700. Still more preferred the mutated HN gene encodes for a hemagglutinin-neuramidase in which the amino acid phenylalanine (F) at position 277 of the hemagglutinin-neuramidase is substituted to leucine (L), the optionally mutated M gene encodes for a matrix protein in which the amino acid glycine (G) at position 165 is substituted to the amino acid tryptophane (W), the mutated F gene encodes for a fusion protein in which the amino acid phenylalanine (F) at position 117 is substituted to the amino acid serine (S) and/or the amino acid phenylalanine (F) at position 190 is substituted to the amino acid leucine (L), and the mutated L gene encodes for a L protein in which the amino acid valine (V) at position 757 is substituted to the amino acid isoleucine (I), and/or the amino acid phenylalanine (F) at position 1551 is substituted to the amino acid serine (S), and/or the amino acid arginine (R) at position 1700 is substituted to the amino acid leucine (L). Still more preferred the L protein comprises all three mutations at positions 757, 1551 and 1700.

Also within the scope of the present invention are those nucleic acids comprising, in addition to the mutation in the HN gene, preferably in combination with one or more mutation in the M gene, the F gene and the L gene, as specified supra, a mutation in the F gene resulting in an amino acid substitution at position 289 of the amino acid sequence, and/or at least one mutation in the L gene resulting in an amino acid substitution at position 1717 and/or at position 1910 of the L proteine, and/or a mutation in the P gene as specified supra.

In one embodiment of the present invention the nucleic acid consists of or comprises at least one of the nucleic acids according to SEQ ID No. 2 to 23 or parts thereof. In a more preferred embodiment the nucleic acid of the present invention has a sequence identity of at least 75 %, particularly of at least 90 %, more particularly of at least 95 % to any of the sequences according to SEQ ID No. 2 to 23.

In one embodiment of the present invention the nucleic acid is derived from a recombinant NDV as described supra.

The skilled artisan knows how to use the disclosed sequences to produce synthetic DNAs (e.g. vectors, in particular in rg-NDV vectors), and how to use the synthetic DNAs to reconstruct and express the viral genome. Thus, the skilled artisan is enabled to produce virus particles by using the information disclosed herein.

Recombinant viral genomes, which can be used to rescue virus particles, can for example be obtained by a method called 'reverse genetics'. Therefore, in another aspect the present invention also provides a method (herein also called 'reverse genetics') for providing cloned full-length cDNA of the recombinant NDV strains according to the present invention, and, in a further embodiment, the invention also provides infectious virus particles obtained from said full-length cDNA of the recombinant NDV strains according to the present invention. These recombinant NDV strains have an unexpected beneficial replication capability in cell cultures, eggs or animals that are used to propagate the virus for pharmaceutical purposes, and/or have an improved viral safety profile, giving production advantages to such improved rgNDV strains. Furthermore, the higher replication rate is also particularly useful to obtain the desired oncolytic effects in vivo in that more virus particles will be produced for subsequent rounds of infection of cancer cells that were missed in the first round of infection. What is more, higher levels (yields) of the foreign gene or the foreign genes encoded by the transgenic construct will be achieved for providing the desired enhanced oncolytic effects in vivo. Thus, by modifying the HN protein within a transgene-expressing NDV higher yields of the transgenic construct protein can be obtained, which is very useful in anti-tumor immunotherapy. By modifying the F protein and/or the L protein, as specified supra, also the viral safety can be improved.

The recombinant NDV strains according to the present invention can be obtained by a reverse genetic method for preparing an rgNDV encoding at least one foreign gene and having improved replication in a cancer cell, and preferably also improved viral safety, over a parent NDV.

The method according to the present invention comprises providing a nucleic acid construct encoding a HN gene with a mutation, particularly wherein the mutation in the HN gene leads to a change in the expression of the hemagglutinin-neuraminidase, wherein the amino acid, particularly phenylalanine (F), in position 277 is substituted, particularly wherein the amino acid in position 277 is substituted to an amino acid with a hydrophobic side chain, more particularly wherein the amino acid in position 277 is substituted to leucine (L) at position 277 of the HN gene product / HN protein.

The method further comprises a step of providing a nucleic acid encoding a rgNDV further comprising a transgenic construct encoding an antibody directed to the protein PD-L1 or an antigen-binding part that is directed to the protein PD-L1, preferably Atezolizumab, an antigen-binding part of Atezolizumab or a variant of Atezolizumab or its antigen-binding part, an antibody directed to the growth factor protein VEGF-A or an antigen-binding part that is directed to the growth factor protein VEGF-A, preferably Bevacizumab, an antigen-binding part of Bevacizumab or a variant of Bevacizumab or its antigen-binding part, an antibody directed to the killer-cell immunoglobulin-like receptors (KIRs), in particular KIR2DL1/2L3, or an antigen-binding part that is directed to the killer-cell immunoglobulin-like receptors (KIRs), in particular KIR2DL1/2L3, preferably Lirilumab, an antigen-binding part of Lirilumab or a variant of Lirilumab or its antigen-binding part, an antibody directed to and inhibiting LAG-3, or an antigen-binding part that is directed to and inhibiting LAG-3, preferably Relatlimab, an antigen-binding part of Relatlimab or a variant of Relatlimab or its antigen-binding part, an antibody directed to NKG2A, or an antigen-binding part that is directed to NKG2A, preferably Monalizumab, an antigen-binding part of Monalizumab or a variant of Monalizumab or its antigen-binding part, an antibody directed to the glucocorticoid-induced TNF-superfamily receptor (GITR), or an antigen-binding part that is directed to the glucocorticoid-induced TNF-superfamily receptor (GITR), preferabyl TRX518, an antigen-binding part of TRX518 or a variant of TRX518 or its antigen-binding part, an antibody directed to and activating the protein OX40, or an antigen-binding part that is directed to and activating the protein OX40, preferably BMS 986178, an antigen-binding part of BMS 986178 or a variant of BMS 986178 or its antigen-binding part, a membrane protein which is the receptor for the proteins CD28 and CTLA-4, and which membrane protein is involved in the costimulatory signal essential for T-lymphocyte activation, or part thereof which is the receptor for the proteins CD28 and CTLA-4, and which membrane protein is involved in the costimulatory signal essential for T-lymphocyte activation, preferably CD80, a part of CD80 or a variant of CD80 or its part, a human interleukin 12 (hIL-12), or part thereof, preferably (ns)hIL-12, a part of (ns)hIL-12 or a variant of (ns)hIL-12 or its part, and/or a green fluorescent protein, or part thereof, preferably eGFP, a part of eGFP or a variant of eGFP or its part.

In the next step of the claimed method the nucleic acid construct with said mutation in the HN gene (optionally with further mutations, in particular those as specified herein) is incorporated in the nucleic acid encoding a rgNDV comprising the transgenic construct in order to obtain a full length cDNA of the NDV, which additionally comprises a transgenic construct as specified supra. In a preferred embodiment this method step results in a nucleic acid as disclosed supra.

The nucleic acid encoding a rgNDV further comprising a transgenic construct as specified supra can be obtained by generating sub-genomic cDNA fragments and assembling the full length cDNA of the recombinant NDV from these fragments.

Following these method steps the thus obtained nucleic acid encoding a recombinant and mutated rgNDV according to the present invention is used to produce infectious rgNDV particles, which replication characteristics and expression rates of the encoded foreign protein(s) in cancer cells are compared with the replication characteristics and expressions rates of the parent NDV used for designing the recombinant and mutated NDV strain.

Finally, said rgNDV is selected for further use, when it shows an improved replication characteristic over the parent NDV and/or an improved viral safety, as well as a sufficient expression of the gene product or gene products of the at least one foreign genes.

If it is intended to introduce further to the mutation in the HN gene, at least one further mutation in a viral gene, preferably the M gene, the F gene, the L gene and/or the P gene, the selected recombinant and mutated rgNDV comprising a transgenic construct as specified supra and a mutated HN gene is used for incorporating a nucleic acid construct encoding a mutated M gene, F gene, L gene or P gene, and the method for introducing the mutation in the HN gene is repeated respectively. Alternatively there is already provided a nucleic acid encoding a rgNDV with the transgenic construct and carrying a mutation in the M gene, F gene, L gene or P gene, particularly wherein said mutation is capable of improving oncolytic potential of said rgNDV.

According to a preferred embodiment of the present invention the mutation in the M gene comprises or is a mutation which encodes a matrix protein with an amino acid substitution at position 165 to an amino acid with an aromatic side chain. Still more preferred is a substitution of the amino acid glycine (G) at position 165 of the M gene product to the amino acid tryptophane (W).

According to a further preferred embodiment of the present invention the at least one mutation in the F gene comprises or is a mutation which encodes a fusion protein / F protein with an amino acid substitution at position 117 to an amino acid with a hydroxylated side chain and/or with an amino acid substitution at position 190 to an amino acid with an aliphatic side chain. Still more preferred is a substitution of the amino acid phenylalanine (F) at position 117 of the F gene product to the amino acid serine (S) and/or a substitution of the amino acid phenylalanine (F) at position 190 of the F gene product to the amino acid leucine (L). Optionally, the F gene may also comprise a mutation which encodes a fusion protein with an amino acid substitution at position 289 to an amino acid with an aliphatic side chain other than leucine, preferably a substitution of the amino acid leucine (L) at position 289 of the F gene product to the amino acid alanine (A).

According to a still further preferred embodiment of the present invention the at least one mutation in the L gene comprises or is a mutation which encodes a L protein with an amino acid substitution at position 757 to an amino acid with an aliphatic side chain other than valine, and/or with an amino acid substitution at position 1551 to an amino acid with a hydroxylated side chain, and/or with an amino acid substitution at position 1700 to an amino acid with an aliphatic side chain. Still more preferred is a substitution of the amino acid valine (V) at position 757 of the L gene product to the amino acid isoleucine (I) and/or a substitution of the amino acid phenylalanine (F) at position 1551 of the L gene product to the amino acid serine (S), and/or a substitution of the amino acid arginine (R) at position 1700 of the L gene product to the amino acid leucine (L). Optionally, the L gene may also comprise a mutation which encodes a L protein with an amino acid substitution at position 1717 to an amino acid with an aromatic side chain other than tyrosine, preferably a substitution of the amino acid tyrosine (Y) at position 1717 of the L gene product to the amino acid histidine (H), and/or a mutation which encodes a L protein with an amino acid substitution at position 1910 to an amino acid with a basic side chain, preferably a substitution of the amino acid glutamic acid (E) at position 1910 of the L gene product to the amino acid lysine (K).

According to another preferred embodiment of the present invention the mutation is in the RNA-editing sequence of the P gene. More preferably, the mutation abolishes and/or decreases the expression of the V protein.

It is also within the scope of the present invention that the viral genome comprises a combination of two or more mutations, which are selected from a mutation in the HN gene, a mutation in the M gene, at least one mutation in the F gene, at least one mutation in the L gene, and a mutation in the P gene. For the details of the respective mutations, reference to the detailed disclosure of each of these mutations given supra and in tables 1 is made.

The invention also provides an isolated, recombinant nucleic acid encoding a rgNDV obtainable by a method for preparing an rgNDV having improved replication in a cancer cell in comparison to that of a parent NDV, as described supra.

In one preferred embodiment the isolated and recombinant nucleic acid encodes a rgNDV provided with a mutation in the HN gene resulting in a change of the amino acid phenylalanine (F) at amino acid position 277 of the hemagglutinin-neuraminidase into a leucine (L), said mutation (a F277L mutation) allowing replication of said rgNDV in a human cancer cell to a higher level than replication of an otherwise identical rgNDV having the amino acid phenylalanine (F) at position 277 in the HN gene product in combination with a transgenic construct comprising any one of the foreign gene of the present invention, as defined supra. For example the said isolated and recombinant nucleic acid encoding an antibody or an antigen-binding part thereof directed to the protein PD-L1, preferably Atezolizumab, an antigen-binding part thereof or a variant of Atezolizumab or its antigen-binding part. The rgNDV may further comprise at least one or more mutation(s) selected from a mutation in the M gene, preferably G165W, a mutation at amino acid position 117, 190 and/or 289 in the F gene, preferably F117S, F190L, and/or L289A, a mutation at amino acid position 757, 1551, 1700, 1717 and/or 1910 in the L gene, preferably V757I, F1551S, R1700L, Y1717H and/or E1910K, and a mutation in the P gene, preferably the mutation in the P gene abolishes and/or decreases the expression of the V protein.

In another much preferred embodiment the isolated and recombinant nucleic acid encodes a rgNDV provided with a mutation in the HN gene resulting in a change of the amino acid phenylalanine (F) at amino acid position 277 of the hemagglutinin-neuraminidase into a leucine (L), said mutation (a F277L mutation) allowing replication of said rgNDV in a human cancer cell to a higher level than replication of an otherwise identical rgNDV having the amino acid phenylalanine (F) at position 277 in the HN gene in combination with a transgenic construct encoding an antibody or an antigen-binding part thereof directed to the growth factor protein VEGF-A, preferably Bevacizumab, an antigen-binding part thereof or a variant of Bevacizumab or its antigen-binding part. The rgNDV may further comprise at least one or more mutation(s) selected from a mutation in the M gene, preferably G165W, a mutation at amino acid position 117, 190 and/or 289 in the F gene, preferably F117S, F190L, and/or L289A, a mutation at amino acid position 757, 1551, 1700, 1717 and/or 1910 in the L gene, preferably V757I, F1551S, R1700L, Y1717H and/or E1910K, and a mutation in the P gene, preferably the mutation in the P gene abolishes and/or decreases the expression of the V protein.

According to a particularly preferred embodiment the nucleic acid used to engineer a recombinant NDV genome is derived from the NDV strain MTH-68/H. Thus the present invention provides in one embodiment nucleic acids, preferably obtainable by the above specified method, which are rgNDV-Mut HN(F277L)-Atezolizumab, rgNDV-Mut HN(F277L)-Bevacizumab, rgNDV-Mut HN(F277L)/M(G165W)-Atezolizumab (= rgNDV-NoThaBene-1-Atezolizumab), rgNDV-Mut HN(F277L)/M(G165W)-Bevacizumab (= rgNDV-NoThaBene-1-Bevacizumab). In another enmbodiment the present invention provides nucleic acids, preferably obtainable by the above specified method, which are rgNDV-MutHN(F277L)/M(G165W)/F(F117S)/F(F190L)/L(V757I)/L(F1551S)/L(R1700L)-Atezolizumab (= rgNDV-NoThaBene-2-Atezolizumab), rgNDV-Mut HN(F277L)/M(G165W)/F(F117S)/F(F190L)/L(V757I)/L(F1551S)/L(R1700L)-Bevacizumab (= rgNDV-NoThaBene-2-Bevacizumab).

Other embodiments of the nucleis acid as provided by the present invention are:
- rgNDV-Mut HN(F277L)-Lirilumab,
- rgNDV-Mut HN(F277L)/M(G165W)-Lirilumab,
- rgNDV-Mut HN(F277L)/M(G165W)/F(F117S)/F(F190L)/L(V757I)/ L(F 1551 S)/L(R1700L)-Lirilumab,

- rgNDV-Mut HN(F277L)-Relatlimab,
- rgNDV-Mut HN(F277L)/M(G165W)-Relatlimab,
- rgNDV-Mut HN(F277L)/M(G165W)/F(F117S)/F(F190L)/L(V757I)/ L(F 1551 S)/L(R1700L)-Relatlimab,

- rgNDV-Mut HN(F277L)-Monalizumab,
- rgNDV-Mut HN(F277L)/M(G165W)-Monalizumab,
- rgNDV-Mut HN(F277L)/M(G165W)/F(F117S)/F(F190L)/L(V757I)/ L(F 1551 S)/L(R1700L)-Monalizumab,

- rgNDV-Mut HN(F277L)-TRX518,
- rgNDV-Mut HN(F277L)/M(G165W)-TRX518,
- rgNDV-Mut HN(F277L)/M(G165W)/F(F117S)/F(F190L)/L(V757I)/ L(F1551S)/L(R1700L)-TRX518,

- rgNDV-Mut HN(F277L)-BMS 986178,
- rgNDV-Mut HN(F277L)/M(G165W)-BMS 986178,
- rgNDV-Mut HN(F277L)/M(G165W)/F(F117S)/F(F190L)/L(V757I)/ L(F1551S)/L(R1700L)-BMS 986178,

- rgNDV-Mut HN(F277L)-CD80,
- rgNDV-Mut HN(F277L)/M(G165W)-CD80,
- rgNDV-Mut HN(F277L)/M(G165W)/F(F117S)/F(F190L)/L(V757I)/ L(F1551S)/L(R1700L)-CD80,

- rgNDV-Mut HN(F277L)-(ns)hIL-12,
- rgNDV-Mut HN(F277L)/M(G165W)-(ns)hIL-12,
- rgNDV-Mut HN(F277L)/M(G165W)/F(F117S)/F(F190L)/L(V757I)/ L(F1551S)/L(R1700L)-(ns)hIL-12,

- rgNDV-Mut HN(F277L)-eGFP,
- rgNDV-Mut HN(F277L)/M(G165W)-eGFP,
- rgNDV-Mut HN(F277L)/M(G165W)/F(F117S)/F(F190L)/L(V757I)/ L(F1551S)/L(R1700L)-eGFP.

A NDV or rgNDV according to the present invention can be provided in a suitable cell or cell line, for example a HeLa cell line or in cancer cells, or an embryonated egg that is susceptible to a NDV infection.

In these infected cells the virus is grown to sufficient quantities, and preferably under sufficient conditions such that the virus is free from exogenous contamination, and the progeny virus particles are collected by suitable methods well known to those skilled in the art.

In a further aspect the present invention is concerned with the medical use of the recombinant NDV strains (the virus particles) of the present invention. In this regard the invention provides an oncolytic NDV for use in oncological treatment (this term is used herein also replaceable with "for use as a medicament, especially in a method of treatment of cancer"; or the use of said oncolytic NDV in the preparation of a pharmaceutical formulation for use in said method of treatment) in humans (or more generically animals, such as mammalian animals).

In this regard the present invention is first concerned with a recombinant NDV according to the present invention for use in medicine. Secondly, the present invention is more particulary concerned with a recombinant NDV according to the present invention for use in a method of treating cancer in a subject considered in need thereof.

In particular one or more of the recombinant and mutated NDV strains of the present invention are used in a method for treating one or more indications selected from the group consisting of brain tumors, like glioblastoma, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose, throat tumors, tongue tumors, and skin tumors, like melanoma.

In a preferred embodiment of the present invention the subject to be treated is a mammal, particularly a mammalian animal or a human subject. Still more preferred the one or more oncolytic NDV strains of the present invention may be used for the treatment of adults and/or children, preferably human adults and/or human children.

In one embodiment only one recombinant and mutated NDV or rgNDV strain according to the present invention is used in a method for treating cancer, in particular a cancer selected from the specific cancers as described and/or mentioned in the claims herein. Alternatively in another preferred embodiment of the present invention a combination of at least two of the recombinant and mutated NDV or rgNDV strains according to the present invention is used in a method for treating cancer, in particular a cancer selected from the specific cancers as described and/or mentioned in the claims herein. In particular it is preferred to use a combination of virus particles, wherein each virus particle encodes and expresses another foreign gene selected from the group of the foreign genes of the present invention, as defined above. For example, the foreign gene encoding an antibody directed to the protein PD-L1 or an antigen-binding part thereof directed to the protein PD-L1, preferably Atezolizumab, an antigen-binding part therof or a variant of Atezolizumab or its antigen-binding part, or encoding an antibody directed to the growth factor protein VEGF-A or an antigen-binding part thereof directed to the growth factor protein VEGF-A, preferably Bevacizumab, an antigen-binding part therof or a variant of Bevacizumab or its antigen-binding part. The use of such a combination of foreign genes results in an active combination of proteins beneficially improving the immunological response with the cancer.

Also within the scope of the present invention is the use of one or more NDV or rgNDV strains according to the present invention in combination with one or more other therapies suitable for the treatment of cancer. In a preferred embodiment the one or more NDV or rgNDV strains or a pharmaceutical formulation comprising the same is used in a method for treating cancer, especially a cancer selected from the specific cancers as described and/or mentioned in the claims herein.

The one or more NDV or rgNDV strains and one or more other therapies can be used concurrently or sequentially. In certain embodiments, the one or more NDV or rgNDV and the one or more other therapies are administered in the same ("fixed") pharmaceutical formulation. In other embodiments, the one or more NDV or rgNDV strains and the one or more other therapies are administered in different formulations. The one or more NDV or rgNDV strains of the present invention and the one or more other therapies can be administered by the same or different routes of administration to the subject considered in need thereof. Another therapy within the meaning of the present invention also comprises an administration of other oncolytical virus strains, for example recombinant NDV or rgNDV strains encoding and expressing foreign genes other than those as disclosed within the present invention. Another foreign gene expressed by the NDV or rgNDV may be selected from the group consisiting of a gene encoding an antibody directed to protein PD-1 (anti-PD-1), preferably a gene encoding Nivolumab, a gene encoding an antibody directed to the surface protein CTLA-4 (anti-CTLA-4), preferably a gene encoding Ipilimumab, a gene encoding a protein which improves the cellular immune response and the ability of T cells to enter tumor cells, preferably a gene encoding interleukin-12 (IL-12), a gene encoding a protein with the ability to modulate the virus replication cycle, preferably a gene encoding the non-structural protein NS1 of influenza A virus, a gene encoding a protein with the ability to selectively induce apoptosis in human tumor cells, but not in normal human cells, preferably a gene encoding Apoptin, a gene encoding a protein that reduces or inhibits IFN expression such as an IFN-beta receptor, preferably a gene encoding the viral protein B18R from vaccinia virus, any antigen-binding part of these antibodies, any parts of these other proteins, variants of these antibodies and these other proteins, and variants of these antigen-binding parts or parts. Particularly preferred NDV strains and rgNDV which may be used in combination are disclosed in the copending European patent application no. 18166400.4 and the copending European patent application no. 19154204.2. For details of these strains reference to these patent applications is made.

Particulary preferred is the combined use of recombinant NDV or rgNDV strains encoding and expressing as foreign genes a gene encoding an antibody directed to protein PD-1 or an antigen-binding part directed to protein PD-1 (anti-PD-1), preferably a gene encoding Nivolumab, an antigen-binding part of Nivolumab, a variant of Nivolumab or a variant of an antigen-binding part of Nivolumab, a recombinant NDV or rgNDV strain encoding and expressing as foreign genes a gene encoding an antibody directed to the surface protein CTLA-4 or an antigen-binding part directed to protein CTLA-4 (anti-CTLA-4), preferably a gene encoding Ipilimumab, an antigen-binding part of Ipilimumab, a variant of Ipilimumab or a variant of an antigen-binding part of Ipilimumab, and a recombinant NDV or rgNDV strain encoding and expressing as foreign genes a gene encoding an antibody directed to protein PD-L1 or an antigen-binding part directed to protein PD-L1 (anti-PD-L1), preferably a gene encoding Atezolizumab, an antigen-binding part of Atezolizumab, a variant of Atezolizumab or a variant of an antigen-binding part of Atezolizumab. Such a combination results in decreased side effects, as compared to directly administering the respective proteins to a patient in need thereof. For example the combination of the following NDV strains may be used in accordance with the present invention:
- NDV-Mut HN(F277L)/M(G165W)-Nivolumab,
- NDV-Mut HN(F277L)/M(G165W)-Ipilimumab, and
- NDV-Mut HN(F277L)/M(G165W)/F(F117S)/F(F190L)/L(V757I)/ L(F1551S)/L(R1700L)-Atezolizumab
or
- NDV-Mut HN(F277L)/M(G165W)/F(F117S)/F(F190L)/L(V757I)/ L(F 1551 S)/L(R1700L)-Nivolumab,
- NDV-Mut HN(F277L)/M(G165W)/F(F117S)/F(F190L)/L(V757I)/ L(F1551S)/L(R1700L)-Ipilimumab, and
- NDV-Mut HN(F277L)/M(G165W)/F(F117S)/F(F190L)/L(V757I)/ L(F 1551 S)/L(R1700L)-Atezolizumab

In a further aspect the present invention also provides a pharmaceutical formulation comprising a recombinant and mutated NDV strain, preferably an rgNDV, as described herein, without or preferably with at least one pharmaceutically acceptable carrier material.

For the indications mentioned herein, the appropriate dosage will, of course, vary depending upon, for example, the particular molecule of the invention to be employed, the mode of administration and the nature and severity of the condition being treated. In general, the dosage, in one embodiment of the invention, can preferably be in the range of 10⁷ to 10⁹ pfu per dose.

The recombinant and mutated NDV strains according to the present invention provide on the one hand an improved replication capacity of the NDV particles in cancer cells and/or an improved safety profile, preferably in humans, which is associated with increased cancer cell lysis and increased anti-cancer activitity, because the recombinant and mutated NDV strains of the present invention are still selective for cancer cells. The low to sero activity towards normal cells is associated with an increased therapeutic window or safety margin, as is commonly determined for cancer therapeutics. Beside the virological activity the recombinant mutated NDV strains of the present invention are in addition beneficial for the treatment of cancer from an immunological point of view. This is because the foreign gene carried by NDV strains of the present invention beneficially influences the ability of the immune system of the subject to be treated to attack and destroy cancer cells. Advantageuously the foreign gene is expressed inside the tumor to be treated, that is directly in the side of need.

The pharmaceutical formulations or pharmaceutical compositions of the present invention may be manufactured in any conventional manner, which are known to those skilled in the art. For example a pharmaceutical composition according to the present invention comprising a recombinant virus particle as decribed within this description can be provided in a dried, preferably in a lyophilized form and can be complemented with a suitable solvent, e.g. an aqueous carrier for injection at the time when used for administration. The dried form is very stable. Thus, in one embodiment the pharmaceutical composition is present as a dried form, still more preferred in a lyophilized form, and is reconstituted before administration by adding a suitable solvent. That is the present invention also relates to the above described dried form of the pharmaceutical composition for use as a medicament, preferably for use in a method of treating cancer. The process parameters for drying, preferably lyophilisation must be chosen such that the dried/lyophilized product contains live, replication competent viral particles, which can be grown to standardized titers, when needed. A person skilled in the art knows how to set these parameters.

For administration of a dried form, the dried form is directly dissolved in a suitable solvent or pharmaceutically acceptable carrier for injection and/or stabilization of the NDV or rg NDV and/or for improving its delivery to cancer cells. The solvent or carrier is preferably an aqueous carrier, for example sterile water for injection or sterile buffered physiological saline or another, preferably isotonic buffer system having a pH in the range of 7.2 to 7.6. The reconstitution can be performed ideally at or close to the intended time of administration in order to avoid any contaminations with microbes, etc. The skilled person is familiar with the handling of pharmaceutical compositions for reconstitution and reconstituted solutions.

In another embodiment, the present invention is concerned with pharmaceutical formulations comprising cancer cells infected with a transgene-expressing NDV or transgene-expressing rgNDV strain as described herein, and a pharmaceutically acceptable carrier. In specific embodiments, the cancer cells have been treated with gamma radiation prior to incorporation into the pharmaceutical formulation. In specific embodiments, the cancer cells have been treated with gamma radiation before infection with the NDV strain (e.g., rgNDV). In other specific embodiments, the cancer cells have been treated with gamma radiation after infection with the NDV (e.g., rgNDV). In another embodiment, presented herein are pharmaceutical formulations comprising a protein concentrate from lysed NDV-infected cancer cells (e.g., rgNDV infected cancer cells), and a pharmaceutically acceptable carrier.

The pharmaceutical formulation of the present invention can be prepared by a method comprising or consisting of the steps of:
- propagating an NDV, preferably an rgNDV, according to the present invention in at least one cell or embryonated egg that is susceptible to a NDV infection;
- collecting the progeny virus particles, wherein the virus is grown to sufficient quantities and under sufficient conditions that the virus is free from exogenous contamination, such that the progeny virus is suitable for formulation into a pharmaceutical formulation; and
- preferably manufacturing a pharmaceutical formulation comprising said progeny virus particles in the absence of or after addition of at least one pharmaceutically acceptable carrier material.

Thus in a further aspect the present invention also provides a method for producing the above mentioned pharmaceutical formulation. It is also within the scope of the present invention that for manufacturing the said pharmaceutical formulation a nucleic acid according to the present invention is used, from which virus particles can be rescued which are then propagated in at least one cell or embryonated egg that is susceptible to a NDV infection.

The step of collecting progeny virus particles may in one embodiment also comprise a step of processing the collected virus-containing material to enrich virus particles and/or to eliminate host cell DNA.

In a further aspect the present invention also provides a cell or cell line or an embryonated egg comprising a NDV, preferably a rgNDV, according to the present invention.

In still a further aspect the present invention is also concerned with a method for treating cancer, especially a cancer selected from the specific cancers as described and/or mentioned in the claims herein, in a subject considered in need thereof. The method for treating cancer utilizes a transgene-expressing NDV or transgene-expressing rgNDV described herein or any combination thereof or a pharmaceutical formulation comprising such a NDV or rgNDV or any combination thereof, especially in a therapeutically effective amount. Within the meaning of the present patent application a therapeutically effective amount also includes an effective amount for preventing a cancer, in particular a cancer selected from the specific cancers as described and/or mentioned in the claims herein.

The method of treatment comprises or consists of administering to a subject in need thereof a NDV or an rgNDV according to the present invention or any combination thereof in a sufficient amount for infecting and destroying some or all of the cancer cells. In a specific embodiment the method for treating cancer comprises or consists of infecting a cancer cell in a subject with a NDV or rgNDV of the present invention or any combination thereof or with a pharmaceutical composition of the present invention. In another embodiment the one or more NDV or rgNDV or a pharmaceutical composition comprising the same is administered to a subject in need thereof by intravenous, intra-arterial, intratumoral, intramuscular, intradermal, subcutaneous, or any other medically relevant route of administration. According to another embodiment of the present invention the NDV or rgNDV according to the present invention or any combination thereof is administering to a subject in need thereof by administering cancer cells infected with a NDV or rgNDV according to the present invention, especially in a therapeutically effective amount, or pharmaceutical formulation thereof by intravenous, intra-arterial, intratumoral, intramuscular, intradermal, subcutaneous, or any other medically relevant route of administration. In specific embodiments, the cancer cells have been treated with gamma radiation prior to administration to the subject or incorporation into the pharmaceutical formulation. In still another embodiment, a method for treating cancer comprises or consists of administering to a subject in need thereof protein concentrates or plasma membrane fragments from cancer cells infected with a NDV or rgNDV or a pharmaceutical formulation according to the present invention.

In a particular preferred embodiment the method for treating a cancer, in particular a cancer selected from the specific cancers as described and/or mentioned in the claims herein, comprises administering a mixture of at least two recombinant and mutated NDV or rgNDV strains according to the present invention. In particular it is preferred to use a combination of virus particles, wherein each virus particle encodes and expresses another foreign gene selected from the group the foreign genes of the present invention, as defined above. For example, the encoded and expressed foreign genes are a gene encoding an antibody directed to the protein PD-L1 or an antigen-binding part directed to the protein PD-L1, preferably Atezolizumab, an antigen-binding part of Atezolizumab or a variant of Atezolizumab or its antigen-binding part, and a gene encoding an antibody directed to the growth factor protein VEGF-A or an antigen-binding part directed to the growth factor protein VEGF-A, preferably Bevacizumab, an antigen-binding part of Bevacizumab or a variant of Bevacizumab or its antigen-binding part. The administration of such a combination of foreign genes results in an active combination of proteins beneficially improving the immunological response with the cancer. The administration of this mixture of NDVs or rgNDVs according to the present invention can be achieved as specified supra.

Also within the scope of the present invention is a method of treating cancer, especially a cancer selected from the specific cancers as described and/or mentioned in the claims herein, which utilizes a NDV or rgNDV according to the present invention in combination with one or more other therapies. In a preferred embodiment the NDV or rgNDV or a pharmaceutical formulation comprising the same is administered to a subject in need thereof in a therapeutically effective amount. The NDV or rgNDV and one or more other therapies can be administered concurrently or sequentially to the subject (meaning they are jointly therapeutically active). In certain embodiments, the NDV or rgNDV and one or more other therapies are administered in the same ("fixed") pharmaceutical formulation. In other embodiments, the NDV or rgNDV and one or more other therapies are administered in different formulations. The NDV or rgNDV of the present invention and one or more other therapies can be administered by the same or different routes of administration to the subject considered in need thereof. Another therapy within the meaning of the present invention also comprises an administration of other oncolytical virus strains, for example recombinant NDV or rgNDV strains encoding and expressing foreign genes other than those as disclosed within the present invention. Another foreign gene expressed by the NDV or rgNDV may be selected from the group consisting of a gene encoding an antibody directed to protein PD-1 (anti-PD-1), preferably a gene encoding Nivolumab, a gene encoding an antibody directed to the surface protein CTLA-4 (anti-CTLA-4), preferably a gene encoding Ipilimumab, a gene encoding a protein which improves the cellular immune response and the ability of T cells to enter tumor cells, preferably a gene encoding interleukin-12 (IL-12), a gene encoding a protein with the ability to modulate the virus replication cycle, preferably a gene encoding the non-structural protein NS1 of influenza A virus, a gene encoding a protein with the ability to selectively induce apoptosis in human tumor cells, but not in normal human cells, preferably a gene encoding Apoptin, a gene encoding a protein that reduces or inhibits IFN expression such as an IFN-beta receptor, preferably a gene encoding the viral protein B18R from vaccinia virus, any antigen-binding part of these antibodies, any parts of these other proteins, variants of these antibodies and these other proteins, and variants of these antigen-binding parts or parts. Particularly preferred NDV strains and rgNDV which may be used in combination are disclosed in the copending European patent application no. 18166400.4 and the copending European patent application no. 19154204.2. For details of these strains reference to these patent applications is made. Particulary preferred is the combined use of recombinant NDV or rgNDV strains encoding and expressing as foreign genes anti-PD-1, preferably Nivolumab, anti-CTLA-4, preferably Ipilimumab, anti-PD-Ll, preferably Atezolizumab, or parts or variants thereof. For example the following combinations are in accordance with the present invention:
- NDV-Mut HN(F277L)/M(G165W)-Nivolumab,
- NDV-Mut HN(F277L)/M(G165W)-Ipilimumab, and
- NDV-Mut HN(F277L)/M(G165W)/F(F117S)/F(F190L)/L(V757I)/ L(F1551S)/L(R1700L)-Atezolizumab
or
- NDV-Mut HN(F277L)/M(G165W)/F(F117S)/F(F190L)/L(V757I)/ L(F 1551 S)/L(R1700L)-Nivolumab,
- NDV-Mut HN(F277L)/M(G165W)/F(F117S)/F(F190L)/L(V757I)/ L(F1551S)/L(R1700L)-Ipilimumab, and
- NDV-Mut HN(F277L)/M(G165W)/F(F117S)/F(F190L)/L(V757I)/ L(F 1551 S)/L(R1700L)-Atezolizumab

Another therapy may also comprise or may be radiotherapy for cancer.

### Figures

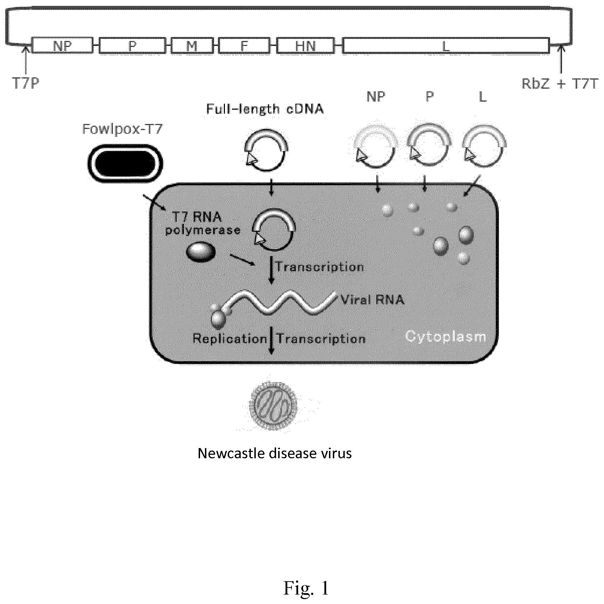

Figure 1 is a schematic presentation of the NDV reverse genetics system. The upper part shows the composition of the full-length cDNA plasmid which contains the full-length NDV cDNA (encoding the nucleocapsid protein (NP), the phosphoprotein (P), the matrix protein (M), the fusion protein (F), the hemagglutinin-neuraminidase (HN), and the RNA-dependent RNA polymerase (L)) cloned behind the bacteriophage T7 RNA Polymerase promoter (T7P; yellow triangle) and followed by a ribozyme sequence (Rz) and T7 transcription termination signal (T7T).

A suitable host cell (shaded round-cornered box) is infected with a recombinant Fowlpox virus that expresses T7 DNA-dependent RNA polymerase (Fowlpox-T7) and subsequently co-transfected with the full-length cDNA plasmid and three helper plasmids containing the genes encoding the NDV NP, P and L proteins, respectively. Transcription of the full-length cDNA results in the generation of the NDV antigenome RNA which is encapsidated by NP protein then transcribed and replicated by the RNA-leading to the generation of infectious NDV.

Figure 2 is a schematic presentation of the genome of the rgNDV-mutants according to the present invention. The figure shows the position of the respective foreign gene which is inserted as extra transcription unit into the rgNDV-mutant genome between the P and M genes.

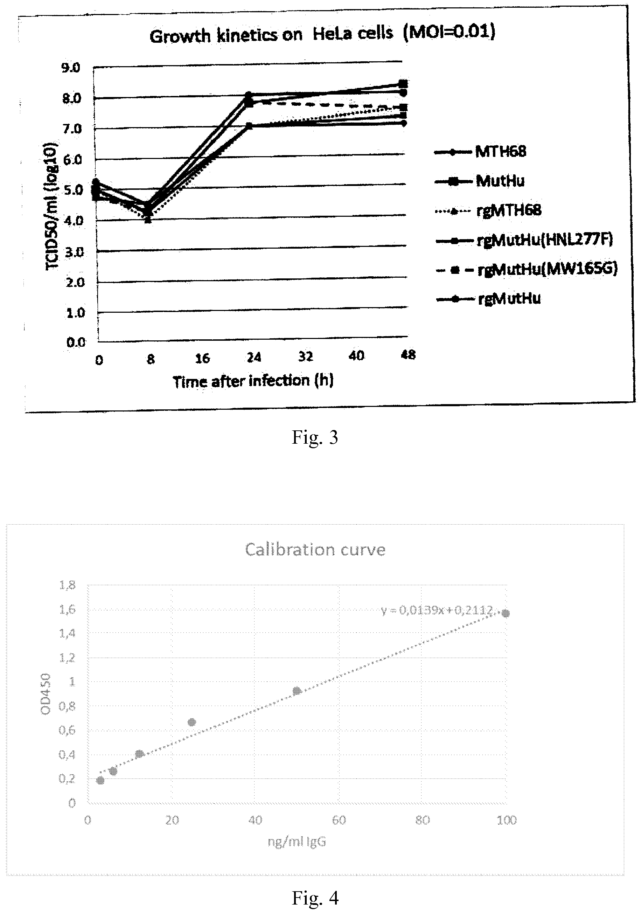

Figure 3 shows growth kinetics in HeLa cells. HeLa cells were infected at a multiplicity of infection (MOI) of 0.01 with the indicated viruses. At 0h, 8h, 24h and 48h after infection, the amount of infectious virus in the supernatant was determined by end-point titration on QM5 cells. MTH68: NDV strain MTH-68/H; MutHu: NDV-Mut HN(F277L)/M(G165W); rgMTH68: NDV strain MTH-68/H derived by reverse genetics from cloned full-length cDNA; rgMutHu: NDV strain NDV-Mut HN(F277L)/M(G165W) derived by reverse genetics from cloned full-length cDNA; rgMutHu(HNL277F): rgMutHu in which the amino acid mutation at position 277 in the HN gene was converted from L back to F; rgMutHu(MW165G): rgMutHu in which the amino acid mutation at position 165 in the M gene was converted from W back to G.

Figure 4 shows a calibration curve of a human IgG ELISA used for determing and quantifying the expression of Atezolizumab and Bevacizumab from a recombinant NDV strain according to the present invention.

### Examples

### Example 1. Nucleotide sequence analysis of mutant NDV-Mut HN(F277L)/M(G165W).

We identified a spontaneous mutant of an oncolytic NDV strain MTH-68/H (Csatary et al., 1999, Anticancer Res. 19:635-638.; further called MTH68). The replication capacity of the mutant strain (designated NDV-Mut HN(F277L)/M(G165W) in a variety of human neoplastic cell lines, as well as autologous primary tumors, is greatly enhanced as compared to the original MTH-68/H strain (also referred to as MTH68 strain). We analyzed its nucleotide sequence and found that, compared to MTH68, NDV-HN(F277L)/M(G165W) has two nucleotide mutations, one leading to an amino acid substitution in the M protein (G165W) and the other in the HN protein (F277L).

### Example 2. A reverse genetics system that allows genetic modification of NDV-strains.

### 2.1 Reverse genetics

In order to be able to genetically modify the genome of an RNA virus such as NDV, a manipulatable genetic system must be developed that uses a copy of the full viral RNA (vRNA) genome in the form of DNA. This full-length cDNA is amenable to genetic modification by using recombinant DNA techniques. The authentic or modified cDNA can be converted back into vRNA in cells, which in the presence of the viral replication proteins results in the production of a new modified infectious virus. Such 'reverse genetics systems' have been developed in the last few decades for different classes of RNA viruses. This system enables the rapid and facile introduction of mutations and deletions and the insertion of a transgene transcriptional unit, thereby enabling the changing of the biological properties of the virus.

Reverse genetics systems for several NDV strains, including lentogenic as well as velogenic strains, were developed by the Central Veterinary Institute (CVI), part of Wageningen University and Research, currently Wageningen Bioveterinary Research (WBVR) under the supervision of Dr. Ben Peeters (Peeters et al., 1999, J. Virol. 73:5001-9; de Leeuw et al., 2005, J. Gen. Virol. 86:1759-69; Dortmans et al., 2009, J. Gen. Virol. 90:2746-50). In order to generate a reverse genetics system for providing the NDV nucleic acids and strains according to the present invention, a similar approach was used. Details of the procedure can be found in the above cited papers and in the paragraphs below. Briefly, the system consists of 4 components, i.e., a transcription plasmid containing the full-length (either authentic or genetically modified) cDNA of the virus, which is used to generate the vRNA, and 3 expression plasmids ('helper plasmids') containing the NP, P and L genes of NDV respectively, which are used to generate the vRNA-replication complex (consisting of NP, P and L proteins). Transcription of the cDNA (i.e. conversion of the cDNA into vRNA) and expression of the NP, P and L genes by the helper plasmids is driven by a T7 promoter. The corresponding T7 DNA-dependent RNA polymerase (T7-RNAPol) is provided by a helper-virus (Fowlpox-T7).

In order to rescue virus, the 4 plasmids are co-transfected into Fowlpox-T7 infected cells (Fig. 1). Three to five days after transfection, the supernatant is inoculated into specific-pathogen-free embryonated chicken eggs (ECE) and incubated for 3 days. Infectious virus that is produced by transfected cells will replicate in the ECE and progeny virus can be harvested from the allantois fluid.

In order to develop a reverse genetics system for NDV the following steps were followed:
- Generation of sub-genomic NDV and foreign gene cDNA's by RT-PCR
- Assembly of full-length cDNA in a transcription vector
- Cloning of each of the NP, P and L genes into an expression vector
- Verify nucleotide sequence of full-length cDNA and helper-plasmids
- Repair nucleotide differences resulting from the cloning procedure, if necessary
- Rescue of infectious virus from cDNA using co-transfection (Fig. 1)

### 2.2 Construction of full-length NDV-Mut HN(F277L)/M(G165W) cDNA and helper plasmids

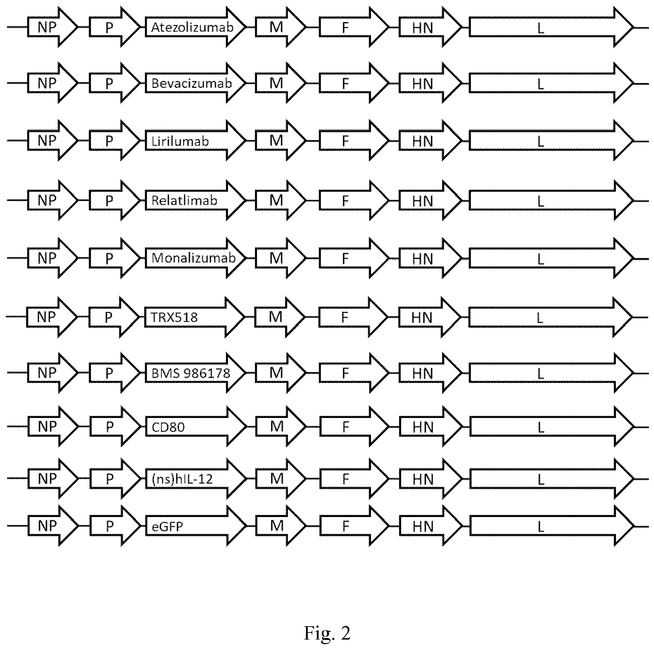

NDV-Mut HN(F277L)/M(G165W) (passage 28 HeLa cells) was used for the isolation of vRNA using standard procedures. The vRNA was used to generate first-strand cDNA by means of Reverse Transcriptase followed by PCR to generate 4 sub-genomic cDNA fragments (designated C1, C2, C3 and C8). The full-length cDNA of NDV-MutHu was assembled from these fragments and cloned in the transcription vector pOLTV5 (Peeters et al., 1999, J. Virol. 73:5001-5009) by a combination of In-Fusion® cloning and classical cloning using restriction enzymes. An overview of the procedure is shown in Fig. 2, and further details can be found in Appendix 1 of this reference. The NP, P and L-genes of NDV-MutHu were obtained by RT-PCR (Appendix 1) and cloned in the expression plasmid pCVI which was derived by deletion of a ClaI restriction fragment from pCI-neo (Promega).

### 2.3 Nucleotide sequence analysis

Nucleotide sequence analysis was used to verify that the sequence of pFL-NDV Mut HN(F277L)/M(G165W) was correct. A few nucleotides which differed from the Reference sequence were repaired. Silent mutations (i.e., not leading to an amino acid change) may be left unchanged.

### 2.4 Rescue of infectious virus from pFL-NDV Mut HN(F277L)/M(G165W)

In order to generate infectious virus, we used the co-transfection system described above (and illustrated in Fig. 1) to transfect QM5 cells (derived from Quail) using plasmid pFL-NDV_Mut HN(F277L)/M(G165W) and the helper plasmids pCVI-NP^{Mut HN(F277L)/M(G165W)}, pCVI-P^{Mut HN(F277L)/M(G165W)} and pCVI-L^{Mut HN(F277L)/M(G165W)}. Rescue of infectious virus was successful as demonstrated by the presence of infectious virus in the allantoic fluid of ECE that were inoculated with the transfection supernatant (data not shown). The identity of the rescued virus was determined by RT-PCR followed by sequencing. The rescued virus was designated rgMut HN(F277L)/M(G165W). NDV-Mut HN(F277L)/M(G165W) and rgMut HN(F277L)/M(G165W) have similar growth kinetics in HeLa cells.

### Example 3. Identify whether one or both of the amino acid substitutions in Mut HN(F277L)/M(G165W) are responsible for the difference in growth kinetics between Mut HN(F277L)/M(G165W) and the parent strain MTH68.

### 3.1 Growth kinetics in HeLa cells

The rescued rg-viruses (Table 1) as well as the original Mut HN(F277L)/M(G165W) and MTH68 viruses were used to determine their growth-kinetics in HeLa cells. Briefly, 4x10⁶ HeLa cells were seeded in 25 cm² cell culture flasks and grown overnight. The cells were infected using a MOI of 0.01 (i.e., 1 infectious virus particle per 100 cells), and at 8, 24 and 48 hours after infection the virus titer in the supernatant was determined by end-point titration on QM5 cells.

The data (Fig. 3) indicate that strains Mut HN(F277L)/M(G165W) and rgMut HN(F277L)/M(G165W) yield at least 10-fold higher virus titers than MTH68. Furthermore, the data indicate that the mutation at amino acid position 277 in the HN gene is responsible for this effect. The M mutation does not seem to have an effect. This can be best seen when looking at the virus titers 24h after infection, or even better when comparing the increase in virus titer between 8h and 24h (the exponential growth phase). The virus titer shows an increase of 3.5 (log10) for Mut HN(F277L)/M(G165W), rgMut HN(F277L)/M(G165W) and rgMut HN(F277L)/M(W165G), whereas this is 2.5 for MTH68, 2.7 for rgMut HN(L277F) and 3.0 for rgMTH68 (Table 3).

rgMut HN(F277L)/M(W165G) is a strain in which the mutation in the M gene has been restored in accordance with the NDV MTH-68/H.

**Table 1. Virus titers (log10 TCID50/ml)**

| | Time after infection (h) | | | |
|---|---|---|---|---|
| Virus | 0 h | 8 h | 24 h | 48 h |
| MTH68 | 4.8 | 4.5 | 7.0 | 7.0 |
| Mut HN(F277L)/M(G165W) | 5.0 | 4.3 | 7.8 | 8.3 |
| rgMTH68 | 5.0 | 4.0 | 7.0 | 7.5 |
| rgMut HN(L277F) | 5.0 | 4.3 | 7.0 | 7.3 |
| rgMut M(W165G) | 4.8 | 4.3 | 7.8 | 7.5 |
| rgMutHu | 5.3 | 4.5 | 8.0 | 8.0 |

### Example 4. Generation of recombinant NDV-strains with enhanced oncolytic and immune stimulating properties due to the expression of different therapeutic proteins.

To this end, three different rgMut HN(F277L)/M(G165W)/F(F117S)/F(F190L)/ L(V757I)/L(F1551S)/L(R1700L) strains were generated, expressing the genes for:
1) Atezolizumab
2) Bevacizumab

### 4.1 Generation of recombinant viruses

Recombinant NDV-Mut HN(F277L)/M(G165W)/F(F117S)/F(F190L)/L(V757I)/ L(F1551S)/L(R1700L) viruses (rgNDV-Mut HN(F277L)/M(G165W/F(F117S)/ F(F190L)/L(V757I)/L(F1551S)/L(R1700L)) expressing Atezolizumab or Bevacizumab were generated by means of the previously established reverse genetics system described above. The genes encoding the heavy- and light-chain of Atezolizumab or the genes encoding the heavy- and light-chain of Bevacizumab were inserted into the full-length cDNA of NDV-Mut HN(F277L)/M(G165W)/F(F117S)/ F(F190L)/L(V757I)/L(F1551S)/L(R1700L) between the P and the M genes. To this end the open reading frames of the foreign genes were fused via a 2A sequence. The genes were provided with the necessary NDV gene-start and gene-end sequences in order to allow transcription by the vRNA polymerase.

Figure 2 shows the final constellation of the recombinant virus that was rescued from cloned full-length cDNA by means of the NDV reverse-genetics systems.

Infectious virus was rescued for both constructs, and virus stocks were prepared by two passages in HeLa cells. The nucleotide sequences of the inserted genes in the different recombinant viruses were verified by means of nucleotide sequence analysis and found to be correct.

Expression of Atezolizumab and Bevacizumab was determined and quantified by using a total human IgG ELISA (Invitrogen). The amount of Atezolizumab and Bevacizumab, respectively that is secreted into the medium of HeLa cells infected with the above mentioned recombinant NDV strains encoding Atezolizumab or Bevacizumab was determined by analyzing the culture supernatant of the 2^{nd} passage on HeLa cells.

As can be seen from the OD450 values given in Table 2 below, the production of Atezolizumab reached approximately 15.3 µg/ml and the production of Bevacizumab reached approximately 155.7 µg/ml.

**Table 2: OD450 values of a human IgG ELISA. As negative control the respectively mutated NDV-strain without an inserted foreign gene has been used.**

| Calibration | | Negative control | rgMutHu2-Atezolizumab | rgMutHu2-Bevacizumab | Dilution |
|---|---|---|---|---|---|
| IgG ng/ml | | | | | |
| 100 | 1.565 | 0.071 | 2.192 | 3.51 | 1:5 |
| 50 | 0.923 | 0.081 | 1.657 | 3.441 | 1:50 |
| 25 | 0.665 | 0.079 | 0.636 | 1.573 | 1:500 |
| 12.5 | 0.401 | 0.078 | 0.307 | 0.644 | 1:5000 |
| 6.25 | 0.258 | | | | |
| 3.12 | 0.186 | | | | |
| 0 | 0.081 | | | | |
| 0 | 0.082 | | | | |

### References:

- Blach-Olszewska et al., (1977) Why HeLa cells do not produce interferon? Arch Immunol Ther Exp (Warsz) 25:683-91.
- Chng et al., (2015) Cleavage efficient 2A peptides for high level monoclonal antibody expression in CHO cells, mAbs, 7:2, 403-412; http://dx.doi.org/10.1080/19420862.2015.1008351.
- Enoch et al., (1986) Activation of the Human beta-Interferon Gene Requires an Interferon-Inducible Factor, Mol. Cell. Biol. 6:801-10.
- Haryadi et al., (2015) Optimization of Heavy Chain and Light Chain Signal Peptides for High Level Expression of Therapeutic Antibodies in CHO Cells. PLoS ONE 10(2): e0116878.doi:10.1371/journal.pone.0116878.
- Schirrmacher, (2015) Oncolytic Newcastle disease virus as a prospective anti-cancer therapy. A biologic agent with potential to break therapy resistance. Expert Opin. Biol. Ther. 15:17 57-71
- Zamarin et al., (2014) Localized oncolytic virotherapy overcomes systemic tumor resistance to immune checkpoint blockade immunotherapy. Sci. Transl. Med. 6(226).
- Zamarin & Palese, (2017) Oncolytic Newcastle Disease Virus for cancer therapy: old challenges and new directions. Future Microbiol. 7:347-67.

### Appendix 1: primers used for the generation of cDNA fragments and helper-plasmids

### cDNA fragments

| Fragment | Size | Primer | Sequence (5'-3') |
|---|---|---|---|
| C1 | 3.6 kb | Noss-09 (SEQ ID No. 28) | ACGACTCACTATAGGaccaaacagagaatccgtgag |
| | | Noss-121 (SEQ ID No. 29) | CCGGGAAGATCCAGGgcactcttcttgcatgttac |
| C2 | 3.7 kb | N oss-122 (SEQ ID No. 30) | GGGCCTGCCTCACTAtggtggtaacatgcaagaag |
| | | Noss-123 (SEQ ID No. 31) | TGCATGTTACCACCAatgtgtcattgtatcgcttg |
| C3 | 5.7 kb | Noss-125 (SEQ ID No. 32) | CAAGAAGGGAGATACgtaatatacaagcgatacaatg |
| | | N oss-126 (SEQ ID No. 33) | TCGCTTG TAT ATT ACttgttgtagcaaagagcacc |
| C8 | 2.0 kb | Noss-133 (SEQ ID No. 34) | GGCCTGGATCTTCCCattatgctgtctgtatacggtgc |
| | | Noss-10 (SEQ ID No. 35) | ATGCCATGCCGACCCaccaaacaaagacttggtgaatg |
| iPCR pOLTV5 (StuI) | 2.5 kb | Noss-17 (SEQ ID No. 36) | CCTATAGTGAGTCGTATTAATTTC |
| | | Noss-128 (SEQ ID No. 37) | CCTGGATCTTCCCGGGTCGG |
| iPCR pOLTV5 (SmaI) | 2.5 kb | Noss-137 (SEQ ID No. 38) | GGGTCGGCATGGCATCTCCACC |
| | | Noss-138 (SEQ ID No. 39) | GGGAAGATCCAGGCCTATAGTG |

### Helper-plasmids (generated by In-Fusion® cloning in pCVI)

| Gene | primer | sequence |
|---|---|---|
| NP | Noss-22 (SEQ ID No. 40) | CTCTAGAGTCGACCCttctgccaacatgtcttccg |
| | Noss-23 (SEQ ID No. 41) | GGGAAGCGGCCGCCCgtcggtcagtatccccagtc |
| P | Noss-24 (SEQ ID No. 42) | CTCTAGAGTCGACCCcagagtgaagatggccaccttc |
| | Noss-25n (SEQ ID No. 43) | GGGAAGCGGCCGCCCgtagtagtgatcagccattc |
| L | Noss-26 (SEQ ID No. 44) | CTCTAGAGTCGACCCgggtaggacatggcgggctc |
| | Noss-27 (SEQ ID No. 45) | GGGAAGCGGCCGCCCtgcctttaagagtcacagttac |

## Claims

1. A recombinant Newcastle Disease Virus (NDV), wherein the recombinant NDV carries as a foreign gene at least one gene selected from the group consisting of
- a gene encoding an antibody directed to protein PD-L1 or an antigen-binding part directed to protein PD-L1 (anti-PD-L1), preferably a gene encoding Atezolizumab, an antigen-binding part of Atezolizumab, a variant of Atezolizumab or a variant of an antigen-binding part of Atezolizumab,
- a gene encoding an antibody directed to the growth factor protein VEGF-A or an antigen-binding part directed to the growth factor protein VEGF-A (anti-VEGF-A), preferably a gene encoding Bevacizumab, an antigen-binding part of Bevacizumab, a variant of Bevacizumab or a variant of an antigen-binding part of Bevacizumab,
- a gene encoding an antibody directed to killer-cell immunoglobulin-like receptors (KIRs) or an antigen-binding part directed to killer-cell immunoglobulin-like receptors (KIRs) (anti-KIR), preferably a gene encoding Lirilumab, an antigen-binding part of Lirilumab, a variant of Lirilumab or a variant of an antigen-binding part of Lirilumab,
- a gene encoding an antibody directed to and inhibiting LAG-3 or an antigen-binding part directed to and inhibiting LAG-3 (anti-LAG-3), preferably a gene encoding Relatlimab, an antigen-binding part of Relatlimab, a variant of Relatlimab or a variant of an antigen-binding part of Relatlimab,
- a gene encoding an antibody directed to NKG2A or an antigen-binding part directed to NKG2A (anti-NKG2A), preferably a gene encoding Monalizumab, an antigen-binding part of Monalizumab, a variant of Monalizumab or a variant of an antigen-binding part of Monalizumab,
- a gene encoding an antibody directed to the glucocorticoid-induced TNF-superfamily receptor (GITR) or an antigen-binding part directed to the glucocorticoid-induced TNF-superfamily receptor (GITR) (anti-GITR), preferably a gene encoding TRX518, an antigen-binding part of TRX518, a variant of TRX518 or a variant of an antigen-binding part of TRX518,
- a gene encoding an antibody directed to and activating the protein OX40 or an antigen-binding part directed to and activating the protein OX40 (anti-OX40), preferably a gene encoding BMS 986178, an antigen-binding part of BMS 986178, a variant of BMS 986178 or a variant of an antigen-binding part of BMS 986178,
- a gene encoding a membrane protein which is the receptor for the proteins CD28 and CTLA-4, and which membrane protein is involved in the costimulatory signal essential for T-lymphocyte activation or a part of the membrane protein which is the receptor for the proteins CD28 and CTLA-4, and which membrane protein is involved in the costimulatory signal essential for T-lymphocyte activation, preferably a gene encoding CD80, a part of CD80, a variant of CD80 or a variant of a part of CD80,
- a gene encoding a human interleukin 12 (hIL-12) or a part of a human interleukin 12 (hIL-12), preferably a gene encoding (ns)hIL-12, a part of (ns)hIL-12, a variant of (ns)hIL-12 or a variant of a part of (ns)hIL-12,
- a gene encoding a green fluorescent protein or a part of a green fluorescent protein, preferably a gene encoding eGFP, a part of eGFP, a variant of eGFP or a variant of a part of eGFP, and
- any combination of these genes, parts or variants, wherein
said recombinant NDV comprises a mutated HN gene and the encoded hemagglutinin-neuramidase with an amino acid substitution at position 277 to an amino acid with a hydrophobic side chain other than phenylalanine, preferably where phenylalanine (F) is substituted to leucine (L) at position 277 of the HN gene.

2. The recombinant NDV according to claim 1, wherein the NDV further comprises a mutation in the M gene and the thus encoded matrix protein, preferably wherein the mutated M gene encodes a matrix protein with an amino acid substitution at position 165 to an amino acid with an aromatic side chain, particulary where glycine (G) is substituted to tryptophane (W) at position 165 of the M protein.

3. The recombinant NDV according to claim 1 or claim 2, wherein the NDV comprises a mutated F gene and the encoded fusion protein with an amino acid substitution at position 117 to an amino acid with a hydroxylated amino acid side chain, preferably where phenylalanine (F) is substituted to serine (S) at position 117 of the F protein, and/or with an amino acid substitution at position 190 to an amino acid with an aliphatic amino acid side chain, preferably where phenylalanine (F) is substituted to leucine (L) at position 190 of the F protein.

4. The recombinant NDV according to any one of claims 1 to 3, wherein the NDV comprises a mutated L gene and the encoded L protein with an amino acid substitution at position 757 to an amino acid with an aliphatic amino acid side chain other than valine (V), preferably where valine (V) is substituted to isoleucine (I) at position 757 of the L protein, and/or with an amino acid substitution at position 1551 to an amino acid with a hydroxylated side chain, preferably where phenylalanine (F) is substituted to serine (S) at position 1551 of the L protein, and/or with an amino acid substitution at position 1700 to an amino acid with an aliphatic side chain, preferably where arginine (R) is substituted to leucine (L) at position 1700 of the L protein, preferably wherein the NDV comprises a mutated L gene and the encoded L protein with the amino acid substitution at position 757, position 1551 and position 1700.

5. The recombinant NDV according to any one of claims 1 to 4, wherein the recombinant NDV is encoded by and/or comprises at least one of the nucleic acids according to SEQ ID No. 2 to 23 or variants thereof, preferably the variants having a sequence identity of at least 75 %, particularly of at least 90 %, more particularly of at least 95 %.

6. The recombinant NDV according to any one of claims 1 to 5, wherein the NDV comprises a mutated F gene and the encoded fusion protein with a amino acid substitution at position 289 to an amino acid with a aliphatic side chain other than leucine (L), preferably where leucine (L) is substituted to alanine (A) at position 289 of the F protein, and/or wherein the NDV comprises a mutated L gene and the encoded large protein with a amino acid substitution at position 1717 to an amino acid with aromatic side chain other than tyrosine (Y), preferably where tyrosine (Y) is substituted to histidine (H) at position 1717 of the L protein, and/or at position 1910 to an amino acid with a basic side chain, preferably where glutamic acid (E) is substituted to lysine (L) at position 1910 of the L protein.

7. A recombinant NDV according to any one of claims 1 to 6 for use in medicine.

8. A recombinant NDV according to any one of claims 1 to 7 for use in a method of treating cancer in a subject, preferably in a mammal, more preferably a mammalian animal or a human subject, considered in need thereof, in particular for the treatment of one or more indications selected from the group consisting of brain tumors, like glioblastoma, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose, throat tumors, tongue tumors, and skin tumors, like melanoma.

9. A nucleic acid encoding a recombinant NDV, preferably a rgNDV, the nucleic acid comprising a transgenic construct, wherein said transgenic construct encodes a protein selected from the group consisting of
- an antibody directed to protein PD-L1 or an antigen-binding part directed to protein PD-L1 (anti-PD-L1), preferably Atezolizumab, an antigen-binding part of Atezolizumab, a variant of Atezolizumab or a variant of an antigen-binding part of Atezolizumab,
- an antibody directed to the growth factor protein VEGF-A or an antigen-binding part directed to the growth factor protein VEGF-A (anti-VEGF-A), preferably Bevacizumab, an antigen-binding part of Bevacizumab, a variant of Bevacizumab or a variant of an antigen-binding part of Bevacizumab,
- an antibody directed to killer-cell immunoglobulin-like receptors (KIRs) or an antigen-binding part directed to killer-cell immunoglobulin-like receptors (KIRs) (anti-KIR), preferably Lirilumab, an antigen-binding part of Lirilumab, a variant of Lirilumab or a variant of an antigen-binding part of Lirilumab,
- an antibody directed to and inhibiting LAG-3 or an antigen-binding part directed to and inhibiting LAG-3 (anti-LAG-3), preferably Relatlimab, an antigen-binding part of Relatlimab, a variant of Relatlimab or a variant of an antigen-binding part of Relatlimab,
- an antibody directed to NKG2A or an antigen-binding part directed to NKG2A (anti-NKG2A), preferably Monalizumab, an antigen-binding part of Monalizumab, a variant of Monalizumab or a variant of an antigen-binding part of Monalizumab,
- an antibody directed to the glucocorticoid-induced TNF-superfamily receptor (GITR) or an antigen-binding part directed to the glucocorticoid-induced TNF-superfamily receptor (GITR) (anti-GITR), preferably TRX518, an antigen-binding part of TRX518, a variant of TRX518 or a variant of an antigen-binding part of TRX518,
- an antibody directed to and activating the protein OX40 or an antigen-binding part directed to and activating the protein OX40 (anti-OX40), preferably BMS 986178, an antigen-binding part of BMS 986178, a variant of BMS 986178 or a variant of an antigen-binding part of BMS 986178,
- a membrane protein which is the receptor for the proteins CD28 and CTLA-4, and which membrane protein is involved in the costimulatory signal essential for T-lymphocyte activation or a part of the membrane protein which is the receptor for the proteins CD28 and CTLA-4, and which membrane protein is involved in the costimulatory signal essential for T-lymphocyte activation, preferably CD80, a part of CD80, a variant of CD80 or a variant of a part of CD80,
- a human interleukin 12 (hIL-12) or a part of a human interleukin 12 (hIL-12), preferably (ns)hIL-12, a part of (ns)hIL-12, a variant of (ns)hIL-12 or a variant of a part of (ns)hIL-12,
- a green fluorescent protein or a part of a green fluorescent protein, preferably a eGFP, a part of eGFP, a variant of eGFP or a variant of a part of eGFP, and
- any combination of these proteins, parts or variants, wherein
the nucleic acid in addition having a mutation in the HN gene, the sequence of the mutated HN gene encoding hemagglutinin-neuramidase with an amino acid substitution at position 277 to an amino acid with a hydrophobic side chain other than phenylalanine, particulary where the mutated HN gene encodes HN^{F277L}.

10. The nucleic acid according to claim 9, wherein the sequence encoding the recombinant NDV further comprises a mutation in the M gene, preferably wherein the mutated M gene encodes a matrix protein with an amino acid substitution at position 165 to an amino acid with an aromatic side chain, particulary where the mutated M gene encodes M^{G165W}.

11. The nucleic acid according to claim 9 or claim 10, wherein the sequence encoding the recombinant NDV further comprises at least one mutation in the F gene, preferably wherein the encoded mutated F gene encodes a fusion protein with an amino acid substitution at position 117 to an amino acid with a hydroxylated side chain, preferably where the encoded mutated F gene encodes F^{F117S}, and/or with an amino acid substitution at position 190 to an amino acid with an aliphatic side chain, preferably where the mutated F gene encodes F^{F190L}.

12. The nucleic acid according to any one of claims 9 to 11, wherein the sequence encoding the recombinant NDV further comprises at least one mutation in the L gene, preferably wherein the encoded mutated L gene encodes a large protein with an amino acid substitution at position 757 to an amino acid with an aliphatic side chain, preferably where the mutated L gene encodes L^{V757I}, and/or with an amino acid amino acid substitution at position 1551 to an amino acid with an aliphatic side chain, preferably where the encoded mutated L gene encodes L^{F1551S}, and/or with an amino acid amino acid substitution at position 1700 to an amino acid with an aliphatic side chain, preferably where the encoded mutated L gene encodes L^{R1700L}, more preferably wherein the encoded mutated L gene encodes a large protein with the amino acid substitution at position 757, position 1551 and position 1700.

13. The nucleic acid according to any one of claims 9 to 12, wherein the nucleic acid consists of or comprises at least one of the nucleic acids according to SEQ ID No. 2 to 23 or variants thereof, preferably the variants having a sequence identity of at least 75 %, particularly of at least 90 %, more particularly of at least 95 %.

14. The nucleic acid according to any one of claims 9 to 13, wherein the sequence encoding the recombinant NDV comprises a mutation in the F gene, wherein the mutated F gene encodes a fusion protein with an amino acid substitution at position 289 to an amino acid with an aliphatic side chain other than leucine (L), preferably where the encoded mutated F gene encodes F^{L289A}, and/or wherein the sequence encoding the recombinant NDV comprises at least one mutation in the L gene, wherein the encoded mutated L gene encodes a large protein with a amino acid substitution at position 1717 to an amino acid with aromatic side chain other than tyrosine (Y), preferably where the mutated L gene encodes L^{Y1717H}, and/or wherein the encoded mutated L gene encodes a large protein with a amino acid substitutionat position 1910 to an amino acid with a basic side chain, preferably where the mutated L gene encodes L^{E1910K}.

15. The nucleic acid according to any one of claims 9 to 14, wherein the nucleic acid is derived from a recombinant NDV according to any one of claims 1 to 8.

16. A pharmaceutical formulation comprising particles of the recombinant NDV according to any one of claims 1 to 6 and/or a nucleic acid according to any one of claims 9 to 15, preferably for use in oncological treatment of a subject considered in need thereof, in particular for the treatment of one or more indications selected from the group consisting of brain tumors, bone tumors, like osteosarcoma and/or Ewing's sarcoma, soft tissue tumors, like rhabdomyosarcoma, gynecological tumors, like breast cancer, ovary cancer and/or cervix cancer, gastrointestinal tumors, like esophageal tumors, stomach tumors, colon tumors, pancreas tumors, prostate tumors, lung tumors, ear, nose, throat tumors, tongue tumors, and skin tumors, like melanoma.
